(19) Europäisches Patentamt
European Patent Office
Office européen des brevets

(11) EP 1 623 704 A1

(12) # EUROPEAN PATENT APPLICATION
published in accordance with Art. 158(3) EPC

(43) Date of publication:
**08.02.2006 Bulletin 2006/06**

(21) Application number: **04730641.0**

(22) Date of filing: **30.04.2004**

(51) Int Cl.:
***A61K 31/121*** *(2000.01)* ***A61K 31/222*** *(2000.01)*
***A61K 31/343*** *(2000.01)* ***A61K 31/341*** *(2000.01)*
***A61K 31/365*** *(1985.01)* ***A61K 31/351*** *(2000.01)*
***A61K 31/381*** *(2000.01)* ***A61K 31/366*** *(2000.01)*
***A61K 31/352*** *(2000.01)* ***A61P 43/00*** *(2000.01)*
***A61P 3/10*** *(2000.01)* ***A61P 3/08*** *(2000.01)*
***A61P 5/50*** *(2000.01)* ***C07D 493/08*** *(1985.01)*
***C07D 309/12*** *(1985.01)* ***C07D 311/32*** *(1985.01)*
***C07C 49/84*** *(1974.07)* ***C07C 49/835*** *(1980.01)*
***C07C 69/18*** *(1968.09)* ***C07C 69/157*** *(1980.01)*
***C07C 69/16*** *(1968.09)*

(86) International application number:
**PCT/JP2004/006282**

(87) International publication number:
**WO 2004/096198 (11.11.2004 Gazette 2004/46)**

(84) Designated Contracting States:
**AT BE BG CH CY CZ DE DK EE ES FI FR GB GR HU IE IT LI LU MC NL PL PT RO SE SI SK TR**

(30) Priority: **02.05.2003 JP 2003127517**
**19.05.2003 JP 2003140821**
**20.06.2003 JP 2003177280**
**25.07.2003 JP 2003202118**
**05.12.2003 JP 2003408213**
**25.03.2004 JP 2004090656**

(71) Applicant: **TAKARA BIO INC.**
**Otsu-shi,**
**Shiga 520-2193 (JP)**

(72) Inventors:
- **ENOKI, Tatsuji**
**Kyotanabe-shi, Kyoto 610-0313 (JP)**
- **KOBAYASHI, Eiji**
**Otsu-shi, Shiga 520-2153 (JP)**
- **OGAWA, Kinuko**
**Otsu-shi, Shiga 520-2134 (JP)**
- **KUDO, Yoko**
**Otsu-shi, Shiga 520-2134 (JP)**
- **TANABE, Masashige**
**Otsu-shi, Shiga 520-2133 (JP)**
- **SUGIYAMA, Katsumi**
**Otsu-shi, Shiga 520-2134 (JP)**
- **OHNOGI, Hiromu**
**Kusatsu-shi, Shiga 525-0025 (JP)**
- **SAGAWA, Hiroaki**
**Kusatsu-shi, Shiga 525-0025 (JP)**
- **KATO, Ikunoshin**
**Koka-gun, Shiga 529-1851 (JP)**

(74) Representative: **Vossius & Partner**
**Siebertstrasse 4**
**81675 München (DE)**

(54) **THERAPEUTIC AGENT**

(57) A therapeutic agent and prophylactic agent for a disease accompanying an abnormality in an amount of insulin or insulin response, an agent for an insulin-mimetic action, a food, beverage and feed, an agent for enhancing glucose uptake into a cell, and an agent for inducing differentiation into an adipocyte, **characterized in that** each comprises as an effective ingredient at least one compound selected from the group consisting of a chalcone compound, an acetophenone compound, a coumarin compound, a phthalide compound, derivatives thereof, and pharmacologically acceptable salts thereof.



Printed by Jouve, 75001 PARIS (FR)

## Description

TECHNICAL FIELD

The present invention relates to a medicament, food; beverage or feed which is useful in treating or preventing a disease associated with insulin in a living body, for example, diabetes, obesity or the like.

BACKGROUND ART

Insulin is a hormone necessary for normal metabolism of carbohydrates, proteins and fats in a mammal. Since human suffering from type I diabetes does not sufficiently produce insulin, which is a hormone sustaining life, administration of insulin from the external is required for survival. Human suffering from type II diabetes needs administering with insulin or an agent for enhancing insulin secretion in order to control the glucose level in blood from an inappropriate level, due to causations such as deficiency of the amount of insulin produced and insulin resistance, to an appropriate level. However, among humans suffering from type II diabetes, therapeutic effects may not be found in cases where insulin or the agent for enhancing insulin secretion is administered to diabetic patients of which cause is insulin resistance caused by hyperinsulinemia, abnormality in insulin receptor, abnormality of a downstream signal of the insulin receptor or the like.

In recent years, in order to solve the side effects of insulin and the above-mentioned problems, developments have been made on a substance having physiological functions similar to those of insulin (hereinafter referred to as insulin-mimetic substance in some cases). It has been found that a synthetic benzoquinone derivative is an insulin-mimetic substance (for example, WO 99/51225), and that shikonin derived from *Lithospermum erythrorhizon* is an insulin-mimetic substance (for example, Kamei R. and seven others, *Biochem. Biophys. Res. Commun.,* 2002, Vol. 292, P642-651). These insulin-mimetic substances as mentioned above have been expected to ameliorate symptoms by exhibiting physiological activities similar to those of insulin, in not only type I diabetic patients but also type II diabetic patients, and even more type II diabetic patients of which cause is insulin resistance.

Chalcone compounds are known to have a variety of physiological activities, such as cytotoxicity, anticancer activity, anti-bacterial action, and antiviral action, have been known (for example, see J. R. Dimmock and three others, *Current Medicinal Chemistry,* 1999, Vol. 6, 1125-1149). Also, it has been known that these chalcones have enhancing actions for NGF production (see, for example, WO 01/54682), and it is described in the publication that chalcone compounds ameliorate diabetic retinopathy by enhancing NGF production. Diabetic retinopathy is a secondary disease of diabetes, and the publication does not show that chalcone compounds ameliorate diabetes itself, but simply discloses that the compounds locally treat retinopathy induced as a secondary disease of diabetes. An insulin-mimetic action such as anti-diabetic action or anti-obesity action by the chalcone compound has not been known so far.

Acetophenone compounds are known to have a suppressive action for cyclooxygenase-2 (COX-2) expression (for example, WO 01/030341), and the publication discloses that acetophenones are useful in preventing large intestine cancer or the like by exhibiting suppressive action for COX-2 expression. An insulin-mimetic action such as anti-diabetic action or anti-obesity action by the acetophenone has not been known so far.

Coumarin compounds are known to have an enhancing action for NGF production (for example, WO 02/083660), and the publication describes that coumarin compounds ameliorate diabetic retinopathy by enhancing NGF production. Diabetic retinopathy is a secondary disease of diabetes, and the publication does not show that coumarin compounds ameliorate diabetes itself, but simply discloses that the compounds locally treat retinopathy induced as a secondary disease of diabetes. An insulin-mimetic action such as anti-diabetic action or anti-obesity action by the coumarin compound has not been known so far.

Phthalide compounds are known to have anticonvulsant action. However, an insulin-mimetic action such as anti-diabetic action or anti-obesity action by the phthalide compound has not been known so far.

**DISCLOSURE OF INVENTION**

An object of the present invention is to develop a substance having insulin-mimetic action suitable as food materials and medicament materials, which is safe and capable of being conveniently taken, and to provide a medicament, food, beverage or feed using the composition or substance.

Summarizing the present invention, a first invention of the present invention relates to a therapeutic agent or prophylactic agent for a disease accompanying an abnormality in an amount of insulin or insulin response,

characterized in that the therapeutic agent or prophylactic agent comprises as an effective ingredient at least one compound selected from the group consisting of a compound represented by the following general formula (Formula 1):

(Formula 1)

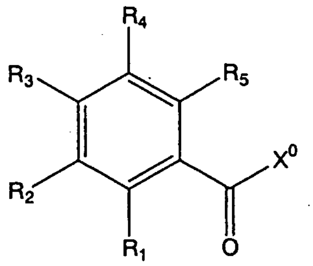

wherein each of $R_1$ to $R_5$, which may be the identical or different, is a hydrogen atom, or a hydroxyl group which may be esterified or etherified, a halogen group, an acyl group, an amino group, a nitro group, a hydroperoxy group, an aliphatic group, an aromatic group, an aromatic aliphatic group or a sugar residue, or one or more pairs selected from $R_1$ and $R_2$, $R_2$ and $R_3$, $R_3$ and $R_4$, and $R_4$ and $R_5$ may form a ring containing one or more atoms selected from a carbon atom, an oxygen atom, a nitrogen atom and a sulfur atom in a possible range, wherein a hydroxyl group which may be esterified or etherified, a halogen group, an acyl group, an amino group, a nitro group, a hydroperoxy group, an aliphatic group, an aromatic group, an aromatic aliphatic group, or a sugar residue may be bound to the above ring; and wherein $X^0$ is a hydrogen atom, an aliphatic group, an aromatic group, or an aromatic aliphatic group, wherein a hydroxyl group which may be esterified or etherified, a halogen group, an acyl group, an amino group, a nitro group, a hydroperoxy group, an aliphatic group, an aromatic group, an aromatic aliphatic group, or a sugar residue may be bound to the aliphatic group, the aromatic group, or the aromatic aliphatic group;
a compound represented by the following general formula (Formula 2):

(Formula 2)

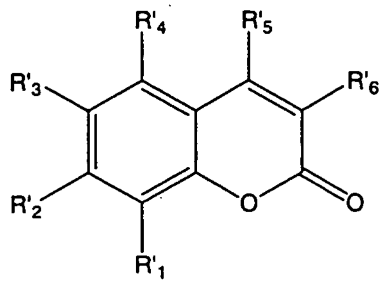

wherein each of $R'_1$ to $R'_6$, which may be identical or different, is a hydrogen atom, a hydroxyl group which may be esterified or etherified, a halogen group, an acyl group, an amino group, a nitro group, a hydroperoxy group, an aliphatic group, an aromatic group, an aromatic aliphatic group, or a sugar residue, one or more pairs selected from $R'_1$ and $R'_2$, $R'_2$ and $R'_3$, $R'_3$ and $R'_4$, $R'_4$ and $R'_5$, and $R'_5$ and $R'_6$ may form a ring containing one or more atoms selected from a carbon atom, an oxygen atom, a nitrogen atom and a sulfur atom in a possible range, wherein a hydroxyl group which may be esterified or etherified, a halogen group, an acyl group, an amino group, a nitro group, a hydroperoxy group, an aliphatic group, an aromatic group, an aromatic aliphatic group, or a sugar residue may be bound to the above ring;
a compound represented by the following general formula (Formula 3):

(Formula 3)

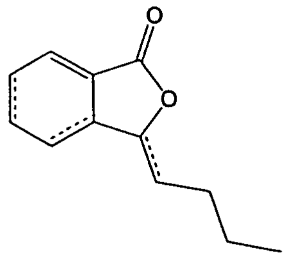

wherein a bond containing a dotted line is a single bond or a double bond; derivatives thereof; and pharmacologically acceptable salts thereof.

In the first invention of the present invention, the compound represented by the above general formula (Formula 1) is exemplified by a compound represented by the following general formula (Formula 4):

(Formula 4)

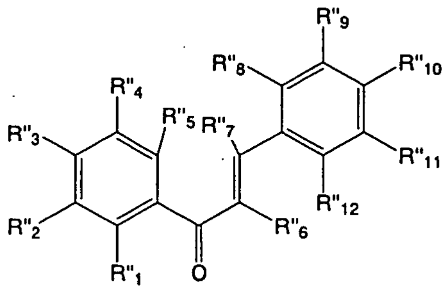

wherein each of $R''_1$ to $R''_{12}$, which may be identical or different, is a hydrogen atom, a hydroxyl group which may be esterified or etherified, a halogen group, an acyl group, an amino group, a nitro group, a hydroperoxy group, an aliphatic group, an aromatic group, an aromatic aliphatic group, or a sugar residue, or one or more pairs selected from $R''_1$ and $R''_2$, $R''_2$ and $R''_3$, $R''_3$ and $R''_4$, $R''_4$ and $R''_5$, $R''_8$ and $R''_9$, $R''_9$ and $R''_{10}$, $R''_{10}$ and $R''_{11}$, and $R''_{11}$ and $R''_{12}$ may form a ring containing one or more atoms selected from a carbon atom, an oxygen atom, a nitrogen atom, and a sulfur atom in a possible range, wherein a hydroxyl group which may be esterified or etherified, a halogen group, an acyl group, an amino group, a nitro group, a hydroperoxy group, an aliphatic group, an aromatic group, an aromatic aliphatic group, or a sugar residue may be bound to the above ring,
and/or
a compound represented by the following general formula (Formula 5):

(Formula 5)

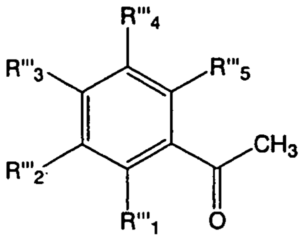

wherein each of $R'''_1$ to $R'''_5$, which may be identical or different, is a hydrogen atom, a hydroxyl group which may be esterified or etherified, a halogen group, an acyl group, an amino group, a nitro group, a hydroperoxy group, an aliphatic group, an aromatic group, an aromatic aliphatic group, or a sugar residue, or one or more pairs selected from $R'''_1$ and $R'''_2$, $R'''_2$ and $R'''_3$, $R'''_3$ and $R'''_4$, and $R'''_4$ and $R'''_5$ may form a ring containing one or more atoms selected from a carbon

atom, an oxygen atom, a nitrogen atom, and a sulfur atom in a possible range, wherein a hydroxyl group which may be esterified or etherified, a halogen group, an acyl group, an amino group, a nitro group, a hydroperoxy group, an aliphatic group, an aromatic group, an aromatic aliphatic group, or a sugar residue may be bound to the above ring.

In the first invention of the present invention, the compound represented by the above general formula (Formula 4) is exemplified by a compound represented by the following general formula (Formula 6):

(Formula 6)

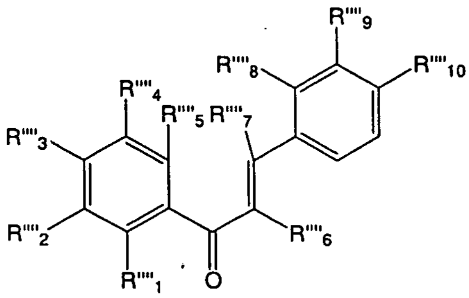

wherein each of $R''''_1$, $R''''_3$, $R''''_4$, and $R''''_5$, which may be identical or different, is a hydrogen atom, a hydroxyl group, a methyl group, a methoxy group, a prenyl group, a geranyl group, a halogen group, an acetoxy group, a methylbutyl group, a farnesyl group, an ethoxy group, a benzyl group or a benzyloxy group; $R''''_2$ is a hydrogen atom, a hydroxyl group, a methyl group, a methoxy group, a halogen group, an acetoxy group, an ethoxy group, a benzyl group, a benzyloxy group, or an aliphatic group having 5 to 15 carbon atoms; each of $R''''_6$ and $R''''_7$, which may be identical or different, is a hydrogen atom or a hydroxyl group; each of $R''''_8$, $R''''_9$, and $R''''_{10}$, which may be identical or different, is a hydrogen atom, a hydroxyl group, a tetrahydropyranyloxy group, an acetoxy group, a methoxy group, a geranyloxy group, a prenyl group, a halogen group or a nitro group, with proviso that a case where each of $R''''_3$, $R''''_9$ and $R''''_{10}$ is a hydroxyl group at the same time is excluded, wherein $R''''_1$ and $R''''_2$, or $R''''_2$ and $R''''_3$ may together form a ring structure represented by the following formula (Formula 7):

(Formula 7)

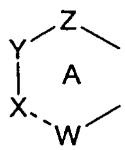

,

wherein each of W and Z is a carbon atom or an oxygen atom; X is a carbon atom; Y is 0 or 1 carbon atom; a dotted line is a single bond or a double bond; and the above ring A is a 5-membered ring or a 6-membered ring;

when the ring A is a 5-membered ring, $R''''_1$ is W, and $R''''_2$ is Z, or $R''''_2$ is W and $R''''_3$ is Z; when $R''''_1$ is W and $R''''_2$ is Z, W is an oxygen atom, a W-X bond is a single bond, each of X and Z is a carbon atom, and Y is not present; and further in this case, 1-hydroxy-1-methylethyl group is bound to X, and when $R''''_2$ is W and $R''''_3$ is Z, W is a carbon atom, a W-X bond is a single bond, X is a carbon atom, Y is not present, and Z is an oxygen atom; and further in this case, a 1-hydroxy-1,5-dimethyl-4-hexenyl group is bound to X;

when the ring A is a 6-membered ring, $R''''_1$ is W and $R''''_2$ is Z, or $R''''_2$ is W and $R''''_3$ is Z: when $R''''_1$ is W and $R''''_2$ is Z, W is an oxygen atom, a W-X bond is a single bond, and each of X, Y and Z is a carbon atom at the same time, and further in this case, one or more members selected from a hydrogen atom, a hydroxyl group, a methyl group and an isohexenyl group are bound to X and Y, or X and Y together form a hydroxydimethylcyclohexane ring wherein a methyl group is bound to X; when $R''''_2$ is W and $R''''_3$ is Z, each of W, X and Y is a carbon atom, a W-X bond is a double bond, and Z is an oxygen atom, and further in this case, a methyl group and an isohexenyl group are bound to Y.

In the first invention of the present invention, the compound represented by the above general formula (Formula 1) is exemplified by at least one compound selected from the group consisting of xanthoangelol, 4-hydroxyderricin, xanthoangelol H, 1-(5,6,7,8,8a,10a-hexahydro-1,7-dihydroxy-8,8,10a-trimethyl-9H-xanthen-4-yl)-3-(4-hydroxyphenyl)-2-propen-1-one, 1-(3,4-dihydro-3,5-dihydroxy-2-(3-isohexenyl)-2-methyl-2H-benzopyran-8-yl)-3-(4-hydroxyphenyl)-2-propen-1-one, 1-[2,3-dihydro-4-hydroxy-2-(1-hydroxy-1,5-dimethyl-4-hexenyl)-benzofuran-5-yl]-3-(4-hydroxyphenyl)-2-propen-1-one, 1-[2,3-dihydro-2-(1-hydroxy-1-methylethyl)-4-methoxy-benzofuran-7-yl]-3-(4-hydroxyphenyl)-2-propen-1-one, 1-[2,4-dihydroxy-3-(6,7-dihydroxy-3,7-dimethyl-2-octenyl)phenyl]-3-(4-hydroxyphenyl)-2-propen-1-one, 1-[3-(7-ethoxy-6-hydroxy-3,7-dimethyl-2-octenyl)-2,4-dihydroxyphenyl]-3-(4-hydroxyphenyl)-2-propen-1-one, 1-[3-(2,5-epoxy-2,6,6-trimethyl-cyclohexylmethyl)-2-hydroxy-4-methoxyphenyl]-3-(4-hydroxyphenyl)-2-propen-1-one, 1-[2-hydroxy-3-(7-hydroperoxy-3,7-dimethyl-2,5-octadienyl)-4-methoxyphenyl]-3-(4-hydroxyphenyl)-2-propen-1-one, 1-[2-hydroxy-3-(6-hydroperoxy-3,7-dimethyl-2,7-octadienyl)-4-methoxyphenyl]-3-(4-hydroxyphenyl)-2-propen-1-one,
1-[2-hydroxy-3-(7-hydroxy-3,7-dimethyl-2,5-octadienyl)-4-methoxyphenyl]-3-(4-hydroxyphenyl)-2-propen-1-one, xanthoangelol G, xanthoangelol F, lespeol, isobavachalcone, xanthohumol, 4,2'-dihydroxy-4'-methoxychalcone, 4,2'-dihydroxy-3'-methyl-4'-methoxychalcone, bavachalcone, 4-tetrahydropyranyloxy-2'-hydroxy-3'-prenyl-4'-methoxychalcone, 3,4,2'-trihydroxy-3'-prenyl-4'-methoxychalcone, 4,2'-diacetoxy-3'-prenyl-4'-methoxychalcone, 4,4'-dimethoxy-2'-hydroxy-3'-prenylchalcone, 3,4-dihydroxy-2',4'-dichlorochalcone, 4,2'-dihydroxy-3'-(3-methylbutyl)-4'-methoxychalcone, 4,4',6'-trimethoxy-2'-hydroxy-3'-prenylchalcone, 4,2'-dihydroxy-3'-prenyl-4'-ethoxychalcone, 4,2'-dihydroxy-3'-geranyl-4'-ethoxychalcone, 4,2'-dihydroxy-3'-farnesyl-4'-methoxychalcone, 4,2'-dihydroxy-3'-benzyl-4'-methoxychalcone, 4,2',4'-trihydroxy-3'-benzylchalcone, 4,2',4'-trihydroxy-3'-farnesylchalcone, 4,2',4'-triacetoxy-3'-geranylchalcone, 4,2'-diacetoxy-3'-geranyl-4'-methoxychalcone, 2'-acetoxy-3'-geranyl-4-hydroxy-4'-methoxychalcone, 2,2'-dihydroxy-3,3'-diprenyl-4,4'-dimethoxychalcone, 4,2'-dihydroxy-3'-geranyl-4'-benzyloxychalcone, 4-chloro-2',4'-dihydroxy-3'-geranylchalcone, 4-chloro-2',4'-dihydroxy-3'-prenylchalcone, 3-nitro-2'-hydroxy-3'-prenyl-4'-methoxychalcone, 4-hydroxy-2'-benzyloxy-3'-prenyl-4'-methoxychalcone, 4-acetoxy-2'-hydroxy-3'-prenyl-4'-methoxychalcone, 4-chloro-2'-acetoxy-3'-prenyl-4'-methoxychalcone, 4-chloro-2'-acetoxy-3'-geranyl-4'-methoxychalcone, 3,4,2'-trihydroxychalcone, 3,4,2',5'-tetrahydroxychalcone, chalcone, 4-chloro-2'-benzyloxychalcone, xanthoangelol B, xanthoangelol C, xanthoangelol D, xanthoangelol E and bavachromanol.

In the first invention of the present invention, the derivative of the compound represented by the above general formula (Formula 1) is exemplified by at least one compound selected from the group consisting of 4'-*O*-geranylnaringenin, isobavachin, prostratol F, 8-geranyl-4'-hydroxy-7-methoxyflavanone, 1-(2-hydroxy-3-(3-methylbutyl)-4-methoxyphenyl)-3-(4-hydroxyphenyl)-propan-1-one, 7-methoxy-8-prenyl-4'-hydroxyflavanone, 1-adamantyl-3-(3,4-dihydroxyphenyl)-2-propen-1-one, 1-adamantyl-3-hydroxy-4-(3,4-dihydroxyphenyl)-butan-1-one, and 1-adamantyl-4-(3,4-dihydroxyphenyl)-3-buten-1-one.

In the first invention of the present invention, the compound represented by the above general formula (Formula 5) is exemplified by a compound represented by the following general formula (Formula 8):

(Formula 8)

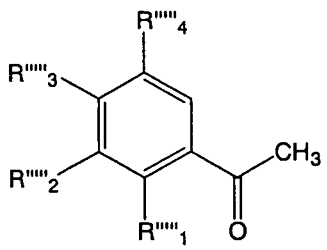

,

wherein $R'''''_1$ is a hydroxyl group, a methyl group or a benzyloxy group; each of $R'''''_2$ and $R'''''_4$, which may be identical or different, is a hydrogen atom, a hydroxyl group, a methyl group, a methoxy group, an ethoxy group, a prenyl group, a methylbutyl group, a prenyloxy group, a geranyl group, a farnesyl group, a benzyl group, a benzyloxy group or a tetrahydropyranyloxy group; and $R'''''_3$ is a hydrogen atom, a hydroxyl group, a methoxy group, an ethoxy group, an acetoxy group, a tetrahydropyranyloxy group, a benzyloxy group or a prenyloxy group.

In the first invention of the present invention, the compound represented by the above general formula (Formula 1) is exemplified by at least one compound selected from the group consisting of

2',4'-dihydroxy-5'-prenylacetophenone,
2'-hydroxy-4'-methoxy-5'-prenylacetophenone,
2'-hydroxy-3'-prenyl-4'-methoxyacetophenone,
2'-hydroxy-3'-methyl-4'-methoxyacetophenone,
2',4'-dihydroxy-3'-geranylacetophenone,
2'-hydroxy-3'-geranyl-4'-methoxyacetophenone,

2'-hydroxy-3'-(3-methylbutyl)-4'-methoxyacetophenone,
2'-hydroxy-3'-prenyl-4'-tetrahydropyranyloxyacetophenone,
2'-hydroxy-3'-geranyl-4'-tetrahydropyranyloxyacetophenone,
2'-hydroxy-3'-prenyl-4'-ethoxyacetophenone,
2'-hydroxy-3'-geranyl-4'-ethoxyacetophenone,
2',4'-dihydroxy-3'-farnesylacetophenone,
2'-hydroxy-3'-farnesyl-4'-methoxyacetophenone,
2',4'-dihydroxy-3'-benzylacetophenone,
2'-hydroxy-3'-benzyl-4'-methoxyacetophenorie,
2'-methyl-4'-prenyloxyacetophenone,
2'-benzyloxyacetophenone and
2'-hydroxy-3'-geranyl-4'-benzyloxyacetophenone.

In the first invention of the present invention, the compound represented by the above general formula (Formula 1) is exemplified by a compound in which, in the general formula, $X^0$ is a hydrogen atom, a 2-(2-furyl)vinyl group, a 2-(2-thienyl)vinyl group, a 4-phenyl-1,3-butadienyl group, or a 4-methyl-1,3-pentadienyl group; $R_1$ is a hydroxyl group; $R_2$ is a hydrogen atom or a prenyl group; $R_3$ is a methoxy group; and each of $R_4$ and $R_5$ is a hydrogen atom.

In the first invention of the present invention, the compound represented by the above general formula (Formula 1) is exemplified by at least one compound selected from the group consisting of 1-(2-hydroxy-4-methoxy-3-prenylphenyl)-3-(2-thienyl)-2-propen-1-one, 1-(2-hydroxy-4-methoxy-3-prenylphenyl)-5-phenyl-2,4-pentadien-1-one, 3-(2-furyl)-1-(2-hydroxy-3-prenyl-4-methoxyphenyl)-2-propen-1-one, 2-hydroxy-3-prenyl-4-methoxybenzaldehyde, 1-(2-hydroxy-3-prenyl-4-methoxyphenyl)-5-methyl-2,4-hexadien-1-one and 2-hydroxy-4-methoxybenzaldehyde.

In the first invention of the present invention, the derivative of the compound represented by the above general formula (Formula 1) is exemplified by 2-(2-furyl)-3-(2-furylmethylene)-2,3-dihydro-7-methoxy-8-prenyl-4H-1-benzopyran-4-one.

In the first invention of the present invention, the compound represented by the above general formula (Formula 2) is exemplified by a compound represented by the following general formula (Formula 9):

(Formula 9)

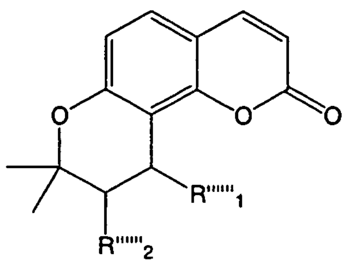

,

wherein each of $R''''''_1$ and $R''''''_2$, which may be identical or different, is a hydrogen atom, a hydroxyl group, an acetoxy group, or an angeloyloxy group.

In the first invention of the present invention, the compound represented by the above general formula (Formula 2) is exemplified by at least one compound selected from the group consisting of 3'-acetoxy-4'-angeloyloxy-3',4'-dihydroseselin, 3'-angeloyloxy-3',4'-dihydroseselin, 3'-angeloyloxy-4'-hydroxy-3',4'-dihydroseselin and xanthotoxin.

In the first invention of the present invention, the compound represented by the above general formula (Formula 3) is exemplified by sedanolide and/or n-butylidenephthalide.

Second to fifth inventions of the present invention relate to an agent for an insulin-mimetic action, a food, beverage or feed for treating or preventing a disease accompanying an abnormality in an amount of insulin or insulin response, an agent for enhancing glucose uptake into a cell, and an agent for inducing differentiation into an adipocyte, characterized in that each comprises an effective ingredient of the first invention of the present invention.

A sixth invention of the present invention relates to compounds represented by the following formulas (Formula 10) to (Formula 44):

(Formula 10)

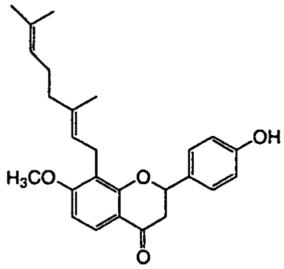

,

(Formula 11)

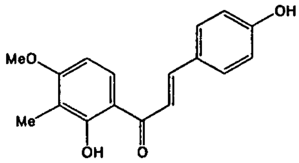

,

(Formula 12)

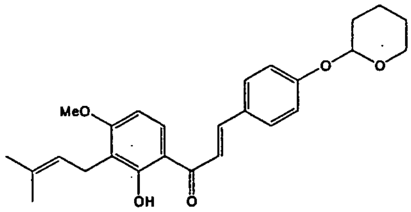

,

(Formula 13)

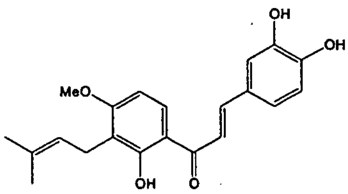

,

(Formula 14)

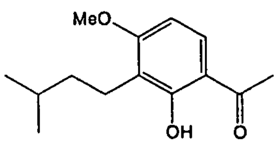

,

(Formula 15)

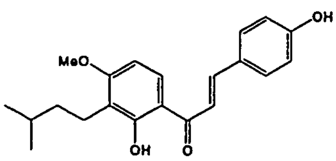

,

(Formula 16)

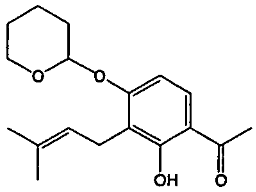

,

(Formula 17)

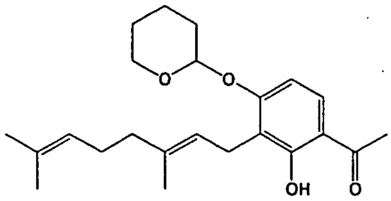

,

(Formula 18)

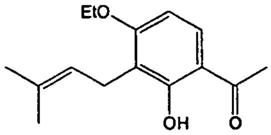

,

(Formula 19)

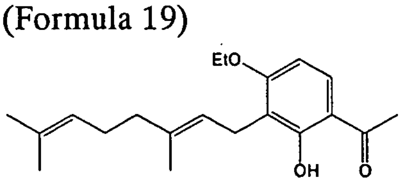

,

(Formula 20)

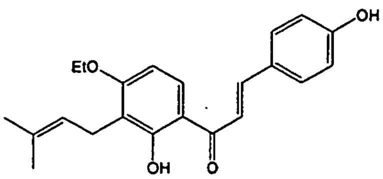

,

(Formula 21)

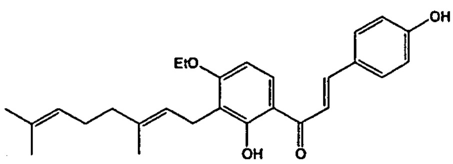

,

(Formula 22)

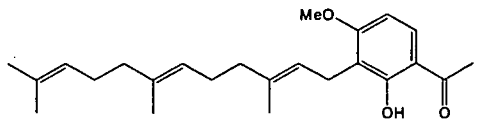

,

(Formula 23)

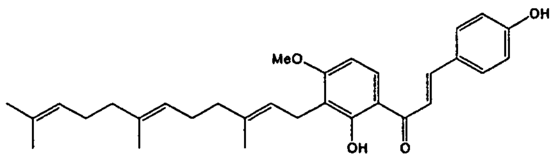

,

(Formula 24)

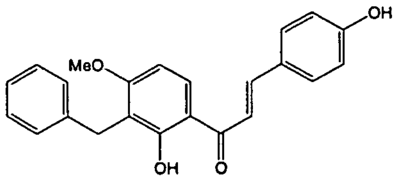

,

(Formula 25)

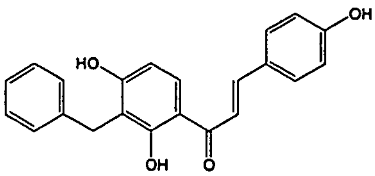

,

(Formula 26)

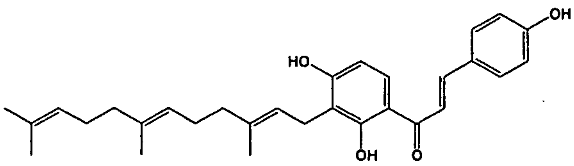

,

(Formula 27)

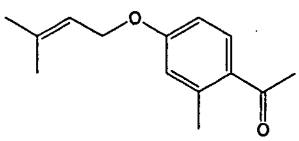

,

(Formula 28)

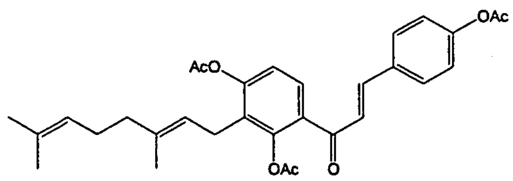

,

(Formula 29-1)

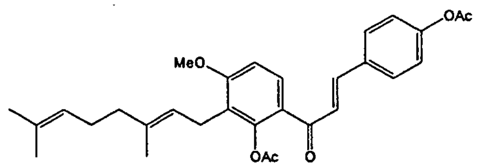

,

(Formula 29-2)

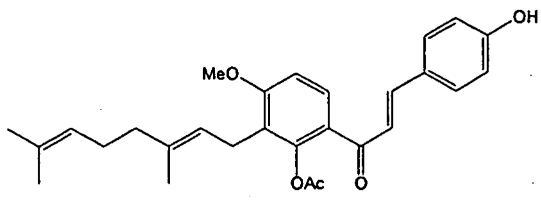

,

(Formula 30)

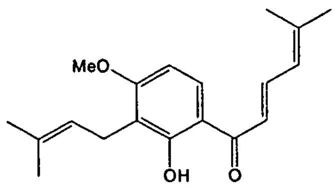

,

(Formula 31)

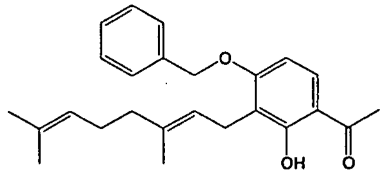

,

(Formula 32)

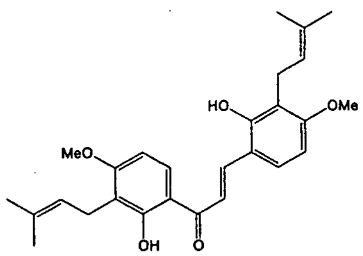

,

(Formula 33)

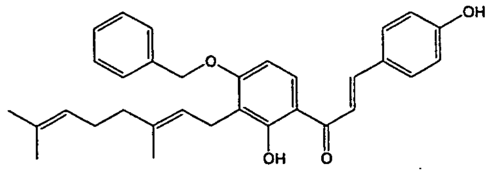

,

(Formula 34)

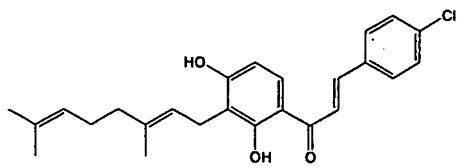

,

(Formula 35)

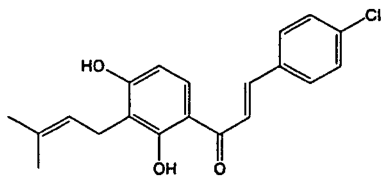

,

(Formula 36)

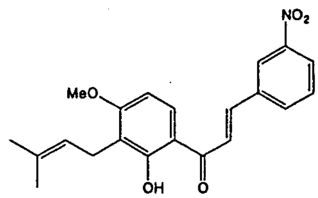

,

(Formula 37)

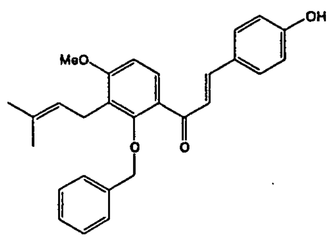

,

(Formula 38)

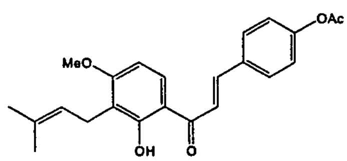

,

(Formula 39)

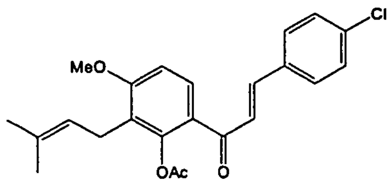

,

(Formula 40)

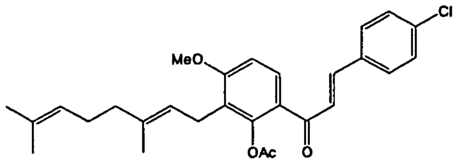

,

(Formula 41)

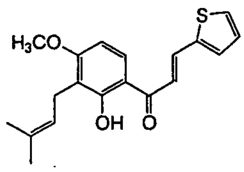

,

(Formula 42)

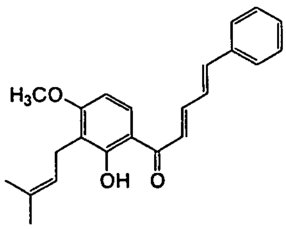

,

(Formula 43)

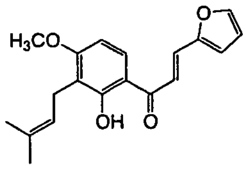

,

(Formula 44)

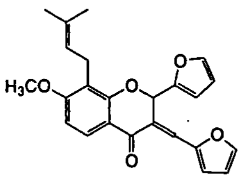

,

or a salt thereof.

The present invention relates to a medicament, food, beverage, or feed for treating or preventing a disease accompanying an abnormality in an amount of insulin or insulin response. The medicament is useful as a therapeutic agent or prophylactic agent for a disease accompanying an abnormality in an amount of insulin or insulin response, such as diabetes or obesity. Also, according to the food or beverage, by taking it as foodstuff on a daily basis, symptoms of a disease accompanying an abnormality in an amount of insulin or insulin response can be ameliorated and the like. Therefore, the foodstuff of the present invention can be said to be functional foodstuff, and are effective in maintaining homeostasis of a living body by their insulin-mimetic actions. In addition, the feed of the present invention can also exhibit similar effects.

## BRIEF DESCRIPTION OF THE DRAWINGS

Figure 1 is a chart showing $^{1}$H-NMR spectrum of TB1.
Figure 2 is a chart showing $^{13}$C-NMR spectrum of TB1.
Figure 3 is a chart showing $^{1}$H-NMR spectrum of TB2.
Figure 4 is a chart showing $^{13}$C-NMR spectrum of TB2.
Figure 5 is a chart showing $^{1}$H-NMR spectrum of TB3.
Figure 6 is a chart showing $^{13}$C-NMR spectrum of TB3.
Figure 7 is a chart showing $^{1}$H-NMR spectrum of TB4.
Figure 8 is a chart showing $^{13}$C-NMR spectrum of TB4.
Figure 9 is a chart showing $^{1}$H-NMR spectrum of TB5.
Figure 10 is a chart showing $^{13}$C-NMR spectrum of TB5.
Figure 11 is a chart showing $^{1}$H-NMR spectrum of TB6.
Figure 12 is a chart showing $^{13}$C-NMR spectrum of TB6.
Figure 13 is a chart showing $^{1}$H-NMR spectrum of TB7.
Figure 14 is a chart showing $^{13}$C-NMR spectrum of TB7.
Figure 15 is a chart showing $^{1}$H-NMR spectrum of TB8.
Figure 16 is a chart showing $^{13}$C-NMR spectrum of TB8.
Figure 17 is a chart showing $^{1}$H-NMR spectrum of TB9.
Figure 18 is a chart showing $^{13}$C-NMR spectrum of TB9.
Figure 19 is a chart showing $^{1}$H-NMR spectrum of a compound C081.
Figure 20 is a chart showing $^{13}$C-NMR spectrum of a compound C081.
Figure 21 is a chart showing $^{1}$H-NMR spectrum of a compound C082.
Figure 22 is a chart showing $^{13}$C-NMR spectrum of a compound C082.
Figure 23 is a chart showing $^{1}$H-NMR spectrum of a coumarin compound A.
Figure 24 is a chart showing $^{13}$C-NMR spectrum of a coumarin compound A.
Figure 25 is a chart showing $^{1}$H-NMR spectrum of a coumarin compound B.
Figure 26 is a chart showing $^{13}$C-NMR spectrum of a coumarin compound B.
Figure 27 is a chart showing $^{1}$H-NMR spectrum of a coumarin compound C.
Figure 28 is a chart showing $^{1}$H-NMR spectrum of a compound (C023).
Figure 29 is a chart showing $^{1}$H-NMR spectrum of a compound (C030).
Figure 30 is a chart showing $^{1}$H-NMR spectrum of a compound (C031).
Figure 31 is a chart showing $^{1}$H-NMR spectrum of a compound (C041).
Figure 32 is a chart showing $^{1}$H-NMR spectrum of a compound (C043).
Figure 33 is a chart showing $^{1}$H-NMR spectrum of a compound (C044).
Figure 34 is a chart showing $^{1}$H-NMR spectrum of a compound (C045).
Figure 35 is a chart showing $^{1}$H-NMR spectrum of a compound (C047).
Figure 36 is a chart showing $^{1}$H-NMR spectrum of a compound (C048).
Figure 37 is a chart showing $^{1}$H-NMR spectrum of a compound (C049).
Figure 38 is a chart showing $^{1}$H-NMR spectrum of a compound (C050).
Figure 39 is a chart showing $^{1}$H-NMR spectrum of a compound (C052).
Figure 40 is a chart showing $^{1}$H-NMR spectrum of a compound (C053).
Figure 41 is a chart showing $^{1}$H-NMR spectrum of a compound (C056).
Figure 42 is a chart showing $^{1}$H-NMR spectrum of a compound (C057).
Figure 43 is a chart showing $^{1}$H-NMR spectrum of a compound (C058).
Figure 44 is a chart showing $^{1}$H-NMR spectrum of a compound (C059).
Figure 45 is a chart showing $^{1}$H-NMR spectrum of a compound (C061).

Figure 46 is a chart showing $^1$H-NMR spectrum of a compound (C064-1).
Figure 47 is a chart showing $^1$H-NMR spectrum of a compound (C069).
Figure 48 is a chart showing $^1$H-NMR spectrum of a compound (C070).
Figure 49 is a chart showing $^1$H-NMR spectrum of a compound (C072).
Figure 50 is a chart showing $^1$H-NMR spectrum of a compound (C073).
Figure 51 is a chart showing $^1$H-NMR spectrum of a compound (C074).
Figure 52 is a chart showing $^1$H-NMR spectrum of a compound (C075).
Figure 53 is a chart showing $^1$H-NMR spectrum of a compound (C076).
Figure 54 is a chart showing $^1$H-NMR spectrum of a compound (C077).
Figure 55 is a chart showing $^1$H-NMR spectrum of a compound (C078).
Figure 56 is a chart showing $^1$H-NMR spectrum of a compound (C079).
Figure 57 is a chart showing $^1$H-NMR spectrum of a compound (C080).
Figure 58 is a chart showing $^1$H-NMR spectrum of a compound (C-THP).
Figure 59 is a chart showing $^1$H-NMR spectrum of a compound (C-CIN).
Figure 60 is a chart showing $^{13}$C-NMR spectrum of a compound (C-CIN).
Figure 61 is a chart showing $^1$H-NMR spectrum of a compound (FUR-1).
Figure 62 is a chart showing $^1$H-NMR spectrum of a compound (FUR-2).
Figure 63 is a chart showing $^{13}$C-NMR spectrum of a compound (FUR-2).
Figure 64 is a chart showing $^1$H-NMR spectrum of a compound (C064-2).
Figure 65 is a graph showing a synergistic effect of activities of 4-hydroxyderricin and insulin to enhance glucose uptake.
Figure 66 is a graph showing an enhancing action of 4-hydroxyderricin on glucose uptake inhibited by cytochalasin B.
Figure 67 is a graph showing an enhancing action of insulin or 4-hydroxyderricin on glucose uptake in a mature adipocyte which is induced to be differentiated by xanthoangelol.
Figure 68 is a graph showing that 4-hydroxyderricin, xanthoangelol, xanthohumol, xanthoangelol F and xanthoangelol H do not have activating ability for peroxisome proliferating agent-responsive receptor $\gamma$ (PPAR$\gamma$).
Figure 69 is a graph showing an enhancing action of 4-hydroxyderricin on glucose uptake using a cell caused to have insulin resistance by TNF$\alpha$.

## BEST MODE FOR CARRYING OUT THE INVENTION

The medicament, food, beverage, feed or the like provided by the present invention comprises as an effective ingredient at least one compound selected from the group consisting of a compound represented by the above general formula (Formula 1), a compound represented by the above general formula (Formula 2), a compound represented by the above general formula (Formula 3), derivatives thereof, and pharmacologically acceptable salts thereof. The desired effects of the present invention as described below are exhibited based on the insulin-mimetic action exhibited by these effective ingredients.

In the present invention, the insulin-mimetic action is not particularly limited as long as at least one of the physiological activities possessed by insulin is exhibited. For example, the insulin-mimetic action is exemplified by at least one of metabolic regulatory actions such as enhancement of uptake of a sugar or an amino acid in a cell, and synthesis and degradation inhibition of glycogen or protein. Also, the presence or absence of the insulin-mimetic action can be conveniently determined in accordance with the method described in Example 82 or 83 set forth below. Since the effective ingredient of the present invention has an insulin-mimetic action, there can be exhibited a therapeutic effect or prophylactic effect for all sorts of diseases for which the use of insulin is effective from the therapeutic or prophylactic viewpoint.

The halogen group as used herein is not particularly limited. The halogen group includes, for example, a fluoro group, a chloro group, a bromo group, an iodo group, an iodosyl group, an iodyl group, and a dichloroiodo group.

The hydroxyl group which may be esterified or etherified is not particularly limited. The hydroxyl group includes, for example, a hydroxyl group, a methoxy group, an ethoxy group, a benzyloxy group, an acetoxy group, a tetrahydropyranyloxy group, an angeloyloxy group, a prenyloxy group, a gelanyloxy group, and a farnesyloxy group.

The acyl group is not particularly limited. The acyl group includes, for example, an aldehyde group, a carboxymethyl group, a carboxyl group, an acetyl group, an aroyl group and the like.

The aliphatic group refers to a saturated or unsaturated linear, branched or cyclic hydrocarbon group to which an optional functional group (including a substituent) may be added. The hydrocarbon group used herein is not particularly limited. The preferred hydrocarbon group is exemplified by a linear alkyl group, a branched alkyl group, a linear alkenyl group, a branched alkenyl group, and a cyclic alkyl group, each having 1 to 30 carbon atoms. Further, the aliphatic group as used herein also encompasses those prepared by adding the hydroxyl group which may be esterified or etherified, the halogen group, the acyl group, the amino group, the nitro group or the hydroperoxy group mentioned above to these

aliphatic groups; and these aliphatic groups containing an epoxy structure.

Specifically, the aliphatic group as used herein is exemplified by, for example, a methyl group, an ethyl group, a n-propyl group, an isopropyl group, an isobutyl group, a sec-butyl group, a tert-butyl group, an isopentyl group, a neopentyl group, a tert-pentyl group, an ethenyl group, an allyl group, a trans-1-propenyl group, a cis-1-propenyl group, a methylbutyl group, a prenyl group, an isohexenyl group, a geranyl group, a farnesyl group, an isopropenyl group, a cis-1-methyl-1-propenyl group, a trans-1-methyl-1-propenyl group, a trans-1-methyl-1-propenyl group, a trans-1-ethyl-1-propenyl group, an adamantyl group, a 4-methyl-1,3-pentadienyl group, a 6,7-dihydroxy-3,7-dimethyl-2-octenyl group, a 7-ethoxy-6-hydroxy-3,7-dimethyl-2-octenyl group, a 2,5-epoxy-2,6,6-trimethyl-cyclohexylmethyl group, a 7-hydroperoxy-3,7-dimethyl-2,5-octadienyl group, a 6-hydroperoxy-3,7-dimethyl-2,7-octadienyl group, a 7-hydroxy-3,7-dimethyl-2,5-octadienyl group, a 6-hydroxy-3,7-dimethyl-2,7-octadienyl group, a 3-methyl-6-oxo-2-hexenyl group, a 2-hydroxy-3-methyl-3-butenyl group, a 2-hydroperoxy-3-methyl-3-butenyl group, a 1-hydroxy-1,5-dimethyl-4-hexenyl group, and a 1-hydroxy-1-methylethyl group.

Here, the aliphatic group exempifying $X^0$ in the above general formula (Formula 1) preferably includes a methyl group and a 4-methyl-1,3-pentadienyl group.

In addition, the aliphatic group having 5 to 15 carbon atoms in the above general formula (Formula 6) is exemplified by a methylbutyl group, a prenyl group, a geranyl group, a farnesyl group, a 6,7-dihydroxy-3,7-dimethyl-2-octenyl group, a 7-ethoxy-6-hydroxy-3,7-dimethyl-2-octenyl group, a 2,5-epoxy-2,6,6-trimethyl-cyclohexylmethyl group, a 7-hydroperoxy-3,7-dimethyl-2,5-octadienyl group, a 6-hydroperoxy-3,7-dimethyl-2,7-octadienyl group, a 7-hydroxy-3,7-dimethyl-2,5-octadienyl group, a 6-hydroxy-3,7-dimethyl-2,7-octadienyl group, a 3-methyl-6-oxo-2-hexenyl group, a 2-hydroxy-3-methyl-3-butenyl group, and a 2-hydroperoxy-3-methyl-3-butenyl group.

In addition, the aromatic group includes, for example, a phenyl group, a furyl group, a thienyl group, a naphthyl group, a biphenyl group, and a pyrrolyl group, a pyridyl group, an indolyl group, an imidazolyl group, a tolyl group, a xylyl group, and the like. Also, the aromatic group as used herein encompasses those prepared by adding a hydroxyl group which may be esterified or etherified, a halogen group, an acyl group, an amino group, a nitro group, or a hydroperoxy group to these aromatic groups.

Preferred examples of the aromatic aliphatic group are exemplified by aromatic aliphatic groups having a saturated or unsaturated linear or branched hydrocarbon group having 1 to 20 carbon atoms, and the aromatic aliphatic group includes, for example, a phenylalkyl group of which alkyl group has 1 to 20 carbon atoms (e.g., a benzyl group, a phenethyl group), a 2-phenylvinyl group, a 2-(4-hydroxyphenyl)vinyl group, a 2-(2-furyl)vinyl group, a 2-(2-thienyl)vinyl group, a 4-phenyl-1,3-butadienyl group, a styryl group, a cinnamyl group and the like. In addition, the aromatic aliphatic group as used herein also encompasses those prepared by adding a hydroxyl group which may be esterified or etherified, a halogen group, an acyl group, a nitro group, an amino group, or a hydroperoxy group to these aromatic aliphatic groups.

Here, the aromatic aliphatic group exemplifying $X^0$ in the above general formula (Formula 1) preferably includes an aromatic aliphatic group containing a 2-phenylvinyl group in the structure, a 2-(2-furyl)vinyl group, a 2-(2-thienyl)vinyl group, and a 4-phenyl-1,3-butadienyl group.

The sugar constituting the sugar residue includes, for example, monosaccharides such as glucose, threose, ribose, apiose, allose, rhamnose, arabinopyranose, ribulose, xylose, galactose, 3,6-anhydrogalactose, mannose, talose, fucose, fructose, glucuronic acid, and galacturonic acid; disaccharides such as gentiobiose, neohesperidose, rutinose, agarobiose, isomaltose, sucrose, xylobiose, nigerose, maltose, and lactose; an oligosaccharide derived from a polysaccharide such as agarose, fucoidan, and starch; polysaccharides such as agarose, fucoidan, and starch; and the like. In addition, the sugar residue includes a compound in which a sugar is O-, N-, S-, or C-glycoside-bound, and a compound in which a sugar is bound via a C-C bond to a carbon other than a reducing end of a sugar. In addition, the sugar residue as used herein also encompasses those prepared by adding a hydroxyl group which may be esterified or etherified, a halogen group, an acyl group, a nitro group, an amino acid, a hydroperoxy group, a sulfate group, a phosphate group or the like to these sugar residues.

In the present specification, the phrase "within a possible range" in the explanation of the general formulas means within a range that the compound can be naturally present, or that the compound can be artificially synthesized.

The derivative of the compound represented by the above general formula (Formula 1), the compound represented by the above general formula (Formula 2), or the compound represented by the above general formula (Formula 3), which is an effective ingredient of the present invention, is a compound which has an insulin-mimetic action, and can be synthesized from those compounds, or an analogue of those compounds, preferably those in which a part of a structure of those compounds has been modified by substitution, deletion or the like, and various derivatives as mentioned below are included.

The compound represented by the above general formula (Formula 4), which is one embodiment of the compound represented by the above general formula (Formula 1) of the present invention, is a chalcone compound. In the present invention, the chalcone compound is exemplified by the compound represented by the above general formula (Formula 6). In the formula of the above general formula (Formula 6), especially preferably, $R''''_1$ is exemplified by a hydroxyl group, a methoxy group, an acetoxy group, a halogen group, a benzyloxy group or a hydrogen atom; $R''''_2$ is

exemplified by an aliphatic group having 5 to 15 carbon atoms, a hydrogen atom, a methyl group, or a benzyl group; $R''''_3$ is exemplified by a hydroxyl group, a methoxy group, a halogen group, an ethoxy group, an acetoxy group, a benzyloxy group or a hydrogen atom; $R''''_4$ is exemplified by a hydrogen atom, a prenyl group or a hydroxyl group; $R''''_5$ is exemplified by a hydrogen atom, a hydroxyl group or a methoxy group; each of $R''''_6$, $R''''_7$ and $R''''_8$, which may be identical or different, is exemplified by a hydrogen atom or a hydroxyl group; $R''''_9$ is exemplified by a hydrogen atom, a hydroxyl group, a prenyl group or a nitro group; $R''''_{10}$ is exemplified by a hydroxyl group, a hydrogen atom, a tetrahydropyranyloxy group, an acetoxy group, a methoxy group, a geranyloxy group or a halogen group, with proviso that a case where each of $R''''_3$, $R''''_9$ and $R''''_{10}$ is a hydroxyl group at the same time is excluded. In addition, $R''''_1$ and $R''''_2$, or $R''''_2$ and $R''''_3$ may together form a ring structure represented by the following formula (Formula 7):

(Formula 7)

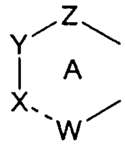

,

wherein each of W and Z is a carbon atom or an oxygen atom; X is a carbon atom; Y is 0 or 1 carbon atom; a dotted line is a single bond or a double bond; and the above ring A is a 5-membered ring or a 6-membered ring;
when the ring A is a 5-membered ring, $R''''_1$ is W, and $R''''_2$ is Z, or $R''''_2$ is W and $R''''_3$ is Z; when $R''''_1$ is W and $R''''_2$ is Z, W is an oxygen atom, a W-X bond is a single bond, each of X and Z is a carbon atom, and Y is not present; and further in this case, 1-hydroxy-1-methylethyl group is bound to X; alternatively, when $R''''_2$ is W and $R''''_3$ is Z, W is a carbon atom, a W-X bond is a single bond, X is a carbon atom, Y is not present, and Z is an oxygen atom; and further in this case, a 1-hydroxy-1,5-dimethyl-4-hexenyl group is bound to X;
when the ring A is a 6-membered ring, $R''''_1$ is W and $R''''_2$ is Z, or $R''''_2$ is W and $R''''_3$ is Z; when $R''''_1$ is W and $R''''_2$ is Z, W is an oxygen atom, a W-X bond is a single bond, and each of X, Y and Z is a carbon atom at the same time, and further in this case, one or more members selected from a hydrogen atom, a hydroxyl group, a methyl group and an isohexenyl group are bound to X and Y, or X and Y together form a hydroxydimethylcyclohexane ring wherein a methyl group is bound to X; alternatively when $R''''_2$ is W and $R''''_3$ is Z, each of W, X and Y is a carbon atom, a W-X bond is a double bond, and Z is an oxygen atom, and further in this case, a methyl group and an isohexenyl group are bound to Y.

In addition, the chalcone compound used in the present invention is especially preferably exemplified by xanthoangelol, 4-hydroxyderricin, xanthoangelol H, 1-(5,6,7,8,8a,10a-hexahydro-1,7-dihydroxy-8,8,10a-trimethyl-9H-xanthen-4-yl)-3-(4-hydroxyphenyl)-2-propen-1-one (TB1), 1-(3,4-dihydro-3,5-dihydroxy-2-(3-isohexenyl)-2-methyl-2H-benzopyran-8-yl)-3-(4-hydroxyphenyl)-2-propen-1-one (TB2), 1-[2,3-dihydro-4-hydroxy-2-(1-hydroxy-1,5-dimethyl-4-hexenyl)-benzofuran-5-yl]-3-(4-hydroxyphenyl)-2-propen-1-one (TB3), 1-[2,3-dihydro-2-(1-hydroxy-1-methylethyl)-4-methoxy-benzofuran-7-yl]-3-(4-hydroxyphenyl)-2-propen-1-one (TB4), 1-[2,4-dihydroxy-3-(6,7-dihydroxy-3,7-dimethyl-2-octenyl)phenyl]-3-(4-hydroxyphenyl)-2-propen-1-one (TB5), 1-[3-(7-ethoxy-6-hydroxy-3,7-dimethyl-2-octenyl)-2,4-dihydroxyphenyl]-3-(4-hydroxyphenyl)-2-propen-1-one (TB6), 1-[3-(2,5-epoxy-2,6,6-trimethyl-cyclohexylmethyl)-2-hydroxy-4-methoxyphenyl]-3-(4-hydroxyphenyl)-2-propen-1-one (TB7), 1-[2-hydroxy-3-(7-hydroperoxy-3,7-dimethyl-2,5-octadienyl)-4-methoxyphenyl]-3-(4-hydroxyphenyl)-2-propen-1-one (TB8), 1-[2-hydroxy-3-(6-hydroperoxy-3,7-dimethyl-2,7-octadienyl)-4-methoxyphenyl]-3-(4-hydroxyphenyl)-2-propen-1-one (TB9), 1-[2-hydroxy-3-(7-hydroxy-3,7-dimethyl-2,5-octadienyl)-4-methoxyphenyl]-3-(4-hydroxyphenyl)-2-propen-1-one (C081), xanthoangelol G, xanthoangelol F, lespeol, isobavachalcone, xanthohumol, 4,2'-dihydroxy-4'-methoxychalcone (C020), 4,2'-dihydroxy-3'-methyl-4'-methoxychalcone (C023), bavachalcone, 4-tetrahydropyranyloxy-2'-hydroxy-3'-prenyl-4'-methoxychalcone (C030), 3,4,2'-trihydroxy-3'-prenyl-4'-methoxychalcone (C031), 4,2'-diacetoxy-3'-prenyl-4'-methoxychalcone (C032), 4,4'-dimethoxy-2'-hydroxy-3'-prenylchalcone (C035), 3,4-dihydroxy-2',4'-dichlorochalcone (C011), 4,2'-dihydroxy-3'-(3-methylbutyl)-4'-methoxychalcone (C043), 4,4',6'-trimethoxy-2'-hydroxy-3'-prenylchalcone (C046), 4,2'-dihydroxy-3'-prenyl-4'-ethoxychalcone (C049), 4,2'-dihydroxy-3'-geranyl-4'-ethoxychalcone (C050), 4,2'-dihydroxy-3'-farnesyl-4'-methoxychalcone (C053), 4,2'-dihydroxy-3'-benzyl-4'-methoxychalcone (C056), 4,2',4'-trihydroxy-3'-benzylchalcone (C057), 4,2',4'-trihydroxy-3'-farnesylchalcone (C058), 4,2',4'-triacetoxy-3'-geranylchalcone (C061), 4,2'-diacetoxy-3'-geranyl-4'-methoxychalcone (C064-1), 2'-acetoxy-3'-geranyl-4-hydroxy-4'-methoxychalcone (C064-2), 4-chloro-2'-benzyloxychalcone (C066), 2,2'-dihydroxy-3,3'-diprenyl-4,4'-dimethoxychalcone (C072), 4,2'-dihydroxy-3'-geranyl-4'-benzyloxychalcone (C073), 4-chloro-2',4'-dihydroxy-3'-geranylchalcone (C074), 4-chloro-2',4'-dihydroxy-3'-prenylchalcone (C075), 3-nitro-2'-hydroxy-3'-prenyl-4'-methoxychalcone (C076), 4-hydroxy-2'-benzyloxy-3'-prenyl-4'-methoxychalcone (C077), 4-acetoxy-2'-hydroxy-3'-prenyl-4'-methoxychalcone (C078), 4-chloro-2'-acetoxy-3'-prenyl-4'-methoxychalcone (C079), 4-chloro-2'-acetoxy-3'-geranyl-4'-methoxychalcone (C080), 3,4,2'-trihydroxychalcone (C005), 3,4,2',5'-tetrahydroxychalcone (C006), chalcone, xanthoangelol B, xanthoangelol C, xanthoangelol D, xanthoangelol E and bavachromanol.

The structural formulas for these compounds are shown in Tables 1 to 6. In Tables 1 to 6, the listing of Examples on the rightmost column shows the names used in Examples given below for each of the compounds.

In the present specification, "Me" stands for a methyl group, "MeO" or "OMe" stands for a methoxy group, "EtO" stands for an ethoxy group, and "OAc" stands for an acetoxy group.

| Table 1 | | |
| --- | --- | --- |
| Name of Compound | Structural Formula | Example |
| Xanthoangelol | 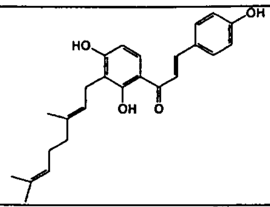 | |
| 4-Hydroxyderricin | 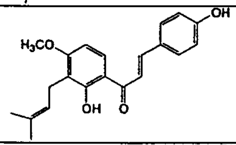 | |
| Xanthoangelol H | 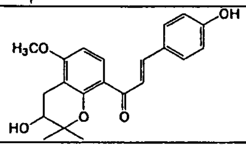 | |
| 1-(5,6,7,8,8a,10a-Hexahydro-1,7-dihydroxy-8,8,10a-trimethyl-9H-xanthen-4-yl)-3-(4-hydroxyphenyl)-2-propen-1-one | 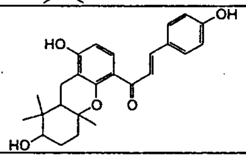 | TB1 |
| 1-(3,4-Dihydro-3,5-dihydroxy-2-(3-isohexenyl)-2-methyl-2H-benzopyran-8-yl)-3-(4-hydroxyphenyl)-2-propen-1-one | 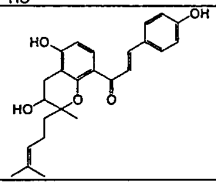 | TB2 |
| 1-[2,3-Dihydro-4-hydroxy-2-(1-hydroxy-1,5-dimethyl-4-hexenyl)-benzofuran-5-yl]-3-(4-hydroxyphenyl)-2-propen-1-one | 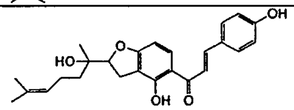 | TB3 |
| 1-[2,3-Dihydro-2-(1-hydroxy-1-methylethyl)-4-methoxybenzofuran-7-yl]-3-(4-hydroxyphenyl)-2-propen-1-one | 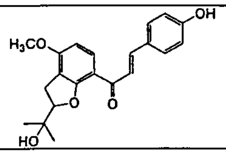 | TB4 |
| 1-[2,4-Dihydroxy-3-(6,7-dihydroxy-3,7-dimethyl-2-octenyl)phenyl]-3-(4-hydroxyphenyl)-2-propen-1-one | 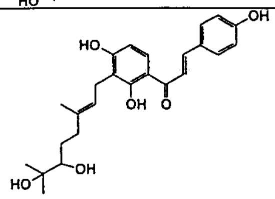 | TB5 |
| 1-[3-(7-Ethoxy-6-hydroxy-3,7-dimethyl-2-octenyl)-2,4-dihydroxyphenyl]-3-(4-hydroxyphenyl)-2-propen-1-one | 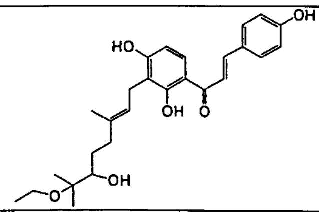 | TB6 |
| 1-[3-(2,5-Epoxy-2,6,6-trimethyl-cyclohexylmethyl)-2-hydroxy-4-methoxyphenyl]-3-(4-hydroxyphenyl)-2-propen-1-one | 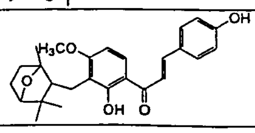 | TB7 |

| Table 2 | | |
|---|---|---|
| Name of Compound | Structural Formula | Example |
| 1-[2-Hydroxy-3-(7-hydroperoxy-3,7-dimethyl-2,5-octadienyl)-4-methoxyphenyl]-3-(4-hydroxyphenyl)-2-propen-1-one | 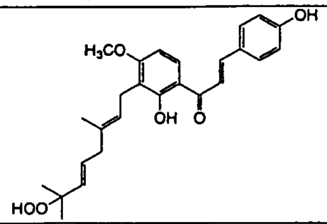 | TB8 |
| 1-[2-Hydroxy-3-(6-hydroperoxy-3,7-dimethyl-2,7-octadienyl)-4-methoxyphenyl]-3-(4-hydroxyphenyl)-2-propen-1-one | 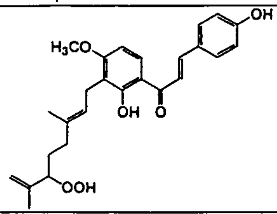 | TB9 |
| 1-[2-Hydroxy-3-(7-hydroxy-3,7-dimethyl-2,5-octadienyl)-4-methoxyphenyl]-3-(4-hydroxyphenyl)-2-propen-1-one | 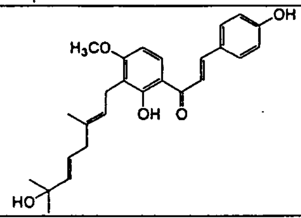 | C081 |
| Xanthoangelol G | 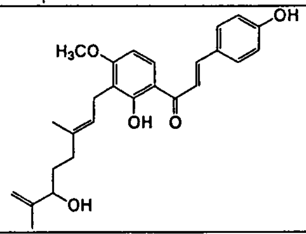 | |
| Xanthoangelol F | 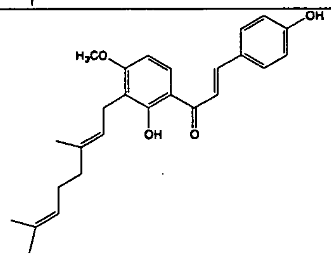 | |
| Lespeol | 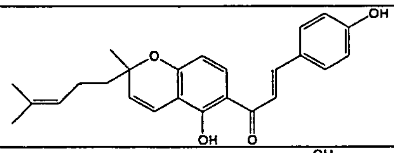 | |
| Isobavachalcone | 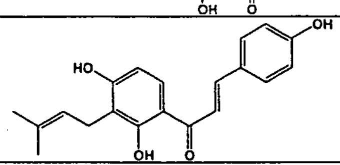 | |
| Xanthohumol | 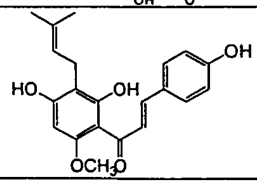 | |

| Table 3 | | |
|---|---|---|
| Name of Compound | Structural Formula | Example |
| 4,2'-Dihydroxy-4'-methoxychalcone | 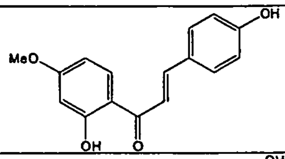 | C020 |
| 4,2'-Dihydroxy-3'-methyl-4'-methoxychalcone | 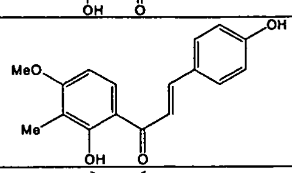 | C023 |
| Bavachalcone | 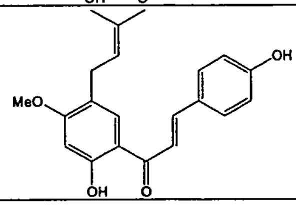 | |
| 4-Tetrahydropyranyloxy-2'-hydroxy-3'-prenyl-4'-methoxychalcone | 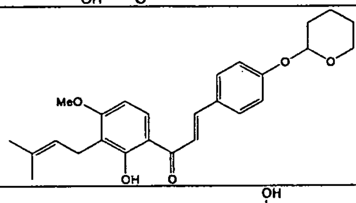 | C030 |
| 3,4,2'-Trihydroxy-3'-prenyl-4'-methoxychalcone | 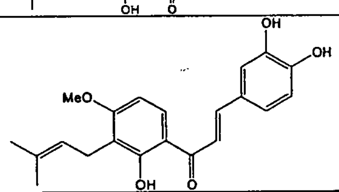 | C031 |
| 4,2'-Diacetoxy-3'-prenyl-4'-methoxychalcone | 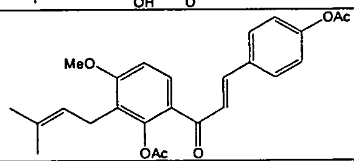 | C032 |
| 4,4'-Dimethoxy-2'-hydroxy-3'-prenylchalcone | 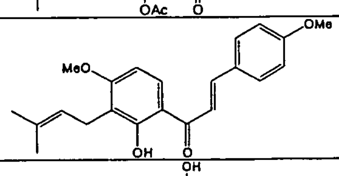 | C035 |
| 3,4-Dihydroxy-2',4'-dichlorochalcone | 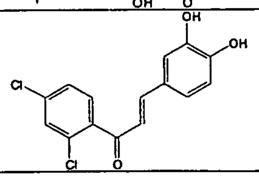 | C011 |
| 4,2'-Dihydroxy-3'-(3-methylbutyl)-4'-methoxychalcone | 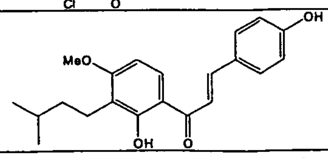 | C043 |
| 4,4',6'-Trimethoxy-2'-hydroxy-3'-prenylchalcone | 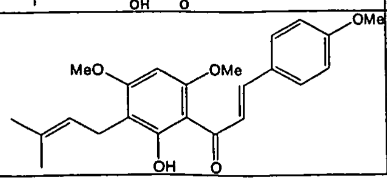 | C046 |

| Table 4 | | |
|---|---|---|
| Name of Compound | Structural Formula | Example |
| 4,2'-Dihydroxy-3'-prenyl-4'-ethoxychalcone | 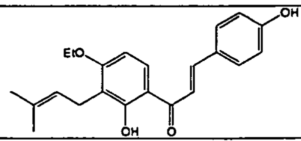 | C049 |
| 4,2'-Dihydroxy-3'-geranyl-4'-ethoxychalcone | 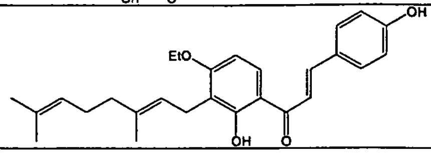 | C050 |
| 4,2'-Dihydroxy-3'-farnesyl-4'-methoxychalcone | 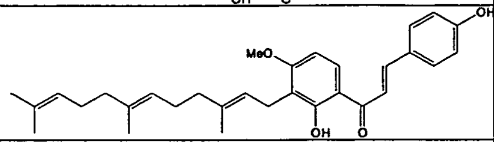 | C053 |
| 4,2'-Dihydroxy-3'-benzyl-4'-methoxychalcone | 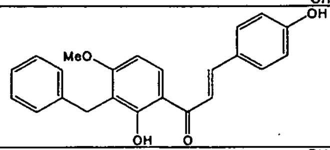 | C056 |
| 4,2',4'-Trihydroxy-3'-benzylchalcone | 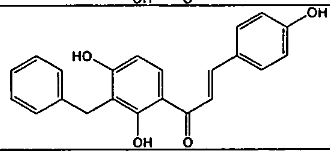 | C057 |
| 4,2',4'-Trihydroxy-3'-farnesylchalcone | 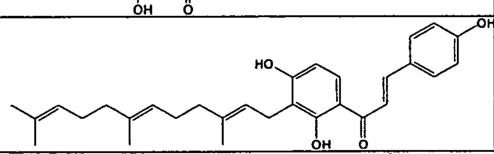 | C058 |
| 4,2',4'-Triacetoxy-3'-geranylchalcone | 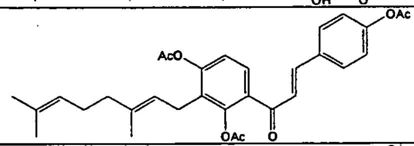 | C061 |
| 4,2'-Diacetoxy-3'-geranyl-4'-methoxychalcone | 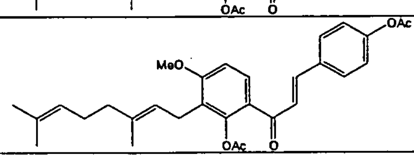 | C064-1 |
| 2'-Acetoxy-3'-geranyl-4-hydroxy-4'-methoxychalcone | 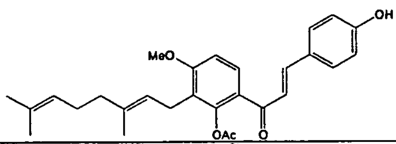 | C064-2 |
| 4-Chloro-2'-benzyloxychalcone | 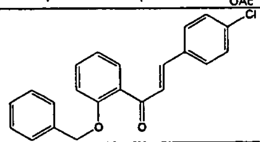 | C066 |
| 2,2'-Dihydroxy-3,3'-diprenyl-4,4'-dimethoxychalcone | 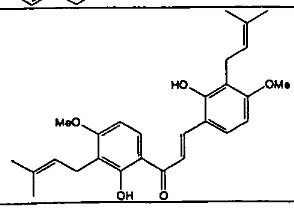 | C072 |

| Table 5 | | |
|---|---|---|
| Name of Compound | Structural Formula | Example |
| 4,2'-Dihydroxy-3'-geranyl-4'-benzyloxychalcone | 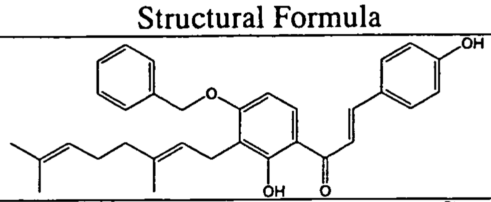 | C073 |
| 4-Chloro-2',4'-dihydroxy-3'-geranylchalcone | 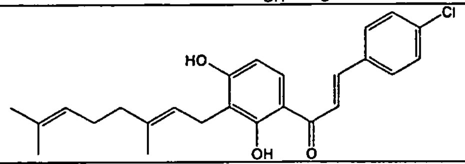 | C074 |
| 4-Chloro-2',4'-dihydroxy-3'-prenylchalcone | 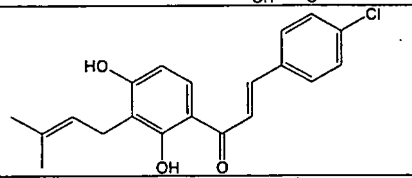 | C075 |
| 3-Nitro-2'-hydroxy-3'-prenyl-4'-methoxychalcone | 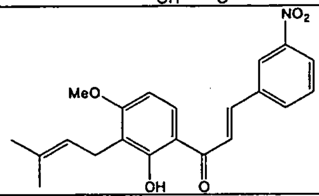 | C076 |
| 4-Hydroxy-2'-benzyloxy-3'-prenyl-4'-methoxychalcone | 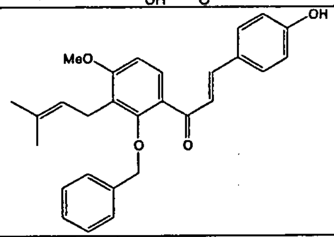 | C077 |
| 4-Acetoxy-2'-hydroxy-3'-prenyl-4'-methoxychalcone | 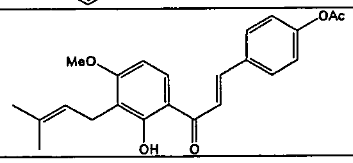 | C078 |
| 4-Chloro-2'-acetoxy-3'-prenyl-4'-methoxychalcone | 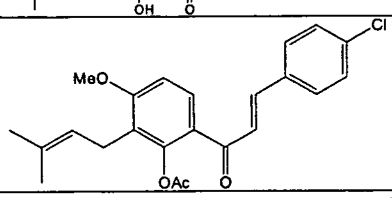 | C079 |
| 4-Chloro-2'-acetoxy-3'-geranyl-4'-methoxychalcone | 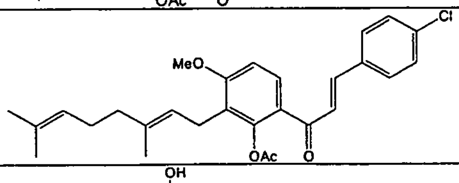 | C080 |
| 3,4,2'-Trihydroxychalcone | 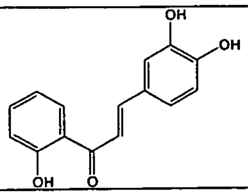 | C005 |

| Table 6 | | |
|---|---|---|
| Name of Compound | Structural Formula | Example |
| 3,4,2',5'-Tetrahydroxychalcone | 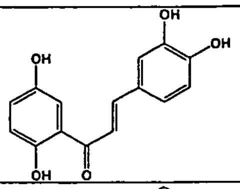 | C006 |
| Chalcone | 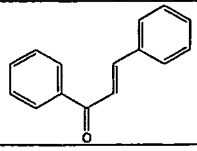 | |
| Xanthoangelol B | 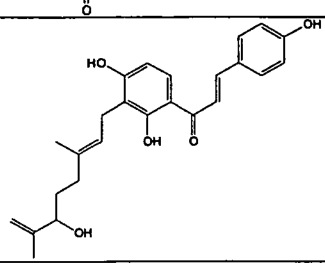 | |
| Xanthoangelol C | 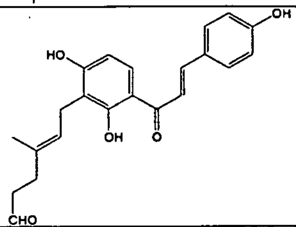 | |
| Xanthoangelol D | 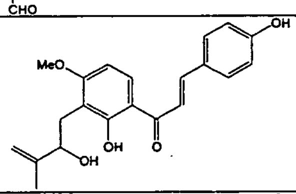 | |
| Xanthoangelol E | 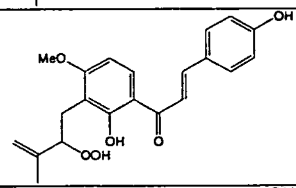 | |
| Bavachromanol | 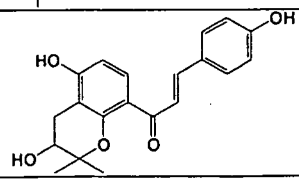 | |

As the chalcone compound used in the present invention, a commercially available compound can be utilized. Alternatively, the chalcone compound can be synthesized or semi-synthesized by a known method, or the chalcone compound can be obtained by extraction and purification from a plant according to a conventional process. For example, the chalcone compound can be obtained by fractionation and purification from a plant belonging to Umbelliferae such as *Angelica keiskei* koidz. by various chromatographies or the like.

Specifically, when, for example, xanthoangelol, 4-hydroxyderricin, xanthoangelol H, TB1, TB2, TB3, TB4, TB5, TB6, TB7, TB8, TB9, xanthoangelol F, lespeol, isobavachalcone, xanthoangelol B, xanthoangelol C, xanthoangelol D, xanthoangelol E, or xanthoangelol G is purified, these compounds can be purified by carrying out extraction from *Angelica keiskei* koidz. with ethyl acetate as a solvent, and appropriate fractionation by silica chromatography and reverse phase

chromatography, in reference to Examples given below. In addition, when xanthohumol is purified from *Humulus lupulus*, xanthohumol can be purified by, for example, carrying out extraction from *Humulus lupulus* with ethyl acetate as a solvent, and appropriate fractionation by silica chromatography.

In addition, when the chalcone compound used in the present invention is synthesized, the chalcone compound may be synthesized by a known process. For example, when chalcone compounds listed in the above-mentioned Tables 1 to 6 are synthesized, these compounds can be synthesized according to the processes described in Examples given below.

In addition, as the chalcone compound used in the present invention, besides the compounds listed in the above-mentioned Tables 1 to 6, 3,2'-dihydroxy-3'-prenyl-4'-methoxychalcone, α-hydroxy-4-hydroxyderricin, β-hydroxy-4-hydroxyderricin, or 4-hydroxy-2',4'-dimethoxy-3'-prenylchalcone may be used. The structural formulas of these compounds are shown in the following Table 7.

| Table 7 | Structural Formula |
|---|---|
| 3,2'-Dihydroxy-3'-prenyl-4'-methoxychalcone | 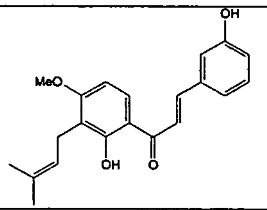 |
| α-Hydroxy-4-hydroxyderricin | 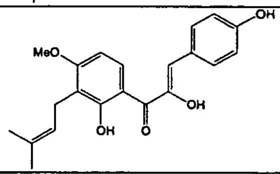 |
| β-Hydroxy-4-hydroxyderricin | 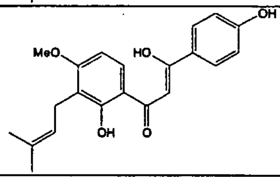 |
| 4-Hydroxy-2',4'-dimethoxy-3'-prenylchalcone | 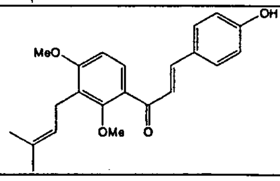 |

3,2'-Dihydroxy-3'-prenyl-4'-methoxychalcone can be prepared by subjecting 3-hydroxybenzaldhyde and 2'-hydroxy-3'-prenyl-4'-methoxyacetophenone to Claisen condensation reaction in the presence of barium hydroxide, to be used in the present invention.

α-Hydroxy-4-hydroxyderricin can be prepared by treating 4-hydroxyderricin in water/dimethyl sulfoxide which is weakly alkaline.

β-Hydroxy-4-hydroxyderricin can be prepared by treating

4-hydroxyderricin in water/dimethyl sulfoxide which is weakly alkaline, to be used in the present invention.

4-Hydroxy-2',4'-dimethoxy-3'-prenylchalcone can be prepared by subjecting 4-hydroxybenzaldehyde and 2',4'-dimethoxy-3'-prenylacetophenone to Claisen condensing reaction in the presence of barium hydroxide, to be used in the present invention.

In addition, as the effective ingredient of the present invention, the derivative of the compound represented by the above formula (Formula 4), that is, the derivative of the chalcone compound can be used. The derivative of the chalcone compound is not particularly limited as long as the derivative can be synthesized from a chalcone compound, or is an analogue of a chalcone compound, wherein the derivative gives the desired effects of the present invention. For example, in the compound represented by the above formula (Formula 4), when $R''_1$ is a hydroxyl group and $R''_7$ is a hydrogen atom, a compound which can be obtained by condensing this hydroxyl group and the hydrogen atom can be also used as a derivative of the chalcone compound in the present invention. Here, the chalcone compound is preferably

exemplified by, for example, the compound represented by the above formula (Formula 6). The derivative of the chalcone compound as mentioned above is not particularly limited, and is exemplified by, for example, 4'-*O*-geranylnaringenin, isobavachin, prostratol F, 8-geranyl-4'-hydroxy-7-methoxyflavanone (C082), and 7-methoxy-8-prenyl-4'-hydroxyflavanone (C034). In addition, other derivatives of the chalcone compound are not particularly limited, and, for example, 1-(2-hydroxy-3-(3-methylbutyl)-4-methoxyphenyl)-3-(4-hydroxyphenyl)-propan-1-one (C033), 1-adamantyl-3-(3,4-dihydroxyphenyl)-2-propen-1-one (C013), 1-adamantyl-3-hydroxy-4-(3,4-dihydroxyphenyl)-butan-1-one (C014) and 1-adamantyl-4-(3,4-dihydroxyphenyl)-3-buten-1-one (C015) can be used. The structural formulas of these compounds are shown in Tables 8 and 9. In Tables 8 and 9, the listing of Examples on the rightmost column shows the names which are used in Examples given below for each of the compounds.

| Table 8 | | |
|---|---|---|
| Name of Compound | Structural Formula | Example |
| 4'-O-Geranylnaringenin | 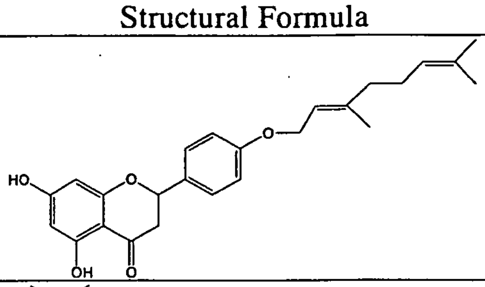 | |
| Isobavachin | 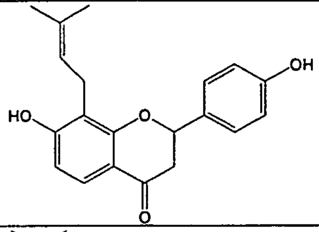 | |
| Prostratol F | 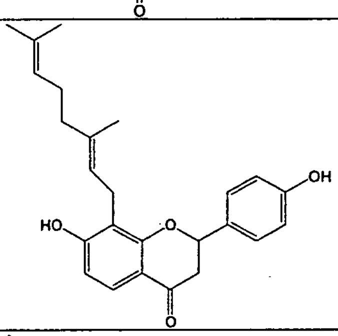 | |
| 8-Geranyl-4'-hydroxy-7-methoxy-flavanone | 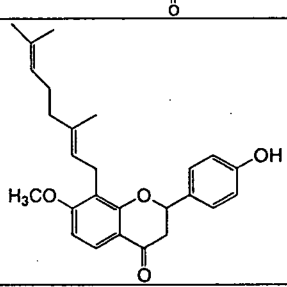 | C082 |
| 1-(2-Hydroxy-3-(3-methylbutyl)-4-methoxyphenyl)-3-(4-hydroxyphenyl)-propan-1-one | 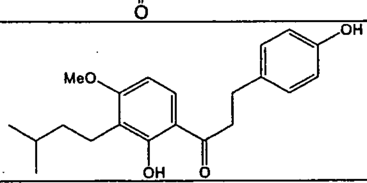 | C033 |
| 7-Methoxy-8-prenyl-4'-hydroxyflavanone | 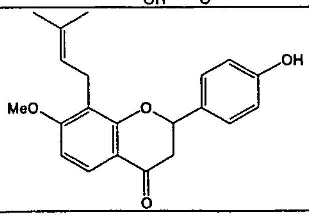 | C034 |

| Table 9 | | |
|---|---|---|
| Name of Compound | Structural Formula | Example |
| 1-Adamantyl-3-(3,4-dihydroxyphenyl)-2-propen-1-one | 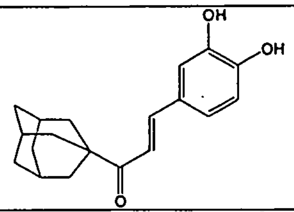 | C013 |
| 1-Adamantyl-3-hydroxy-4-(3,4-dihydroxyphenyl)-butan-1-one | 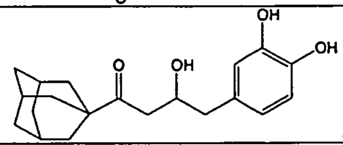 | C014 |
| 1-Adamantyl-4-(3,4-dihydroxyphenyl)-3-buten-1-one | 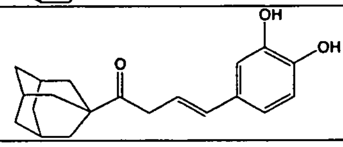 | C015 |

As the derivative of the chalcone compound used in the present invention, a commercially available compound can be utilized. Alternatively, the derivative can be synthesized or semi-synthesized by a known process, or the derivative can be obtained by extraction and purification from a plant according to a conventional process. For example, the derivative can be obtained by fractionation and purification from a plant belonging to Umbelliferae such as *Angelica keiskei* koidz. by various chromatographies or the like.

Specifically, when, for example, 4'-O-geranylnaringenin, isobavachin, prostratol F, or 7-methoxy-8-prenyl-4'-hydroxyflavanone (also named munduleaflavanone A) is purified, these compounds can be purified by carrying out extraction from *Angelica keiskei* koidz. with ethyl acetate as a solvent, and appropriate fractionation by silica chromatography and reverse phase chromatography in reference to Examples given below.

In addition, when the derivative of the chalcone compound used in the present invention is synthesized, the derivative may be synthesized by a known process. For example, when the derivatives of the chalcone compounds listed in the above-mentioned Tables 8 and 9 are synthesized, the derivatives can be synthesized according to the processes described in Examples given below.

In addition, as the derivative of the chalcone compound besides the compounds listed in the above-mentioned Tables 8 and 9, a compound represented by the following formula (Formula 45) can be used in the present invention.

(Formula 45)

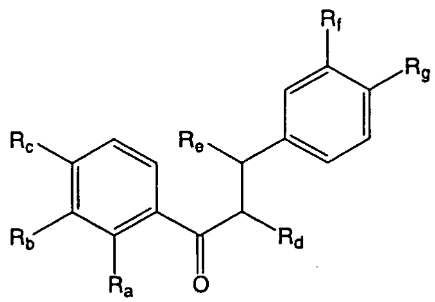

,

wherein each of $R_a$, $R_b$ and $R_c$ is a hydrogen atom, a hydroxyl group, a methyl group, a methoxy group, an acetoxy group, a prenyl group, a geranyl group or a methylbutyl group, at least two of which are functional groups other than a hydrogen atom, wherein $R_a$, $R_b$ and $R_c$ may be different from, or identical to each other with proviso that each is other than a hydrogen atom; $R_d$ and $R_e$ each is a hydrogen atom or a hydroxyl group, at least one of which is a hydrogen atom, or alternatively, $R_d$ and $R_e$ may be a hydrogen atom at the same time; and $R_f$ and Rg each is any one member selected from a hydrogen atom, a hydroxyl group or an acetoxy group, at least one of which is a functional group other

than a hydrogen atom, wherein $R_f$ and Rg may be different from, or identical to each other, with proviso that each is other than a hydrogen atom.

In addition, the compound represented by the above general formula (Formula 45) is exemplified by, for example, 2',4-dihydroxy-4'-methoxydihydrochalcone, 2',4-dihydroxy-4'-methoxy-3'-methyldihydrochalcone, 2',4-dihydroxy-4'-methoxy-3'-prenyldihydrochalcone, 2',3-dihydroxy-4'-methoxy-3'-prenyldihydrochalcone, 2',3,4-trihydroxy-4'-methoxy-3'-prenyldihydrochalcone, 2',4,4'-trihydroxy-3'-prenyldihydrochalcone, 3'-geranyl-2',4-dihydroxy-4'-methoxydihydrochalcone, 2',4-diacetoxy-4'-methoxy-3'-prenyldihydrochalcone, α,2' ,4-trihydroxy-4'-methoxy-3'-prenyldihydrochalcone, β,2' ,4-trihydroxy-4'-methoxy-3'-prenyldihydrochalcone, and 4-hydroxy-2',4'-dimethoxy-3'-prenyldihydrochalcone. The structural formulas of these compounds are shown in Table 10.

| Table 10 | Structural Formula |
|---|---|
| 2',4-Dihydroxy-4'-methoxydihydrochalcone | 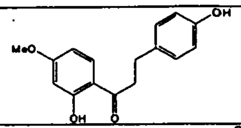 |
| 2',4-Dihydroxy-4'-methoxy-3'-methyldihydrochalcone | 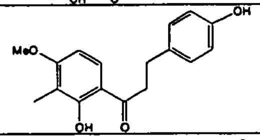 |
| 2',4-Dihydroxy-4'-methoxy-3'-prenyldihydrochalcone | 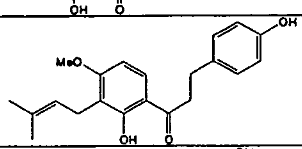 |
| 2',3-Dihydroxy-4'-methoxy-3'-prenyldihydrochalcone | 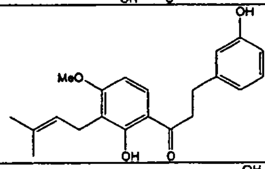 |
| 2',3,4-Trihydroxy-4'-methoxy-3'-prenyldihydrochalcone | 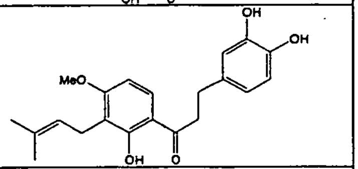 |
| 2',4,4'-Trihydroxy-3'-prenyldihydrochalcone | 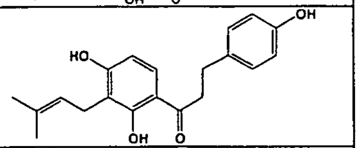 |
| 3'-Geranyl-2',4-dihydroxy-4'-methoxydihydrochalcone | 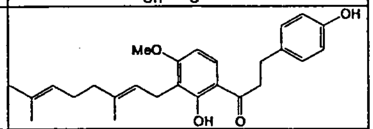 |
| 2'4-Diacetoxy-4'-methoxy-3'-prenyldihydrochalcone | 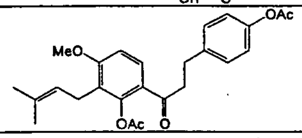 |
| α,2',4-Trihydroxy-4'-methoxy-3'-prenyldihydrochalcone | 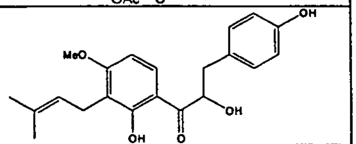 |
| β,2',4-Trihydroxy-4'-methoxy-3'-prenyldihydrochalcone | 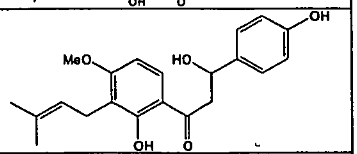 |
| 4-Hydroxy-2',4'-dimethoxy-3'-prenyldihydrochalcone | 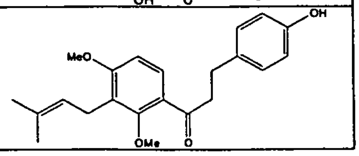 |

2',4-Dihydroxy-4'-methoxydihydrochalcone can be prepared by hydrogenating 4,2'-dihydroxy-4'-methoxychalcone mentioned above using palladium as a catalyst.

2',4-Dihydroxy-4'-methoxy-3'-methyldihydrochalcone can be prepared by hydrogenating 4,2'-dihydroxy-3'-methyl-4'-methoxychalcone using palladium as a catalyst.

2',4-Dihydroxy-4'-methoxy-3'-prenyldihydrochalcone can be prepared by hydrogenating 4-hydroxyderricin

mentioned above using palladium as a catalyst.

2',3-Dihydroxy-4'-methoxy-3'-prenyldihydrochalcone can be prepared by hydrogenating 2',3-dihydroxy-4'-methoxy-3'-prenylchalcone using palladium as a catalyst.

2',3,4-Trihydroxy-4'-methoxy-3'-prenyldihydrochalcone can be prepared by hydrogenating 3,4,2'-trihydroxy-3'-prenyl-4'-methoxychalcone mentioned above using palladium as a catalyst.

2',4,4'-Trihydroxy-3'-prenyldihydrochalcone can be prepared by hydrogenating isobavachalcone mentioned above using palladium as a catalyst.

3'-Geranyl-2',4-dihydroxy-4'-methoxydihydrochalcone can be prepared by hydrogenating xanthoangelol F mentioned above using palladium as a catalyst.

2',4-Diacetoxy-4'-methoxy-3'-prenyldihydrochalcone can be prepared by hydrogenating 4,2'-diacetoxy-3'-prenyl-4'-methoxychalcone mentioned above using palladium as a catalyst.

$\alpha$,2',4-Trihydroxy-4'-methoxy-3'-prenyldihydrochalcone can be prepared by hydrogenating $\alpha$-hydroxy-4-hydroxyderricin mentioned above using palladium as a catalyst.

$\beta$,2',4-Trihydroxy-4'-methoxy-3'-prenyldihydrochalcone can be prepared by hydrogenating the $\beta$-hydroxy-4-hydroxyderricin mentioned above using palladium as a catalyst.

4-Hydroxy-2',4'-dimethoxy-3'-prenyldihydrochalcone can be prepared by hydrogenating 4-hydroxy-2',4'-dimethoxy-3'-prenylchalcone using palladium as a catalyst.

Further, as the derivative of the chalcone compound besides the derivatives mentioned above, for example, a derivative (prodrug) which can be easily hydrolyzed in a body to exhibit the desired effects, such as an ester can be prepared. The prodrug may be prepared in accordance with a known process. In addition, the derivative of the compound used in the present invention also encompasses a derivative obtained by adding a protecting group such as a tetrahydropyranyl group to a hydroxyl group. In addition, the derivative of the present invention also encompasses a derivative obtained by administering the compound of the present invention to a mammal, to give a product via metabolism. Here, the derivative may be a salt thereof.

As the salt of the chalcone compound or a derivative thereof of the present invention, a pharmacologically acceptable salt may be used. Alternatively, the salt may be a derivative of the compound which can function as a prodrug as described above. Therefore, the chalcone compound relating to the present invention encompasses a derivative of the chalcone compound and a salt thereof, as long as the desired effects of the present invention can be obtained. In addition, various isomers such as an optical isomer, a keto-enol tautomer, and a geometrical isomer of the chalcone compound, and isolated products of each of isomers can be all used in the present invention as long as these isomers have insulin-mimetic actions.

The pharmacologically acceptable salt of the compounds as described herein is exemplified by, for example, alkali metal salts, alkaline earth metal salts, salts with organic bases, and the like. The pharmacologically acceptable salt used in the present invention means a salt of a compound which is substantially atoxic to an organism and has an insulin-mimetic action. The salt includes, for example, salts with sodium, potassium, calcium, magnesium, ammonium, protonated benzathine (N,N'-di-benzylethylenediamine), choline, ethanolamine, diethanolamine, ethylenediamine, meglamine (N-methylglucamine), benethamine (N-benzylphenethylamine), piperazine, tolomethamine (2-amino-2-hydroxymethyl-1,3-propanediol) or the like.

As the chalcone compound used as the effective ingredient in the present invention, a fraction containing in a high concentration the chalcone compound obtained by fractionating from a plant belonging to Umbelliferae such as *Angelica keiskei* koidz. by a known process can also be used. The fractionating means includes extraction, separation by precipitation, column chromatography, thin layer chromatography and the like. The chalcone compound may be isolated by further progressing the purification of the resulting fraction using, as an index, the activity of inducing differentiation into an adipocyte or the enhancing action on glucose uptake into a cell, as illustrated in Examples 82 and 83 given below.

The compound represented by the above general formula (Formula 5), which is one embodiment of the above general formula (Formula 1) of the present invention, is an acetophenone compound. In the present invention, the acetophenone compound is exemplified by the compound represented by the above general formula (Formula 8). In the above general formula (Formula 8), especially preferably, $R''''_1$ is exemplified by a hydroxyl group, a methyl group or a benzyloxy group; $R''''_2$ is exemplified by a hydrogen atom, a prenyl group, a methyl group, a geranyl group, a methylbutyl group, a farnesyl group, or a benzyl group; $R''''_3$ is exemplified by a hydrogen atom, a hydroxyl group, a methoxy group, a tetrahydropyranyloxy group, an ethoxy group, a benzyloxy group or a prenyloxy group; and $R''''_4$ is exemplified by a hydrogen atom or a prenyl group.

In addition, especially preferably, the acetophenone compound used in the present invention is exemplified by 2',4'-dihydroxy-5'-prenylacetophenone (C025), 2'-hydroxy-4'-methoxy-5'-prenylacetophenone (C027), 2'-hydroxy-3'-prenyl-4'-methoxyacetophenone (C026), 2'-hydroxy-3'-methyl-4'-methoxyacetophenone (C022), 2',4'-dihydroxy-3'-geranylacetophenone (C036), 2'-hydroxy-3'-geranyl-4'-methoxyacetophenone (C038), 2'-hydroxy-3'-(3-methylbutyl)-4'-methoxyacetophenone (C041), 2'-hydroxy-3'-prenyl-4'-tetrahydropyranyloxyacetophenone (C044), 2'-hydroxy-3'-geranyl-4'-tetrahydropyranyloxyacetophenone (C045), 2'-hydroxy-3'-prenyl-4'-ethoxyacetophenone (C047), 2'-hydroxy-3'-geranyl-4'-ethoxyacetophenone (C048), 2',4'-dihydroxy-3'-farnesylacetophenone (C051), 2'-hydroxy-3'-farnesyl-4'-methoxyacetophenone (C052), 2',4'-dihydroxy-3'-benzylacetophenone (C054), 2'-hydroxy-3'-benzyl-4'-methoxyacetophenone (C055), 2'-methyl-4'-prenyloxyacetophenone (C059), 2'-benzyloxyacetophenone (C065) or 2'-hydroxy-3'-geranyl-4'-benzyloxyacetophenone (C070). The structural formulas of these compounds are shown in Tables 11 and 12. In Tables 11 and 12, the listing of Examples on the rightmost column shows the names used in Examples given below for each of the compounds.

| Table 11 | | |
|---|---|---|
| Name of Compound | Structural Formula | Example |
| 2'4'-Dihydroxy-5'-prenylacetophenone | 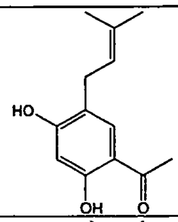 | C025 |
| 2'-Hydroxy-4'-methoxy-5'-prenylacetophenone | 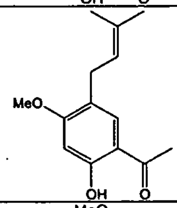 | C027 |
| 2'-Hydroxy-3'-prenyl-4'-methoxyacetophenone | 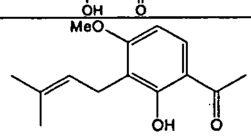 | C026 |
| 2'-Hydroxy-3'-methyl-4'-methoxyacetophenone | 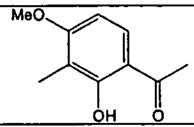 | C022 |
| 2',4'-Dihydroxy-3'-geranylacetophenone | 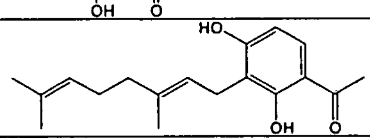 | C036 |
| 2'-Hydroxy-3'-geranyl-4'-methoxyacetophenone | 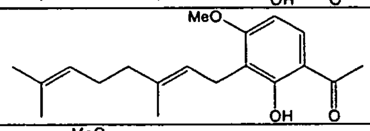 | C038 |
| 2'-Hydroxy-3'-(3-methylbutyl)-4'-methoxyacetophenone | 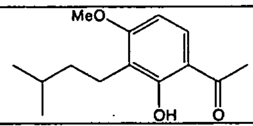 | C041 |
| 2'-Hydroxy-3'-prenyl-4'-tetrahydropyranyloxyacetophenone | 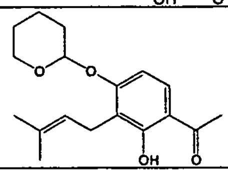 | C044 |
| 2'-Hydroxy-3'-geranyl-4'-tetrahydropyranyloxyacetophenone | 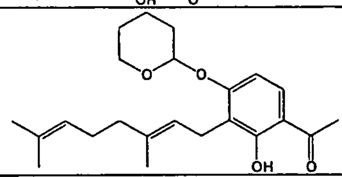 | C045 |
| 2'-Hydroxy-3'-prenyl-4'-ethoxyacetophenone | 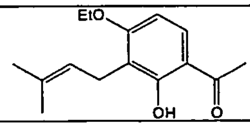 | C047 |

| Table 12 | | |
|---|---|---|
| Name of Compound | Formula | Example |
| 2'-Hydroxy-3'-geranyl-4'-ethoxyacetophenone | 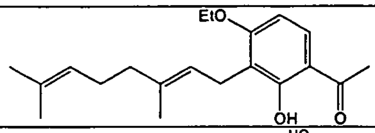 | C048 |
| 2',4'-Dihydroxy-3'-farnesylacetophenone | 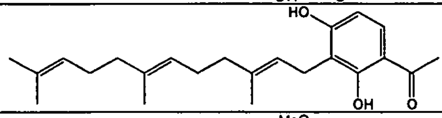 | C051 |
| 2'-Hydroxy-3'-farnesyl-4'-methoxyacetophenone | 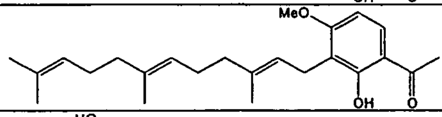 | C052 |
| 2',4'-Dihydroxy-3'-benzylacetophenone | 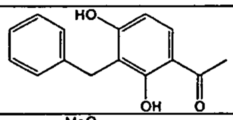 | C054 |
| 2'-Hydroxy-3'-benzyl-4'-methoxyacetophenone | 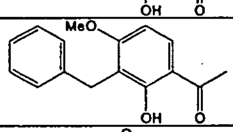 | C055 |
| 2'-Methyl-4'-prenyloxyacetophenone | 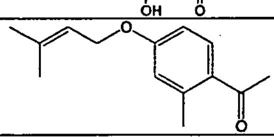 | C059 |
| 2'-Benzyloxyacetophenone | 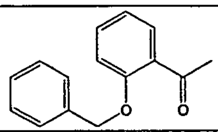 | C065 |
| 2'-Hydroxy-3'-geranyl-4'-benzyloxyacetophenone | 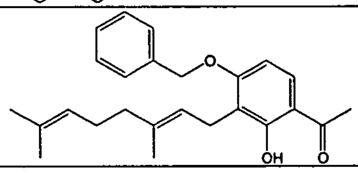 | C070 |

As the acetophenone compound used in the present invention, a commercially available compound can be utilized. Alternatively, the acetophenone compound can be synthesized by a known process. For example, when the acetophenone compound listed in the above-mentioned Table 11 or 12 is synthesized, the acetophenone compound can be synthesized according to the processes described in Examples given below.

In addition, as the effective ingredient of the present invention, the derivative of the compound represented by the above formula (Formula 5), that is, the derivative of the acetophenone compound can also be used. The derivative of the acetophenone compound is not particularly limited, as long as the derivative can be synthesized from the acetophenone compound, or is an analogue of the acetophenone compound, wherein the derivative gives the desired effects of the present invention.

As the derivative of the acetophenone compound used in the present invention, a commercially available compound can be utilized. Alternatively, the derivative can be obtained by synthesizing by a known process.

As the derivative of the acetophenone compound, for example, a derivative (prodrug) which can be easily hydrolyzed in a body to exhibit the desired effects, such as an ester can be prepared. The prodrug may be prepared in accordance with a known process. In addition, the derivative of the compound used in the present invention encompasses a derivative obtained by adding a protecting group such as a tetrahydropyranyl group to a hydroxyl group. In addition, the derivative of the present invention encompasses a derivative obtained by administering the compound of the present invention to a mammal, to give a product via metabolism. Here, the derivative may be a salt thereof.

In addition, as the salt of the acetophenone compound or a derivative thereof used in the present invention, the same salts as the salts of the chalcone compound mentioned above can be used. Alternatively, the salt may be a derivative of the compound which can function as a prodrug, as described above. Therefore, the acetophenone compound relating to the present invention encompasses a derivative of the acetophenone compound and a salt thereof as long

as the desired effects of the present invention can be obtained. In addition, various isomers such as an optical isomer, a keto-enol tautomer, and a geometrical isomer of the acetophenone compound, and isolated products of each of isomers can be all used in the present invention as long as these isomers have insulin-mimetic actions.

In the present invention, a compound represented by the above general formula (Formula 1) which is different from the chalcone compound and the acetophenone compound mentioned above is also exemplified. Specifically, a compound in the above formula (Formula 1) in which $X^0$ is a hydrogen atom, a 2-(2-furyl)vinyl group, a 2-(2-thienyl)vinyl group, a 4-phenyl-1,3-butadienyl group, or a 4-methyl-1,3-pentadienyl group; $R_1$ is a hydroxyl group; $R_2$ is a hydrogen atom or a prenyl group; $R_3$ is a methoxy group, and each of $R_4$ and $R_5$ is a hydrogen atom, and a derivative thereof are used in the present invention.

The compound is especially preferably exemplified by 1-(2-hydroxy-4-methoxy-3-prenylphenyl)-3-(2-thienyl)-2-propen-1-one (C-THP), 1-(2-hydroxy-4-methoxy-3-prenylphenyl)-5-phenyl-2,4-pentadien-1-one (C-CIN), 3-(2-furyl)-1-(2-hydroxy-3-prenyl-4-methoxyphenyl)-2-propen-1-one (FUR-1), 2-hydroxy-3-prenyl-4-methoxybenzaldehyde (C063), 1-(2-hydroxy-3-prenyl-4-methoxyphenyl)-5-methyl-2,4-hexadien-1-one (C069) and 2-hydroxy-4-methoxybenzaldehyde. In addition, the derivative of the compound, for example, a derivative of the FUR-1 mentioned above, is exemplified by 2-(2-furyl)-3-(2-furylmethylene)-2,3-dihydro-7-methoxy-8-prenyl-4H-1-benzopyran-4-one (FUR-2). The structural formulas of these compounds and a derivative thereof are shown in Table 13. In Table 13, the listing of Examples on the rightmost column shows names used in Examples given below of each of the compounds.

Table 13

| Name of Compound | Structural Formula | Example |
| --- | --- | --- |
| 1-(2-Hydroxy-4-methoxy-3-prenylphenyl)-3-(2-thienyl)-2-propen-1-one | 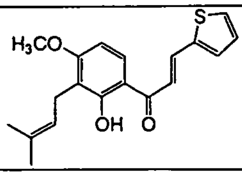 | C-THP |
| 1-(2-Hydroxy-4-methoxy-3-prenylphenyl)-5-phenyl-2,4-pentadien-1-one | 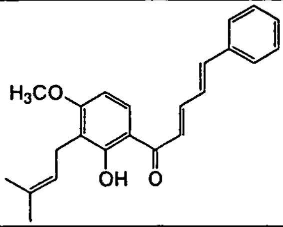 | C-CIN |
| 3-(2-Furyl)-1-(2-hydroxy-3-prenyl-4-methoxyphenyl)-2-propen-1-one | 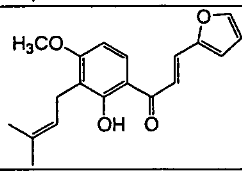 | FUR-1 |
| 2-(2-Furyl)-3-(2-furylmethylene)-2,3-dihydro-7-methoxy-8-prenyl-4H-1-benzopyran-4-one | 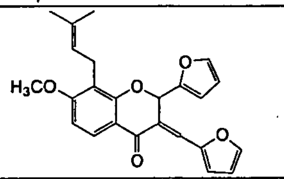 | FUR-2 |
| 2-Hydroxy-3-prenyl-4-methoxybenzaldehyde | 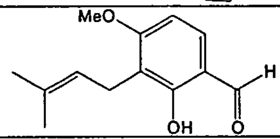 | C063 |
| 1-(2-Hydroxy-3-prenyl-4-methoxyphenyl)-5-methyl-2,4-hexadien-1-one | 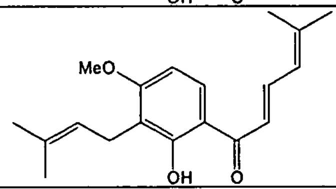 | C069 |
| 2-Hydroxy-4-methoxybenzaldehyde | 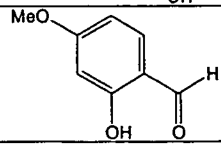 | |

As these compounds and derivatives thereof, a commercially available compound can be utilized. Alternatively, these compounds can be synthesized by a known process. When the compound listed in the above-mentioned Table 13 is synthesized, the compound can be synthesized by referring to the processes described in Examples given below.

In addition, as the derivative of the compound, besides the above-mentioned derivatives, a derivative (prodrug) which can be easily hydrolyzed in a body to exhibit the desired effects, such as an ester can be prepared. The prodrug may be prepared in accordance with a known process. In addition, the derivative of the compound used in the present invention encompasses a compound obtained by adding a protecting group such as a tetrahydropyranyl group to a hydroxyl group. In addition, the derivative of the present invention encompasses a derivative obtained by administering the compound of the present invention to a mammal, to give a product via metabolism. Here, the derivative may be a salt thereof.

In addition, as the salt of the compound or a derivative thereof, the same salts as the salts of the chalcone compound mentioned above can be used. Alternatively, the salt may be a derivative of the compound which can function as a prodrug as described above. Therefore, the compound relating to the present invention encompasses a derivative of the compound and a salt thereof as long as the desired effects of the present invention can be obtained. In addition,

various isomers such as an optical isomer, a keto-enol tautomer, and a geometrical isomer of the compound, and isolated products of each of isomers can be all used in the present invention, as long as these isomers have insulin-mimetic actions.

In the present invention, the compound represented by the above general formula (Formula 2) is a coumarin compound. In the present invention, the coumarin compound is preferably exemplified by the compound represented by the above general formula (Formula 9). In the above general formula (Formula 9), especially preferably, $R''''''''_1$ is exemplified by a hydrogen atom, a hydroxyl group or an angeloyloxy group; and $R''''''''_2$ is exemplified by an acetoxy group or an angeloyloxy group.

In addition, the coumarin compound used in the present invention is especially preferably exemplified by 3'-acetoxy-4'-angeloyloxy-3',4'-dihydroseselin (coumarin compound A), 3'-angeloyloxy-3',4'-dihydroseselin (coumarin compound B), 3'-angeloyloxy-4'-hydroxy-3',4'-dihydroseselin (coumarin compound C) or xanthotoxin. The structural formulas of these compounds are shown in Table 14. In Table 14, the listing of Examples on the rightmost column shows names used in Examples given below for each of the compounds.

| Table 14 | | |
|---|---|---|
| Name of Compound | Structural Formula | Example |
| 3'-Acetoxy-4'-angeloyloxy-3',4'-dihydroseselin | 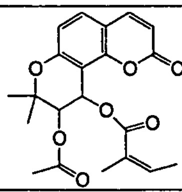 | Coumarin Compound A |
| 3'-Angeloyloxy-3',4'-dihydroseselin | 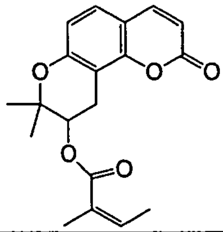 | Coumarin Compound B |
| 3'-Angeloyloxy-4'-hydroxy-3',4'-dihydroseselin | 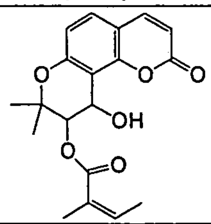 | Coumarin Compound C |
| Xanthotoxin | 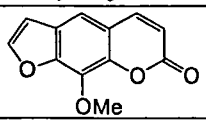 | |

As the coumarin compound used as the effective ingredient in the present invention, a commercially available compound can be utilized. Also, the coumarin compound can be synthesized by a known process, or alternatively, the compound can be also obtained by extraction and purification from a plant according to a conventional process. For example, 3'-acetoxy-4'-angeloyloxy-3',4'-dihydroseselin (coumarin compound A), 3'-angeloyloxy-3',4'-dihydroseselin (coumarin compound B), 3'-angeloyloxy-4'-hydroxy-3',4'-dihydroseselin (coumarin compound C) and xanthotoxin are all coumarin compounds contained in *Angelica keiskei* koidz., which is a plant belonging to Umbelliferae. When these compounds are prepared, these compounds can be extracted from *Angelica keiskei* koidz. with ethyl acetate as a solvent, and the extract is properly fractionated by reverse phase chromatography to purify these compounds, to be used in the present invention as described in Examples given below.

As the derivative of the coumarin compound, a derivative (prodrug) which can be easily hydrolyzed in a body to exhibit the desired effects, such as an ester, can be prepared. The prodrug may be prepared in accordance with a known process. In addition, the derivative of the compound used in the present invention encompasses a compound obtained by adding a protecting group such as a tetrahydropyranyl group to a hydroxyl group. In addition, the derivative of the present invention encompasses a derivative obtained by administering the compound of the present invention to

a mammal, to give a product via metabolism. Here, the derivative may be a salt thereof.

In addition, as the salt of the coumarin compound used in the present invention or a derivative thereof, the same salts as the salts of the chalcone compound mentioned above can be used. Alternatively, the salt may be a derivative of the compound which can function as a prodrug as mentioned above. Therefore, the coumarin compound relating to the present invention encompasses a derivative of the coumarin compound and a salt thereof as long as the desired effects of the present invention can be obtained. In addition, various isomers such as an optical isomer, a keto-enol tautomer, and a geometrical isomer of the coumarin compound, and isolated products of each of isomers can be all used in the present invention as long as the isomers have insulin-mimetic actions.

In the present invention, the compound represented by the above general formula (Formula 3) is a phthalide compound. As the compound represented by the above general formula (Formula 3), sedanolide or n-butylidenephthalide is especially preferably exemplified. The structural formulas of these compounds are shown in Table 15.

Table 15

| Name of Compound | Structural Formula |
|---|---|
| Sedanolide | 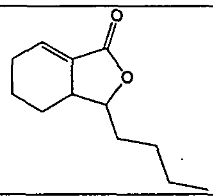 |
| n-Butylidenephthalide | 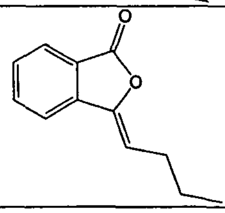 |

As the compound represented by the above general formula (Formula 3) used as the effective ingredient in the present invention, a commercially available compound can be used. Also, the compound can be synthesized by a known process, to be used in the present invention, or alternatively, the compound can be obtained by extracting and purifying from a plant in accordance with a conventional process. For example, sedanolide is a component contained in *Apium* of a plant belonging to Umbelliferae, and n-butylidenephthalide is a component contained in *Angelica acutiloba* or *Cnidium officinale,* which is a plant belonging to Umbelliferae. These compounds can be all purified from a plant by carrying out various solvent extractions, or various chromatographies by a known method, to be used in the present invention.

As the derivative of the compound represented by the above general formula (Formula 3), a derivative (prodrug) which can be easily hydrolyzed in a body to exhibit the desired effects, such as an ester can be prepared. The prodrug may be prepared in accordance with a known process. Also, the derivative of the compound used in the present invention encompasses a compound obtained by adding a protecting group such as a tetrahydropyranyl group to a hydroxyl group. In addition, the derivative of the present invention encompasses a derivative obtained by administering the compound of the present invention to a mammal, to give a product via metabolism. Here, the derivative may be a salt thereof.

In addition, as the salt of the compound represented by the above general formula (Formula 3) or a derivative thereof used in the present invention, the same salts as the salts of the chalcone compound mentioned above can be used. Also, the salt may be a derivative of the compound which can function as a prodrug as described above. Therefore, the compound represented by the above general formula (Formula 3) relating to the present invention encompasses a derivative of the compound and a salt thereof, as long as the desired effects of the present invention can be obtained. In addition, various isomers such as an optical isomer, a keto-enol tautomer, and a geometrical isomer of the compound represented by the above general formula (Formula 3), and isolated products of each of the isomers can be all used in the present invention, as long as these isomers have insulin-mimetic actions.

The present invention also provides novel compounds which are referred to as C082, C023, C030, C031, C041, C043, C044, C045, C047, C048, C049, C050, C052, C053, C056, C057, C058, C059, C061, C064-1, C064-2, C069, C070, C072, C073, C074, C075, C076, C077, C078, C079, C080, C-THP, C-CIN, FUR-1, and FUR-2, respectively, in Examples given below, which are used as the effective ingredient of the present invention, in addition to a medicament, a food, a beverage, a feed or the like, each containing the effective ingredient as mentioned above.

In the present specification, the above general formula (Formula 1), the above general formula (Formula 2), the above general formula (Formula 3), a derivative thereof and a pharmacologically acceptable salt thereof are referred to as the effective ingredient of the present invention, and a therapeutic agent or prophylactic agent for a disease accompanying an abnormality in an amount of an insulin or insulin response, which comprises the effective ingredient of the present invention may be referred to as the therapeutic agent or prophylactic agent of the present invention in some cases. In addition, the therapeutic agent, the prophylactic agent, and the agent for an insulin-mimetic action are collectively referred to as a medicament of the present invention in some cases.

No toxicity is especially found in the effective ingredient according to the present invention as mentioned later. Also, there is no risk of the onset of side effects. For these reasons, the disease can be safely and appropriately treated or prevented. Therefore, the therapeutic agent, the prophylactic agent, the food, the beverage or the feed, each comprising the effective ingredient, is effective in treating or preventing a disease accompanying an abnormality in an amount of insulin or insulin response.

In the present invention, the disease accompanying an abnormality of an amount of insulin or insulin response includes diseases characterized by a factor selected from change in insulin level in blood, change in activity level of insulin or an insulin receptor, aberrance in downstream signal of an insulin receptor and combinations thereof. The disease is exemplified by, for example, diabetes, obesity, hypertension, arteriosclerosis, cocaine withdrawal symptoms, static cardiac incompetence, amnesia, cardiovascular spasm, cerebral angiospasm, chromaffinoma, ganglioneuroblastoma, Huntington's disease, and hyperlipemia. The diabetes may be exemplified by any of type I diabetes and type II diabetes. In addition, the type II diabetes encompasses a disease of which causation is insulin resistance for which a therapeutic effect is not found even when insulin or an agent for enhancing insulin secretion is administered.

In the state of insulin resistance, a signal from an insulin receptor by insulin is inhibited, so that diversified functions possessed by insulin are not exhibited, whereby generating various dysbolisms. The effective ingredient used in the present invention can exhibit an insulin-mimetic effect also for a symptom of insulin resistance, as described in Examples 88 to 92, 94 and 95. Specifically, by using the prophylactic agent or therapeutic agent of the present invention, a therapeutic or prophylactic effect can be exhibited also for a disease caused by insulin resistance, for example, type II diabetes for which a therapeutic effect is not seen even when insulin or an agent for enhancing insulin secretion is administered. In addition, the effective ingredient of the present invention can also exhibit the effect of lowering the amount of insulin in blood. In other words, the medicament of the present invention can be also used as a therapeutic agent or prophylactic agent for a disease requiring lowering of the amount of insulin for treatment or prevention. The disease is not particularly limited, and is exemplified by hyperinsulinemia, Alzheimer's disease and the like. In addition, since reports have been made that the stimulation via an insulin receptor and the effect of extended longevity are closely related *(Science,* vol. 299, P572-574 (2003); *Nature,* vol. 424, P277-284 (2003)), the medicament of the present invention can also be used as an agent for anti-aging.

There have been known that insulin enhances induction of differentiation of preadipocytes into adipocytes, and that glucose uptake takes place in matured adipocytes thereby accumulating triglyceride in the adipocytes *(J. Biol. Chem.,* Vol. 253, No. 20, P7570-7578 (1978)). Specifically, utilizing this method, an insulin-mimetic action of a test substance can be determined by administering the test substance in place of insulin, and determining an adipocyte differentiation and the amount of triglyceride in the adipocytes.

In addition, there have been known that insulin has an enhancing action on glucose uptake, and that glucose uptake into a cell is enhanced by the action of insulin in a matured adipocyte *(J. Biol. Chem.,* Vol. 253, No. 20, P7579-7583 (1978)). Specifically, utilizing this method, an insulin-mimetic action of a test substance can be determined by administering the test substance in place of insulin, and determining the amount of glucose uptake into a matured adipocyte.

In addition, as an especially preferable form of the therapeutic agent or prophylactic agent of the present invention, a compound having a strong enhancing action on glucose uptake into a cell, and a compound having a strong action for inducing adipocyte differentiation can be used together. Specifically, a synergistic effect can be exhibited by using together 4-hydroxyderricin and xanthoangelol as described in Examples 86, 90 and 91.

The therapeutic agent or prophylactic agent of the present invention includes ones formed into a preparation by combining the above-mentioned effective ingredient according to the present invention with a known pharmaceutical carrier. In the embodiment of the present invention, as the effective ingredient in the form of a salt, a pharmacologically acceptable salt is used. Also, as the therapeutic agent or prophylactic agent of the present invention, the above-mentioned effective ingredient can be combined with other components which can be used for the same applications as those of the effective ingredients, for example, insulin preparation, an agent for enhancing insulin secretion, an agent for improving insulin resistance, an agent for ameliorating postprandial hyperglycemia, an agent for insulin-mimetic action or the like which is known in the art.

The therapeutic agent or prophylactic agent of the present invention is usually manufactured by combining the above-mentioned effective ingredient with a pharmacologically acceptable liquid or solid carrier. A solvent, a dispersant, an emulsifier, a buffer, a stabilizer, an excipient, a binder, a disintegrant, a lubricant, or the like can be optionally added thereto, to form a solid agent such as a tablet, a granule, a powder, an epipastic, and a capsule, or a liquid agent such

as a common liquid agent, a suspension agent or an emulsion agent. In addition, there can be also formed into a dry product which can be liquefied by adding an appropriate carrier before use, or also into an external preparation.

The pharmaceutical carrier can be selected depending upon the administration form and preparation form of the therapeutic agent or prophylactic agent. In the case of an orally administered preparation comprising a solid composition, the preparation can be produced in the form of a tablet, a pill, a capsule, a powder, a fine powder, a granule or the like, and there can be utilized, for example, starch, lactose, saccharose, mannitol, carboxymethyl cellulose, cornstarch, an inorganic salt or the like. In addition, during the preparation of the orally administered preparation, a binder, a disintegrant, a surfactant, a lubricant, a fluidity accelerator, a corrective, a colorant, a flavor, and the like can be further combined therewith. For example, in the case of forming into a tablet or a pill, the tablet or pill may be covered with a sugar-coating made of sucrose, gelatin or hydroxypropyl cellulose, or with a film made of a substance soluble in the stomach or intestine as desired. In the case of an orally administered preparation comprising a liquid composition, the preparation can be prepared in the form of a pharmaceutically acceptable emulsion, solution, suspension, syrup, or the like. In this case, for example, purified water, ethanol or the like is utilized as a carrier. Furthermore, an auxiliary agent such as a wetting agent or a suspending agent, a sweetener, a flavor, an antiseptic, or the like may be added as desired.

On the other hand, in the case of a parenterally administered preparation, the preparation can be prepared by dissolving or suspending the above-mentioned effective ingredient of the present invention in a diluent such as distilled water for injection, physiological saline, an aqueous solution of glucose, vegetable oil for injection, sesame oil, peanut oil, soybean oil, corn oil, propylene glycol or polyethylene glycol, in accordance with a conventional method, and adding a microbicide, a stabilizer, an osmotic regulator, a soothing agent, or the like if needed. It is also possible to produce a solid composition and dissolve the composition in sterile water or a sterile solvent for injection before use.

The external preparation includes solid, semi-solid or liquid preparations for percutaneous administration or transmucosal (intraoral or intranasal) administration. The external preparation also includes suppositories and the like. For example, the external preparation may be prepared as liquid preparations including emulsions, suspensions such as lotions, external tinctures, and liquid agents for transmucosal administration; ointments such as oily ointments and hydrophilic ointments; patches for percutaneous administration or transmucosal administration such as films, tapes and poultices; and the like.

Each of the above-mentioned various preparations can be appropriately produced in accordance with conventional methods by utilizing known pharmaceutical carriers and the like. Also, the content of the effective ingredient in the preparation is not particularly limited, as long as the content is in an amount in which the effective ingredient can be preferably administered within the dose range described below in consideration of administration form, administration method and the like of the preparation. A typical content of the effective ingredient in the preparation is from 0.1 to 100% by weight or so.

The therapeutic agent or prophylactic agent of the present invention is administered via an administration route appropriate for each of the preparation form. The administration method is also not limited to specific one. The agent can be administered internally, externally (or topically) or by injection. The injection can be administered, for example, intravenously, intramuscularly, subcutaneously, intracutaneously, or the like. As to an external preparation, for example, a suppository may be administered according to its proper administration method.

The dose of the therapeutic agent or prophylactic agent of the present invention is changeable and properly set depending upon its preparation form, administration method, purpose of use, and age, body weight, symptom or the like of a patient to which the therapeutic agent or prophylactic agent is administered, or the like. Generally, the dose of the agent is, in terms of the dose of the effective ingredient of the present invention contained in the preparation, for example, from 0.1 μg to 10 g/kg weight, preferably from 1 μg to 5 g/kg weight, and even more preferably from 10 μg to 1 g/kg weight, per day for adult. As a matter of course, the dose varies depending upon various conditions, so that an amount smaller than the dose mentioned above may be sufficient, or an amount exceeding the dose range may be required. Administration may be carried out once or in several divided portions in a day within the desired dose range. The administration period is also not particularly limited. Also, the therapeutic agent or prophylactic agent of the present invention can be directly orally administered, or the agent can be added to any foodstuffs to be taken on a daily basis.

In addition, the present invention can provide an agent for insulin-mimetic action comprising the above-mentioned effective ingredient. The agent for insulin-mimetic action may be the above-mentioned effective ingredient itself, or a composition comprising the above-mentioned effective ingredient. In the embodiment of the present invention, as the effective ingredient in the form of a salt, a pharmacologically acceptable salt is used. The agent for insulin-mimetic action may be prepared by, for example, combining the above-mentioned effective ingredient with other component, for example, insulin preparation, an agent for enhancing insulin secretion, an agent for improving insulin resistance, an agent for ameliorating postprandial hyperglycemia, an agent for insulin-mimetic action or the like which is known in the art which can be used for the same application as the effective ingredient, and forming into a form of reagent usually used according to the above-mentioned process for preparing the therapeutic agent or prophylactic agent. The content of the above-mentioned effective ingredient in the agent for insulin-mimetic action is not particularly limited, as long as

the content is in an amount in which the desired effects of the present invention can be exhibited in consideration of administration method, purpose of use or the like of the agent for insulin-mimetic action. A typical content of the effective ingredient in the agent for insulin-mimetic action is from 0.1 to 100% by weight or so. Also, the amount of the agent for insulin-mimetic action used is not particularly limited, as long as the desired effects of the present invention can be exhibited. Especially in the case where the agent for insulin-mimetic action is administered to a living body, the agent for insulin-mimetic action may be preferably used in an amount in which the effective ingredient can be administered within the dose range of the effective ingredient for the above-mentioned therapeutic agent or prophylactic agent. The agent for insulin-mimetic action is useful in treating or preventing a disease accompanying an abnormality of an amount of insulin or insulin response. In addition, the agent for insulin-mimetic action is useful in screening of drugs for diseases accompanying an abnormality in an amount of insulin or insulin response. Furthermore, the agent for insulin-mimetic action is useful in studies on mechanisms of an action on cells by insulin, or functional studies relating to physical changes in the cells. In addition, the agent for an insulin-mimetic action can be used by adding the agent in place of or together with serum or insulin preparation to a medium for culturing a cell, a tissue, or an organ. The medium is very useful as a medium for culturing a cell, a tissue or an organ that has reduced level of, or contains no serum or insulin preparation.

In addition, the amount of insulin in blood can be expected to be lowered by administering the agent for insulin-mimetic action of the present invention to human. In other words, the agent for insulin-mimetic action of the present invention can also be used as a therapeutic or prophylactic agent for a disease requiring the lowering of the amount of insulin for the treatment or prevention. The disease is not particularly limited, and is exemplified by hyperinsulinemia, Alzheimer's disease and the like. In addition, since reports have been made that the stimulation via an insulin receptor and the effect of extended longevity are closely related *(Science,* vol. 299, P572-574 (2003); *Nature,* vol. 424, P277-284 (2003)), the agent for insulin-mimetic action of the present invention can also be used as an agent for anti-aging.

No toxicity is especially found in the effective ingredient according to the present invention as described later. Also, there is no risk of the onset of side effects. For these reasons, according to the medicament and the like of the present invention, the insulin-mimetic action can be safely and appropriately exhibited. Therefore, the medicament, food, beverage or feed of the present invention comprising the effective ingredient is effective in treating or preventing a disease accompanying an abnormality of an amount of insulin or insulin response.

In addition, the present invention provides a food, beverage or feed for treating or preventing a disease accompanying an abnormality of an amount of insulin or insulin response, wherein the food, beverage or feed comprises the above-mentioned effective ingredient (hereinafter referred to as the food or beverage of the present invention in some cases). In the embodiment of the present invention, as the effective ingredient in the form of a salt, a pharmacologically acceptable salt is used, and a salt of the same level of safety is also suitably used. Since the food, beverage or feed of the present invention has an insulin-mimetic action, the food, beverage or feed is very useful in amelioration of symptoms or prevention of a disease accompanying an abnormality of an amount of insulin or insulin response. Furthermore, the food or beverage of the present invention is a food or beverage for lowering blood glucose level, having the action of lowering a blood glucose level, so that the food or beverage is useful as a functional food or beverage effective in an individual who cares about his/her blood glucose level or an individual who cares about his/her body fat.

The food, beverage or feed of the present invention can be combined with another substance which is known to have an anti-diabetic action, for example, a substance having an insulin-mimetic action, a substance having enhancing action for insulin secretion, a substance having an action for improving insulin resistance, or a substance having an action for ameliorating postprandial hyperglycemia, which is commonly known. The food, beverage or feed can also be combined with, for example, hardly digestible dextrin or the like.

The term "comprising" in the food, beverage or feed of the present invention encompasses the meanings of containing(ed), adding(ed) and/or diluting(ed). As used herein, the term "containing(ed)" refers to an embodiment of containing the effective ingredient used in the present invention in the food, beverage or feed; the term "adding(ed)" refers to an embodiment of adding the effective ingredient used in the present invention to a raw material for the food, beverage or feed; and the term "diluting(ed)" refers to an embodiment of adding a raw material for the food, beverage or feed to the effective ingredient used in the present invention.

The process for preparing the food, beverage or feed of the present invention is not particularly limited. For example, combination, cooking, processing, and the like can be carried out in accordance with those generally employed for foods, beverages or feeds, and the food, beverage or feed of the present invention can be prepared by the methods for preparing a food, beverage or feed, as long as the resulting food, beverage or feed contains the above-mentioned effective ingredient of the present invention having an insulin-mimetic action.

The food or beverage of the present invention is not particularly limited. The food or beverage includes, for example, processed agricultural and forest products, processed livestock products, processed marine products and the like, including processed grain products such as processed wheat products, processed starch products, processed premix products, noodles, macaronis, bread, bean jam, buckwheat noodles, wheat-gluten bread, rice noodle, *fen-tiao*, and packed rice cake; processed fat and oil products such as plastic fat and oil, tempura oil, salad oil, mayonnaise, and dressing; processed soybean products such as tofu products, soybean paste, and fermented soybeans; processed meat

products such as ham, bacon, pressed ham, and sausage; marine products such as frozen ground fish, steamed fish paste, tubular roll of steamed fish paste, cake of ground fish, deep-fried patty of fish paste, fish ball, fascia and tendon, fish meat ham, sausage, dried bonito, products of processed fish egg, canned marine products, and preserved food boiled down in soy sauce (*tsukudani*); milk products such as raw material milk, cream, yogurt, butter, cheese, condensed milk, powder milk, and ice cream; processed vegetable and fruit products such as paste, jam, pickled vegetables, fruit beverages, vegetable beverages, and mixed beverages; confectionaries such as chocolates, biscuits, sweet bun, cake, rice cake snacks and rice snacks; alcohol beverages such as *sake,* Chinese liquor, wine, whiskey, Japanese distilled liquor *(shochu),* vodka, brandy, gin, rum, beer, refreshing alcoholic beverages, fruit liquor, and liqueur; luxury drinks such as green tea, tea, oolong tea, coffee, green juice *(aojiru*), refreshing beverages and lactic acid beverages; seasonings such as soy sauce, sauce, vinegar, and sweet rice wine; canned, bottled or pouched foods such as rice topped with cooked beef and vegetable, rice boiled together with meat and vegetables in a small pot, steamed rice with red beans, curry roux and rice, and other precooked foods; semi-dry or concentrated foods such as liver pastes and other spreads, soups for buckwheat noodles or wheat noodles, and concentrated soups; dry foods such as instant noodles, instant curry roux, instant coffee, powder juice, powder soup, instant soybean paste *(miso)* soup, precooked foods, precooked beverages, and precooked soup; frozen foods such as *sukiyaki,* pot-steamed hotchpotch, split and grilled eel, hamburger steak, *shao-mai,* dumpling stuffed with minced pork, various sticks, and fruit cocktails; solid foods; liquid foods (soups); spices; and the like, in which each of the foods and beverages comprises the above-mentioned ingredient according to the present invention.

In the food or beverage of the present invention, the above-mentioned effective ingredient is contained, added and/or diluted, alone or in plurality, and its shape is not particularly limited, as long as the effective ingredient is contained in an amount necessary for exhibiting its insulin-mimetic action. For example, the shape includes those which can be taken orally such as tablets, granules and capsules.

The content of the above-mentioned effective ingredient in the food or beverage of the present invention is not particularly limited, and the content can be appropriately selected from the viewpoints of sensory aspect and exhibition of activity. The content of the effective ingredient is, for example, preferably 0.00001% by weight or more, more preferably from 0.0001 to 10% by weight, even more preferably from 0.0006 to 6% by weight, of the food. The content is, for example, preferably 0.00001% by weight or more, more preferably from 0.0001 to 10% by weight, even more preferably from 0.0006 to 6% by weight, of the beverage. Also, the food or beverage of the present invention may be taken so that the effective ingredient contained therein is taken in an amount of, for example, from 0.1 μg to 10 g/kg weight, preferably from 1 μg to 5 g/kg weight, and more preferably from 10 μg to 1 g/kg weight, per day for adult.

In addition, the present invention provides a feed for an organism having insulin-mimetic action, prepared by containing, adding and/or diluting the above-mentioned effective ingredient (hereinafter referred to as the feed of the present invention in some cases). In still another embodiment, the present invention also provides a method of feeding an organism, characterized by administering the above-mentioned effective ingredient to the organism. In still yet another embodiment, the present invention provides an organism-feeding agent characterized in that the organism-feeding agent comprises the above-mentioned effective ingredient.

In these inventions, the organisms are, for example, cultured or bred animals, pet animals, and the like. The cultured or bred animal is exemplified by livestock, laboratory animals, poultry, pisces, crustacea or shellfish. The feed is exemplified by a feed for sustenance of and/or amelioration in physical conditions. The organism-feeding agent is exemplified by immersion agents, feed additives, and beverage additives.

According to these inventions, the same effects can be expected to be exhibited as those of the above-mentioned therapeutic agent or prophylactic agent of the present invention, on the basis of the insulin-mimetic action of the above-mentioned effective ingredient used in the present invention, in the organism exemplified above to which these are applied. In other words, the above-mentioned feed or the like has a therapeutic or prophylactic effect in treating or preventing a disease accompanying an abnormality in an amount of insulin or insulin response in the organism.

When the feed or the organism-feeding agent of the present invention is used, the above-mentioned effective ingredient is usually administered in an amount of preferably from 0.01 to 2000 mg per day per 1 kg of the body weight of a subject organism. The administration can be carried out, for example, by previously adding and mixing the effective ingredient in a raw material for an artificially formulated feed to be given to a subject organism, or mixing the effective ingredient with a powder raw material for an artificially formulated feed, and thereafter further adding and mixing the mixture with other raw materials. The content of the above-mentioned effective ingredient in the feed is not particularly limited. The content can be appropriately set in accordance with its purposes, and typically, the content is preferably in a ratio of from 0.001 to 15% by weight. The content of the effective ingredient in the organism-feeding agent may be adjusted to the same level as the feed.

The process for preparing the feed and the organism-feeding agent according to the present invention is not particularly limited, and its composition may be set in accordance with a general feed, as long as the above-mentioned effective ingredient according to the present invention having insulin-mimetic action may be contained in the feed prepared.

The organism to which the present invention can be applied is not limited. The cultured or bred animals include livestock such as *Equus, Bos, Porcus, Ovis, Capra, Camelus,* and *Lama;* laboratory animals such as mice, rats, guinea pigs, and rabbits; poultry such as *Chrysolophus,* ducks, *Meleagris,* and *Struthioniformes;* and the pet animals include dogs, cats, and the like, so that the feed can be widely applied.

By allowing a subject organism to take the feed comprising the above-mentioned effective ingredient used in the present invention having insulin-mimetic action, or immersing a subject organism into a solution containing the above-mentioned effective ingredient used in the present invention having insulin-mimetic action (prepared by dissolving the above immersing agent to pool or the like), the physical conditions of the livestock, laboratory animals, poultry, pet animals or the like can be well sustained or ameliorated. These embodiments of administering the effective ingredient of the present invention to a subject organism are encompassed as one embodiment of the feeding method of an organism in the present invention.

In addition, the present invention can provide an agent for enhancement of glucose uptake into a cell comprising the above-mentioned effective ingredient. The agent for enhancement of glucose uptake may be the above-mentioned effective ingredient itself, or a composition comprising the above-mentioned effective ingredient. In the embodiment of the present invention, as the effective ingredient in the form of a salt, a pharmacologically acceptable salt is used. The agent for enhancement of glucose uptake may be prepared by, for example, combining the above-mentioned effective ingredient with other components, for example, insulin preparation, an agent for enhancing insulin secretion, an agent for improving insulin resistance, an agent for ameliorating postprandial hyperglycemia, an agent for insulin-mimetic action or the like that is commonly known, which can be used for the same application as the effective ingredient, and forming into a form of reagent usually used according to the above-mentioned process for preparing the therapeutic agent or prophylactic agent. The content of the above-mentioned effective ingredient in the agent for enhancement of glucose uptake is not particularly limited, as long as the content is in an amount in which the desired effects of the present invention can be exhibited in consideration of administration method, purpose of use or the like of the agent for enhancement of glucose uptake. A typical content of the effective ingredient in the agent for enhancement of glucose uptake is from 0.1 to 100% by weight or so. Also, the amount of the agent for enhancement of glucose uptake used is not particularly limited, as long as the desired effects of the present invention can be exhibited. Especially in the case where the agent for enhancement of glucose uptake is used by administering to a living body, the agent for enhancement of glucose uptake may be preferably used in an amount in which the effective ingredient can be administered within the dose range of the effective ingredient for the above-mentioned therapeutic agent or prophylactic agent. The agent for enhancement of glucose uptake is useful in treating or preventing a disease requiring an enhancing action on glucose uptake into a cell for the treatment or prevention. The disease is exemplified by, for example, the above-mentioned disease requiring an insulin-mimetic action, as well as cardiac diseases, especially cardiac infarction and post-ischemic injury of the heart, and the like. In addition, since the agent for enhancement of glucose uptake enhances glucose uptake by a cell, when the action is exhibited in a muscle cell, an action for enhancing muscles or an action for recovery from fatigue can be induced. Also, the agent for enhancement of glucose uptake can be used for the manufacture of a food, beverage or feed for treating or preventing these diseases. The food, beverage, or feed can be used according to the above-mentioned food, beverage or feed for treating or preventing a disease requiring an insulin-mimetic action mentioned above. In addition, the agent for enhancement of glucose uptake is also useful in screening of drugs for diseases requiring an enhancing action on glucose uptake into a cell for the treatment or prevention mentioned above. Furthermore, the agent for enhancement of glucose uptake is useful in studies on mechanisms of action of glucose uptake by the cell, or functional studies on physical changes in the cells and the like.

In addition, the present invention can provide an agent for induction of an adipocyte differentiation comprising the above-mentioned effective ingredient. The precursor cell that can be induced to differentiate into adipocyte by the agent for induction of differentiation is not particularly limited, as long as the cell is capable of differentiating into adipocytes. The precursor cell includes, for example, preadipocyte, fibroblast, mesenchymal stem cell and the like. The agent for induction of differentiation may be the above-mentioned effective ingredient itself, or a composition comprising the above-mentioned effective ingredient. In the embodiment of the present invention, as the effective ingredient in the form of a salt, a pharmacologically acceptable salt is used. The agent for induction of differentiation may be prepared by, for example, combining the above-mentioned effective ingredient with other component, for example, insulin preparation, an agent for enhancing insulin secretion, an agent for improving insulin resistance, an agent for ameliorating postprandial hyperglycemia, an agent for insulin-mimetic action or the like which is commonly known, which can be used for the same application as the effective ingredient, and forming into a form of reagent usually used according to the above-mentioned process for preparing the therapeutic agent or prophylactic agent. The content of the above-mentioned effective ingredient in the agent for induction of differentiation is not particularly limited, as long as the content is in an amount in which the desired effects of the present invention can be exhibited in consideration of administration method, method of use or the like of the agent for induction of differentiation. A typical content of the effective ingredient in the agent for induction of differentiation is from 0.1 to 100% by weight or so. Also, the amount of the agent for induction of differentiation used is not particularly limited, as long as the desired effects of the present invention can be exhibited. Especially in the case

where the agent for induction of differentiation is administered to a living body, the agent for induction of differentiation may be preferably used in an amount in which the effective ingredient can be administered within the dose range of the effective ingredient for the above-mentioned therapeutic agent or prophylactic agent. The agent for induction of differentiation is useful in treating or preventing a disease requiring an action for induction of an adipocyte differentiation for the treatment or prevention. The disease is exemplified by, for example, the above-mentioned disease requiring an insulin-mimetic action, as well as gout, fatty liver, cholecystolithiasis, menoxenia, infertility, and the like. The agent for induction of differentiation can be used for the manufacture of a food, beverage or feed for treating or preventing these diseases. The food, beverage, or feed can be used according to the above-mentioned food, beverage or feed in treating or preventing the above-mentioned disease requiring an insulin-mimetic action. In addition, the agent for induction of differentiation is also useful in screening of drugs for diseases requiring an action for induction of an adipocyte differentiation for the treatment or prevention mentioned above. Furthermore, the agent for induction of differentiation is useful in studies on mechanisms of action for induction of an adipocyte differentiation, or functional studies on physical changes in the cells and the like.

No toxicity is found even when the above-mentioned effective ingredient used in the present invention is administered in an amount effective in the exhibition of its action. For example, in the case of oral administration, no cases of deaths are found even when a compound listed in Tables 1 to 15 mentioned above, an optically active form thereof, or a salt thereof is administered to a mouse at 1 g/kg body weight in a single dose. In addition, no cases of deaths are found even when the above-mentioned effective ingredient is orally administered to a rat at 1 g/kg weight in a single dose.

## EXAMPLES

The present invention will be described more concretely hereinbelow by means of the examples, but the present invention is by no means limited to these descriptions. Unless specified otherwise, % in these examples means % by weight.

### Example 1 Preparation of Xanthoangelol

(1) Fifteen liters of ethanol was added to 5 kg of a dry powder of root portions *of Angelica keiskei* koidz., and extracted at room temperature for 30 minutes. After suction filtration, the ethanol and the residue were separated. The same procedures were repeated twice for the residue. Thereafter, the ethanol extracts were combined, and the combined extract was concentrated under reduced pressure, to give a concentrate of an ethanol extract.

(2) The concentrate of an ethanol extract obtained in item (1) of Example 1 was dissolved in 2 L of a 25% ethanol solution, and thereafter fractionated by using reverse phase chromatography. As the resin, Cosmosil 140 C18-OPN (manufactured by Nakalai Tesque, Inc.: 400 mL) was used. The elution was carried out with 1 L of a 30% aqueous ethanol solution, 5 L of a 40% aqueous ethanol solution, 4 L of a 75% aqueous ethanol solution, 3 L of a 100% aqueous ethanol solution in that order.

(3) The fraction eluted with the 75% aqueous ethanol solution obtained in item (2) of Example 1 was concentrated under reduced pressure, and adsorbed on a silica gel (BW-300SP: manufactured by Fuji Silysia Chemical Ltd.: 350 mL). The elution was carried out stepwise with chloroform : hexane at a solvent ratio of 2:1 (800 mL), and 10:4 (1800 mL), and ethyl acetate (1400 mL) in that order. The eluates were fractionated 200 mL each for the fractions 1 to 5, 150 mL for the fraction 6, 100 mL each for the fractions 7 to 10, 200 mL each for the fractions 11 to 16, and 1000 mL for the fraction 17 in that order.

(4) The fraction number 17 obtained in item (3) of Example 1 was concentrated under reduced pressure, and adsorbed on a silica gel (350 mL). The elution was carried out stepwise with chloroform : hexane at a solvent ratio of 10:3 (1000 mL), 10:1 (2100 mL) and 20:1 (1000 mL), and ethyl acetate (500 mL) in that order. After the initial 2300 mL was eluted, the eluates were fractionated for 100 mL each.

(5) The fraction numbers 4 to 22 obtained in item (4) of Example 1 were concentrated under reduced pressure, and the concentrates were dissolved in chloroform. Subsequently, the recrystallization was carried out with hexane, and the formed precipitates and supernatant were separated. The precipitates obtained were dried, to give xanthoangelol.

### Example 2 Preparation of 4-Hydroxyderricin

The silica fraction numbers 10 to 15 obtained in item (3) of Example 1 were collected and concentrated under reduced pressure, and the concentrate was dissolved in chloroform. Subsequently, the recrystallization with hexane was carried out, and the formed precipitates and supernatant were separated. The precipitates obtained were dried, to

give 4-hydroxyderricin.

Example 3 Preparation of Xanthoangelol H

(1) The fraction eluted with the 40% aqueous ethanol solution obtained in item (2) of Example 1 was concentrated under reduced pressure, and the concentrate was adsorbed on a silica gel (350 mL). The elution was carried out stepwise with chloroform : hexane at a solvent ratio of 50:1 (960 mL), 40:1 (520 mL), 20:1 (1000 mL), 10:1 (840 mL) and 5:1 (520 mL) in that order. The eluates were fractionated 8 mL each.
(2) The silica fraction numbers 142 to 164 obtained in item (1) of Example 3 were collected and concentrated to dryness, and the concentrate was dissolved in ethyl acetate. Subsequently, the recrystallization with hexane was carried out, and the formed precipitates and supernatant were separated. The precipitates obtained were dried, to give xanthoangelol H.

Example 4 Preparation of TB1

(1) The silica fraction numbers 303 to 325 obtained in item (1) of Example 3 were collected and concentrated to dryness, and the concentrate was dissolved in ethyl acetate. Subsequently, the recrystallization with hexane was carried out, and the formed precipitates were dried, to give a yellow substance.
(2) The structure of the yellow substance obtained in item (1) of Example 4 was analyzed by measuring various kinds of NMR spectra with a nuclear magnetic resonance (NMR) spectrometer (Model AVANCE 600: manufactured by Bruker BIOSPIN). The signals of NMR are shown below. In addition, the numbers of the peaks are shown in the following formula (Formula 46).

(Formula 46)

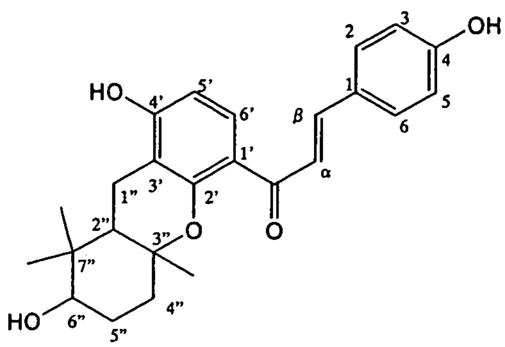

$^1$H-NMR(deuterated dimethyl sulfoxide): δ 0.81 (3H, s, $CH_3$-7"), 1.03 (3H, s, $CH_3$-7"), 1.24 (3H, s, $CH_3$-3"), 1.54 (1H, m, H-5"), 1.61 (1H, dd, J=4.8 Hz, J=13.2 Hz, H-2"), 1.71 (1H, m, H-5"), 1.75 (1H, m, H-4"), 1.87(1H, m, H-4"), 2.34 (1H, dd, J=13.2 Hz, J=16.8 Hz, H-1"), 2.67 (1H, dd, J=4.8 Hz, J=16.8 Hz, H-1"), 3.27 (1H, m, H-6"), 4.65 (1H, d, J=4.8 Hz, OH-6"), 6.47 (1H, d, J=8.4 Hz, H-5'), 6.83 (2H, d, J=8.4 Hz, H-3 and H-5), 7.39 (1H, d, J=8.4 Hz, H-6'), 7.42 (1H, d, J=15.6 Hz, H-β), 7.48 (1H, d, J=15.6 Hz, H-α), 7.51 (2H, d, J=8.4 Hz, H-2 and H-6), 9.97(1H, br-s, OH-4), 10.22(1H, br-s, OH-4')

Figure 1 shows $^1$H-NMR spectrum.

$^{13}$C-NMR (deuterated dimethyl sulfoxide): δ15.3 ($CH_3$-7"), 18.8 (C-1"), 20.7 ($CH_3$-3"), 28.1 ($CH_3$-7"), 28.9 (C-5"), 38.3 (C-4"), 38.9 (C-7"), 46.4 (C-2"), 76.8 (C-6"), 77.9 (C-3"), 107.7 (C-5'), 110.4 (C-3'), 116.8 (C-3 and C-5), 120.8 (C-1'), 125.2 (C-α), 127.1 (C-1), 130.2(C-6'), 130.8 (C-2 and C-6), 141.2 (C-β), 154.9 (C-2'), 160.3 (C-4), 160.6 (C-4'), 189.8(C=O)

Figure 2 shows $^{13}$C-NMR spectrum.

Next, the mass spectrum (MS) of the yellow substance obtained in item (1) of Example 4 was measured with a mass spectrometer (DX302: manufactured by JEOL LTD.) by FAB-MS technique.

As the matrix, FAB-MS: m/z 407 $(M-H)^-$ metanitrobenzyl alcohol was used.

From the above results of the NMR spectrum analysis and mass spectrum analysis, the yellow substance obtained in item (1) of Example 4 was identified to be 1-(5,6,7,8,8a,10a-hexahydro-1,7-dihydroxy-8,8,10a-trimethyl-9H-xanthen-4-yl)-3-(4-hydroxyphenyl)-2-propen-1-one (molecular weight: 408, hereinafter referred to as TB1).

Example 5 Preparation of TB2

(1) The silica fraction numbers 283 to 302 obtained in item (1) of Example 3 were collected and concentrated to dryness, and the concentrate was dissolved in ethyl acetate. Subsequently, the recrystallization with hexane was carried out, and the formed precipitates were dried, to give a yellow substance.

(2) The NMR spectra and the mass spectrum of the yellow substance obtained in item (1) of Example 5 were determined in the same manner as in item (2) of Example 4. The signals of NMR are shown below. In addition, the numbers of the peaks are shown in the following formula (Formula 47).

(Formula 47)

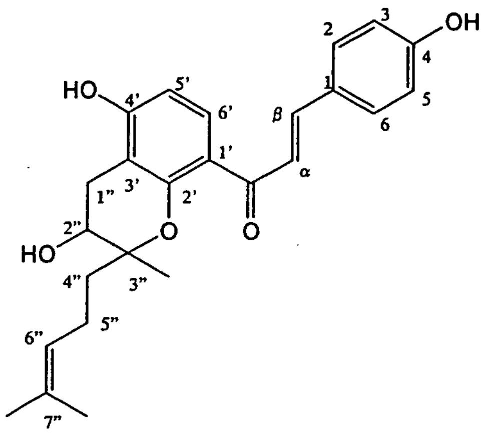

$^{1}$H-NMR(deuterated dimethyl sulfoxide): δ1.20(3H, s, $CH_3$-3"), 1.36 (3H, s, $CH_3$-7"), 1.57 (3H, s, $CH_3$-7"), 1.68 (2H, m, H-4"), 2.10 (2H, m, H-5"), 2.41 (1H, dd, J=9.0 Hz, J=16.8 Hz, H-1"), 2.85 (1H, dd, J=6.0 Hz, J=16.8 Hz, H-1"), 3.76 (1H, m, H-2"), 5.01 (1H, m, H-6"), 5.23 (1H, d, J=4.8 Hz, OH-2"), 6.47 (1H, d, J=8.4 Hz, H-5'), 6.80 (2H, d, J=8.4 Hz, H-3 and H-5), 7.38 (1H, d, J=8.4 Hz, H-6'), 7.44 (1H, d, J=15.6 Hz, H-β), 7.47 (1H, d, J=15.6 Hz, H-α), 7.50 (2H, d, J=8.4 Hz, H-2 and H-6), 9.96 (1H, s, OH-4), 10.19 (1H, s, OH-4')

Figure 3 shows $^{1}$H-NMR spectrum.

$^{13}$C-NMR (deuterated dimethyl sulfoxide): δ18.1 ($CH_3$-3"), 18.2 ($CH_3$-7"), 22.1 (C-5"), 26.3 ($CH_3$-7"), 27.2 (C-1"), 38.7 (C-4"), 66.7 (C-2"), 80.2 (C-3"), 107.8 (C-5'), 109.1 (C-3'), 116.7 (C-3 and C-5), 121.0 (C-1'), 125.1 (C-6"), 125.1 (C-α), 127.0 (C-1), 130.3 (C-6'), 130.8 (C-2 and C-6), 131.6 (C-7"), 141.5 (C-β), 154.6 (C-2'), 160.4(C-4), 160.4 (C-4'), 189.9(C=O)

Figure 4 shows $^{13}$C-NMR spectrum.

As the matrix, FAB-MS: m/z 407 $(M-H)^-$ metanitrobenzyl alcohol was used.

From the above results of the NMR spectrum analysis and mass spectrum analysis, the yellow substance obtained in item (1) of Example 5 was identified to be 1-(3,4-dihydro-3,5-dihydroxy-2-(3-isohexenyl)-2-methyl-2H-benzopyran-8-yl)-3-(4-hydroxyphenyl)-2-propen-1-one (molecular weight: 408, hereinafter referred to as TB2).

Example 6 Preparation of TB3

(1) The fraction numbers 23 and 24 obtained in item (4) of Example 1 were concentrated under reduced pressure, and the concentrate was dissolved in chloroform. The recrystallization with hexane was carried out, to give a yellow substance.

(2) The NMR spectra and the mass spectrum of the yellow substance obtained in item (1) of Example 6 were determined in the same manner as in item (2) of Example 4. The signals of NMR are shown below. In addition, the

numbers of the peaks are shown in the following formula (Formula 48).

(Formula 48)

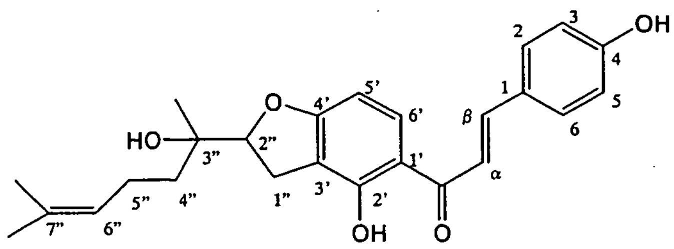

$^1$H-NMR(deuterated chloroform): δ1.34 (3H, s, $CH_3$-3"), 1.57 (2H, m, H-4"), 1.65 (3H, s, $CH_3$-7"), 1.71 (3H, s, $CH_3$-7"), 1.79(1H, s, OH-3"), 2.11 (1H, m, H-5"), 2.19 (1H, m, H-5"), 3.19 (2H, d, J=8.7 Hz, H-1"), 4.82 (1H, t, J=8.7 Hz, H-2"), 5.15 (1H, t, J=6.7 Hz, H-6"), 5.21 (1H, s, OH-4), 6.44 (1H, d, J=8.4 Hz, H-5'), 6.89 (2H, d, J=7.2 Hz, H-3 and H-5), 7.46 (1H, d, J=15.0 Hz, H-α), 7.58 (2H, d, J=7.2 Hz, H-2 and H-6), 7.80 (1H, d, J=8.4 Hz, H-6'), 7.84 (1H, d, J=15.0 Hz, H-β), 13.51 (1H, s, OH-2')

Figure 5 shows $^1$H-NMR spectrum.

$^{13}$C-NMR (deuterated chloroform): δ18.1 ($CH_3$-7"), 22.4 (C-5"), 23.2 ($CH_3$-3"), 26.1 ($CH_3$-7"), 27.3 (C-1"), 37.1 (C-4"), 74.2 (C-3"), 91.6 (C-2"), 102.1 (C-5'), 114.2 (C-3'), 115.4 (C-1'), 116.4 (C-3 and C-5), 118.6 (C-α), 124.4 (C-6"), 128.2 (C-1), 130.9 (C-2 and C-6), 132.1 (C-6'), 132.7 (C-7"), 144.3 (C-β), 158.3 (C-4), 161.9 (C-2'), 167.0 (C-4'), 192.5(C=O)

Figure 6 shows $^{13}$C-NMR spectrum.

As the matrix, FAB-MS: m/z 407 $(M-H)^-$ metanitrobenzyl alcohol was used.

From the above results of the NMR spectrum analysis and mass spectrum analysis, the yellow substance obtained in item (1) of Example 6 was identified to be 1-[2,3-dihydro-4-hydroxy-2-(1-hydroxy-1,5-dimethyl-4-hexenyl)-benzofuran-5-yl]-3-(4-hydroxyphenyl)-2-propen-1-one (molecular weight: 408, hereinafter referred to as TB3).

Example 7 Preparation of TB4

(1) The silica fraction numbers 118 to 132 obtained in item (1) of Example 3 were collected and concentrated to dryness, to give a yellow substance.

(2) The NMR spectra and the mass spectrum of the yellow substance obtained in item (1) of Example 7 were determined in the same manner as in item (2) of Example 4. The signals of NMR are shown below. In addition, the numbers of the peaks are shown in the following formula (Formula 49).

(Formula 49)

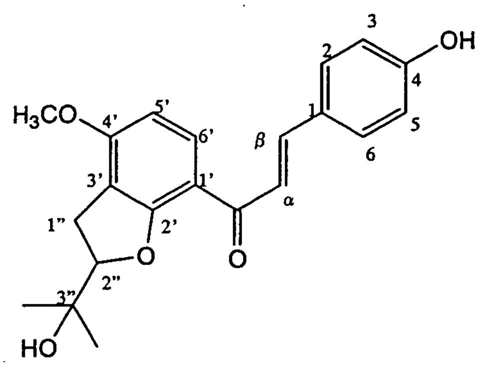

$^1$H-NMR(deuterated dimethyl sulfoxide): δ1.18 (3H, s, $CH_3$-3"), 1.28 (3H, s, $CH_3$-3"), 3.07 (2H, m, H-1"), 3.87 (3H, s, $OCH_3$-4'), 4.72 (1H, s, OH-3"), 4.78 (1H, t, J=8.7 Hz, H-2"), 6.65 (1H, d, J=9.0 Hz, H-5'), 6.82 (2H, d, J=8.4 Hz,

H-3 and H-5), 7.57 (2H, d, J=8.4 Hz, H-2 and H-6), 7.59 (1H, d, J=15.6 Hz, H-β), 7.69 (1H, d, J=9.0 Hz, H-6'), 7.81 (1H, d, J=15.6 Hz, H-α), 10.02 (1H, s, OH-4)

Figure 7 shows $^{1}$H-NMR spectrum.

$^{13}$C-NMR (deuterated dimethyl sulfoxide): δ26.2 ($CH_3$-3"), 26.8 ($CH_3$-3"), 27.6 (C-1"), 56.5 ($OCH_3$-4'), 70.9 (C-3"), 91.5 (C-2"), 105.2 (C-5'), 115.7 (C-3'), 116.0 (C-1'), 116.7 (C-3 and C-5), 123.8 (C-α), 127.0 (C-1), 131.0 (C-2 and C-6), 131.3 (C-6'), 142.7 (C-β), 160.5 (C-4'), 160.6 (C-4), 161.8 (C-2'), 186.5 (C=O)

Figure 8 shows $^{13}$C-NMR spectrum.

As the matrix, FAB-MS: m/z 353 $(M-H)^-$ metanitrobenzyl alcohol was used.

From the above results of the NMR spectrum analysis and mass spectrum analysis, the yellow substance obtained in item (1) of Example 7 was identified to be 1-[2,3-dihydro-2-(1-hydroxy-1-methylethyl)-4-methoxybenzofuran-7-yl]-3-(4-hydroxyphenyl)-2-propen-1-one (molecular weight: 354, hereinafter referred to as TB4).

Example 8 Preparation of TB5

(1) The silica fraction numbers 335 to 349 obtained in item (1) of Example 3 were collected and concentrated to dryness, and thereafter fractionated by using reverse phase chromatography. As the resin, Cosmosil 140 C18-OPN (30 mL) was used. The elution was carried out with 200 mL each of a 10% aqueous ethanol solution, a 15% aqueous ethanol solution, a 20% aqueous ethanol solution, a 25% aqueous ethanol solution, and a 30% aqueous ethanol solution, 500 mL of a 35% aqueous ethanol solution, and 200 mL of a 75% aqueous ethanol solution in that order, and the eluates were fractionated for every 100 mL.

(2) The fraction numbers 6 and 7 obtained in item (1) of Example 8 were collected and concentrated to dryness, to give a yellow substance.

(3) The NMR spectra and the mass spectrum of the yellow substance obtained in item (2) of Example 8 were determined in the same manner as in item (2) of Example 4. The signals of NMR are shown below. In addition, the numbers of the peaks are shown in the following formula (Formula 50).

(Formula 50)

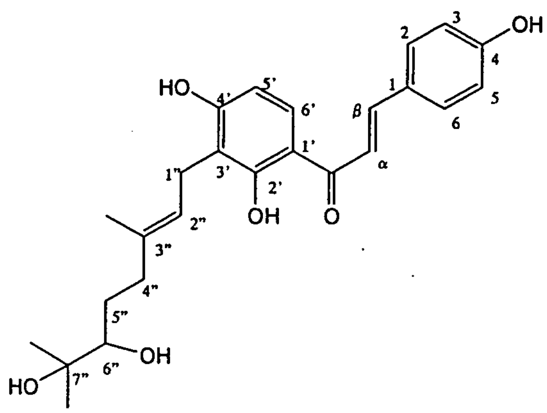

$^{1}$H-NMR(deuterated dimethyl sulfoxide): δ0.96 (3H, s, $CH_3$-7"), 1.02 (3H, s, $CH_3$-7"), 1.16 (1H, m, H-5"), 1.61 (1H, m, H-5"), 1.73 (3H, s, $CH_3$-3"), 1.85 (1H, m, H-4"), 2.15 (1H, m, H-4"), 3.01 (1H, m, H-6"), 3.24 (1H, m, H-1"), 3.31 (1H, m, H-1"), 4.00 (1H, s, OH-7"), 4.23 (1H, d, J=6.0 Hz, OH-6"), 5.19 (1H, t, J=7.2 Hz, H-2"), 6.47 (1H, d, J=8.4 Hz, H-5'), 6.84 (2H, d, J=8.4 Hz, H-3 and H-5), 7.75 (1H, d, J=5.4 Hz, H-α), 7.75 (1H, d, J=5.4 Hz, H-β), 7.75 (2H, d, J=8.4 Hz, H-2 and H-6), 8.03 (1H, d, J=8.4 Hz, H-6'), 10.11 (1H, s, OH-4), 10.55 (1H, s, OH-4'), 14.00 (1H, s, OH-2')

Figure 9 shows $^{1}$H-NMR spectrum.

$^{13}$C-NMR (deuterated dimethyl sulfoxide): δ17.0 ($CH_3$-3"), 22.1 (C-1"), 25.4 ($CH_3$-7"), 27.2 ($CH_3$-7"), 30.3 (C-5"), 37.5 (C-4"), 72.4 (C-7"), 78.0 (C-6"), 108.2 (C-5'), 113.6 (C-1'), 115.4 (C-3'), 116.7 (C-3 and C-5), 118.3 (C-α), 122.4 (C-2"), 126.7 (C-1), 130.7 (C-6'), 132.0 (C-2 and C-6), 135.7 (C-3"), 145.0 (C-β), 161.1 (C-4), 163.2 (C-4'), 164.4 (C-2'), 192.6 (C=O)

Figure 10 shows $^{13}$C-NMR spectrum.

As the matrix, FAB-MS: m/z 425 $(M-H)^-$ metanitrobenzyl alcohol was used.

From the above results of the NMR spectrum analysis and mass spectrum analysis, the yellow substance obtained in item (2) of Example 8 was identified to be 1-[2,4-dihydroxy-3-(6,7-dihydroxy-3,7-dimethyl-2-octenyl)phenyl]-3-(4-hydroxyphenyl)-2-propen-1-one (molecular weight: 426, hereinafter referred to as TB5).

Example 9 Preparation of TB6

(1) The concentrate of the supernatant obtained in item (2) of Example 3 was adsorbed on the silica gel (100 mL). The elution was carried out with a solvent of hexane : ethyl acetate = 7:5, and the eluates were fractionated for every 8 mL.

(2) The silica fraction numbers 41 to 51 obtained in item (1) of Example 9 were collected and concentrated to dryness, to give a yellow substance.

(3) The NMR spectra and the mass spectrum of the yellow substance obtained in item (2) of Example 9 were determined in the same manner as in item (2) of Example 4. The signals of NMR are shown below. In addition, the numbers of the peaks are shown in the following formula (Formula 51).

(Formula 51)

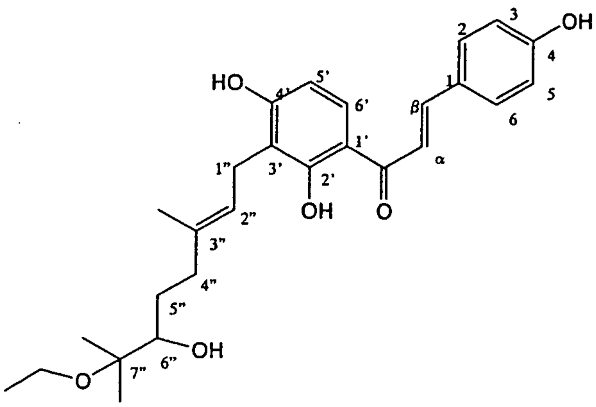

$^1$H-NMR(deuterated dimethyl sulfoxide): δ0.96 (3H, s, $CH_3$-7"), 0.99 (3H, t, J=6.9 Hz, -O-$CH_2$-$\underline{CH_2}$), 1.04 (3H, s, $CH_3$-7"), 1.15 (1H, m, H-5"), 1.60 (1H, m, H-5"), 1.72 (3H, s, $CH_3$-3"), 1.89 (1H, m, H-4"), 2.13 (1H, m, H-4"), 3.18 (1H, m, H-6"), 3.24 (2H, m, H-1"), 3.29 (2H, m, -O-$\underline{CH_2}$-$CH_3$), 4.27 (1H, d, J=6.0 Hz, OH-6"), 5.20 (1H, t, J=6.9 Hz, H-2"), 6.47 (1H, d, J=9.0 Hz, H-5'), 6.84 (2H, d, J=8.4 Hz, H-3 and H-5), 7.75 (1H, d, J=4.8 Hz, H-α), 7.75 (1H, d, J=4.8 Hz, H-β), 7.75 (2H, d, J=8.4 Hz, H-2 and H-6), 8.31 (1H, d, J=9.0 Hz, H-6'), 10.11 (1H, s, OH-4), 10.55 (1H, s, OH-4'), 14.00 (1H, s, OH-2')

Figure 11 shows $^1$H-NMR spectrum.

$^{13}$C-NMR (deuterated dimethyl sulfoxide): δ17.0 ($CH_3$-3"), 17.0 (-O-$CH_2$-$\underline{C}H_3$), 21.1 ($CH_3$-7"), 22.1 (C-1"), 23.3 ($CH_3$-7"), 29.9(C-5"), 37.2 (C-4"), 56.6 (-O-$\underline{C}H_2$-$CH_3$), 75.1(C-6"), 77.5 (C-7"), 108.2 (C-5'), 113.6 (C-1'), 115.4 (C-3'), 116.7 (C-3 and C-5), 118.3 (C-α), 122.7 (C-2"), 126.7 (C-1), 130.6 (C-6'), 132.0 (C-2 and C-6), 135.5 (C-3"), 145.0 (C-β), 161.1 (C-4), 163.1 (C-4'), 164.4 (C-2'), 192.6 (C=O)

Figure 12 shows $^{13}$C-NMR spectrum.

As the matrix, FAB-MS: m/z 453 $(M-H)^-$ metanitrobenzyl alcohol was used.

From the above results of the NMR spectrum analysis and mass spectrum analysis, the yellow substance obtained in item (2) of Example 9 was identified to be 1-[3-(7-ethoxy-6-hydroxy-3,7-dimethyl-2-octenyl)-2,4-dihydroxyphenyl]-3-(4-hydroxyphenyl)-2-propen-1-one (molecular weight: 454, hereinafter referred to as TB6).

Example 10 Preparation of TB7

(1) The concentrate of the supernatant obtained in item (5) of Example 1 was adsorbed on the silica gel (350 mL). The elution was carried out stepwise with chloroform : hexane at a solvent ratio of 100:1 (1500 mL), 50:1 (2600

mL), and 20:1 (2600 mL), and ethyl acetate (300 mL) in that order, and the eluates were fractionated for every 8 mL.
(2) The fraction numbers 21 to 30 obtained in item (1) of Example 10 were collected and concentrated to dryness, to give a yellow substance.
(3) The NMR spectra and the mass spectrum of the yellow substance obtained in item (2) of Example 10 were determined in the same manner as in item (2) of Example 4. The signals of NMR are shown below. In addition, the numbers of the peaks are shown in the following formula (Formula 52).

(Formula 52)

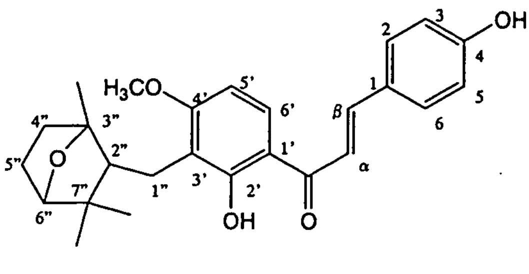

$^1$H-NMR (deuterated dimethyl sulfoxide): δ0.91 (3H, s, $CH_3$-7"), 0.96 (3H, s, $CH_3$-7"), 1.21 (3H, s, $CH_3$-3"), 1.26 (1H, m, H-4"), 1.43 (1H, m, H-4"), 1.53 (1H, m, H-5"), 1.85 (1H, m, H-5"), 2.12 (1H, t, J=7.2 Hz, H-2"), 2.52 (1H, m, H-1"), 2.56 (1H, m, H-1"), 3.62 (1H, d, J=5.4 Hz, H-6"), 3.91 (3H, s, $OCH_3$-4'), 6.67 (1H, d, J=9.0 Hz, H-5'), 6.85 (2H, d, J=8.4 Hz, H-3 and H-5), 7.78 (1H, d, J=15.6 Hz, H-β), 7.78 (2H, d, J=8.4 Hz, H-2 and H-6), 7.83 (1H, d, J=15.6 Hz, H-α), 8.23 (1H, d, J=9.0 Hz, H-6'), 10.15 (1H, s, OH-4), 13.99 (1H, s, OH-2')

Figure 13 shows $^1$H-NMR spectrum.

$^{13}$C-NMR (deuterated dimethyl sulfoxide): δ19.0 ($CH_3$-3"), 21.3(C-1"), 24.5 ($CH_3$-7"), 26.0 ($CH_3$-7"), 26.4 (C-5"), 39.9 (C-4"), 46.3 (C-7"), 53.5 (C-2"), 56.8 ($OCH_3$-4'), 85.8 (C-6"), 86.9 (C-3"), 103.7 (C-5'), 114.8 (C-1'), 116.7 (C-3 and C-5), 117.4 (C-3'), 118.2 (C-α), 126.6 (C-1), 131.3 (C-6'), 132.2 (C-2 and C-6), 145.7 (C-β), 161.3 (C-4), 163.5 (C-2'), 164.1 (C-4'), 193.4 (C=O)

Figure 14 shows $^{13}$C-NMR spectrum.

As the matrix, FAB-MS: m/z 421 $(M-H)^-$ metanitrobenzyl alcohol was used.

From the above results of the NMR spectrum analysis and mass spectrum analysis, the yellow substance obtained in item (2) of Example 10 was identified to be 1-[3-(2,5-epoxy-2,6,6-trimethyl-cyclohexylmethyl)-2-hydroxy-4-methoxyphenyl]-3-(4-hydroxyphenyl)-2-propen-1-one (molecular weight: 422, hereinafter referred to as TB7).

<u>Example 11 Preparation of TB8</u>

(1) The supernatant obtained in Example 2 was concentrated under reduced pressure, and thereafter fractionated using reverse phase chromatography. As the column, TSK gel ODS-80Ts (21.5 mm × 30 cm: manufactured by Tosoh Corporation) was used. The solvent was distilled water : acetonitrile = 15:85, the elution rate was 5 mL/minute, and the detection was carried out at 215 nm. The eluates were fractionated using ultraviolet absorption of the eluates as an index.
(2) The reverse phase chromatography fraction 2 (the fraction containing a detection peak at a retention time of 57.6 minutes) obtained in item (1) of Example 11 were collected and concentrated to dryness, to give a yellow substance.
(3) The NMR spectra and the mass spectrum of the yellow substance obtained in item (2) of Example 11 were determined in the same manner as in item (2) of Example 4. The signals of NMR are shown below. In addition, the numbers of the peaks are shown in the following formula (Formula 53).

(Formula 53)

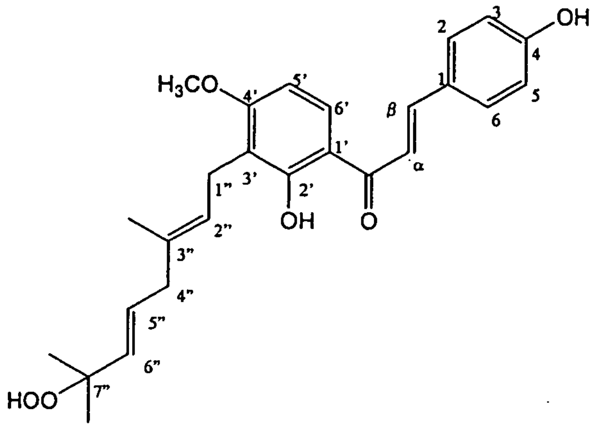

$^1$H-NMR(deuterated dimethyl sulfoxide): δ1.19 (3H, s, $CH_3$-7"), 1.19 (3H, s, $CH_3$-7"), 1.70 (3H, s, $CH_3$-3"), 2.62 (2H, d, J=6.6 Hz, H-4"), 3.29 (1H, m, H-1"), 3.31 (1H, m, H-1"), 3.91 (3H, s, $OCH_3$-4'), 5.19 (1H, t, J=6.9 Hz, H-2"), 5.47 (1H, m, H-5"), 5.55 (1H, d, J=15.6 Hz, H-6"), 6.68 (1H, d, J=9.0 Hz, H-5'), 6.85 (2H, d, J=8.4 Hz, H-3 and H-5), 7.78 (2H, d, J=8.4 Hz, H-2 and H-6), 7.79 (1H, d, J=13.2 Hz, H-β), 7.83 (1H, d, J=13.2 Hz, H-α), 8.23 (1H, d, J=9.0 Hz, H-6'), 10.14 (1H, s, OH-4), 10.81 (1H, s, OOH-7"), 13.81 (1H, s, OH-2')

Figure 15 shows $^1$H-NMR spectrum.

$^{13}$C-NMR (deuterated dimethyl sulfoxide): δ16.8 ($CH_3$-3"), 22.1 (C-1"), 25.5 ($CH_3$-7"), 25.5 ($CH_3$-7"), 43.0 (C-4"), 56.9 ($OCH_3$-4'), 81.1 (C-7"), 103.7 (C-5'), 114.9 (C-1'), 116.6 (C-3'), 116.7 (C-3 and C-5), 118.1 (C-α), 123.5 (C-2"), 126.5 (C-1), 127.9 (C-5"), 131.4 (C-6'), 132.3(C-2 and C-6), 134.3 (C-3"), 137.0 (C-6"), 145.7 (C-β), 161.3 (C-4), 163.0 (C-2'), 163.9 (C-4'), 193.3 (C=O)

Figure 16 shows $^{13}$C-NMR spectrum.

As the matrix, FAB-MS: m/z 437 $(M-H)^-$ metanitrobenzyl alcohol was used.

From the above results of the NMR spectrum analysis and mass spectrum analysis, the yellow substance obtained in item (2) of Example 11 was identified to be 1-[2-hydroxy-3-(7-hydroperoxy-3,7-dimethyl-2,5-octadienyl)-4-methoxyphenyl]-3-(4-hydroxyphenyl)-2-propen-1-one (molecular weight: 438, hereinafter referred to as TB8).

Example 12 Preparation of TB9

(1) The reverse phase chromatography fraction 3 obtained in item (1) of Example 11 (fraction containing a detection peak at a retention time of 61.2 minutes) was concentrated to dryness, to give a yellow substance.

(2) The NMR spectra and the mass spectrum of the yellow substance obtained in item (1) of Example 12 were determined in the same manner as in item (2) of Example 4. The signals of NMR are shown below. In addition, the numbers of the peaks are shown in the following formula (Formula 54).

(Formula 54)

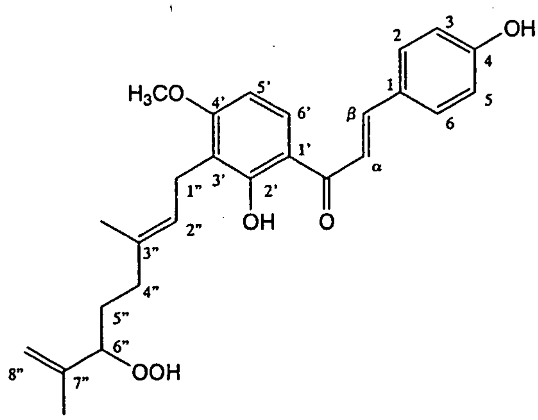

$^1$H-NMR(deuterated dimethyl sulfoxide): δ1.40 (1H, m, H-5"), 1.56 (1H, m, H-5"), 1.62 (3H, s, $CH_3$-7"), 1.72 (3H, s, $CH_3$-3"), 1.89 (2H, m, H-4"), 3.27 (1H, m, H-1"), 3.31 (1H, m, H-1"), 3.91 (3H, s, $OCH_3$-4'), 4.07 (1H, t, J=6.9 Hz, H-6"), 4.79 (1H, s, H-8"), 4.84 (1H, s, H-8"), 5.14 (1H, t, J=6.6 Hz, H-2"), 6.68 (1H, d, J=9.0 Hz, H-5'), 6.85 (2H, d, J=8.4 Hz, H-3 and H-5), 7.78 (2H, d, J=8.4 Hz, H-2 and H-6), 7.78 (1H, d, J=15.0 Hz, H-β), 7.83 (1H, d, J=15.0 Hz, H-α), 8.24 (1H, d, J=9.0 Hz, H-6'), 10.15 (1H, s, OH-4), 11.25 (1H, s, OOH-6"), 13.81 (1H, s, OH-2')

Figure 17 shows $^1$H-NMR spectrum.

$^{13}$C-NMR (deuterated dimethyl sulfoxide): δ16.7 ($CH_3$-3"), 17.7 ($CH_3$-7"), 22.0 (C-1"), 29.5 (C-5"), 36.0 (C-4"), 56.9 ($OCH_3$-4'), 88.2 (C-6"), 103.6 (C-5'), 114.0 (C-8"), 114.9 (C-1'), 116.7 (C-3'), 116.7 (C-3 and C-5), 118.1 (C-α), 122.9 (C-2"), 126.5 (C-1), 131.3 (C-6'), 132.3 (C-2 and C-6), 134.9 (C-3"), 145.3 (C-7"), 145.7 (C-β), 161.3 (C-4), 163.0 (C-2'), 163.8 (C-4'), 193.3 (C=O)

Figure 18 shows $^{13}$C-NMR spectrum.

As the matrix, FAB-MS: m/z 437 $(M-H)^-$ metanitrobenzyl alcohol was used.

From the above results of the NMR spectrum analysis and mass spectrum analysis, the yellow substance obtained in item (1) of Example 12 was identified to be 1-[2-hydroxy-3-(6-hydroperoxy-3,7-dimethyl-2,7-octadienyl)-4-methoxyphenyl]-3-(4-hydroxyphenyl)-2-propen-1-one (molecular weight: 438, hereinafter referred to as TB9).

Example 13 Preparation of Compound (C081)

(1) One-hundred milligrams of TB8 obtained in item (2) of Example 11 was dissolved in 50 mL of methanol, and 60 mg of triphenylphosphine (manufactured by Tokyo Kasei Kogyo Co., Ltd.) was added to the solution, and the mixture was reacted at room temperature for 1 hour. The reaction mixture was concentrated under reduced pressure, and the concentrate was subjected to thin layer chromatography using chloroform : methanol = 10:1 as a developing solvent. Next, an ultraviolet absorption portion was scraped off, and extracted with the developing solvent, and thereafter the extracted was concentrated to dryness, to give 57.2 mg of a yellow substance.

(2) The NMR spectra and the mass spectrum of the yellow substance obtained in item (1) of Example 13 were determined in the same manner as in item (2) of Example 4. The signals of NMR are shown below. In addition, the numbers of the peaks are shown in the following formula (Formula 55).

(Formula 55)

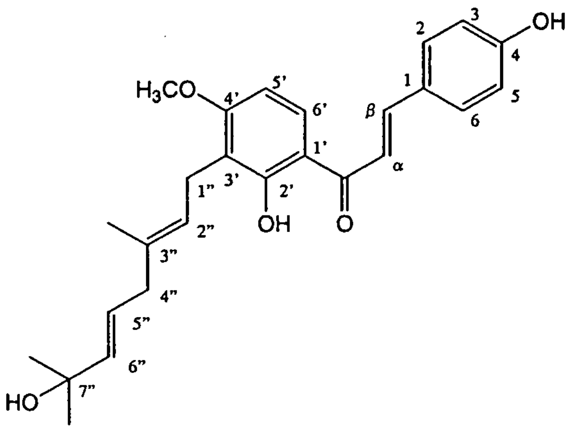

$^1$H-NMR(deuterated dimethyl sulfoxide): δ1.13 (3H, s, $CH_3$-7"), 1.13 (3H, s, $CH_3$-7"), 1.70 (3H, s, $CH_3$-3"), 2.59 (2H, d, J=7.2 Hz, H-4"), 3.28 (2H, d, J=7.2 Hz, H-1"), 3.91 (3H, s, $OCH_3$-4'), 4.42 (1H, s, OH-7"), 5.17 (1H, t, J=7.2 Hz, H-2"), 5.42 (1H, m, H-5"), 5.52 (1H, d, J=15.0 Hz, H-6"), 6.68 (1H, d, J=9.0 Hz, H-5'), 6.85 (2H, d, J=9.0 Hz, H-3 and H-5), 7.77 (1H, d, J=15.0 Hz, H-β), 7.78 (2H, d, J=9.0 Hz, H-2 and H-6), 7.83 (1H, d, J=15.0 Hz, H-α), 8.24 (1H, d, J=9.0 Hz, H-6'), 10.17 (1H, s, OH-4), 13.80 (1H, s, OH-2')

Figure 19 shows $^1$H-NMR spectrum.

$^{13}$C-NMR (deuterated dimethyl sulfoxide): δ16.8 ($CH_3$-3"), 22.1 (C-1"), 31.0 ($CH_3$-7"), 31.0 ($CH_3$-7"), 42.7 (C-4"), 56.9 ($OCH_3$-4'), 69.7 (C-7"), 103.6 (C-5'), 114.9 (C-1'), 116.7 (C-3'), 116.7 (C-3 and C-5), 118.1 (C-α), 123.2 (C-2"), 123.9 (C-5"), 126.6 (C-1), 131.3 (C-6'), 132.3 (C-2 and C-6), 134.6 (C-3"), 141.5 (C-6"), 145.7 (C-β), 161.3 (C-4), 163.0 (C-2'), 163.8 (C-4'), 193.3 (C=O)

Figure 20 shows $^{13}$C-NMR spectrum.

As the matrix, FAB-MS: m/z 421 $(M-H)^-$ metanitrobenzyl alcohol was used.

From the above results of the NMR spectrum analysis and mass spectrum analysis, the yellow substance obtained in item (1) of Example 13 was identified to be 1-[2-hydroxy-3-(7-hydroxy-3,7-dimethyl-2,5-octadienyl)-4-methoxyphenyl]-3-(4-hydroxyphenyl)-2-propen-1-one (molecular weight: 422, hereinafter referred to as a compound (C081)).

Example 14 Preparation of Xanthoangelol G

(1) One-hundred milligrams of TB9 obtained in item (1) of Example 12 was dissolved in 50 mL of methanol, and 60 mg of the triphenylphosphine was added to the solution, and the mixture was reacted at room temperature for 1 hour. The reaction mixture was concentrated under reduced pressure, and the concentrate was subjected to thin layer chromatography using chloroform : methanol = 10:1 as a developing solvent. Next, an ultraviolet absorption portion was scraped off, and extracted with the developing solvent, and thereafter the extracted was concentrated to dryness, to give 57.2 mg of xanthoangelol G.

Example 15 Preparation of Xanthoangelol F

The silica fraction numbers 6 to 9 obtained in item (3) of Example 1 were collected and concentrated under reduced pressure, and thereafter the concentrates were dissolved in chloroform. Subsequently, the recrystallization was carried out with hexane, and the formed precipitates and supernatant were separated. The precipitates obtained were dried, to give xanthoangelol F.

Example 16 Preparation of 4'-O-Geranylnaringenin

(1) The supernatant obtained in Example 15 was concentrated under reduced pressure, and thereafter fractionated using reverse phase chromatography. As the column, TSK gel ODS-80Ts (21.5 mm × 30 cm) was used. The elution

was carried out for 45 minutes with distilled water : acetonitrile = 15:85, and a volume ratio of acetonitrile was linearly changed to 100% in the subsequent 50 minutes. The elution rate was 5 mL/minute, and the detection was carried out at 215 nm. The eluates were fractionated using ultraviolet absorption of the eluates as an index.
(2) The reverse phase chromatography fraction 2 (the fraction containing a detection peak at a retention time of 33 minutes) obtained in item (1) of Example 16 were concentrated to dryness, to give 4'-O-geranylnaringenin.

Example 17 Preparation of Lespeol

The reverse phase chromatography fraction 5 (the fraction containing a detection peak at a retention time of 49 minutes) obtained in item (1) of Example 16 were concentrated to dryness, to give lespeol.

Example 18 Preparation of Isobavachalcone

(1) The fraction eluted with the 75% aqueous ethanol solution obtained in item (2) of Example 1 was concentrated under reduced pressure, and adsorbed on a silica gel (BW-300SP: 350 mL). The elution was carried out stepwise with chloroform : hexane at a solvent ratio of 2:1 (2600 mL), and 10:3, 15:1, and 20:1 (600 mL each), 100:1 (1000 mL) and ethyl acetate (500 mL) in that order. The eluates were fractionated every 200 mL.
(2) The fraction numbers 23 to 30 obtained in item (1) of Example 18 were concentrated under reduced pressure, to give isobavachalcone.

Example 19 Preparation of Isobavachin

The silica fraction numbers 108 to 114 obtained in item (1) of Example 3 were collected and concentrated under reduced pressure, to give isobavachin.

Example 20 Preparation of Prostratol F

One-hundred milligrams of the xanthoangelol obtained in item (5) of Example 1 was dissolved in 100 mL of a 2% aqueous sodium hydroxide, and reacted at 50°C for 2 hours. The reaction mixture was neutralized, and thereafter fractionated using reverse phase chromatography. As the resin, Cosmosil 140 C18-OPN (100 mL) was used. The elution was carried out with 200 mL each of a 40% aqueous ethanol solution, a 50% aqueous ethanol solution, and a 60% aqueous ethanol solution in that order. Next, the fraction eluted with the 50% aqueous ethanol solution was concentrated to dryness, to give 63 mg of prostratol F.

Example 21 Preparation of Compound (C082)

(1) One-hundred milligrams of the xanthoangelol F obtained in Example 15 was dissolved in 100 ml of a 2% aqueous sodium hydroxide, and reacted at 50°C for 2 hours. The reaction mixture was neutralized, and thereafter fractionated using reverse phase chromatography. As the resin, Cosmosil 140 C18-OPN (100 ml) was used. The elution was carried out with 200 mL each of a 40% aqueous ethanol solution, a 50% aqueous ethanol solution, a 60% aqueous ethanol solution and a 70% aqueous ethanol solution in that order. The fraction eluted with the 60% aqueous ethanol solution was concentrated to dryness, to give 22 mg of a yellow substance.
(2) The NMR spectra and the mass spectrum of the yellow substance obtained in item (1) of Example 21 were determined in the same manner as in item (2) of Example 4. The signals of NMR are shown below. In addition, the numbers of the peaks are shown in the following formula (Formula 56).

(Formula 56)

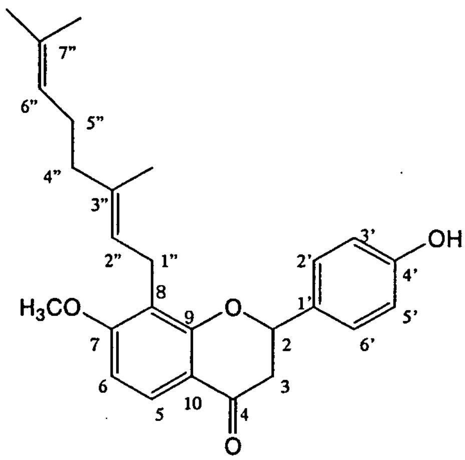

$^1$H-NMR(deuterated dimethyl sulfoxide): δ1.51(3H, s, $CH_3$-7"), 1.57 (3H, s, $CH_3$-3"), 1.58 (3H, s, $CH_3$-7"), 1.88 (2H, m, H-4"), 1.98 (2H, m, H-5"), 2.72 (1H, dd, J=16.8 Hz, J=2.4 Hz, H-3), 3.10 (1H, dd, J=12.6 Hz, J=16.8 Hz, H-3), 3.23 (2H, d, J=6.6 Hz, H-1"), 3.86 (3H, s, $OCH_3$-7), 5.01 (1H, t, J=6.0 Hz, H-6"), 5.09 (1H, t, J=6.6 Hz, H-2"), 5.45 (1H, dd, J=2.4 Hz, J=12.6 Hz, H-2), 6.78 (1H, d, J=8.4 Hz, H-6), 6.79 (2H, d, J=9.0 Hz, H-3' and H-5'), 7.31 (2H, d, J=9.0 Hz, H-2' and H-6'), 7.68. (1H, d, J=8.4 Hz, H-5), 9.54(1H, s, OH-4')

Figure 21 shows $^1$H-NMR spectrum.

$^{13}$C-NMR (deuterated dimethyl sulfoxide): δ 16.7 ($CH_3$-3"), 18.4 ($CH_3$-7"), 22.5 (C-1"), 26.3 ($CH_3$-7"), 27.0 (C-5"), 40.2 (C-4"), 44.0 (C-3), 57.0 ($OCH_3$-7), 79.7 (C-2), 106.0 (C-6), 115.8 (C-10), 115.9 (C-3' and C-5'), 117.5 (C-8), 122.5 (C-2"), 124.8 (C-6"), 126.5 (C-5), 128.8 (C-2' and C-6'), 130.8 (C-1'), 131.5 (C-7"), 135.4 (C-3"), 158.4 (C-4'), 160.5 (C-9), 163.5 (C-7), 191.8 (C-4)

Figure 22 shows $^{13}$C-NMR spectrum.

As the matrix, FAB-MS: m/z 405 $(M-H)^-$ metanitrobenzyl alcohol was used.

From the above results of the NMR spectrum analysis and mass spectrum analysis, the yellow substance obtained in item (1) of Example 21 was identified to be 8-geranyl-4'-hydroxy-7-methoxy-flavanone (molecular weight: 406, hereinafter referred to as a compound (C082)).

Example 22 Preparation of Coumarin Compound A

(1) The fraction eluted with the 40% aqueous ethanol solution obtained in item (2) of Example 1 was concentrated under reduced pressure, and thereafter fractionated using reverse phase chromatography. As the column, TSK gel ODS-80Ts (21.5 mm X 30 cm) was used. The solvent was distilled water : acetonitrile = 1:3, the elution rate was 5 mL/minute, and the detection was carried out at 215 nm. The eluates were fractionated using ultraviolet absorption of the eluates as an index.

(2) The NMR spectra and the mass spectrum of the reverse phase chromatography fraction 5 (fraction containing a peak detection at a retention time of 30.5 minutes) obtained in item (1) of Example 22 were determined in the same manner as in item (2) of Example 4. The signals of NMR are shown below. In addition, the numbers of the peaks are shown in the following formula (Formula 57).

(Formula 57)

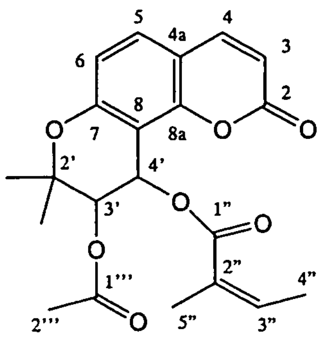

$^1$H-NMR(deuterated dimethyl sulfoxide): δ1.40 (3H, s, $CH_3$-2'), 1.41 (3H, s, $CH_3$-2'), 1.79 (3H, br-t, J=1.5 Hz, H-5"), 1.89 (3H, br-dd, J=1.2 Hz, J=7.2 Hz, H-4"), 2.04 (3H, s, H-2'''), 5.30 (1H, d, J=5.0 Hz, H-3'), 6.08 (1H, br-q, J=7.2 Hz, H-3"), 6.31 (1H, d, J=9.6 Hz, H-3), 6.50 (1H, d, J=5.0 Hz, H-4'), 6.90 (1H, d, J=9.0 Hz, H-6), 7.66 (1H, d, J=9.0 Hz, H-5), 8.00 (1H, d, J=9.6 Hz, H-4)

Figure 23 shows $^1$H-NMR spectrum.

$^{13}$C-NMR (deuterated dimethyl sulfoxide): δ16.0 (C-4"), 20.8 (C-5"), 21.3 ($CH_3$-2'), 23.0 (C-2'''), 25.3 ($CH_3$-2'), 61.1 (C-4'), 70.4 (C-3'), 78.1 (C-2'), 107.4 (C-8), 113.4 (C-4a), 113.4 (C-3), 115.0 (C-6), 128.0 (C-2"), 130.9 (C-5), 138.0 (C-3"), 145.3 (C-4), 154.4 (C-8a), 156.9 (C-7), 160.2 (C-2), 167.1(C-1"), 170.3 (C-1''')

Figure 24 shows $^{13}$C-NMR spectrum.

As the matrix, FAB-MS: m/z 387 $(M+H)^+$metanitrobenzyl alcohol was used.

From the above results of the NMR spectrum analysis and mass spectrum analysis, the reverse phase chromatography fraction 5 was identified to be 3'-acetoxy-4'-angeloyloxy-3',4'-dihydroseselin (molecular weight: 386, hereinafter referred to as Coumarin Compound A).

**Example 23 Preparation of Coumarin Compound B**

The NMR spectra and the mass spectrum of the reverse phase chromatography fraction 7 (fraction containing a peak detection at a retention time of 32.4 minutes) obtained in item (1) of Example 22 were determined in the same manner as in item (2) of Example 4. The signals of NMR are shown below. In addition, the numbers of the peaks are shown in the following formula (Formula 58).

(Formula 58)

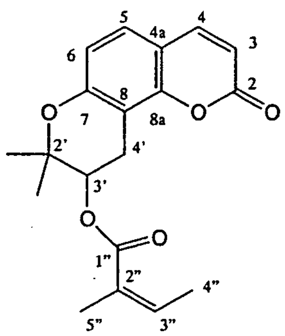

$^1$H-NMR(deuterated dimethyl sulfoxide): δ1.33 (3H, s, $CH_3$-2'), 1.37 (3H, s, $CH_3$-2'), 1.78 (3H, br-t, J=1.5 Hz, H-5"), 1.80 (3H, br-dd, J=1.8 Hz, J=7.2 Hz, H-4"), 2.92 (1H, dd, J=4.2 Hz, J=18.0 Hz, H-4'), 3.20 (1H, dd, J=4.8 Hz, J=18.0 Hz, H-4'), 5.18 (1H, dd, J=4.2 Hz, J=4.8 Hz, H-3'), 6.12 (1H, br q, J=7.2 Hz, H-3"), 6.29 (1H, d, J=9.6 Hz, H-3), 6.85 (1H, d, J=9.0 Hz, H-6), 7.50 (1H, d, J=9.0 Hz, H-5), 7.98 (1H, d, J=9.6 Hz, H-4)

Figure 25 shows $^1$H-NMR spectrum.

$^{13}$C-NMR (deuterated dimethyl sulfoxide): δ16.0 (C-4"), 21.0 (C-5"), 23.3 (C-4'), 24.2 ($CH_3$-2'), 24.9 ($CH_3$-2'), 69.8 (C-3'), 77.4 (C-2'), 107.1 (C-8), 112.8 (C-4a), 112.9 (C-3), 114.5 (C-6), 127.9 (C-2"), 128.1 (C-5), 139.2 (C-3"), 145.5 (C-4), 153.8 (C-7), 156.6 (C-8a), 161.0 (C-2), 167.2 (C-1")

Figure 26 shows $^{13}$C-NMR spectrum.

As the matrix, FAB-MS: m/z 329 $(M+H)^+$ metanitrobenzyl alcohol was used.

From the above results of the NMR spectrum analysis and mass spectrum analysis, the reverse phase chromatography fraction 7 was identified to be 3'-angeloyloxy-3',4'-dihydroseselin (molecular weight: 328, hereinafter referred to as Coumarin Compound B).

Example 24 Preparation of Coumarin Compound C

The NMR spectra and the mass spectrum of the reverse phase chromatography fraction 3 (fraction containing a peak detection at a retention time of 23.9 minutes) obtained in item (1) of Example 22 were determined in the same manner as in item (2) of Example 4. The signals of NMR are shown below. In addition, the numbers of the peaks are shown in the following formula (Formula 59).

(Formula 59)

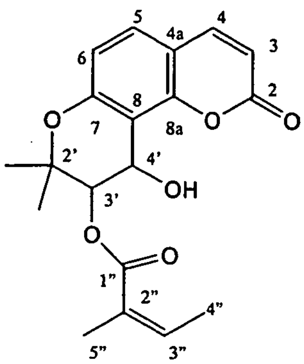

$^{1}$H-NMR(deuterated dimethyl sulfoxide): δ1.39 (3H, s, $CH_3$-2'), 1.43 (3H, s, $CH_3$-2'), 1.91 (3H, br-s, H-5"), 1.98 (3H, br-d, J=7.2 Hz, H-4"), 4.93 (1H, d, J=5.0 Hz, H-4'), 5.24 (1H, t, J=5.0 Hz, H-3'), 5.78 (1H, d, J=5.0 Hz, OH-4'), 6.20 (1H, br-q, J=7.2 Hz, H-3"), 6.31 (1H, d, J=9.6 Hz, H-3), 6.82 (1H, d, J=8.4 Hz, H-6), 7.57 (1H, d, J=8.4 Hz, H-5), 7.99 (1H, d, J=9.6 Hz, H-4)

Figure 27 shows $^{1}$H-NMR spectrum.

As the matrix, FAB-MS: m/z 345 $(M+H)^+$ metanitrobenzyl alcohol was used.

From the above results of the NMR spectrum analysis and mass spectrum analysis, the reverse phase chromatography fraction 3 was identified to be 3'-angeloyloxy-4'-hydroxy-3',4'-dihydroseselin (molecular weight: 344, hereinafter referred to as Coumarin Compound C).

Example 25 Preparation of Xanthohumol

(1) Ten liters of ethanol was added to 1 kg of dry powder of *Humulus lupulus,* and extraction was carried out at room temperature for 2 hours. Next, 5 L of ethanol was added to the extraction residue, and extraction was carried out at room temperature for 30 minutes. The resulting extracts were combined and concentrated under reduced pressure with a rotary evaporator, to give 800 mL of an ethanol extract of *Humulus lupulus.*

(2) Distilled water and hexane were added to the ethanol extract of *Humulus lupulus* obtained in item (1) of Example 25 while mixing, and thereafter the mixture was fractionated into a hexane layer and an aqueous layer. Next, chloroform was added to the aqueous layer while mixing, and the mixture was fractionated into an aqueous layer and a chloroform layer. The chloroform layer was concentrated under reduced pressure, and thereafter the concentrate was dissolved in 60 mL of chloroform.

(3) The chloroform layer obtained in item (2) of Example 25 was adsorbed on a silica gel (BW-300SP, 100 mL). Next, elution was carried out with chloroform (100 mL), and chloroform : ethyl acetate = 1:1 (100 mL) in that order.

(4) Recrystallization was carried out with ethyl acetate and hexane from the fraction eluted with chloroform : ethyl

acetate = 1:1 obtained in item (3) of Example 25, to give a xanthohumol.

Example 26 Preparation of 4,2'-Dihydroxy-4'-methoxychalcone (hereinafter referred to as Compound (C020))

2'-Hydroxy-4'-methoxyacetophenone (manufactured by Aldrich) and 4-hydroxybenzaldehyde (manufactured by Aldrich) were subjected to Claisen condensation, to give a compound (C020). The structure of the compound (C020) was analyzed with nuclear magnetic resonance (NMR) spectrometer (Model AVANCE 600: manufactured by Bruker BIOSPIN), and with a mass spectroscopic (MS) (DX302: manufactured by JEOL LTD.) mass analyzer.
$^1$H-NMR(deuterated dimethyl sulfoxide): δ3.84 (3H, s, $-OCH_3$), 6.50 (1H, d, J=2.4 Hz, H-3'), 6.55 (1H, dd, J=9 Hz, H-5'), 6.86 (2H, d, J=8.4 Hz, H-3,5), 7.79 (4H, m), 8.25 (1H, d, H-6'), 10.17 (1H, s, -OH-4), 13.65 (1H, s, -OH-2')
As the matrix, FAB-MS: m/z 271 $(M+H)^+$ glycerol was used.

Example 27 Preparation of 2'4'-Dihydroxy-5'-prenylacetophenone (hereinafter referred to as Compound (C025)) and 2'-Hydroxy-4'-methoxy-5'-prenylacetophenone(hereinafter referred to as Compound (C027))

2',4'-Dihydroxyacetophenone (manufactured by Wako Pure Chemical Industries, Ltd.) was reacted with 2-methyl-3-buten-2-ol (manufactured by Wako Pure Chemical Industries, Ltd.) in anhydrous dioxane at room temperature for 1 hour under an argon atmosphere in the presence of boron trifluoride•ether complex (manufactured by Wako Pure Chemical Industries, Ltd.), to give a compound (C025).
$^1$H-NMR(deuterated chloroform): δ1.67, 1.68 (6H, 2s, $(C\underline{H}_3)_2C=$), 2.50 (3H, s, $-CO-C\underline{H}_3$), 3.15 (2H, d, J=7.2 Hz, $Ar-C\underline{H}_2-CH=$), 5.27 (1H, m, $-CH_2-(C\underline{H}=$), 6.29 (1H, s, H-5'), 7.53 (1H, s, H-6'), 10.63 (1H, s, -OH-4'), 12.45 (1H, s,-OH-2')
As the matrix, FAB-MS: m/z 221 $(M+H)^+$ glycerol was used.
The compound (C025) was reacted with dimethyl sulfate (manufactured by Wako Pure Chemical Industries, Ltd.) with thermally refluxing in anhydrous acetone in the presence of potassium carbonate, to give a compound (C027).
$^1$H-NMR(deuterated chloroform): δ1.72, 1.77 (6H, 2s, $(C\underline{H}_3)_2C=$), 2.56 (3H, s, $-CO-CH_3$), 3.24 (2H, d, J=7.2 Hz, $Ar-C\underline{H}_2-CH=$), 3.88 (3H, s, $-OCH_3$), 5.27 (1H, m, $-CH_2-C\underline{H}=$), 6.41 (1H, s, H-5'), 7.42 (1H, s, H-6'), 12.72 (1H, s, -OH-2')
As the matrix, FAB-MS: m/z 235 $(M-H)^+$ glycerol was used.

Example 28 Preparation of 2'-Hydroxy-3'-prenyl-4'-methoxyacetophenone (hereinafter referred to as compound (C026))

2',4'-Dihydroxyacetophenone was dissolved in 2M KOH/methanol, and 1-bromo-2-methyl-2-butene (Aldrich) was added thereto while ice-cooling for 15 minutes, and reacted at room temperature for 45 minutes, to give 2',4'-dihydroxy-3'-prenylacetophenone (hereinafter referred to as compound (C024)). The compound (C024) was treated in the same manner as in Example 27, to give a compound (C026).
$^1$H-NMR(deuterated chloroform): δ1.69, 1.80 (6H, 2s, $(C\underline{H}_3)_2C=$), 2.58 (3H, s, $-CO-CH_3$), 3.37 (2H, d, J=7.2 Hz, $Ar-C\underline{H}_2-CH=$), 3.91 (3H, s, $-OCH_3$), 5.22 (1H, m, $-CH_2-CH=$), 6.47 (1H, d, J=9 Hz, H-5'), 7.62 (1H, d, H-6'), 12.75 (1H, s, -OH-2')
As the matrix, FAB-MS: m/z 235 $(M+H)^+$ glycerol was used.

Example 29 Preparation of 2'-Hydroxy-3'-methyl-4'-methoxyacetophenone (hereinafter referred to as compound (C022))

2',4'-Dihydroxy-3'-methylacetophenone (Aldrich) was treated in the same manner as in Example 27, to give a compound (C022).
$^1$H-NMR(deuterated chloroform): δ2.11 (3H, s, $-C\underline{H}_3$), 2.58 (3H, s, $-CO-CH_3$), 3.91 (3H, s, $-OCH_3$), 6.47 (1H, d, J=9 Hz, H-5'), 7.62 (1H, s, H-6'), 12.77 (1H, s, -OH-2')
As the matrix, FAB-MS: m/z 181 $(M+H)^+$ metanitrobenzyl alcohol was used.

Example 30 Preparation of 4,2'-Dihydroxy-3'-methyl-4'-methoxychalcone (hereinafter referred to as compound (C023))

2'-Hydroxy-3'-methyl-4'-methoxyacetophenone (C022) and 4-hydroxybenzaldehyde (Aldrich) were subjected to Claisen condensation, to give a compound (C023).
$^1$H-NMR(deuterated dimethyl sulfoxide): δ2.02 (3H, s, $-CH_3$) 3.91 (3H, s, $-OCH_3$), 6.68 (1H, d, J=9 Hz, H-5'), 6.85 (2H, d, J=8.4 Hz, H-3,5), 7.78 (2H, d, H-2,6), 7.79 (1H, d, J=15.6 Hz, $CO-C\underline{H}=CH-$), 7.83 (1H, d, $CO-CH=C\underline{H}-$), 8.23 (1H, d, H-6'), 10.15 (1H, s, -OH-4), 13.78 (1H, s, -OH-2')
Figure 28 shows $^1$H-NMR spectrum of the compound (C023).
As the matrix, FAB-MS: m/z 285 $(M+H)^+$ metanitrobenzyl alcohol was used.

Example 31 Preparation of 4,2'-Dihydroxy-4'-methoxy-5'-prenylchalcone (Bavachalcone)

2'-Hydroxy-4'-methoxy-5'-prenylacetophenone (C027) and 4-hydroxybenzaldehyde (Aldrich) were subjected to Claisen condensation, to give bavachalcone.
$^1$H-NMR(deuterated dimethyl sulfoxide): δ1.67, 1.71 (6H, 2s, (C$\underline{H}$3)$_2$C=), 3.24 (2H, d, J=7.2 Hz, Ar-C$\underline{H}_2$-CH=), 3.86 (3H, s, -$OCH_3$), 5.23 (1H, m, -$CH_2$-C$\underline{H}$=), 6.52 (1H, s, H-3'), 6.86 (2H, d, J=8.4 Hz, H-3,5), 7.76 (2H, s, CO-C$\underline{H}$=C$\underline{H}$-), 7.77 (2H, d, H-2,6), 8.02 (1H, s, H-6'), 10.14 (1H, s,-OH-4), 13.61(1H, s,-OH-2')
As the matrix, FAB-MS: m/z 339 $(M+H)^+$ metanitrobenzyl alcohol was used.

Example 32 Preparation of 4-Tetrahydropyranyloxy-2'-hydroxy-3'-prenyl-4'-methoxychalcone (hereinafter referred to as compound (C030))

4,2'-Dihydroxy-3'-prenyl-4'-methoxychalcone was dissolved in dichloromethane containing a catalytic amount of pyridinium-p-toluenesulfonate (Tokyo Kasei Kogyo Co., Ltd.), and the mixture was stirred at room temperature for 30 minutes. Thereafter, 3,4-dihydro-2H-pyrane (Tokyo Kasei Kogyo Co., Ltd.) was added to the mixture, and the resulting mixture was stirred at room temperature for additional 3 hours, to give a compound (C030).
$^1$H-NMR(deuterated dimethyl sulfoxide): δ1.50-1.80 (6H, m, THP), 1.62, 1.73 (6H, 2s, (C$\underline{H}_3$)$_2$CH=), 3.27 (2H, d, J=7.2 Hz, Ar-C$\underline{H}_2$-CH=), 3.59 (1H, m, THP), 3.75 (1H, m, THP), 3.91 (3H, s, -$OCH_3$), 5.13 (1H, m, -$CH_2$-C$\underline{H}$=), 5.60 (1H, m, THP), 6.69 (1H, d, J=9.6 Hz, H-5'), 7.11 (2H, d, J=9 Hz, H-3,5), 7.81 (1H, d, J=15 Hz, CO-C$\underline{H}$=CH-), 7.88 (2H, d, H-2,6), 7.92 (1H, d, CO-CH=C$\underline{H}$-), 8.26 (1H, d, H-6'), 13.72 (1H, s, -OH-2')
Figure 29 shows $^1$H-NMR spectrum of the compound (C030).
As the matrix, FAB-MS: m/z 423 $(M+H)^+$ metanitrobenzyl alcohol was used.

Example 33 Preparation of 3.4.2'-Trihydroxy-3'-prenyl-4'-methoxychalcone (hereinafter referred to as compound (C031))

2'-Hydroxy-3'-prenyl-4'-methoxyacetophenone (C026) and 3,4-dihydroxybenzaldehyde (Aldrich) were subjected to Claisen condensation, to give a compound (C031).
$^1$H-NMR(deuterated dimethyl sulfoxide): δ1.62, 1.72 (6H, 2s, (C$\underline{H}_3$)$_2$C=), 3.26 (2H, d, J=6.6 Hz, Ar-C$\underline{H}_2$-CH=), 3.90 (3H, s, -$OCH_3$), 5.13 (1H, m,-$CH_2$-C$\underline{H}$=), 6.67 (1H, d, J=9 Hz, H-5'), 6.82 (1H, d, J=7.8 Hz, H-5), 7.23 (1H, dd, J=1.8 Hz, H-6), 7.30 (1H, d, H-2), 7.70 (1H, d, J=15.6 Hz, CO-C$\underline{H}$=CH-), 7.73 (1H, d, CO-CH=C$\underline{H}$-), 8.21 (1H, d, H-6'), 13.79 (1H, s, -OH-2')
Figure 30 shows $^1$H-NMR spectrum of the compound (C031).
As the matrix, FAB-MS: m/z 355 $(M+H)^+$ metanitrobenzyl alcohol was used.

Example 34 Preparation of 4,2'-Diacetoxy-3'-prenyl-4'-methoxychalcone (hereinafter referred to as compound (C032))

4,2'-Dihydroxy-3'-prenyl-4'-methoxychalcone was reacted with acetic anhydride in dichloromethane for 1 hour, in the presence of triethylamine and a catalytic amount of dimethylaminopyridine (DMAP), to give a compound (C032).
$^1$H-NMR(deuterated dimethyl sulfoxide): δ1.62, 1.71 (6H, 2S, (C$\underline{H}_3$)$_2$C=), 2.24, 2.29 (6H, 2s, -CO-$CH_3$), 3.22 (2H, d, J=7.2 Hz, Ar-C$\underline{H}_2$-CH=), 3.91 (3H, s, -$OCH_3$), 5.03 (1H, m, -$CH_2$-C$\underline{H}$=), 7.03 (1H, d, J=8.4 Hz, H-5'), 7.22 (2H, d, J=8.4 Hz, H-3,5), 7.54 (1H, d, J=15.6 Hz, CO-C$\underline{H}$=CH-), 7.58 (1H, d, CO-CH=C$\underline{H}$-), 7.86 (2H, d, H-2,6), 7.94 (1H, d, H-6')
As the matrix, FAB-MS: m/z 423 $(M+H)^+$ metanitrobenzyl alcohol was used.

Example 35 Preparation of 1-(2-Hydroxy-3-(3-methylbutyl)-4-methoxyphenyl)-3-(4-hydroxyphenyl)-propan-1-one hereinafter referred to as compound (C033))

4,2'-Dihydroxy-3'-prenyl-4'-methoxychalcone was hydrogenated in methanol at room temperature for 30 minutes, in the presence of Palladium Black (manufactured by Nakalai Tesque, Inc.), to give a compound (C033).
$^1$H-NMR(deuterated dimethyl sulfoxide): δ0.89, 0.90 (6H, 2s, (C$\underline{H}_3$)$_2$CH-), 1.29 (2H, m, (C$\underline{H}_3$)$_2$-CH-C$\underline{H}_2$-), 1.50 (1H, m, $(CH_3)_2$C$\underline{H}$-$CH_2$-), 2.55 (2H, m, Ar-C$\underline{H}_2$-$CH_2$-), 2.83 (2H, t, J=7.2 Hz, CO-C$\underline{H}_2$-$CH_2$-), 3.26 (2H, t, CO-$CH_2$-C$\underline{H}_2$-), 3.85 (3H, s, -$OCH_3$), 6.60 (1H, d, J=9 Hz, H-5'), 6.73 (2H, d, J=8.4 Hz, H-3,5), 7.06 (2H, d, H-2,6), 7.84(1H, d, H-6'), 9.15 (1H, s, -OH-4), 12.88 (1H, s,-OH-2')
As the matrix, FAB-MS: m/z 343 $(M+H)^+$ metanitrobenzyl alcohol was used.

Example 36 Preparation of 4,4'-Dimethoxy-2'-hydroxy-3'-prenylchalcone (hereinafter referred to as compound (C035))

4,2'-Dihydroxy-3'-prenyl-4'-methoxychalcone was treated in the same manner as in Example 27 to give a

compound (C035).

$^1$H-NMR(deuterated dimethyl sulfoxide): δ1.62, 1.73 (6H, 2s, ($C\underline{H}_3$)$_2$C=), 3.27 (2H, d, J=7.2 Hz, Ar-C$\underline{H}_2$-CH=), 3.84, 3.91 (6H, 2s, -$OCH_3$), 5.14 (1H, m, -$CH_2$-C$\underline{H}$=), 6.69 (1H, d, J=9.6 Hz, H-5'), 7.04 (2H, d, J=8.4 Hz, H-3,5), 7.83 (1H, d, J=15.6 Hz, CO-C$\underline{H}$=CH-), 7.90 (2H, d, H-2,6), 7.91 (1H, d, CO-CH=C$\underline{H}$-), 8.27 (1H, d, H-6'), 13.74 (1H, s, -OH-2')

As the matrix, FAB-MS: m/z 353 $(M+H)^+$ metanitrobenzyl alcohol was used.

Example 37 Preparation of 3,4-Dihydroxy-2',4'-dichlorochalcone (hereinafter referred to as compound (C011))

2',4'-Dichloroacetophenone (Sigma) and 3,4-dihydroxybenzaldehyde (Tokyo Kasei Kogyo Co., Ltd.) were subjected to Claisen condensation, to give a compound (C011).

$^1$H-NMR(deuterated dimethyl sulfoxide): δ6.76 (1H, d, J=8 Hz, H-5), 6.88 (1H, d, J=15 Hz, CO-C$\underline{H}$=CH-), 7.04 (1H, dd, J=2 Hz, H-6), 7.11 (1H, d, H-2), 7.23 (1H, d, CO-CH=C$\underline{H}$-), 7.55 (2H, m, H-5',6'), 7.74 (1H, s, H-3')

As the matrix, FAB-MS: m/z 309 $(M+H)^+$ metanitrobenzyl alcohol was used.

Example 38 Preparation of 7-Methoxy-8-prenyl-4'-hydroxyflavanone (hereinafter referred to as compound (C034))

The compound (C030) was reacted at 40 degrees in methanol containing 0.5 g/mL sodium hydroxide, to give tetrahydroxypyran (THP) form of a compound (C034). The THP form of the compound (C034) was treated with a catalytic amount of p-toluenesulfonic acid in methanol, to give a compound (C034).

$^1$H-NMR(deuterated dimethyl sulfoxide): δ1.57, 1.59 (6H, 2s, ($C\underline{H}_3$)$_2$C=), 2.72 (1H, dd, J=16.8, 3 Hz, CO-C$\underline{H}_2$-CH), 3.11 (1H, dd, J=12.6 Hz, CO-C$\underline{H}_2$-CH), 3.23 (2H, d, J=7.2 Hz, Ar-C$\underline{H}_2$-CH=), 3.86 (3H, s, -$OCH_3$), 5.09 (1H, m, -$CH_2$-C$\underline{H}$=), 5.47 (1H, dd, CO-$CH_2$-C$\underline{H}$), 6.79 (3H, m, H-3,5,5'), 7.32 (2H, d, J=9 Hz, H-2,6), 7.68 (1H, d, J=9 Hz, H-6'), 9.53 (1H, s, -OH-4)

As the matrix, FAB-MS: m/z 339 $(M+H)^+$ metanitrobenzyl alcohol was used.

Example 39 Preparation of 2',4'-Dihydroxy-3'-geranylacetophenone (hereinafter referred to as compound (C036)) and 2'-Hydroxy-3'-geranyl-4'-methoxyacetophenone (hereinafter referred to as compound (C038))

2',4'-Dihydroxyacetophenone (Wako Pure Chemical Industries, Ltd.) and geranyl bromide (Aldrich) were treated in the same manner as in Example 28, to give 2',4'-dihydroxy-3'-geranylacetophenone (C036).

$^1$H-NMR(deuterated dimethyl sulfoxide): δ1.61, 1.69, 1.84 (9H, 3s, ($C\underline{H}_3$)$_2$C=, -C(C$\underline{H}_3$)=CH-), 2.11 (4H, m, -$CH_2$-C$\underline{H}_2$-C($CH_3$)=, =CH-C$\underline{H}_2$-$CH_2$-), 2.57 (3H, s, -CO-C$\underline{H}_3$), 3.47 (2H, d, J=7.2 Hz, Ar-C$\underline{H}_2$-CH=), 5.07 (1H, m, ($CH_3$)$_2$C=C$\underline{H}$-$CH_2$-), 5.29 (1H, m, -C($CH_3$)=C$\underline{H}$-), 6.40 (1H, d, J=9 Hz, H-5'), 7.56 (1H, d, H-6'), 13.13 (1H, s, -OH-2')

As the matrix, FAB-MS: m/z 289 $(M+H)^+$ metanitrobenzyl alcohol was used.

The compound (C036) was treated with dimethyl sulfate (Wako Pure Chemical Industries, Ltd.) in the same manner as in Example 27, to give a compound (C038).

$^1$H-NMR(deuterated dimethyl sulfoxide): δ1.50, 1.57, 1.70 (9H, 3s, ($C\underline{H}_3$)$_2$C=, -C(C$\underline{H}_3$)=CH-), 1.89 (2H, m, -$CH_2$-C$\underline{H}_2$-C($CH_3$)=), 1.98 (2H, m, =CH-C$\underline{H}_2$-$CH_2$-), 2.57 (3H, s, -CO-$CH_3$), 3.23 (2H, d, J=7.2 Hz, Ar-C$\underline{H}_2$-CH=), 3.89 (3H, s, -$OCH_3$), 5.00 (1H, m, ($CH_3$)$_2$C=C$\underline{H}$-$CH_2$-), 5.10 (1H, m, -C($CH_3$)=C$\underline{H}$-), 6.64 (1H, d, J=9 Hz, H-5'), 7.82 (1H, d, H-6'), 12.84 (1H, s, -OH-2')

As the matrix, FAB-MS: m/z 303 $(M+H)^+$ metanitrobenzyl alcohol was used.

Example 40 Preparation of 2'-Hydroxy-3'-(3-methylbutyl)-4'-methoxyacetophenone (hereinafter referred to as compound (C041))

2',4'-Dihydroxy-3'-prenylacetophenone (C024) was treated in the same manner as in Example 35 to give 2',4'-dihydroxy-3'-(3-methylbutyl)acetophenone (C040). The compound (C040) was treated in the same manner as in Example 27, to give a compound (C041).

$^1$H-NMR(deuterated dimethyl sulfoxide): δ0.89, 0.90 (6H, 2s, ($C\underline{H}_3$)$_2$CH-), 1.29 (2H, m, ($CH_3$)$_2$CH-C$\underline{H}_2$-), 1.50 (1H, m, ($CH_3$)$_2$C$\underline{H}$-$CH_2$-), 2.55 (2H, m, Ar-C$\underline{H}_2$-$CH_2$-), 2.57 (3H, s, CO-$CH_3$), 3.87 (3H, s, -$OCH_3$), 6.64 (1H, d, J=9 Hz, H-5'), 7.81 (1H, d, H-6'), 12.83 (1H, s,-OH-2')

Figure 31 shows $^1$H-NMR spectrum of the compound (C041).

As the matrix, FAB-MS: m/z 237 $(M+H)^+$ metanitrobenzyl alcohol was used.

Example 41 Preparation of 4,2'-Dihydroxy-3'-(3-methylbutyl)-4'-methoxychalcone (hereinafter referred to as compound (C043))

2'-Hydroxy-3'-(3-methylbutyl)-4'-methoxyacetophenone (C041) and 4-hydroxybenzaldehyde (Aldrich) were

subjected to Claisen condensation, to give a compound (C043).

$^1$H-NMR(deuterated dimethyl sulfoxide): 80.91, 0.92 (6H, 2s, $(C\underline{H}_3)_2CH$-), 1.32 (2H, m, $(CH_3)_2CH$-$C\underline{H}_2$-), 1.53 (1H, m, $(CH_3)_2C\underline{H}$-$CH_2$-), 2.58 (2H, m, Ar-$C\underline{H}_2$-$CH_2$-), 3.90 (3H, s, -$OCH_3$), 6.67 (1H, d, J=9 Hz, H-5'), 6.84 (2H, d, J=9 Hz, H-3,5), 7.78 (2H, d, H-2,6), 7.78 (1H, d, J=15 Hz, CO-C$\underline{H}$=CH-), 7.83 (1H, d, CO-CH=C$\underline{H}$-), 8.22 (1H, d, H-6'), 10.14 (1H, s, -OH-4), 13.76 (1H, s, -OH-2')

Figure 32 shows $^1$H-NMR spectrum of the compound (C043).

As the matrix, FAB-MS: m/z 341 $(M+H)^+$ metanitrobenzyl alcohol was used.

Example 42 Preparation of 2'-Hydroxy-3'-prenyl-4'-tetrahydropyranyloxyacetophenone (hereinafter referred to as compound (C044))

2',4'-Dihydroxy-3'-prenylacetophenone (C024) was treated in the same manner as in Example 32 to give a compound (C044).

$^1$H-NMR(deuterated dimethyl sulfoxide): δ1.50-1.90 (6H, m, THP), 1.62, 1.73 (6H, 2s, $(C\underline{H}_3)_2C$=), 2.57 (3H, s, -CO-$C\underline{H}_3$), 3.28 (1H, m, Ar-$C\underline{H}_2$-CH=), 3.33 (1H, m, Ar-$C\underline{H}_2$-CH=), 3.57 (1H, m, THP), 3.65 (1H, m, THP), 5.17 (1H, m, -$CH_2$-C$\underline{H}$=), 5.68 (1H, m, THP), 6.72 (1H, d, J=9 Hz, H-5'), 7.77 (1H, d, H-6'), 12.89 (1H, s, -OH-2')

Figure 33 shows $^1$H-NMR spectrum of the compound (C044).

As the matrix, FAB-MS: m/z 305 $(M+H)^+$ metanitrobenzyl alcohol was used.

Example 43 Preparation of 2'-Hydroxy-3'-geranyl-4'-tetrahydropyranyloxyacetophenone (hereinafter referred to as compound (C045))

2',4'-Dihydroxy-3'-geranylacetophenone (C036) was treated in the same manner as in Example 42, to give a compound (C045).

$^1$H-NMR(deuterated dimethyl sulfoxide): δ1.50-1.90 (6H, m, THP), 1.51, 1.58, 1.73 (9H, 3s, (C$\underline{H3}$)$_2$C=, -C($C\underline{H}_3$)=CH-), 1.91 (2H, m, -$CH_2$-$C\underline{H}_2$-C($CH_3$)=), 1.99 (2H, m, =CH-$C\underline{H}_2$-$CH_2$-), 2.57 (3H, s, -CO-$CH_3$), 3.28 (1H, m, Ar-$C\underline{H}_2$-CH=), 3.33 (1H, m, Ar-$C\underline{H}_2$-CH=), 3.57 (1H, m, THP), 3.66 (1H, m, THP), 5.02 (1H, m, $(CH_3)_2C$=C$\underline{H}$-$CH_2$-), 5.17 (1H, m, -C($CH_3$)=C$\underline{H}$-), 5.69 (1H, m, THP), 6.72 (1H, d, J=9 Hz, H-5'), 7.78 (1H, d, H-6'), 12.89 (1H, s, -OH-2')

Figure 34 shows $^1$H-NMR spectrum of the compound (C045).

As the matrix, FAB-MS: m/z 373 $(M+H)^+$ metanitrobenzyl alcohol was used.

Example 44 Preparation of 4,4', 6'-Trimethoxy-2'-hydroxy-3'-prenylchalcone (hereinafter referred to as compound (C046))

Xanthohumol (4,2',4'-trihydroxy-3'-prenyl-6'-methoxychalcone) was treated in the same manner as in Example 27, to give a compound (C046).

$^1$H-NMR(deuterated dimethyl sulfoxide): δ1.61, 1.70 (6H, 2s, $(C\underline{H}_3)_2C$=), 3.17 (2H, d, J=7.2 Hz, Ar-$CH_2$-CH=), 3.82, 3.92, 3.99 (9H, 3s, -$OCH_3$), 5.10 (1H, m, -$CH_2$-C$\underline{H}$=), 6.28 (1H, s, H-5'), 7.03 (2H, d, J=9 Hz, H-3,5), 7.70 (2H, d, H-2,6), 7.72 (1H, d, J=15 Hz, CO-C$\underline{H}$=CH-), 7.79 (1H, d, CO-CH=C$\underline{H}$-), 14.11 (1H, s, -OH-2')

As the matrix, FAB-MS: m/z 383 $(M+H)^+$ metanitrobenzyl alcohol was used.

Example 45 Preparation of 2'-Hydroxy-3'-prenyl-4'-ethoxyacetophenone (hereinafter referred to as compound (C047))

2',4'-Dihydroxy-3'-prenylacetophenone (C024) was treated with diethyl sulfate (Nakalai Tesque, Inc.) in the same manner as in Example 27, to give a compound (C047).

$^1$H-NMR (deuterated chloroform): δ1.46 (3H, t, J=7.2 Hz, $C\underline{H}_3$-$CH_2O$-), 1.69, 1.81 (6H, 2s, $(C\underline{H}_3)_2$-CH=), 2.57 (3H, s, -CO-$CH_3$), 3.38 (2H, d, J=7.2 Hz, Ar-$C\underline{H}_2$-CH=), 4.13 (2H, tt, J=7.2 Hz, $CH_3$-$C\underline{H}_2O$-), 5.24 (1H, m, -$CH_2$-C$\underline{H}$=), 6.45 (1H, d, J=9 Hz, H-5'), 7.60 (1H, d, H-6'), 12.76 (1H, s, -OH-2')

Figure 35 shows $^1$H-NMR spectrum of the compound (C047).

As the matrix, FAB-MS: m/z 249 $(M+H)^+$ metanitrobenzyl alcohol was used.

Example 46 Preparation of 2'-Hydroxy-3'-geranyl-4'-ethoxyacetophenone (hereinafter referred to as compound (C048))

2',4'-Dihydroxy-3'-geranylacetophenone (C036) was treated in the same manner as in Example 45, to give a compound (C048).

$^1$H-NMR(deuterated chloroform): δ1.46 (3H, t, J=7.2 Hz, $C\underline{H}_3$-$CH_2O$-), 1.58, 1.65, 1.80 (9H, 3s, $(C\underline{H}_3)_2C$=, -C($C\underline{H}_3$)=CH-), 1.97 (2H, m, -$CH_2$-$C\underline{H}_2$-C($CH_3$)=), 2.07 (2H, m, =CH-$C\underline{H}_2$-$CH_2$-), 2.57 (3H, s, -CO-$CH_3$), 3.39 (2H, d, J=7.2 Hz, Ar-$C\underline{H}_2$-CH=), 4.13 (2H, tt, J=7.2 Hz, $CH_3$-$C\underline{H}_2O$-), 5.08 (1H, m, $(CH_3)_2C$=C$\underline{H}$-$CH_2$-), 5.24 (1H, m,

$-C(CH_3)=C\underline{H}-$), 6.45 (1H, d, J=9 Hz, H-5'), 7.60 (1H, d, H-6'), 12.76 (1H, s, -OH-2')

Figure 36 shows $^1$H-NMR spectrum of the compound (C048).

As the matrix, FAB-MS: m/z 317 $(M+H)^+$ metanitrobenzyl alcohol was used.

Example 47 Preparation of 4,2'-Dihydroxy-3'-prenyl-4'-ethoxychalcone (hereinafter referred to as compound (C049))

2'-Hydroxy-3'-prenyl-4'-ethoxyacetophenone (C047) and 4-hydroxybenzaldehyde (Aldrich) were subjected to Claisen condensation, to give a compound (C049).

$^1$H-NMR(deuterated dimethyl sulfoxide):
δ1.37 (3H, t, J=7.2 Hz, $C\underline{H}_3$-$CH_2O$-), 1.62, 1.74 (6H, 2s, $(C\underline{H}_3)_2C$=), 3.27 (2H, d, J=7.2 Hz, Ar-$C\underline{H}_2$-CH=), 4.17 (2H, tt, J=7.2 Hz, $CH_3$-$C\underline{H}_2O$-), 5.16 (1H, m, -$CH_2$-C$\underline{H}$=), 6.64 (1H, d, J=9 Hz, H-5'), 6.85 (2H, d, J=8.4 Hz, H-3,5), 7.78 (2H, d, H-2,6), 7.78 (1H, d, J=15 Hz, CO-C$\underline{H}$=CH-), 7.82 (1H, d, CO-CH=C$\underline{H}$-), 8.20 (1H, d, H-6'), 10.14 (1H, s, -OH-4), 13.79 (1H, s, -OH-2')

Figure 37 shows $^1$H-NMR spectrum of the compound (C049).

As the matrix, FAB-MS: m/z 353 $(M+H)^+$ metanitrobenzyl alcohol was used.

Example 48 Preparation of 4,2'-Dihydroxy-3'-geranyl-4'-ethoxychalcone (hereinafter referred to as compound (C050))

2'-Hydroxy-3'-geranyl-4'-ethoxyacetophenone (C048) and 4-hydroxybenzaldehyde (Aldrich) were subjected to Claisen condensation, to give a compound (C050).

$^1$H-NMR(deuterated dimethyl sulfoxide):
δ1.36 (3H, t, J=7.2 Hz, $C\underline{H}_3$-$CH_2O$-), 1.51, 1.57, 1.73 (9H, 3s, $(C\underline{H}_3)_2C$=, -$C(C\underline{H}_3)$=CH-), 1.91 (2H, m, -$CH_2$-$C\underline{H}_2$-$C(CH_3)$=), 2.00 (2H, m, =CH-$C\underline{H}_2$-$CH_2$-), 3.27 (2H, d, J=7.2 Hz, Ar-$C\underline{H}_2$-CH=), 4.17 (2H, tt, J=7.2 Hz, $CH_3$-$C\underline{H}_2O$-), 5.01 (1H, m, $(CH_3)_2C$=C$\underline{H}$-$CH_2$-), 5.16 (1H, m, -$C(CH_3)$=C$\underline{H}$-), 6.65 (1H, d, J=9 Hz, H-5'), 6.85 (2H, d, J=9 Hz, H-3,5), 7.78 (2H, d, H-2,6), 7.78 (1H, d, J=15 Hz, CO-C$\underline{H}$=CH-), 7.82 (1H, d, CO-CH=C$\underline{H}$-), 8.21 (1H, d, H-6'), 10.14 (1H, s, -OH-4), 13.81 (1H, s, -OH-2')

Figure 38 shows $^1$H-NMR spectrum of the compound (C050).

As the matrix, FAB-MS: m/z 421 $(M+H)^+$ metanitrobenzyl alcohol was used.

Example 49 Preparation of 2',4'-Dihydroxy-3'-farnesylacetophenone (hereinafter referred to as compound (C051))

2',4'-Dihydroxyacetophenone (Wako Pure Chemical Industries, Ltd.) and farnesyl bromide (Aldrich) were treated in the same manner as in Example 28, to give a compound (C051).

$^1$H-NMR(deuterated dimethyl sulfoxide):
δ1.51, 1.53, 1.62, 1.71 (12H, 4s, $(C\underline{H}_3)_2C$=, -$C(C\underline{H}_3)$=CH-), 1.80-2.10 (8H, m,), 2.50 (3H, s, -CO-$CH_3$), 3.21 (2H, d, J=7.2 Hz, Ar-$C\underline{H}_2$-CH=), 5.02 (2H, m, -$CH_2$-$CH_2$-C$\underline{H}$=), 5.14 (1H, m, Ar-$CH_2$-C$\underline{H}$=), 6.44 (1H, d, J=9 Hz, H-5'), 7.62 (1H, d, H-6'), 10.50 (1H, s, -OH-4'), 13.03 (1H, s, -OH-2')

As the matrix, FAB-MS: m/z 357 $(M+H)^+$ metanitrobenzyl alcohol was used.

Example 50 Preparation of 2'-Hydroxy-3'-farnesyl-4'-methoxyacetophenone (hereinafter referred to as compound (C052))

2',4'-Dihydroxy-3'-farnesylacetophenone (compound (C051)) was treated with dimethyl sulfate (Wako Pure Chemical Industries, Ltd.) in the same manner as in Example 28, to give a compound (C052).

$^1$H-NMR(deuterated dimethyl sulfoxide):
δ1.49), 1.52, 1.61, 1.71 (12H, 4s, $(C\underline{H}_3)_2C$=, -$C(C\underline{H}_3)$=CH-), 1.80-2.10 (8H, m,), 2.56 (3H, s, -CO-$CH_3$), 3.24 (2H, d, J=7.2 Hz, Ar-$C\underline{H}_2$-CH=), 3.86 (3H, s, -$OCH_3$), 5.01 (2H, m, -$CH_2$-$CH_2$-C$\underline{H}$=), 5.09 (1H, m, Ar-$CH_2$-C$\underline{H}$=), 6.64 (1H, d, J=9 Hz, H-5'), 7.81 (1H, d, H-6'), 12.83 (1H, s, -OH-2')

Figure 39 shows $^1$H-NMR spectrum of the compound (C052).

As the matrix, FAB-MS: m/z 371 $(M+H)^+$ metanitrobenzyl alcohol was used.

Example 51 Preparation of 4,2'-Dihydroxy-3'-farnesyl-4'-methoxychalcone (hereinafter referred to as compound (C053))

2'-Hydroxy-3'-farnesyl-4'-methoxyacetophenone (compound (C052)) and 4-hydroxybenzaldehyde (Aldrich) were subjected to Claisen condensation, to give a compound (C053).

$^1$H-NMR(deuterated dimethyl sulfoxide): δ1.50, 1.51, 1.59, 1.73 (12H, 4s, $(C\underline{H}_3)_2C$=, -$C(C\underline{H}_3)$=CH-), 1.80-2.10 (8H, m), 3.27 (2H, d, J=7.2 Hz, Ar-$C\underline{H}_2$-CH=), 3.90 (3H, s, -$OCH_3$), 5.00 (2H, m, -$CH_2$-$CH_2$-C$\underline{H}$=), 5.13 (1H, m,

Ar-$CH_2$-C<u>H</u>=), 6.67 (1H, d, J=9 Hz, H-5'), 6.85 (2H, d, J=9 Hz, H-3,5), 7.78 (2H, d, H-2,6), 7.78 (1H, d, J=15 Hz, CO-C<u>H</u>=CH-), 7.82 (1H, d, CO-CH=C<u>H</u>-), 8.23 (1H, d, H-6'), 13.79 (1H, s, -OH-2')

Figure 40 shows $^1$H-NMR spectrum of the compound (C053).

As the matrix, FAB-MS: m/z 475 $(M+H)^+$ metanitrobenzyl alcohol was used.

<u>Example 52 Preparation of 2',4'-Dihydroxy-3'-benzylacetophenone (hereinafter referred to as compound (C054))</u>

2',4'-Dihydroxyacetophenone (Wako Pure Chemical Industries, Ltd.) and benzyl bromide (Wako Pure Chemical Industries, Ltd.) were treated in the same manner as in Example 28, to give a compound (C054).

$^1$H-NMR(deuterated dimethyl sulfoxide): δ2.53 (3H, s, -CO-$CH_3$), 3.86 (2H, s, -C<u>H</u>$_2$-$C_6H_5$,), 6.49 (1H, d, J=9 Hz, H-5'), 7.00-7.30 (5H, m, -$C_6H_5$), 7.69 (1H, d, H-6'), 10.68 (1H, s, -OH-4'), 13.13 (1H, s, -OH-2')

As the matrix, FAB-MS: m/z 243 $(M+H)^+$ metanitrobenzyl alcohol was used.

<u>Example 53 Preparation of 2'-Hydroxy-3'-benzyl-4'-methoxyacetophenone (hereinafter referred to as compound (C055))</u>

2',4'-Dihydroxy-3'-benzylacetophenone (C054) was treated with dimethyl sulfate (Wako Pure Chemical Industries, Ltd.) in the same manner as in Example 27, to give a compound (C055).

$^1$H-NMR(deuterated dimethyl sulfoxide): δ2.59 (3H, s, -CO-$CH_3$), 3.86 (5H, s, -C<u>H</u>$_2$-$C_6H_5$, -$OCH_3$), 6.71 (1H, d, J=9 Hz, H-5'), 7.10-7.30 (5H, m, -$C_6H_5$), 7.89 (1H, d, H-6'), 12.93 (1H, s,-OH-2')

As the matrix, FAB-MS: m/z 257 $(M+H)^+$ metanitrobenzyl alcohol was used.

<u>Example 54 Preparation of 4,2'-Dihydroxy-3'-benzyl-4'-methoxychalcone (hereinafter referred to as compound (C056))</u>

2'-Hydroxy-3'-benzyl-4'-methoxyacetophenone (C054) and 4-hydroxybenzaldehyde (Aldrich) were subjected to Claisen condensation, to give a compound (C056).

$^1$H-NMR (deuterated dimethyl sulfoxide): δ3.92 (5H, s, -C<u>H</u>$_2$-$C_6H_5$, -$OCH_3$), 6.73 (1H, d, J=9 Hz, H-5'), 6.85 (2H, d, J=8.4 Hz, H-3,5), 7.10-7.30 (5H, m, -$C_6H_5$), 7.79 (2H, d, H-2,6), 7.80 (1H, d, J=15.6 Hz, CO-C<u>H</u>=CH-), 7.85 (1H, d, CO-CH=C<u>H</u>-), 8.30 (1H, d, H-6'), 10.15 (1H, s, -OH-4), 13.90 (1H, s, -OH-2')

Figure 41 shows $^1$H-NMR spectrum of the compound (C056).

As the matrix, FAB-MS: m/z 361 $(M+H)^+$ metanitrobenzyl alcohol was used.

<u>Example 55 Preparation of 4,2',4'-Trihydroxy-3'-benzylchalcone (hereinafter referred to as compound (C057))</u>

2',4'-Dihydroxy-3'-benzylacetophenone (C053) and 4-hydroxybenzaldehyde (Aldrich) were subjected to Claisen condensation, to give a compound (C057).

$^1$H-NMR(deuterated dimethyl sulfoxide): δ3.89 (2H, s, -C<u>H</u>$_2$-$C_6H_5$), 6.52 (1H, d, J=9 Hz, H-5'), 6.84 (2H, d, J=8.4 Hz, H-3,5), 7.10-7.30 (5H, m, -$C_6H_5$), 7.75 (2H, d, H-2,6), 7.76 (2H, s, CO-C<u>H</u>=C<u>H</u>-), 8.10 (1H, d, H-6'), 14.10 (1H, s, -OH-2')

Figure 42 shows $^1$H-NMR spectrum of the compound (C057).

As the matrix, FAB-MS: m/z 347 $(M+H)^+$ metanitrobenzyl alcohol was used.

<u>Example 56 Preparation of 4,2',4'-Trihydroxy-3'-farnesylchalcone (hereinafter referred to as compound (C058))</u>

2',4'-Dihydroxy-3'-farnesylacetophenone (C051) and 4-hydroxybenzaldehyde (Aldrich) were subjected to Claisen condensation, to give a compound (C058).

$^1$H-NMR(deuterated dimethyl sulfoxide): δ1.51, 1.52, 1.59, 1.73 (12H, 4s, (C<u>H</u>$_3$)$_2$C=, -C(C<u>H</u>$_3$)=CH-), 1.80-2.10 (8H, m), 3.23 (2H, d, J=7.2 Hz, Ar-C<u>H</u>$_2$-CH=), 5.03 (2H, m, -$CH_2$-$CH_2$-C<u>H</u>=), 5.18 (1H, m, Ar-$CH_2$-C<u>H</u>=), 6.45 (1H, d, J=9 Hz, H-5'), 6.83 (2H, d, J=8.4 Hz, H-3,5), 7.73 (2H, s, CO-C<u>H</u>=C<u>H</u>-), 7.74 (2H, d, H-2,6), 8.01 (1H, d, H-6'), 14.01 (1H, s, -OH-2')

Figure 43 shows $^1$H-NMR spectrum of the compound (C058).

As the matrix, FAB-MS: m/z 461 $(M+H)^+$ metanitrobenzyl alcohol was used.

<u>Example 57 Preparation of 2'-Methyl-4'-prenyloxyacetophenone (hereinafter referred to as compound (C059))</u>

2'-Methyl-4'-hydroxyacetophenone (Wako Pure Chemical Industries, Ltd.) and prenyl bromide (Aldrich) were treated in the same manner as in Example 28, to give a compound (C059).

$^1$H-NMR(deuterated dimethyl sulfoxide): δ1.71,1.74 (6H, 2s, (C<u>H</u>$_3$)$_2$C=), 2.45 (3H, s, Ar-$CH_3$), 2.49 (3H, s,

$-OCH_3$), 4.58 (1H, d, J=6.6 Hz, Ar-$C\underline{H}_2$-CH=), 5.42 (1H, m, Ar-$CH_2$-CH=), 6.82 (1H, d, J=3 Hz, H-3'), 6.84 (1H, dd, J=9 Hz, H-5'), 7.83 (1H, d, H-6')

Figure 44 shows $^1$H-NMR spectrum of the compound (C059).

As the matrix, FAB-MS: m/z 219 $(M+H)^+$ metanitrobenzyl alcohol was used.

Example 58 Preparation of 4,2',4'-Triacetoxy-3'-geranylchalcone (hereinafter referred to as compound (C061))

4,2',4'-Trihydroxy-3'-geranylchalcone (xanthoangelol) was treated with acetic anhydride (Nakalai Tesque, Inc.) in dichloromethane in the presence of triethylamine (Nakalai Tesque, Inc.) and a catalytic amount of dimethylaminopyridine (Wako Pure Chemical Industries, Ltd.), to give a compound (C061).

$^1$H-NMR(deuterated dimethyl sulfoxide):
δ1.53, 1.60, 1.70 (9H, 3s, $(C\underline{H}_3)_2C$=, -C($C\underline{H}_3$)=CH-), 1.91 (2H, m, $-CH_2$-$C\underline{H}_2$-C($CH_3$)=), 2.00 (2H, m, =CH-$C\underline{H}_2$-$CH_2$-), 2.21, 2.29, 2.32 (9H, 3s, -OAc), 3.21 (2H, d, J=6.6 Hz, Ar-$C\underline{H}_2$-CH=), 4.93 (1H, m, $(CH_3)_2C$=C$\underline{H}$-$CH_2$-), 5.03 (1H, m, -C($CH_3$)=C$\underline{H}$-), 7.22 (2H, d, J=8.4 Hz, H-3,5), 7.24 (1H, d, J=9 Hz, H-5'), 7.47 (1H, d, J=15.6 Hz, CO-C$\underline{H}$=CH-), 7.59 (1H, d, CO-CH=C$\underline{H}$-), 7.85 (1H, d, H-6'), 7.87 (2H, d, H-2,6)

Figure 45 shows $^1$H-NMR spectrum of the compound (C061).

As the matrix, FAB-MS: m/z 519 $(M+H)^+$ metanitrobenzyl alcohol was used.

Example 59 Preparation of 2-Hydroxy-3-prenyl-4-methoxybenzaldehyde (hereinafter referred to as compound (C063))

2',4'-Dihydroxybenzaldehyde (Wako Pure Chemical Industries, Ltd.) and prenyl bromide (Aldrich) were treated in the same manner as in Example 28, to give 2',4'-dihydroxy-3'-prenylbenzaldehyde (C062). The compound (C062) was treated with dimethyl sulfate (Wako Pure Chemical Industries, Ltd.) in the same manner as in Example 27, to give a compound (C063).

$^1$H-NMR(deuterated dimethyl sulfoxide): δ1.60, 1.70 (6H, 2s, $(C\underline{H}_3)_2C$=), 3.23 (2H, d, J=7.2 Hz, Ar-$C\underline{H}_2$-CH=), 3.89 (3H, s, $-OCH_3$), 5.11 (1H, m, Ar-$CH_2$-C$\underline{H}$=), 6.76 (1H, d, J=9 Hz, H-5), 7.63 (1H, d, H-6), 9.80 (1H, s, -CHO), 11.43 (1H, s, -OH-2)

As the matrix, FAB-MS: m/z 221 $(M+H)^+$ metanitrobenzyl alcohol was used.

Example 60 Preparation of 4,2'-Diacetoxy-3'-geranyl-4'-methoxychalcone (hereinafter referred to as compound (C064-1)) and 2'-Acetoxy-3'-geranyl-4-hydroxy-4'-methoxychalcone (hereinafter referred to as compound (C064-2))

4,2'-Dihydroxy-3'-geranyl-4'-methoxychalcone (xanthoangelol F) was treated with acetic anhydride (Nakalai Tesque, Inc.) in dichloromethane in the presence of triethylamine (Nakalai Tesque, Inc.) and a catalytic amount of dimethylaminopyridine (Wako Pure Chemical Industries, Ltd.), to give a compound (C064-1) and a compound (C064-2).

Compound (C064-1)

$^1$H-NMR(deuterated dimethyl sulfoxide):
δ1.52, 1.59, 1.71 (9H, 3s, $(C\underline{H}_3)_2C$=, -C($CH_3$)=CH-), 1.90 (2H, m, $-CH_2$-$C\underline{H}_2$-C($CH_3$)=), 2.00 (2H, m, =CH-$C\underline{H}_2$-$CH_2$-), 2.23, 2.28 (6H, 2s, -OAc), 3.22 (2H, d, J=7.2 Hz, Ar-$C\underline{H}_2$-CH=), 3.92 (3H, s, $-OC\underline{H}_3$), 5.01 (1H, m, $(CH_3)_2C$=C$\underline{H}$-$CH_2$-), 5.02 (1H, m, -C($CH_3$)=C$\underline{H}$-), 7.04 (1H, d, J=9 Hz, H-5'), 7.21 (2H, d, J=8.4 Hz, H-3,5), 7.53 (1H, d, J=15.6 Hz, CO-C$\underline{H}$=CH-), 7.57 (1H, d, CO-CH=C$\underline{H}$-), 7.86 (2H, d, H-2,6), 7.94 (1H, d, H-6')

Figure 46 shows $^1$H-NMR spectrum of the compound (C064-1).

As the matrix, FAB-MS: m/z 491 $(M+H)^+$ metanitrobenzyl alcohol was used.

Compound (C064-2)

$^1$H-NMR(deuterated chloroform): δ1.59 (3H, s, $(C\underline{H}_3)_2C$=), 1.67 (3H, s, $(C\underline{H}_3)_2C$=), 1.77 (3H, s, -C($C\underline{H}_3$)=CH-), 1.98 (2H, m, $-CH_2$-$C\underline{H}_2$-C($CH_3$)=), 2.06 (2H, m, =CH-$C\underline{H}_2$-$CH_2$-), 2.31 (3H, s, -OAc), 3.34 (2H, m, Ar-$C\underline{H}_2$-CH=), 3.92 (3H, s, $-OC\underline{H}_3$), 5.08 (1H, m, $(CH_3)_2C$=C$\underline{H}$-$CH_2$-), 5.12 (1H, m, -C($CH_3$)=C$\underline{H}$-), 6.03 (1H, s, -OH-4), 6.84 (3H, m, H-3,5, 5'), 7.10 (1H, d, J=15.6 Hz, CO-C$\underline{H}$=CH-), 7.45 (2H, d, J=8.4 Hz, H-2,6), 7.57 (1H, d, J=15.6 Hz, CO-CH=C$\underline{H}$-), 7.68 (1H, d, J=8.4 Hz, H-6')

Figure 64 shows $^1$H-NMR spectrum of the compound (C064-2).

As the matrix, FAB-MS: m/z 449 $(M+H)^+$ metanitrobenzyl alcohol was used.

Example 61 Preparation of 2'-Benzyloxyacetophenone (hereinafter referred to as compound (C065))

2'-Hydroxyacetophenone (Aldrich) and benzyl bromide (Wako Pure Chemical Industries, Ltd.) were thermally refluxed in acetone in the presence of potassium carbonate, to give a compound (C065).

$^1$H-NMR(deuterated dimethyl sulfoxide): δ2.51 (3H, s, -CO-$CH_3$), 5.23 (2H, s, -C$\underline{H}_2$-$C_6H_5$), 7.03 (1H, t, J=7.2 Hz, H-5'), 7.25 (1H, d, J=8.4 Hz, H-3'), 7.30-7.55 (6H, m, H-4', -$C_6H_5$), 7.61 (1H, dd, J=1.8 Hz, H-6')

As the matrix, FAB-MS: m/z 227 $(M+H)^+$ metanitrobenzyl alcohol was used.

Example 62 Preparation of 1-(2'-Hydroxy-3'-prenyl-4'-methoxyphenyl)-5-methyl-2,4-hexadiene-1-one (hereinafter referred to as compound (C069))

2'-Hydroxy-3'-prenyl-4'-methoxyacetophenone (C026) and 3-methyl-2-butenal (Aldrich) were subjected to Claisen condensation, to give a compound (C069).

$^1$H-NMR(deuterated dimethyl sulfoxide):
δ1.61, 1.71, 1.93, 1.94 (12H, 4s, $(C\underline{H}_3)_2C$=), 3.24 (2H, d, J=6.6 Hz, Ar-C$\underline{H}_2$-CH=), 3.88 (3H, s, -$OCH_3$), 5.12 (1H, m, Ar-$CH_2$-C$\underline{H}$=), 6.27 (1H, d, J=12 Hz, CO-CH=CH-C$\underline{H}$=), 6.65 (1H, d, J=9.6 Hz, H-5'), 7.29 (1H, d, J=14.4 Hz, CO-C$\underline{H}$=CH-CH=), 7.70 (1H, dd, CO-CH=C$\underline{H}$-CH=), 7.98 (1H, d, H-6'), 13.66 (1H, s, -OH-2')

Figure 47 shows $^1$H-NMR spectrum of the compound (C069).

As the matrix, FAB-MS: m/z 301 $(M+H)^+$ metanitrobenzyl alcohol was used.

Example 63 Preparation of 2'-Hydroxy-3'-geranyl-4'-benzyloxyacetophenone (hereinafter referred to as compound (C070))

2',4'-Dihydroxy-3'-geranylacetophenone (C036) was thermally refluxed in acetone in the presence of 1.2 equivalents of benzyl bromide (Wako Pure Chemical Industries, Ltd.) and potassium carbonate, to give a compound (C070).

$^1$H-NMR(deuterated dimethyl sulfoxide):
δ1.50, 1.57, 1.62 (9H, 3s, $(C\underline{H}_3)_2C$=, -C(C$\underline{H}_3$)=CH-), 1.89 (2H, m, -$CH_2$-C$\underline{H}_2$-C($CH_3$)=), 1.98 (2H, m, =CH-C$\underline{H}_2$-$CH_2$-), 2.57 (3H, s, -CO-$CH_3$), 3.28 (2H, d, J=7.2 Hz, Ar-C$\underline{H}_2$-CH=), 5.01 (1H, m, $(CH_3)_2C$=C$\underline{H}$-$CH_2$-), 5.13 (1H, m, -C($CH_3$)=C$\underline{H}$-) 5.25 (2H, s, -C$\underline{H}_2$-$C_6H_5$), 6.73 (1H, d, J=9 Hz, H-5'), 7.30-7.50 (5H, m, -$C_6H_5$), 7.81 (1H, d, H-6'), 12.87 (1H, s, -OH-2')

Figure 48 shows $^1$H-NMR spectrum of the compound (C070).

As the matrix, FAB-MS: m/z 379 $(M+H)^+$ metanitrobenzyl alcohol was used.

Example 64 Preparation of 2,2'-Dihydroxy-3,3'-diprenyl-4,4'-dimethoxychalcone (hereinafter referred to as compound (C072))

2'-Hydroxy-3'-prenyl-4'-methoxyacetophenone (C026) and 2-hydroxy-3-prenyl-4-methoxybenzaldehyde (C063) were subjected to Claisen condensation, to give a compound (C072).

$^1$H-NMR(deuterated dimethyl sulfoxide):
δ1.62, 1.72, 1.73 (12H, 3s, $(C\underline{H}_3)_2C$=), 3.27 (2H, d, J=7.2 Hz, Ar-C$\underline{H}_2$-CH=), 3.83, 3.90 (6H, 2s, -$OCH_3$), 5.09, 5.14 (2H, 2m, -$CH_2$-C$\underline{H}$=), 6.66, 6.67 (2H, 2d, J=9 Hz, H-5,5'), 7.82 (1H, d, J=15.6 Hz, CO-C$\underline{H}$=CH-), 7.86, 8.19 (2H, 2d, H-6,6'), 8.24 (1H, d, CO-CH=C$\underline{H}$-), 9.28 (1H, s, -OH-2), 13.87 (1H, s, -OH-2')

Figure 49 shows $^1$H-NMR spectrum of the compound (C072).

As the matrix, FAB-MS: m/z 437 $(M+H)^+$ metanitrobenzyl alcohol was used.

Example 65 Preparation of 4,2'-Dihydroxy-3'-geranyl-4'-benzyloxychalcone (hereinafter referred to as compound (C073))

2'-Hydroxy-3'-geranyl-4'-benzyloxyacetophenone (C070) and 4-hydroxybenzaldehyde (Aldrich) were subjected to Claisen condensation, to give a compound (C073).

$^1$H-NMR(deuterated dimethyl sulfoxide):
δ1.50, 1.57, 1.64 (9H, 3s, $(C\underline{H}_3)_2C$=, -C(C$\underline{H}_3$)=CH-), 1.90 (2H, m, -$CH_2$-C$\underline{H}_2$-C($CH_3$)=), 1.99 (2H, m, =CH-C$\underline{H}_2$-$CH_2$-), 5.02 (1H, m, $(CH_3)_2C$=C$\underline{H}$-$CH_2$-), 5.16 (1H, m, -C($CH_3$)=C$\underline{H}$-), 5.28 (2H, s,-C$\underline{H}_2$-$C_6H_5$), 6.75 (1H, d, J=9 Hz, H-5'), 6.85 (2H, d, J=9 Hz, H-3,5), 7.30-7.50 (5H, m, -$C_6H_5$), 7.78 (2H, d, H-2,6), 7.78 (1H, d, J=15 Hz, CO-C$\underline{H}$=CH-), 7.83 (1H, d, CO-CH=C$\underline{H}$-), 8.22 (1H, d, H-6'), 13.82 (1H, s, -OH-2')

Figure 50 shows $^1$H-NMR spectrum of the compound (C073).

As the matrix, FAB-MS: m/z 483 $(M+H)^+$ metanitrobenzyl alcohol was used.

Example 66 Preparation of 4-Chloro-2',4'-dihydroxy-3'-geranylchalcone (hereinafter referred to as compound (C074))

2',4'-Dihydroxy-3'-geranylacetophenone (C036) and 4-chlorobenzaldehyde (Aldrich) were subjected to Claisen condensation, to give a compound (C074).

$^1$H-NMR(deuterated dimethyl sulfoxide):
δ1.52, 1.58, 1.73 (9H, 3s, $(CH_3)_2C$=, -C(CH$_3$)=CH-), 1.91 (2H, m, =CH-CH$_2$-CH$_2$-), 2.00 (2H, m, =CH-CH$_2$-CH$_2$-), 3.23 (2H, d, J=7.2 Hz, Ar-CH$_2$-CH=), 5.03 (1H, m, $(CH_3)_2C$=CH-CH$_2$-), 5.18 (1H, m, -C(CH$_3$)=CH-), 6.44 (1H, d, J=9 Hz, H-5'), 7.53 (2H, d, J=9 Hz, H-3,5), 7.76 (1H, d, J=15.6 Hz, CO-CH=CH-), 7.94 (2H, d, H-2,6), 7.99 (1H, d, CO-CH=CH-), 8.04 (1H, d, H-6'), 13.85 (1H, s, -OH-2')

Figure 51 shows $^1$H-NMR spectrum of the compound (C074).

As the matrix, FAB-MS: m/z 411 $(M+H)^+$ metanitrobenzyl alcohol was used.

Example 67 Preparation of 4-Chloro-2',4'-dihydroxy-3'-prenylchalcone (hereinafter referred to as compound (C075))

2',4'-Dihydroxy-3'-prenylacetophenone (C024) and 4-chlorobenzaldehyde (Aldrich) were subjected to Claisen condensation, to give a compound (C075).

$^1$H-NMR(deuterated dimethyl sulfoxide):
δ1.62, 1.73 (6H, 2s, $(CH_3)_2C$=), 3.23 (2H, d, J=7.2 Hz, Ar-CH$_2$-CH=), 5.18 (1H, m, Ar-CH$_2$-CH=), 6.48 (1H, d, J=9 Hz, H-5'), 7.53 (2H, d, J=9 Hz, H-3,5), 7.78 (1H, d, J=15.6 Hz, CO-CH=CH-), 7.94 (2H, d, H-2,6), 8.00 (1H, d, CO-CH=CH-), 8.07 (1H, d, H-6'), 13.80 (1H, s, -OH-2')

Figure 52 shows $^1$H-NMR spectrum of the compound (C075).

As the matrix, FAB-MS: m/z 343 $(M+H)^+$ metanitrobenzyl alcohol was used.

Example 68 Preparation of 3-Nitro-2'-hydroxy-3' prenyl-4'-methoxychalcone (hereinafter referred to as compound (C076))

2'-Hydroxy-3'-prenyl-4'-methoxyacetophenone (C026) and 3-nitrobenzaldehyde (Aldrich) were subjected to Claisen condensation, to give a compound (C076).

$^1$H-NMR(deuterated dimethyl sulfoxide): δ1.73, 1.62 (6H, 2s, $(CH_3)_2C$=), 3.28 (2H, d, J=7.2 Hz, Ar-CH$_2$-CH=), 3.93 (3H, s, -OCH$_3$), 5.14 (1H, m, Ar-CH$_2$-CH=), 6.72 (1H, d, J=9 Hz, H-5'), 7.77 (1H, t, J=7.8 Hz, H-5), 7.95 (1H, d, J=15.6 Hz, CO-CH=CH-), 8.27 (1H, d, CO-CH=CH-), 8.28 (1H, d, H-6), 8.36 (1H, d, H-6'), 8.37 (1H, d, H-4), 8.83 (1H, s, H-2), 13.47 (1H, s, -OH-2')

Figure 53 shows $^1$H-NMR spectrum of the compound (C076).

As the matrix, FAB-MS: m/z 368 $(M+H)^+$ metanitrobenzyl alcohol was used.

Example **69** Preparation of 4-Hydroxy-2'-benzyloxy-3'-prenyl-4'-methoxychalcone (hereinafter referred to as compound (C077))

2'-Hydroxy-3'-prenyl-4'-methoxyacetophenone (C026) and benzyl bromide (Wako Pure Chemical Industries, Ltd.) were thermally refluxed in acetone in the presence of potassium carbonate, to give a compound 2'-benzyloxy-3'-prenyl-4'-methoxyacetophenone. The 2'-benzyloxy-3'-prenyl-4'-methoxyacetophenone and 4-hydroxybenzaldehyde (Aldrich) were subjected to Claisen condensation, to give a compound (C077).

$^1$H-NMR(deuterated dimethyl sulfoxide): δ 1.61 (6H, s, $(CH_3)_2C$=), 3.87 (3H, s, -OCH$_3$), 5.10 (1H, m, Ar-CH$_2$-CH=), 4.76 (2H, s, -CH$_2$-C$_6$H$_5$), 6.77 (2H, d, J=8.4 Hz, H-3,5), 6.93 (1H, d, J=8.4 Hz, H-5'), 7.20-7.40 (6H, m, -C$_6$H$_5$, CO-CH=CH-), 7.48 (2H, d, H-2,6), 7.55 (1H, d, J=16.2 Hz, CO-CH=CH-), 7.58 (1H, d, H-6')

Figure 54 shows $^1$H-NMR spectrum of the compound (C077).

As the matrix, FAB-MS: m/z 429 $(M+H)^+$ metanitrobenzyl alcohol was used.

Example 70 Preparation of 4-Acetoxy-2'-hydroxy-3'-prenyl-4'-methoxychalcone (hereinafter referred to as compound (C078))

4,2'-Dihydroxy-3'-prenyl-4'-methoxychalcone (4-hydroxyderricin) was treated with acetic anhydride in dichloromethane under ice-cooling in the presence of triethylamine and a catalytic amount of DMAP, to give a compound (C078).

$^1$H-NMR(deuterated chloroform): δ1.70, 1.82 (6H, 2s, $(CH_3)_2C$=), 2.34 (3H, s, -OAc), 3.41 (2H, d, J=7.2 Hz, Ar-CH$_2$-CH=), 3.93 (3H, s, -OCH$_3$), 5.25 (1H, m, Ar-CH$_2$-CH=), 6.52 (1H, d, J=9 Hz, H-5'), 7.18 (2H, d, J=8.4 Hz, H-3,5), 7.57 (1H, d, J=15.6 Hz, CO-CH=CH-), 7.68 (2H, d, H-2,6), 7.80 (1H, d, H-6'), 7.86 (1H, d, CO-CH=CH-), 13.34 (1H, s, -OH-2')

Figure 55 shows $^1$H-NMR spectrum of the compound (C078).

As the matrix, FAB-MS: m/z 381 $(M+H)^+$ metanitrobenzyl alcohol was used.

Example 71 Preparation of 4-Chloro-2'-acetoxy-3'-prenyl-4'-methoxychalcone (hereinafter referred to as compound (C079))

4-Chloro-2',4'-dihydroxy-3'-prenylchalcone (compound (C075)) was treated with dimethyl sulfate in the same manner as in Example 28, and thereafter the reaction mixture was treated with acetic anhydride in dichloromethane in the presence of triethylamine and a catalytic amount of DMAP, to give a compound (C079).

$^1$H-NMR(deuterated chloroform): δ1.69,1.77 (6H, 2s, $(C\underline{H}_3)_2C=$), 2.30 (3H, s, -OAc), 3.32 (2H, d, J=7.2 Hz, Ar-$C\underline{H}_2$-CH=), 3.93 (3H, s, $-OCH_3$), 5.12 (1H, m, Ar-$CH_2$-C$\underline{H}$=), 6.84 (1H, d, J=9 Hz, H-5'), 7.24 (1H, d, J=15.6 Hz, CO-C$\underline{H}$=CH-), 7.39 (2H, d, J=8.4 Hz, H-3,5), 7.53 (2H, d, H-2,6), 7.58 (1H, d, CO-CH=C$\underline{H}$-), 7.69 (1H, d, H-6')

Figure 56 shows $^1$H-NMR spectrum of the compound (C079).

As the matrix, FAB-MS: m/z 399 $(M+H)^+$ metanitrobenzyl alcohol was used.

Example 72 Preparation of 4-Chloro-2'-acetoxy-3'-geranyl-4'-methoxychalcone (hereinafter referred to as compound (C080))

4-Chloro-2',4'-dihydroxy-3'-geranylchalcone (compound (C074)) was treated with dimethyl sulfate in the same manner as in Example 28, and thereafter the reaction mixture was treated with acetic anhydride in dichloromethane in the presence of triethylamine and a catalytic amount of DMAP, to give a compound (C080).

$^1$H-NMR(deuterated chloroform):

δ1.59, 1.66, 1.77 (9H, 3s, $\underline{(CH_3)}_2C$=, -C($CH_3$)=CH-), 1.98 (2H, m, =CH-$CH_2$-$CH_2$-), 2.06 (2H, m, =CH-$CH_2$-$CH_2$-), 2.30 (3H, s, -OAc), 3.33 (2H, d, J=7.2 Hz, Ar-$CH_2$-C=), 3.93 (3H, s, $-OCH_3$), 5.08 (1H, m, $(CH_3)_2C$=CH-$CH_2$-), 5.12 (1H, m, -C($CH_3$)=C$\underline{H}$-), 6.84 (1H, d, J=8.4 Hz, H-5'), 7.24 (1H, d, J=15.6 Hz, CO-C$\underline{H}$=CH-), 7.39 (2H, d, J=8.4 Hz, H-3,5), 7.53 (2H, d, H-2,6), 7.58 (1H, d, CO-CH=C$\underline{H}$-), 7.69 (1H, d, H-6')

Figure 57 shows $^1$H-NMR spectrum of the compound (C080).

As the matrix, FAB-MS: m/z 467 $(M+H)^+$ metanitrobenzyl alcohol was used.

Example 73 Preparation of 3,4,2'-Trihydroxychalcone (hereinafter referred to as compound (C005))

2'-Hydroxyacetophenone (Tokyo Kasei Kogyo Co., Ltd.) and 3,4-dihydroxybenzaldehyde (Tokyo Kasei Kogyo Co., Ltd.) were subjected to Claisen condensation, to give a compound (C005).

$^1$H-NMR(deuterated dimethyl sulfoxide): δ6.81 (1H, d, J=8 Hz, H-5), 6.98 (2H, m, H-3',5'), 7.22 (1H, dd, J=8 Hz, H-6), 7.29 (1H, d, H-2), 7.53 (1H, td, J=8,8,2 Hz, td, H-4'), 7.71 (2H, m, CO-C$\underline{H}$=C$\underline{H}$-), 8.21 (1H, dd, J=8 Hz, H-6')

As the matrix, FAB-MS: m/z 257 $(M+H)^+$ glycerol was used.

Example 74 Preparation of 3,4,2',5'-Tetrahydroxychalcone (hereinafter referred to as compound (C006))

2',5'-Dihydroxyacetophenone (Tokyo Kasei Kogyo Co., Ltd.) and 3,4-dihydroxybenzaldehyde (Tokyo Kasei Kogyo Co., Ltd.) were subjected to Claisen condensation, to give a compound (C006).

$^1$H-NMR(deuterated dimethyl sulfoxide): δ 6.79 (1H, d, J=8 Hz, H-5), 6.81 (1H, d, J=9 Hz, H-3'), 6.00 (1H, dd, J=3 Hz, H-4'), 7.17 (1H, dd, J=2 Hz, H-6), 7.23 (1H, d, H-2), 7.44 (1H, d, H-6'), 7.55 (1H, d, J=15 Hz, CO-C$\underline{H}$=CH-), 7.66 (1H, d, CO-CH=C$\underline{H}$-)

As the matrix, FAB-MS: m/z 273 $(M+H)^+$ glycerol was used.

Example 75 Preparation of 1-Adamant 1-3- 3,4-dihydroxyphenyl)-2-propen-1-one (hereinafter referred to as compound (C013))

1-Adamantyl-methyl ketone (Sigma) and 3,4-dihydroxybenzaldehyde (Tokyo Kasei Kogyo Co., Ltd.) were subjected to Claisen condensation, to give a compound (C013).

$^1$H-NMR(deuterated dimethyl sulfoxide):

δ1.65-2.04 (15H, m, -adamantyl), 6.74 (1H, d, J=8 Hz, H-5), 7.00 (1H, dd, J=2 Hz, H-6), 7.11 (1H, d, J=16 Hz, CO-C$\underline{H}$=CH-), 7.13 (1H, d, H-2), 7.36 (1H, d, CO-CH=C$\underline{H}$-)

As the matrix, FAB-MS: m/z 299 $(M+H)^+$ glycerol was used.

Example 76 Preparation of 1-Adamantyl-3-hydroxy-4-(3,4-dihydroxyphenyl)-butan-1-one (hereinafter referred to as compound (C014))

The THP-substituted 3',4'-dihydroxyphenylacetic acid (Nakalai Tesque, Inc.) was reduced with 1M DIBAL hexane solution (Nakalai Tesque, Inc.), to give a THP-substituted 3',4'-dihydroxyphenylacetaldehyde. The THP-substituted 3',4'-dihydroxyphenylacetaldehyde was reacted with 1-adamantyl-methyl ketone in hexan containing lithium diisopropylamine while being cooled to -70°C with methanol/dry ice, and thereafter the THP group was deprotected, to give a compound (C014).

$^1$H-NMR(deuterated dimethyl sulfoxide):
δ1.58-1.98 (15H, m, -adamantyl), 2.25 (1H, dd, J=4.5, 16.5 Hz, CO-C$\underline{H_2}$ -CH(OH)-$CH_2$-), 2.41 (1H, dd, J=6.0, 13.5 Hz, CO-C$\underline{H_2}$-CH(OH)-C$\underline{H_2}$ ), 2.61 (1H, dd, J=4.5 Hz, CO-C$\underline{H_2}$-CH(OH)-$CH_2$-), 3.99 (1H, m, CO-$CH_2$-C$\underline{H}$(OH)-$CH_2$-), 6.38 (1H, dd, J=2,8 Hz, H-6), 6.55 (1H, d, H-2), 6.57 (1H, d, H-2)

As the matrix, FAB-MS: m/z 331 $(M+H)^+$ glycerol was used.

Example 77 Preparation of 1-Adamantyl-4-(3,4-dihydroxyphenyhl-3-buten-1-one (hereinafter referred to as compound (C015))

The THP-substituted form of the compound (C014) was treated with acetic anhydride in dichloromethane in the presence of triethylamine and a catalytic amount of DMAP, and thereafter the reaction mixture was treated with DBU in dichloromethane at room temperature, to give a THP-substituted form of a compound (C015). The THP group was deprotected from the THP-substituted form of the compound (C015), to give a compound (C015).

$^1$H-NMR(deuterated dimethyl sulfoxide):
δ1.66-2.00 (15H, m, -adamantyl), 3.37 (2H, dd, J=7,1 Hz, CO-C$\underline{H_2}$-CH=CH-), 5.93 (1H, dt, J=11 Hz, CO-$CH_2$-C$\underline{H}$=CH-), 6.22 (1 H, d, CO-$CH_2$-CH=C$\underline{H}$-), 6.59 (1H, dd, J=2,8 Hz, H-6), 6.63 (1H, d, H-5), 6.75 (1H, d, H-2)

As the matrix, FAB-MS: m/z 313 $(M+H)^+$ glycerol was used.

Example 78 Preparation of 1-(2-Hydroxy-4-methoxy-3-prenylphenyl)-3-(2-thienyl)-2-propen-1-one hereinafter referred to as compound (C-THP))

2'-Hydroxy-4'-methoxy-3'-prenylacetophenone (C026) and 2-thiophenealdehyde (Tokyo Kasei Kogyo Co., Ltd.) were subjected to Claisen condensation, to give a compound (C-THP).

$^1$H-NMR(deuterated chloroform): δ 1.70 (3H, s, $(C\underline{H_3})_2C$=), 1.82 (3H, s, $(C\underline{H_3})_2C$=), 3.40 (2H, d, J=7.2 Hz, Ar-C$\underline{H_2}$-CH=), 3.93 (3H, s, $OCH_3$), 5.25 (1H, t, J=7.2 Hz, Ar-$CH_2$-C$\underline{H}$=), 6.52 (1H, d, J=9.0 Hz, phenyl H-5), 7.12 (1H, dd, J=3.6 Hz, J=4.8 Hz, thienyl H-4), 7.38 (1H, d, J=3.6 Hz, thienyl H-3), 7.40 (1H, d, J=15.0 Hz, -CO-C$\underline{H}$=CH-), 7.45 (1H, d, J=4.8 Hz, thienyl H-5), 7.77 (1H, d, J=9.0 Hz, phenyl H-6), 8.02 (1H, d, J=15.0 Hz, -CO-CH=C$\underline{H}$-), 13.4 (1H, s, OH)

Figure 58 shows $^1$H-NMR spectrum of the compound (C-THP).

As the matrix, FAB-MS: m/z 329 $(M+H)^+$ metanitrobenzyl alcohol was used.

Example 79 Preparation of 1-(2-Hydroxy-4-methoxy-3-prenylphenyl)-5-phenyl-2,4-pentadien-1-one (hereinafter referred to as compound (C-CIN

2'-Hydroxy-4'-methoxy-3'-prenylacetophenone (C026) and trans-cinnamaldehyde (Tokyo Kasei Kogyo Co., Ltd.) were subjected to Claisen condensation, to give a compound (C-CIN). The signals of NMR are shown below. In addition, the numbers of the peaks are shown in the following formula (Formula 60).

(Formula 60)

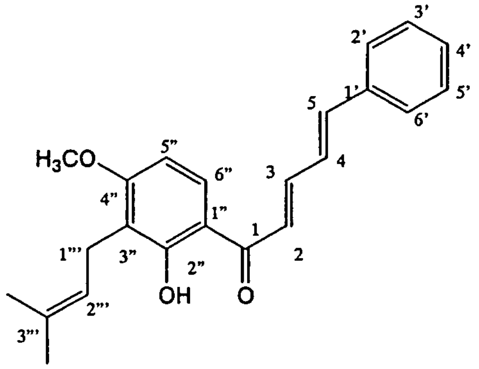

$^1$H-NMR (deuterated chloroform): δ1.70 (3H, s, $CH_3$-3'''), 1.81 (3H, s, $CH_3$-3'''), 3.40 (2H, d, J=7.2 Hz, H-1'''), 3.93 (3H, s, $OCH_3$-4"), 5.24 (1H, t, J=7.2 Hz, H-2'''), 6.51 (1H, d, J=9.0 Hz, H-5"), 7.05 (1H, d, J=3.6 Hz, H-5), 7.05 (1H, d, J=7.2 Hz, H-4), 7.18 (1H, d, J=15.0 Hz, H-2), 7.35 (1H, d, J=7.2 Hz, H-4'), 7.40 (2H, dd, J=7.2 Hz, J=7.2 Hz, H-3' and H-5'), 7.52 (2H, d, J=7.2 Hz, H-2' and H-6'), 7.68 (1H, m, H-3), 7.72 (1H, d, J=9.0 Hz, H-6")

Figure 59 shows $^1$H-NMR spectrum of the compound (C-CIN).

$^{13}$C-NMR (deuterated chloroform): δ18.2 ($CH_3$-3'''), 22.1 (C-1'''), 26.2 ($CH_3$-3'''), 56.2 ($OCH_3$-4"), 102.5 (C-5"), 115.0 (C-1"), 118.0 (C-3"), 122.4 (C-2'''), 124.5 (C-2), 127.3 (C-4), 127.7 (C-3' and C-5'), 129.3 (C-2' and C-6'), 129.5 (C-4'), 129.6 (C-6"), 132.3 (C-3'''), 136.5 (C-1'), 142.3 (C-5), 144.6 (C-3), 163.5 (C-2"), 163.7 (C-4"), 192.6 (C-1)

Figure 60 shows $^{13}$C-NMR spectrum of the compound (C-CIN).

As the matrix, FAB-MS: m/z 349 $(M+H)^+$ metanitrobenzyl alcohol was used.

Example 80 Preparation of 3-(2-Furyl)-1-(2-hdyroxy-3-prenyl-4-methoxyphenyl)-2-propen-1-one (hereinafter referred to as compound (FUR-1) and Preparation of 2-(2-Furyl)-3-(2-furylmethylene)-2,3-dihydro-7-methoxy-8-prenyl-4H-1-benzopyran-4-one (hereinafter referred to as compound (FUR-2)

(1) 2'-Hydroxy-4'-methoxy-3'-prenylacetophenone (C026) and Furfural (Tokyo Kasei Kogyo Co., Ltd.) were subjected to Claisen condensation, to give a compound (FUR-1) and a compound (FUR-2).

(2) The NMR spectra and the mass spectrum of the compound (FUR-1) obtained in item (1) of Example 80 were determined in the same manner as in item (2) of Example 4. The signals of NMR are shown below.

$^1$H-NMR(deuterated chloroform): δ1.70 (3H, s, $(C\underline{H}_3)_2C=$),

1.81 (3H, s, $(C\underline{H}_3)_2C=$), 3.41 (2H, d, J=7.2 Hz, Ar-$C\underline{H}_2$-CH=), 3.92 (3H, s, $OCH_3$), 5.25 (1H, t, J=7.2 Hz, Ar-$CH_2$-C$\underline{H}$=), 6.51 (1H, d, J=9.0 Hz, phenyl H-5), 6.54 (1H, dd, J=3.6 Hz, J=1.8 Hz, furyl H-4), 6.74 (1H, d, J=3.6 Hz, furyl H-3), 7.52 (1H, d, J=15.0 Hz, -CO-C$\underline{H}$=CH-), 7.56 (1H, d, J=1.8 Hz, furyl H-5), 7.65 (1H, d, J=15.0 Hz, -CO-CH=C$\underline{H}$-), 7.81 (1H, d, J=9.0 Hz, phenyl H-6), 13.4 (1H, s, OH)

Figure 61 shows $^1$H-NMR spectrum of the compound (FUR-1).

As the matrix, FAB-MS: m/z 313 $(M+H)^+$ metanitrobenzyl alcohol was used.

(3) The NMR spectra and the mass spectrum of the compound (FUR-2) obtained in item (1) of Example 80 were determined in the same manner as in item (2) of Example 4. The signals of NMR are shown below. In addition, the numbers of the peaks are shown in the following formula (Formula 61).

(Formula 61)

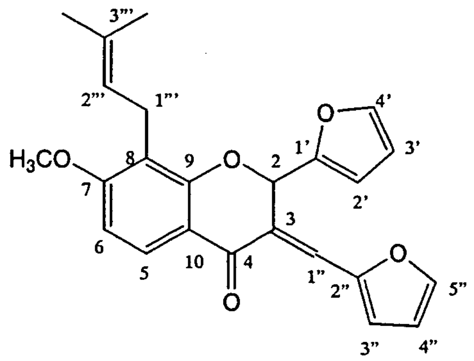

$^{1}$H-NMR(deuterated chloroform): δ1.66 (3H, s, $CH_3$-3'''), 1.80 (3H, s, $CH_3$-3'''), 3.30 (1H, dd, J=7.2 Hz, J=13.8 Hz, H-1'''), 3.36 (1H, dd, J=7.2 Hz, J=13.8 Hz, H-1'''), 3.87 (3H, s, $OCH_3$-7), 5.07 (1H, t, J=7.2 Hz, H-2'''), 6.17 (1H, m, H-2'), 6.20 (1H, m, H-3'), 6.52 (1H, m, H-4"), 6.62 (1H, d, 9.0 Hz, H-6), 6.72 (1H, m, H-3"), 7.15 (1H, s, H-2), 7.37 (1H, m, H-4'), 7.54 (1H, m, H-5"), 7.68 (1H, s, H-1"), 7.90 (1H, d, J=9.0 Hz, H-5)

Figure 62 shows $^{1}$H-NMR spectrum of the compound (FUR-2). $^{13}$C-NMR (deuterated chloroform): δ 18.2-($CH_3$-3'''), 22.4 (C-1'''), 26.2($CH_3$-3'''), 56.2 ($OCH_3$-7), 72.8 (C-2), 105.6 (C-6), 109.9 (C-2'), 110.6 (C-3'), 113.0 (C-4"), 116.8 (C-10), 118.7 (C-8), 119.0 (C-3"), 122.4 (C-2'''), 123.7 (C-1"), 127.2 (C-5), 127.2 (C-3), 132.0 (C-3'''), 143.5 (C-4'), 146.3 (C-5"), 151.4 (C-2"), 152.4 (C-1'), 158.1 (C-9), 163.6 (C-7), 181.2 (C-4)

Figure 63 shows $^{13}$C-NMR spectrum of the compound (FUR-2).

As the matrix, FAB-MS: m/z 391 $(M+H)^+$ metanitrobenzyl alcohol was used.

Example 81 Preparation of 4-Chloro-2'-benzyloxychalcone (hereinafter referred to as compound (C066)

4-Chlorobenzaldehyde (Aldrich) and 2'-hydroxyacetophenone (Tokyo Kasei Kogyo Co., Ltd.) were subjected to Claisen condensation, to give 4-chloro-2'-hydroxychalcone. The 4-chloro-2'-hydroxychalcone was treated in the same manner as in Example 63, to give a compound (C066).

$^{1}$H-NMR(deuterated dimethyl sulfoxide): δ5.22 (2H, s, $-\underline{CH_2}-C_6H_5$), 7.10 (1H, t, J=7.2 Hz), 7.27-7.34 (4H, m), 7.42-7.61 (10H, m)

As the matrix, FAB-MS: m/z 349 $(M+H)^+$ m-nitrobenzyl alcohol was used.

Example 82 Determination of Action for Induction of Adipocyte Differentiation

(1) Induction of Adipocyte Differentiation

Induction of adipocyte differentiation was carried out by partially modifying the above-mentioned method of Rubin C. S. et al. In addition, as a test compound, a compound prepared in Examples mentioned above or a commercially available compound was used. 3T3-L1 cells (ATCC CCL-92.1) were suspended in a 10% fetal bovine serum (manufactured by Bio Whittaker)-containing Dulbecco's modified Eagle's medium (manufactured by Sigma, D6046) containing 200 μM ascorbic acid so as to have a density of 4 x $10^3$ cells/mL, and the suspension was put in each well of a 12-well microtiter plate in an amount of 2 ml per well. The cells were cultured at 37°C for 7 days in the presence of 5% carbon dioxide gas. On the seventh day, the medium was exchanged with a 10% fetal bovine serum (manufactured by Bio Whittaker)-containing Dulbecco's modified Eagle's medium containing 200 μM ascorbic acid and 0.25 μM dexamethasone, and a test compound dissolved in dimethyl sulfoxide in a concentration shown in each of Tables 16 to 19 was added to each well. Here, there were set a group with addition of 4 μL of an aqueous solution containing 5 mg/mL insulin (manufactured by TAKARA BIO INC.) as a positive control, and a group with addition of dimethyl sulfoxide as a negative control. After 45 hours, the medium was exchanged with a 10% fetal bovine serum-containing Dulbecco's modified Eagle's medium containing 200 μM ascorbic acid. A test compound, 2 μL of an aqueous solution containing 5 mg/mL insulin as a positive control, or dimethyl sulfoxide as a negative control was put to each well in the same concentration. The cells were cultured for additional 7 days. In addition, the medium was exchanged on after 2 days and after 5 days, and at that time, a test compound, 2 μL of an aqueous solution

containing 5 mg/mL insulin as a positive control, or dimethyl sulfoxide as a negative control was put to each well in the same concentration.

(2) Determination of Amount of Biosynthesis of Triglyceride

The amount of triglyceride in the adipocytes was determined as an index of induction of differentiation into mature adipocytes, and also, as the evaluation of insulin-mimetic action.

After the termination of the culture, the medium was removed, and the cells were washed twice with a phosphate buffered saline. One milliliter of a solvent of hexane: isopropanol = 3:2 was added to the cells. The mixture was allowed to stand at room temperature for 30 minutes, and thereafter the supernatant was collected. The procedures were repeated again, and 2 mL of the supernatant was concentrated to dryness. The precipitate was dissolved in 100 μL of isopropanol, and the amount of triglyceride contained in 10 μL of the solution was determined using a triglyceride E-test (manufactured by Wako Pure Chemical Industries, Ltd., code 432-40201). In addition, all the determinations were carried out twice. The action for induction of differentiation was calculated by the following calculation formula, and shown by four ranks of +++, ++, + and +/-.

S: Amount of triglyceride in the presence of a test compound;
N: Amount of triglyceride in the negative control; and
P: Amount of triglyceride in the positive control;

$$X = (S - N)/(P - N)$$

(Judgment of Action for Induction of Differentiation)

+++: $1 < X$
++: $0.6<X\leq1$
+: $0.3 < X \leq 0.6$
+/-: $0.15 < X \leq 0.3$

The results are shown in Tables 16 to 19. Tables 16 to 19 give a list of each test compound and the action for induction of differentiation in a given concentration. In Tables 16 to 19, the group with addition of a test compound at each concentration was found to show the induction of biosynthesis of triglyceride. In other words, each test compound listed in the tables was found to show an evident action for induction of differentiation into mature adipocytes.

Table 16

| Name of Test Compound | Concentration (μM) | Action for Induction of Differentiation |
|---|---|---|
| Xanthoangelol | 10 | +++ |
| | 3 | ++ |
| | 1 | +/- |
| TB1 | 10 | ++ |
| | 3 | +++ |
| TB2 | 10 | +++ |
| | 3 | +++ |
| 4-Hydroxyderricin | 10 | +++ |
| | 3 | ++ |
| Xanthoangelol H | 13 | +++ |
| | 4 | ++ |
| | 1.3 | + |
| Xanthohumol | 13 | + |
| | 4 | +/- |

Table continued

| Table 16 | | |
|---|---|---|
| Name of Test Compound | Concentration (μM) | Action for Induction of Differentiation |
| Xanthoangelol F | 10<br>3<br>1 | +++<br>+++<br>++ |
| Xanthoangelol G | 30<br>10<br>3 | +++<br>+++<br>+ |
| Compound (C020) | 13<br>4 | ++<br>+ |
| Compound (C023) | 13<br>4 | ++<br>++ |
| Compound (C032) | 10<br>3<br>1 | +++<br>+++<br>++ |
| Compound (C033) | 10<br>3 | +++<br>+++ |
| Compound (C035) | 10<br>3 | +++<br>++ |
| Compound (C034) | 10<br>3 | +++<br>+++ |
| Isobavachalcone | 10<br>3 | ++<br>+ |
| Bavachalcone | 13<br>4 | +<br>+/- |
| Compound (C046) | 10<br>3 | +++<br>++ |

| Table 17 | | |
|---|---|---|
| Name of Test Compound | Concentration (μM) | Action for Induction of Differentiation |
| Compound (C047) | 30<br>10 | ++<br>+ |
| Compound (C049) | 10<br>3 | +/-<br>+/- |
| Compound (C056) | 10<br>3<br>1 | +++<br>+<br>+/- |
| Compound (C057) | 10<br>3 | +++<br>+ |
| Compound (C061) | 10<br>3 | +++<br>++ |
| Compound (C064-1) | 10<br>3 | +++<br>+++ |

Table continued

| Table 17 | | |
|---|---|---|
| Name of Test Compound | Concentration (μM) | Action for Induction of Differentiation |
| Compound (C064-2) | 10<br>3 | +++<br>+++ |
| Compound (C066) | 10<br>3 | +++<br>++ |
| Compound (C072) | 10<br>3 | +++<br>+++ |
| Compound (C073) | 3 | +/- |
| Compound (C074) | 10<br>3 | +<br>+/- |
| Compound (C075) | 10<br>3 | +<br>+/- |
| Compound (C079) | 10<br>3 | +++<br>++ |
| Compound (C076) | 10<br>3<br>1 | +++<br>+++<br>++ |
| Compound (C077) | 3 | + |
| Compound (C078) | 30<br>10 | +++<br>+ |
| Compound (C080) | 10<br>3<br>1 | +++<br>+++<br>++ |
| Compound (C081) | 30<br>10<br>3 | +++<br>+++<br>++ |

| Table 18 | | |
|---|---|---|
| Name of Test Compound | Concentration (μM) | Action for Induction of Differentiation |
| TB3 | 10<br>3 | +++<br>+++ |
| TB4 | 10<br>3 | +<br>+ |
| TB5 | 10<br>3 | +++<br>+++ |
| TB6 | 10<br>3 | +++<br>+/- |
| TB8 | 10<br>3 | +++<br>+ |
| TB9 | 10 | +++ |
| Compound (C026) | 13<br>4 | ++<br>++ |

Table continued

| Table 18 | | |
|---|---|---|
| Name of Test Compound | Concentration (μM) | Action for Induction of Differentiation |
| | 1.3 | + |
| Compound (C027) | 13<br>4 | +<br>+/- |
| Compound (C022) | 13 | +/- |
| Compound (C036) | 10<br>3 | ++<br>+/- |
| Compound (C038) | 10<br>3 | +++<br>+ |
| Compound (C041) | 10<br>3 | +++<br>+ |
| Compound (C044) | 10<br>3 | ++<br>+ |
| Compound (C048) | 30<br>10 | +++<br>+++ |
| Compound (C051) | 30 | ++ |
| Compound (C052) | 10 | + |
| Compound (C054) | 10<br>3 | +<br>+/- |
| Compound (C055) | 10<br>3 | +<br>+/- |

| Table 19 | | |
|---|---|---|
| Name of Test Compound | Concentration (μM) | Action for Induction of Differentiation |
| Compound (C059) | 30<br>10<br>3 | +++<br>+<br>+/- |
| Compound (C063) | 30<br>10<br>3 | +++<br>++<br>+ |
| Compound (C065) | 30<br>10<br>3 | +++<br>++<br>+ |
| Compound (C069) | 30<br>10<br>3 | +++<br>+++<br>+ |
| Compound (C070) | 30<br>10 | ++<br>+ |
| Compound (C-THP) | 10<br>3<br>1 | +++<br>++<br>+/- |
| Compound (FUR-1) | 10 | +++ |

Table continued

| Table 19 | | |
|---|---|---|
| Name of Test Compound | Concentration (μM) | Action for Induction of Differentiation |
| | 3<br>1 | +<br>+/- |
| Xanthotoxin | 40<br>13 | ++<br>+ |
| 4'-O-Geranylnaringenin | 30<br>10<br>3 | +++<br>++<br>+ |
| Isobavachin | 30<br>10<br>3 | ++<br>+<br>+ |
| Prostratol F | 10<br>3 | ++<br>++ |
| Compound (C082) | 10 | ++ |

Example 83 Determination of Enhancing Action on Glucose Uptake into Mature Adipocytes

(1) Preparation of Mature Adipocytes
The induction of differentiation into mature adipocytes was carried out by partially modifying the above-mentioned method of Rubin C. S. et al. 3T3-L1 cells (ATCC CCL-92.1) were suspended in a 10% fetal bovine serum (manufactured by Bio Whittaker)-containing Dulbecco's modified Eagle's medium (manufactured by Sigma, D6046) containing 200 μM ascorbic acid so as to have a density of 4 x $10^3$ cells/mL, and the suspension was put in each well of a collagen type I-coated 12-well plate (Iwaki Glass Co., Ltd., code 4815-010) in an amount of 2 mL per well. The cells were cultured at 37°C for 7 days in the presence of 5% carbon dioxide gas. On the seventh day, the medium was exchanged with 2 mL of a 10% fetal bovine serum (manufactured by Bio Whittaker)-containing Dulbecco's modified Eagle's medium containing 200 μM ascorbic acid, 0.25 μM dexamethasone, 10 μg/mL insulin (manufactured by TAKARA BIO INC.) and 0.5 mM 3-isobutyl-1-methylxanthine (manufactured by nacalai tesque, 19624-86). After 45 hours, the medium was exchanged with 2 mL of a 10% fetal bovine serum-containing Dulbecco's modified Eagle's medium containing 200 μM ascorbic acid and 5 μg/mL insulin. The medium was exchanged on after 2 days and 5 days, and the cells were cultured for 7 days, to give mature adipocytes.
(2) Determination of Enhancing Action on Glucose Uptake into Mature Adipocytes
As the evaluation of the enhancing action on glucose uptake, and also as the evaluation of the insulin-mimetic action, the amount of 2-deoxyglucose uptake into mature adipocytes during the stimulation of the test compound in the adipocytes was determined. As a test compound, a compound prepared in the Examples mentioned above or a commercially available compound was used.

After the termination of the culture, the medium was removed, and the cells were washed twice with a 0.1% (w/v) bovine serum albumin (manufactured by Sigma, A8022)-containing Dulbecco's modified Eagle's medium. Thereafter, 1 mL of the same medium containing a test compound in a concentration shown in each of Tables 20 to 23 was put to each well. The cells were cultured overnight at 37°C in the presence of 5% carbon dioxide gas. Here, a group without containing the test compound was set as a negative control. After the overnight culture, the cells were washed twice with a HEPES buffer (140 mM NaCl, 5 mM KCl, 2.5 mM MgS04, 1 mM $CaCl_2$, 20 mM HEPES-Na (pH 7.4)). The amount 0.9 mL of the buffer containing a test compound in the same concentration was added to each well. The cells were cultured at 37°C for 75 minutes. During the culture, as a positive control, there was set a group with addition of insulin to the well without addition of a test compound at a point 45 minutes passed so as to have a final concentration of 1 μg/mL. Thereafter, 100 μL of a HEPES buffer containing 0.5 μCi/mL 2-deoxy-[1,2-$^3$H(N)]-glucose (manufactured by PerkinElmer Life Sciences Inc., NET549A) and 1 mM 2-deoxyglucose (manufactured by nacalai tesque, 10722-11) was added thereto. The cells were cultured at 37°C for additional 10 minutes. After the termination of the culture, the supernatant was removed, and the cells were washed three times with a phosphate buffer cooled to 4°C. Thereafter,

0.5 mL of a 1% Nonidet P-40-containing phosphate buffer was added to lyse the cells, whereby 2-deoxy-[1,2-$^3$H(N)]-glucose subjected to uptake into the cells was eluted. The radioactivity was determined with a liquid scintillation counter LS6500 (manufactured by Beckmann) using 25 μL of the supernatant with Ultima Gold (manufactured by PerkinElmer Life Sciences Inc., 6013329) as a scintillation cocktail. The enhancing action on glucose uptake was calculated by the following calculation formula, and shown by four ranks of +++, ++, + and +/-.

S: Amount of 2-deoxyglucose uptake in the presence of a test compound;
N: Amount of 2-deoxyglucose uptake in the negative control; and
P: Amount of 2-deoxyglucose uptake in the positive control;

$$X = (S - N)/(P - N)$$

(Judgment of Enhancing Action for 2-Deoxyglucose Uptake)

+++: $1 < X$
++: $0.6 < X \leq 1$
+: $0.3 < X \leq 0.6$
+/-: $0.15 < X \leq 0.3$

The results are shown in Tables 20 to 23. Tables 20 to 23 give a list of each test compound and the enhancing action for 2-deoxyglucose uptake in a given concentration.
In Tables 20 to 23, the group with addition of a test compound at each concentration was found to show the enhancing action for 2-deoxyglucose uptake. In other words, each test compound listed in the tables was found to show an evident enhancing action on glucose uptake.

Table 20

| Name of Test Compound | Concentration (μM) | Enhancing Action on Glucose Uptake |
|---|---|---|
| 4-Hydroxyderricin | 30 | +++ |
| | 10 | +++ |
| | 3 | ++ |
| Xanthoangelol F | 30 | +++ |
| | 10 | +++ |
| | 3 | + |
| Compound (C020) | 30 | ++ |
| | 10 | + |
| Xanthoangelol | 30 | +++ |
| | 10 | + |
| Chalcone | 30 | + |
| | 10 | + |
| Bavachalcone | 30 | +++ |
| | 10 | + |
| Compound (C030) | 30 | +++ |
| | 10 | + |
| Compound (C031) | 30 | +++ |
| | 10 | +/- |
| Compound (C032) | 30 | +++ |
| | 10 | +++ |

Table continued

| Table 20 | | |
|---|---|---|
| Name of Test Compound | Concentration (μM) | Enhancing Action on Glucose Uptake |
| | 3 | +++ |
| Compound (C033) | 30<br>10<br>3 | ++<br>+<br>+ |
| Compound (C035) | 30<br>10 | ++<br>+ |
| Compound (C011) | 30<br>10<br>3 | +++<br>++<br>+/- |
| Isobavachalcone | 30<br>10<br>3 | +++<br>++<br>+/- |
| Compound (C043) | 10<br>3 | +++<br>+/- |
| Compound (C046) | 30<br>10<br>3 | +++<br>+++<br>+/- |
| Compound (C049) | 30<br>10 | +++<br>+ |

| Table 21 | | |
|---|---|---|
| Name of Test Compound | Concentration (μM) | Enhancing Action on Glucose Uptake |
| Compound (C050) | 10<br>3 | +++<br>+/- |
| Compound (C053) | 30<br>10<br>3 | +++<br>+++<br>+ |
| Compound (C056) | 30<br>10<br>3 | +++<br>+++<br>+/- |
| Compound (C057) | 30<br>10 | +++<br>+/- |
| Compound (C058) | 30 | +++ |
| Compound (C061) | 30 | + |
| Compound (C064-1) | 30<br>10 | +++<br>++ |
| Compound (C064-2) | 30<br>10<br>3 | +++<br>+<br>+/- |
| Compound (C066) | 30<br>10 | ++<br>++ |

Table continued

| Table 21 | | |
|---|---|---|
| Name of Test Compound | Concentration (μM) | Enhancing Action on Glucose Uptake |
| Compound (C072) | 30 | + |
| Compound (C073) | 10<br>3 | +/-<br>+/- |
| Compound (C074) | 30 | + |
| Compound (C075) | 30<br>10 | +<br>+/- |
| Compound (C076) | 30<br>10<br>3 | +++<br>+<br>+/- |
| Compound (C077) | 30<br>10 | +<br>+ |
| Compound (C078) | 30<br>10 | +++<br>+/- |
| Compound (C079) | 30<br>10<br>3 | +++<br>++<br>+++ |
| Compound (C080) | 30<br>10 | ++<br>+ |
| Compound (C005) | 30<br>10 | +<br>+ |
| Compound (C006) | 10 | + |
| TB3 | 10<br>3 | +++<br>+ |
| TB6 | 30 | + |

| Table 22 | | |
|---|---|---|
| Name of Test Compound | Concentration (μM) | Enhancing Action on Glucose Uptake |
| TB7 | 30<br>10 | +++<br>+++ |
| TB8 | 30<br>10<br>3 | ++<br>++<br>+/- |
| TB9 | 30<br>10<br>3 | +<br>+/-<br>+/- |
| Lespeol | 30<br>10 | ++<br>++ |
| Xanthoangelol H | 30 | + |
| Compound (C025) | 30<br>10 | +<br>+/- |

Table continued

| Table 22 | | |
|---|---|---|
| Name of Test Compound | Concentration (μM) | Enhancing Action on Glucose Uptake |
| Compound (C026) | 30<br>10<br>3 | +<br>+<br>+/- |
| Compound (C027) | 30<br>10 | +<br>+/- |
| Compound (C038) | 30<br>10 | ++<br>+ |
| Compound (FUR-1) | 30<br>10 | ++<br>+/- |
| Compound (C041) | 30 | +++ |
| Compound (C044) | 30<br>10 | +++<br>+ |
| Compound (C045) | 30<br>10 | +<br>+/- |
| Compound (C047) | 30<br>10<br>3 | +++<br>++<br>+ |
| Compound (C048) | 30<br>10<br>3 | +++<br>+<br>+/- |
| Compound (C052) | 30<br>10<br>3 | +<br>+<br>+/- |
| Compound (C055) | 30<br>10<br>3 | ++<br>+<br>+/- |
| Compound (C059) | 30<br>10 | +<br>+/- |

| Table 23 | | |
|---|---|---|
| Name of Test Compound | Concentration (μM) | Enhancing Action on Glucose Uptake |
| Compound (C-CIN) | 30<br>10<br>3 | +++<br>+/-<br>+/- |
| Compound (FUR-2) | 30<br>10<br>3 | +<br>+<br>+/- |
| 2-Hydroxy-4-methoxybenzaldehyde | 30<br>10 | +<br>+/- |
| Coumarin Compound A | 30<br>10 | +++<br>+++ |

Table continued

| Table 23 | | |
|---|---|---|
| Name of Test Compound | Concentration (μM) | Enhancing Action on Glucose Uptake |
| Coumarin Compound B | 30<br>10 | +++<br>+++ |
| Coumarin Compound C | 30 | + |
| Compound (C013) | 30 | +++ |
| Compound (C014) | 30<br>10 | +++<br>+ |
| Compound (C015) | 30<br>10<br>3 | +++<br>++<br>+/- |
| Sedanolide | 30<br>10 | ++<br>+/- |
| n-Butylidenephthalide | 30<br>10<br>3 | +++<br>++<br>+ |

Example 84 Synergistic Action of 4-Hydroxyderricin and Insulin to Enhance Glucose Uptake

As the evaluation of synergistic action of 4-hydroxyderricin prepared in Example 2 and a low-concentration insulin to enhance glucose uptake, the amount of 2-deoxyglucose uptake into mature adipocytes during the stimulation with the sample was determined partly in accordance with the method described in Example 83.

The mature adipocytes were prepared according to the method described in item (1) of Example 83.

After the termination of the culture, the medium was removed, and the cells were washed twice with a 0.1% (w/v) bovine serum albumin (manufactured by Sigma, A8022)-containing Dulbecco's modified Eagle's medium. Thereafter, 1 mL of the same medium containing a solution of 4-hydroxyderricin in dimethyl sulfoxide having a final concentration of 5 μM was added thereto. The cells were cultured overnight at 37°C in the presence of 5% carbon dioxide gas. Here, a group without addition of 4-hydroxyderricin was set as a negative control. After the overnight culture, the cells were washed twice with a HEPES buffered saline (140 mM NaCl, 5 mM KCl, 2.5 mM MgS04, 1 mM $CaCl_2$, 20 mM HEPES-Na (pH 7.4)). The amount 0.9 mL of the same buffer containing 4-hydroxyderricin having a final concentration of 5 μM was added thereto. The cells were cultured at 37°C for 45 minutes. Subsequently, insulin was added thereto so as to have a final concentration of 0.01 μg/mL. The cells were cultured for additional 30 minutes. During the culture, as a control, there was set a group with addition of insulin having a final concentration of 0.01μg/mL to the well without addition of 4-hydroxyderricin. Thereafter, 2-deoxy-[1,2-$^3$H(N)]-glucose subjected to uptake into the adipocytes was determined in the same manner as the method described in item (2) of Example 83.

As a result, the group with addition of 4-hydroxyderricin was found to show enhancement of 2-deoxy-[1,2-$^3$H(N)]-glucose uptake in the same manner as in the group with addition of insulin, as compared to the negative control. The group with the addition simultaneously with insulin showed enhancement of glucose uptake more than any of the group with the addition of insulin alone and the group with addition of 4-hydroxyderricin alone. In other words, 4-hydroxyderricin was found to show an action of synergistically increasing the enhancing activity on glucose uptake by the addition simultaneously with insulin. The results are shown in Figure 65. In Figure 65, the axis of abscissas is each sample, and the axis of ordinates is the amount of 2-deoxy-[1,2-$^3$H(N)]-glucose (dpm).

Example 85 Enhancing Action of 4-Hydroxyderricin on Glucose Uptake Inhibited by Cytochalasin B

The influence of cytochalasin B on 2-deoxyglucose uptake into matured adipocytes during the stimulation with the sample in the adipocytes was tested in accordance with the method shown in Example 83 on whether or not the enhancing action of 4-hydroxyderricin on glucose uptake shown in Example 83 is inhibited by cytochalasin B, which is an inhibitor of a glucose transporter.

Specifically, as a sample, a group with addition of a dimethyl sulfoxide solution of 4-hydroxyderricin so as to have a final concentration of 7.8 μM was set. Here, there were set a group without addition of the sample as a negative

control and a group with addition of insulin so as to have a final concentration of 1 μg/mL as a positive control. Furthermore, there was set a group with addition of cytochalasin B (manufactured by nacalai tesque, 10435-81) to each group so as to have a final concentration of 40 μM in the same time as the time of addition of insulin. Thereafter, 2-deoxy-[1,2-$^3$H(N)]-glucose subjected to uptake into the adipocytes was determined in the same manner.

As a result, the group with addition of 4-hydroxyderricin was found to show enhancement of 2-deoxy-[1,2-$^3$H(N)]-glucose uptake in the same manner as in the group with addition of insulin, as compared to the negative control, but 2-deoxy-[1,2-$^3$H(N)]-glucose uptake was almost completely inhibited by the addition of cytochalasin B in every group. In other words, it could be confirmed that activity of 4-hydroxyderricin to enhance glucose uptake was mediated by a glucose transporter in the same manner as in insulin. The results are shown in Figure 66. In Figure 66, the axis of abscissas is each sample, and the axis of ordinates is the amount of 2-deoxy-[1,2-$^3$H(N)]-glucose (dpm).

Example 86 Enhancing Action of Glucose Uptake by Stimulation with Insulin and 4-Hydroxyderricin into Adipocytes Which Were Induced to Be Differentiated by Xanthoangelol

Adipocytes which were induced to be differentiated by xanthoangelol were obtained, and thereafter it was confirmed on whether the glucose uptake into the adipocytes is enhanced by stimulation with insulin or 4-hydroxyderricin.

Specifically, as a sample, there was set a group with addition of a dimethyl sulfoxide solution of xanthoangelol having a final concentration of 10 μM. Thereafter, the medium and the sample were exchanged in the same manner as in the method described in Example 82 except that 3-isobutyl-1-methylxanthine having a final concentration of 0.5 mM was added concurrently with the addition of dexamethasone, to give mature adipocytes which were induced to be differentiated by xanthoangelol.

Next, the amount of 2-deoxyglucose uptake into the mature adipocytes during the stimulation with insulin or 4-hydroxyderricin in the resulting mature adipocytes was determined in the same manner as in the method described in Example 83.

Specifically, there were set the group without the addition and the group with addition of insulin having a final concentration of 1 μg/mL or 4-hydroxyderricin having a final concentration of 20 μM. Thereafter, the amount of 2-deoxy-[1,2-$^3$H(N)]-glucose uptake into the adipocytes was determined in the same manner.

As a result, the mature adipocytes which were induced to be differentiated by xanthoangelol were found to show enhancement of 2-deoxy-[1,2-$^3$H(N)]-glucose uptake by stimulation with insulin or 4-hydroxyderricin. In other words, the mature adipocytes which were induced to be differentiated by xanthoangelol were found to show enhancement of glucose uptake by stimulation with insulin or 4-hydroxyderricin. From the above, it was shown that insulin is not necessary at all from the induction of differentiation to enhancement of glucose uptake by utilizing xanthoangelol and 4-hydroxyderricin. The results are shown in Figure 67. In Figure 67, the axis of abscissas is each sample, and the axis of ordinates is the amount of 2-deoxy-[1,2-$^3$H(N)]-glucose (dpm).

Example 87 Toxicity Test of 4-Hydroxyderricin and Xanthoangelol

The influence of 4-hydroxyderricin and xanthoangelol by forcible oral administration for 3 consecutive days was examined using female 12-week old ICR mice (Japan SLC, Inc.). 4-Hydroxyderricin or xanthoangelol was suspended or dissolved in a 0.5% (w/v) carboxymethyl cellulose solution (CMC) or an olive oil so as to have a concentration of 20% (w/v). The suspension or solution was forcibly orally administered once a day for 3 days in an amount of 5 mL per 1 kg body weight (1 g/kg in terms of 4-hydroxyderricin or xanthoangelol). Three cases were used for each group. No cases of death were found during an experimental period with 4-hydroxyderricin and xanthoangelol in any administration solvent of CMC and the olive oil, and no change was also found in a general symptom.

Example 88 Amelioration Effects on Diabetes of 4-Hydroxyderricin

Pathological amelioration effects of 4-hydroxyderricin were examined using type II diabetes model mice. Experiments were carried out with five male 12-week old KK-Ay mice (CLEA Japan, Inc.) per group. 4-Hydroxyderricin was suspended or dissolved in a 0.5% (w/v) carboxymethyl cellulose solution (CMC) so as to have a concentration of 4% (w/v), and the suspension or solution was forcibly orally administered to the mice once a day at 200 mg/kg for consecutive days. CMC was administered to each mice in the control group at 5 mL/kg in the same manner. On the day before the beginning of administration, on the fourth day and on the eighteenth day, blood was collected from the veins of mouse tails, and the blood glucose level was determined with a simple blood glucose meter, ACCU-CHEK Compact (Roche Diagnostics K.K.). The results are shown in Table 24. By administration of 4-hydroxyderricin (200 mg/kg), the blood glucose level was found to be lowered. Here, during the experimental period, no changes were found in body weight and general symptoms.

Table 24

| | Blood Glucose Level (mg/dL) | | |
|---|---|---|---|
| | Previous Day | Fourth Day | Eighteenth Day |
| Control Group | 263 ± 19 | 261 ± 13 | 276 ± 19 |
| Group Administered with 4-Hydroxyderricin (200 mg/kg) | 262 ± 14 | 187 ± 9 | 191 ± 10 |
| Mean ± Standard Error | | | |

Example 89 Amelioration Effects on Diabetes of Xanthoangelol

Pathological amelioration effects of xanthoangelol were examined using type II diabetes model mice. Experiments were carried out with five female 12-week old KK-Ay mice (CLEA Japan, Inc.) per group. Xanthoangelol was suspended or dissolved in an olive oil so as to have a concentration of 4% (w/v), and the suspension or solution was forcibly orally administered to the mice once a day at 200 mg/kg for consecutive days. The olive oil was administered to mice in the control group at 5 mL/kg in the same manner. On the day before the beginning of administration, on the fourth day and on the twenty-second day, the blood glucose level was determined in the same manner as in Example 88. In addition, on the twenty-second day, blood was simultaneously collected from abdominal cava, and the insulin concentration in serum was determined with Lebis Insulin Kit (Shibayagi Co., Ltd.). The results are shown in Table 25. By administration of xanthoangelol (200 mg/kg), the blood glucose level and the insulin concentration were found to be lowered. In other words, the action of ameliorating insulin resistance was found in xanthoangelol. During the experimental period, no changes were found in body weight and general symptoms.

Table 25

| | Blood Glucose Level (mg/dL) | | | Insulin (ng/mL) |
|---|---|---|---|---|
| | Previous Day | Fourth Day | Twenty-second Day | |
| Control Group | 249 ± 18 | 276 ± 50 | 385 ± 64 | 74 ± 24 |
| Group Administered with Xanthoangelol (200 mg/kg) | 249 ± 13 | 192 ± 16 | 209 ± 8 | 31 ± 7 |
| Mean ± Standard Error | | | | |

Example 90 Amelioration Effects on Diabetes by Combined Use of 4-Hydroxyderricin and Xanthoangelol

Pathological amelioration effects of a combined use of 4-hydroxyderricin and xanthoangelol were examined using type II diabetes model mice. Experiments were carried out with five male 12-week old KK-Ay mice (CLEA Japan, Inc.) per group. Each of 4-hydroxyderricin and xanthoangelol was suspended or dissolved in an olive oil so as to have a concentration of 4% (w/v), and the suspension or solution was forcibly orally administered to the mice once a day so that each administration concentration became 100 mg/kg. The olive oil was administered to the mice in the control group at 5 mL/kg in the same manner. On the day before the beginning of administration, on the fourth day and on the eighteenth day, the blood glucose level was determined in the same manner as in Example 88. The results are shown in Table 26. By a combined administration of 4-hydroxyderricin and xanthoangelol (each 100 mg/kg), the blood glucose level was found to be lowered.

Table 26

| | Blood Glucose Level (mg/dL) | | |
|---|---|---|---|
| | Previous Day | Fourth Day | Eighteenth Day |
| Control Group | 263±16 | 329±38 | 355±20 |
| Group with Combined Administration of 4-Hydroxyderricin + Xanthoangelol (100 mg/kg each) | 262±13 | 181±11 | 215±46 |
| Mean ± Standard Error | | | |

Example 91 Amelioration Action for Glucose Tolerance by 4-hydroxyderricin and Combined Use of 4-hydroxyderricin and xanthoangelol

Amelioration effects for glucose tolerance by 4-hydroxyderricin and combined use of 4-hydroxyderricin and xanthoangelol were examined using a type II diabetes model mouse. Experiments were carried out with five male 12-week old KK-Ay mice (CLEA Japan, Inc.) per group. 4-Hydroxyderricin which was previously suspended or dissolved in an olive oil so as to have a concentration of 4% (w/v) was forcibly orally administered to a group to be administered with 4-hydroxyderricin at 200 mg/kg once a day for consecutive days. 4-Hydroxyderricin and xanthoangelol which were each suspended or dissolved in an olive oil to 4% (w/v) were mixed in equal volumes, and the mixture was forcibly orally administered to a group to be administered with a combined use of 4-hydroxyderricin and xanthoangelol once a day for consecutive days so that each administration concentration became 100 mg/kg. An olive oil was administered to the mice in the control group at 5 mL/kg in the same manner. The mice were fasted over one night spanning from the fifteenth day to the sixteenth day from administration, and a glucose tolerance test was carried out. Four hours after the administration of the sample, a 10% glucose solution was intraperitoneally administered at 1 g/kg. Thirty minutes before glucose tolerance and 30 minutes after the tolerance, blood was collected from veins of the tail, and the blood glucose level was determined with a simple blood glucose meter, ACCU-CHEK Compact (Roche Diagnostic K.K.). The results are shown in Table 27. By the administration of 4-hydroxyderricin alone or the administration of a combined use of 4-hydroxyderricin with xanthoangelol, the amelioration effects for glucose tolerance were found.

Table 27

| | Blood Glucose Level (mg/dL) | |
|---|---|---|
| | Before Glucose Tolerance | 30 Minutes After Tolerance |
| Control Group | 102 ± 8 | 269 ± 19 |
| Group Administered with 4-hydroxyderricin (200 mg/kg) | 93 ± 2 | 224 ± 11 |
| Group Administered with Combined Use of 4-hydroxyderricin + Xanthoangelol (100 mg/kg each) | 91 ± 7 | 211 ± 9 |
| Mean ± Standard Error | | |

Example 92 Amelioration Action for Glucose Tolerance of Xanthoangelol

Amelioration effects for glucose tolerance of xanthoangelol were examined using female 12-week old KK-Ay mice. Xanthoangelol which was previously suspended or dissolved in an olive oil in a concentration of 4% (w/v) was forcibly orally administered to a group to be administered with xanthoangelol at 200 mg/kg once a day for consecutive days. An olive oil was administered to the mice in the control group at 5 mL/kg in the same manner. The mice were fasted over one night spanning the sixteenth day to the seventeenth day from administration, and the glucose tolerance test was carried out in the same manner as in Example 91. The results are shown in Table 28. A suppressive action by xanthoangelol on the elevation in the blood glucose level after the glucose tolerance was found.

Table 28

| | Blood Glucose Level (mg/dL) | |
|---|---|---|
| | Before Glucose Tolerance | 30 Minutes After Tolerance |
| Control Group | 91 ± 3 | 300 ± 23 |
| Group Administered with Xanthoangelol (200 mg/kg) | 106 ± 7 | 234 ± 15 |
| Mean ± Standard Error | | |

Example 93 Determination of Activation Ability for Peroxisome Proliferator-Activated Receptor γ (PPARγ)

The activation ability for $PPAR_{\gamma}$ was determined by a reporter assay using a fusion protein of an yeast GAL4 DNA-binding domain and a human PPARγ ligand-binding site.

(1) Preparation of Plasmid for Reporter Assay
A plasmid (pGAL4/GL3-Promoter) was prepared by repeating five times insertion of a binding site (UASg) of GAL4 into a multicloning site of a pGL3-Promoter vector (Promega). In addition, a ligand-binding site (amino acid sequence

204 to 505) of the $PPAR_\gamma$ gene was cloned from a human liver cDNA library (manufactured by TAKARA BIO INC.) by a polymerase chain reaction, and the ligand-binding site was inserted into a multicloning site of a pFA-CMV plasmid vector (STRATAGENE) to give pFA-CMV (PPARγ).

*Escherichia coli* JM-109 strain was transformed with each of the two kinds of the plasmids obtained by the above-mentioned procedures, and pFA-CMV and pRL-TK (Promega) according to a conventional method. Each of the *Escherichia coli* cells was cultured with shaking at 37°C for 18 hours in an LB medium containing 100 μg/mL ampicillin. Thereafter, the plasmids of each were purified according to the procedure manual of QIAfilter Plasmid midi Kit (QIAGEN).

(2) Gene Transfer into Animal Cells

CV-1 cells were seeded in a 24-well plate so as to have a density of 4 X $10^4$ cells per well. Thereafter, the cells were cultured in a Dulbecco's modified Eagle's medium (manufactured by Sigma, D5796) containing 10% fetal bovine serine (manufactured by Bio Whittaker) for 24 hours under the conditions of 37°C and 5% $CO_2$. Thereafter, in accordance with the procedure manual of LipofectAmine 2000 (Invitrogen), the gene transfer was carried out into the CV-1 cells with 200 ng of pFA-CMV (PPARγ) or pFA-CMV, 400 ng of pGAL4/GL3-promoter, and 200 ng of pRL-TK per well, and thereafter the cells were cultured for 24 hours under the conditions of 37°C and 5% $CO_2$. Thereafter, the medium was exchanged with a Dulbecco's modified Eagle's medium to which a test compound dissolved in dimethyl sulfoxide was previously added so as to have a final concentration of 10 μM. Thereafter, the cells were cultured for additional 24 hours. In addition, there were set a group with addition of dimethyl sulfoxide in place of a test compound as a negative control, and a group with addition of troglytazone (manufactured by Calbiochem) so as to have a final concentration of 3 μM as a positive control. The cells were washed with a phosphate buffered saline, and 5 X PassiveLysisBuffer (Promega) which was previously diluted in 5-folds with distilled water was added thereto in an amount of 100 μL per well. The mixture was shaken at room temperature for 15 minutes to lyse cells. Twenty microliters of this lysed solution was applied in accordance with the procedure manual of Dual Luciferase Assay System (Promega), and a fluorescent luciferase emission intensity and a *Renilla mullerei* luciferase emission intensity were measured using Mithras-LB940 (BERTHOLD TECHNOLOGIES GmbH). The enhancing action for transcriptional activation of each test compound was shown as a relative value obtained by calculating a ratio of the fluorescent luciferase emission intensity to the *Renilla mullerei* luciferase emission intensity, wherein the value of the group with addition of the negative control is defined as 1. The results are shown in Figure 68.

As a result, when pFA-CMV (PPARγ) was used, the enhancing action for transcriptional activation is found in the group with addition of troglytazone, which is a positive control, while no enhancing action for transcriptional activation was found in any of the groups with addition of each test compound of 4-hydroxyderricin, xanthoangelol, xanthohumol, xanthoangelol F and xanthoangelol H. In addition, when pFA-CMV was used, no enhancing action for transcriptional activation was found in cases where any of the compounds were used. It was shown from the above that the enhancing action for transcriptional activation accompanying the PPARγ activation was found in troglytazone, which is a positive control, while no activation ability for PPARγ is found in 4-hydroxyderricin, xanthoangelol, xanthohumol, xanthoangelol F and xanthoangelol H.

Example 94 Suppressive Effects of Xanthoangelol and 4-Hydroxyderricin on Incidence of Diabetes

The suppressive effects of xanthoangelol and 4-hydroxyderricin on incidence of diabetes were examined using type II diabetes model mice. Experiments were carried out with seven male 5-week old KK-Ay mice (CLEA Japan, Inc.) per group. Xanthoangelol and 4-hydroxyderricin were each mixed in a powder feed (CE-2: CLEA Japan, Inc.) so as to have a ratio of 0.15%, and the mice were fed with the blended power feed. The mice in the control group were fed with the powder feed (CE-2). On the day before the beginning of administration, and on the fifteenth day, the blood glucose level was determined in the same manner as in Example 88. In addition, as an index of the incidence of diabetes, amounts of drunk water from the second to eighth days and from the ninth to fifteenth days from the administration were determined, and an average amount of drunk water per day was calculated, respectively. The results are shown in Table 29. By administration of a mixed feed with xanthoangelol or 4-hydroxyderricin, the suppression of increase in a blood glucose level was found. Further, the suppression of increase in the amount of drunk water which is an index of the incidence of diabetes was found. In other words, the effects for suppressing the incidence of diabetes was found in xanthoangelol and 4-hydroxyderricin. During the experimental period, no changes were found in body weight, amount of feed taken and general symptoms.

Table 29

| | Blood Glucose Level (mg/dL) | | Amount of Drunk Water (mL/day) | |
|---|---|---|---|---|
| | Previous Day | Fifteenth Day | Second to Eighth Day | Ninth to Fifteenth Day |
| Control Group | 226 ± 12 | 523 ± 20 | 10.1 ± 0.6 | 16.6 ± 0.7 |
| Group Administered with Xanthoangelol (0.15% Blend Feed) | 232 ± 11 | 426 ± 39 | 10.3 ± 1.3 | 14.6 ± 2.0 |
| Group Administered with 4-hydroxyderricin (0.15% Blend Feed) | 226 ± 14 | 354 ± 24 | 7.6 ± 0.2 | 10.0 ± 0.6 |
| Mean ± Standard Error | | | | |

Example 95 Enhancing Action on Glucose Uptake into Insulin-Resistant Cells

(1) Preparation of Insulin-Resistant Mature Adipocytes
It is said that tumor necrosis factor α (TNF-α) is deeply involved in insulin resistance, and it has been known that a signal from an insulin receptor is inhibited by treating mature adipocytes with TNF-α, so that a reaction of glucose uptake is inhibited by insulin stimulation *(Proc. Natl. Acad. Sci. USA,* vol. 91, p4854-4858 (1994)). The enhancing action by 4-hydroxyderricin on glucose uptake into mature adipocytes in which insulin resistance was induced by TNF-α was evaluated.
The induction of differentiation into mature adipocytes was carried out in the same manner as in the method described in Example 83, except that after preparation of the mature adipocytes, in order to induce insulin resistance in these mature adipocytes, mouse-derived TNF-α (manufactured by Cosmobio, 3410) was added to the medium so as to have a final concentration of 10 ng/mL or 20 ng/mL, and the adipocytes were cultured for additional 2 days. In addition, a well containing no mouse-derived TNF-α was set. Here, during the medium exchange until the cells were lysed, the mouse-derived TNF-α was continuously contained at each concentration.
(2) Determination of Enhancing Action on Glucose Uptake in Insulin-Resistant Mature Adipocytes
The enhancing action on glucose uptake was evaluated in the same manner as in the method described in Example 83. For each of the cells prepared in item (1) of Example 95 mentioned above, there were set a group containing 4-hydroxyderricin at a final concentration of 30 μM, a group without addition of the sample as a negative control, and a group with treatment with insulin, and the radioactivity of each group was determined.

As a result, an increase ratio of the amount of glucose uptake to a negative control upon addition of each sample was obtained from the value of the amount of 2-deoxy-[1,2-$^3$H(N)]-glucose uptake. Further, in order to examine the effects by insulin resistance, the increase was normalized so that the an increase of the amount of glucose uptake by each sample when TNF-α was not added is defined as 100%. As a result, the enhancing action on glucose uptake in the group treated with insulin dropped to around 40% in the mature adipocytes treated with mouse-derived TNF-α, so that these adipocytes were certainly insulin resistant. On the other hand, the enhancing action on glucose uptake in the group treated with 4-hydroxyderricin remained around 80% even in insulin-resistant cells treated with mouse-derived TNF-α. It was shown from the above that 4-hydroxyderricin enhances glucose uptake also in the insulin-resistant mature adipocytes. The results are shown in Figure 69. In other words, Figure 69 shows a change in the increase of the amount of glucose uptake in the insulin-resistant cells, wherein the axis of ordinates is a change in the increase ratio of the amount of glucose uptake, and the axis of abscissas is a concentration of mouse-derived TNFα to be added.

## INDUSTRIAL APPLICABILITY

According to the present invention, there is provided a medicament, a food, a beverage or a feed for the treatment or prevention of a disease accompanying an abnormality in an amount of insulin or insulin response, each comprising at least one compound selected from the group consisting of a compound represented by the above-mentioned general formula (Formula 1), a compound represented by the above-mentioned general formula (Formula 2), a compound represented by the above-mentioned general formula (Formula 3), a derivative thereof and a pharmacologically acceptable salt thereof. The medicament is useful as a therapeutic agent or prophylactic agent for a disease accompanying

an abnormality in an amount of insulin or insulin response, such as diabetes or obesity. In addition, by taking the food or beverage as daily foodstuff and the like, amelioration in symptoms of a disease accompanying an abnormality in an amount of insulin or insulin response can be made. Therefore, functional foodstuff comprising the effective ingredient of the present invention are functional foodstuff useful in maintaining homeostasis of a living body by an insulin-mimetic action. Also, according to the present invention, there is also provided an insulin-mimetic agent comprising at least one compound selected from the group consisting of a compound represented by the above-mentioned general formula (Formula 1), a compound represented by the above-mentioned general formula (Formula 2), a compound represented by the above-mentioned general formula (Formula 3), a derivative thereof and a pharmacologically acceptable salt thereof. The insulin-mimetic agent is useful in functional studies of insulin, or screening of a medicament for a disease associated with insulin. Also, according to the present invention, there is provided an agent for enhancing glucose uptake into a cell, comprising at least one compound selected from the group consisting of a compound represented by the above-mentioned general formula (Formula 1), a compound represented by the above-mentioned general formula (Formula 2), a compound represented by the above-mentioned general formula (Formula 3), a derivative thereof and a pharmacologically acceptable salt thereof. The agent for enhancing glucose uptake is also useful in treating or preventing a disease requiring the enhancing action on glucose uptake into a cell for the treatment or prevention, or preparing a food, beverage or feed for the treatment or prevention of the disease, or screening a drug for a disease requiring the enhancing action on glucose uptake. In addition, according to the present invention, there is provided an agent for inducing differentiation into an adipocyte, comprising at least one compound selected from the group consisting of a compound represented by the above-mentioned general formula (Formula 1), a compound represented by the above-mentioned general formula ( Formula 2), a compound represented by the above-mentioned general formula (Formula 3), a derivative thereof and a pharmacologically acceptable salt thereof. The agent for inducing the differentiation is also useful in treating or preventing a disease requiring the activity of inducing differentiation into an adipocyte for the treatment or prevention, or preparing a food, a drink or a feed for the treatment or prevention of the disease, or screening a drug for a disease requiring the action for inducing differentiation.

## Claims

1. A therapeutic agent or prophylactic agent for a disease accompanying an abnormality in an amount of insulin or insulin response, **characterized in that** the therapeutic agent or prophylactic agent comprises as an effective ingredient at least one compound selected from the group consisting of a compound represented by the following general formula (Formula 1):

(Formula 1)

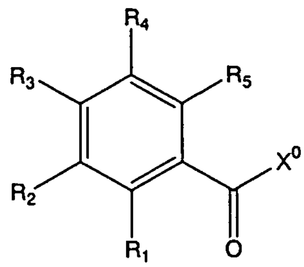

wherein each of $R_1$ to $R_5$, which may be the identical or different, is a hydrogen atom, or a hydroxyl group which may be esterified or etherified, a halogen group, an acyl group, an amino group, a nitro group, a hydroperoxy group, an aliphatic group, an aromatic group, an aromatic aliphatic group or a sugar residue, or one or more pairs selected from $R_1$ and $R_2$, $R_2$ and $R_3$, $R_3$ and $R_4$, and $R_4$ and $R_5$ may form a ring containing one or more atoms selected from a carbon atom, an oxygen atom, a nitrogen atom and a sulfur atom in a possible range, wherein a hydroxyl group which may be esterified or etherified, a halogen group, an acyl group, an amino group, a nitro group, a hydroperoxy group, an aliphatic group, an aromatic group, an aromatic aliphatic group, or a sugar residue may be bound to the above ring; and wherein $X^0$ is a hydrogen atom, an aliphatic group, an aromatic group, or an aromatic aliphatic group, wherein a hydroxyl group which may be esterified or etherified, a halogen group, an acyl group, an amino group, a nitro group, a hydroperoxy group, an aliphatic group, an aromatic group, an aromatic aliphatic group, or a sugar residue may be bound to the aliphatic group, the aromatic group, or the aromatic aliphatic group;
a compound represented by the following general formula (Formula 2):

(Formula 2)

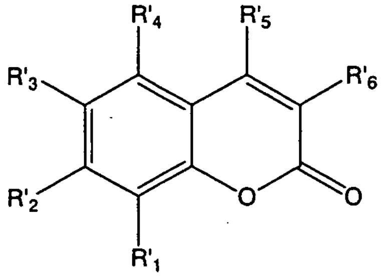

,

wherein each of R'$_1$ to R'$_6$, which may be identical or different, is a hydrogen atom, a hydroxyl group which may be esterified or etherified, a halogen group, an acyl group, an amino group, a nitro group, a hydroperoxy group, an aliphatic group, an aromatic group, an aromatic aliphatic group, or a sugar residue, one or more pairs selected from R'$_1$ and R'$_2$, R'$_2$ and R'$_3$, R'$_3$ and R'$_4$, R'$_4$ and R'$_5$, and R'$_5$ and R'$_6$ may form a ring containing one or more atoms selected from a carbon atom, an oxygen atom, a nitrogen atom and a sulfur atom in a possible range, wherein a hydroxyl group which may be esterified or etherified, a halogen group, an acyl group, an amino group, a nitro group, a hydroperoxy group, an aliphatic group, an aromatic group, an aromatic aliphatic group, or a sugar residue may be bound to the above ring;

a compound represented by the following general formula (Formula 3):

(Formula 3)

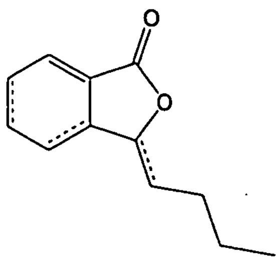

wherein a bond containing a dotted line is a single bond or a double bond; derivatives thereof; and pharmacologically acceptable salts thereof.

2. The therapeutic agent or prophylactic agent according to claim 1, wherein the compound represented by the above general formula (Formula 1) is a compound represented by the following general formula (Formula 4):

(Formula 4)

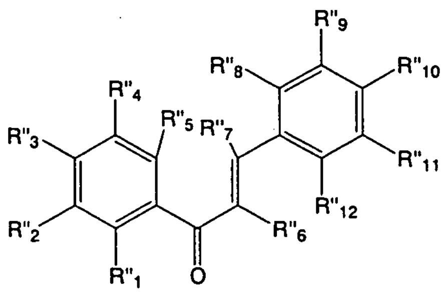

,

wherein each of R"$_1$ to R"$_{12}$, which may be identical or different, is a hydrogen atom, a hydroxyl group which may be esterified or etherified, a halogen group, an acyl group, an amino group, a nitro group, a hydroperoxy group, an aliphatic group, an aromatic group, an aromatic aliphatic group, or a sugar residue, or one or more pairs selected from R"$_1$ and R"$_2$, R"$_2$ and R"$_3$, R"$_3$ and R"$_4$, R"$_4$ and R"$_5$, R"$_8$ and R"$_9$, R"$_9$ and R"$_{10}$, R"$_{10}$ and R"$_{11}$, and R"$_{11}$ and R"$_{12}$ may form a ring containing one or more atoms selected from a carbon atom, an oxygen atom, a nitrogen atom,

and a sulfur atom in a possible range, wherein a hydroxyl group which may be esterified or etherified, a halogen group, an acyl group, an amino group, a nitro group, a hydroperoxy group, an aliphatic group, an aromatic group, an aromatic aliphatic group, or a sugar group may be bound to the above ring, and/or
a compound represented by the following general formula (Formula 5):

(Formula 5)

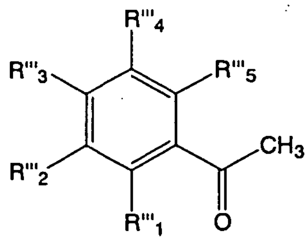
,

wherein each of $R'''_1$ to $R'''_5$, which may be identical or different, is a hydrogen atom, a hydroxyl group which may be esterified or etherified, a halogen group, an acyl group, an amino group, a nitro group, a hydroperoxy group, an aliphatic group, an aromatic group, an aromatic aliphatic group, or a sugar residue, or one or more pairs selected from $R'''_1$ and $R'''_2$, $R'''_2$ and $R'''_3$, $R'''_3$ and $R'''_4$, and $R'''_4$ and $R'''_5$ may form a ring containing one or more atoms selected from a carbon atom, an oxygen atom, a nitrogen atom, and a sulfur atom in a possible range, wherein a hydroxyl group which may be esterified or etherified, a halogen group, an acyl group, an amino group, a nitro group, a hydroperoxy group, an aliphatic group, an aromatic group, an aromatic aliphatic group, or a sugar residue may be bound to the above ring.

**3.** The therapeutic agent or prophylactic agent according to claim 2, wherein the compound represented by the above general formula (Formula 4) is a compound represented by the following general formula (Formula 6):

(Formula 6)

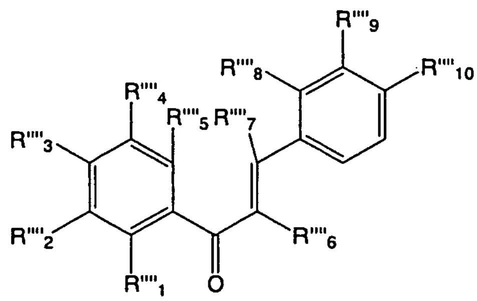
,

wherein each of $R''''_1$, $R''''_3$, $R''''_4$, and $R''''_5$, which may be identical or different, is a hydrogen atom, a hydroxyl group, a methyl group, a methoxy group, a prenyl group, a geranyl group, a halogen group, an acetoxy group, a methylbutyl group, a farnesyl group, an ethoxy group, a benzyl group or a benzyloxy group; $R''''_2$ is a hydrogen atom, a hydroxyl group, a methyl group, a methoxy group, a halogen group, an acetoxy group, an ethoxy group, a benzyl group, a benzyloxy group, or an aliphatic group having 5 to 15 carbon atoms; each of $R''''_6$ and $R''''_7$, which may be identical or different, is a hydrogen atom or a hydroxyl group; each of $R''''_8$, $R''''_9$, and $R''''_{10}$, which may be identical or different, is a hydrogen atom, a hydroxyl group, a tetrahydropyranyloxy group, an acetoxy group, a methoxy group, a geranyloxy group, a prenyl group, a halogen group or a nitro group, with proviso that a case where each of $R''''_3$, $R''''_9$ and $R''''_{10}$ is a hydroxyl group at the same time is excluded, wherein $R''''_1$ and $R''''_2$, or $R''''_2$ and $R''''_3$ may together form a ring structure represented by the following formula (Formula 7):

(Formula 7)

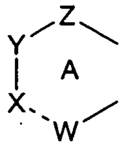

,

wherein each of W and Z is a carbon atom or an oxygen atom; X is a carbon atom; Y is 0 or 1 carbon atom; a dotted line is a single bond or a double bond; and the above ring A is a 5-membered ring or a 6-membered ring; when the ring A is a 5-membered ring, R""$_1$ is W, and R""$_2$ is Z, or R""$_2$ is W and R""$_3$ is Z; when R""$_1$ is W and R""$_2$ is Z, W is an oxygen atom, a W-X bond is a single bond, each of X and Z is a carbon atom, and Y is not present; and further in this case, 1-hydroxy-1-methylethyl group is bound to X, and when R""$_2$ is W and R""$_3$ is Z, W is a carbon atom, a W-X bond is a single bond, X is a carbon atom, Y is not present, and Z is an oxygen atom; and further in this case, a 1-hydroxy-1,5-dimethyl-4-hexenyl group is bound to X;
when the ring A is a 6-membered ring, R""$_1$ is W and R""$_2$ is Z, or R""$_2$ is W and R""$_3$ is Z: when R""$_1$ is W and R""$_2$ is Z, W is an oxygen atom, a W-X bond is a single bond, and each of X, Y and Z is a carbon atom at the same time, and further in this case, one or more atoms selected from a hydrogen atom, a hydroxyl group, a methyl group and an isohexenyl group are bound to X and Y, or X and Y together form a hydroxydimethylcyclohexane ring wherein a methyl group is bound to X; when R""$_2$ is W and R""$_3$ is Z, each of W, X and Y is a carbon atom, a W-X bond is a double bond, and Z is an oxygen atom, and further in this case, a methyl group and an isohexenyl group are bound to Y.

**4.** The therapeutic agent or prophylactic agent according to claim 1, wherein the compound represented by the above general formula (Formula 1) is at least one compound selected from the group consisting of xanthoangelol, 4-hydroxyderricin, xanthoangelol H, 1-(5,6,7,8,8a,10a-hexahydro-1,7-dihydroxy-8,8,10a-trimethyl-9H-xanthen-4-yl)-3-(4-hydroxyphenyl)-2-propen-1-one, 1-(3,4-dihydro-3,5-dihydroxy-2-(3-isohexenyl)-2-methyl-2H-benzopyran-8-yl)-3-(4-hydroxyphenyl)-2-propen-1-one, 1-[2,3-dihydro-4-hydroxy-2-(1-hydroxy-1,5-dimethyl-4-hexenyl)-benzofuran-5-yl]-3-(4-hydroxyphenyl)-2-propen-1-one, 1-[2,3-dihydro-2-(1-hydroxy-1-methylethyl)-4-methoxy-benzofuran-7-yl]-3-(4-hydroxyphenyl)-2-propen-1-one, 1-[2,4-dihydroxy-3-(6,7-dihydroxy-3,7-dimethyl-2-octenyl)phenyl]-3-(4-hydroxyphenyl)-2-propen-1-one, 1-[3-(7-ethoxy-6-hydroxy-3,7-dimethyl-2-octenyl)-2,4-dihydroxyphenyl]-3-(4-hydroxyphenyl)-2-propen-1-one, 1-[3-(2,5-epoxy-2,6,6-trimethyl-cyclohexylmethyl)-2-hydroxy-4-methoxyphenyl]-3-(4-hydroxyphenyl)-2-propen-1-one, 1-[2-hydroxy-3-(7-hydroperoxy-3,7-dimethyl-2,5-octadienyl)-4-methoxyphenyl]-3-(4-hydroxyphenyl)-2-propen-1-one, 1-[2-hydroxy-3-(6-hydroperoxy-3,7-dimethyl-2,7-octadienyl)-4-methoxyphenyl]-3-(4-hydroxyphenyl)-2-propen-1-one, 1-[2-hydroxy-3-(7-hydroxy-3,7-dimethyl-2,5-octadienyl)-4-methoxyphenyl]-3-(4-hydroxyphenyl)-2-propen-1-one, xanthoangelol G, xanthoangelol F, lespeol, isobavachalcone, xanthohumol, 4,2'-dihydroxy-4'-methoxychalcone, 4,2'-dihydroxy-3'-methyl-4'-methoxychalcone, bavachalcone, 4-tetrahydropyranyloxy-2'-hydroxy-3'-prenyl-4'-methoxychalcone, 3,4,2'-trihydroxy-3'-prenyl-4'-methoxychalcone, 4,2'-diacetoxy-3'-prenyl-4'-methoxychalcone, 4,4'-dimethoxy-2'-hydroxy-3'-prenylchalcone, 3,4-dihydroxy-2',4'-dichlorochalcone, 4,2'-dihydroxy-3'-(3-methylbutyl)-4'-methoxychalcone, 4,4',6'-trimethoxy-2'-hydroxy-3'-prenylchalcone, 4,2'-dihydroxy-3'-prenyl-4'-ethoxychalcone, 4,2'-dihydroxy-3'-geranyl-4'-ethoxychalcone, 4,2'-dihydroxy-3'-farnesyl-4'-methoxychalcone, 4,2'-dihydroxy-3'-benzyl-4'-methoxychalcone, 4,2',4'-trihydroxy-3'-benzylchalcone, 4,2',4'-trihydroxy-3'-farnesylchalcone, 4,2',4'-triacetoxy-3'-geranylchalcone, 4,2'-diacetoxy-3'-geranyl-4'-methoxychalcone, 2'-acetoxy-3'-geranyl-4-hydroxy-4'-methoxychalcone, 2,2'-dihydroxy-3,3'-diprenyl-4,4'-dimethoxychalcone, 4,2'-dihydroxy-3'-geranyl-4'-benzyloxychalcone, 4-chloro-2',4'-dihydroxy-3'-geranylchalcone, 4-chloro-2',4'-dihydroxy-3'-prenylchalcone, 3-nitro-2'-hydroxy-3'-prenyl-4'-methoxychalcone, 4-hydroxy-2'-benzyloxy-3'-prenyl-4'-methoxychalcone, 4-acetoxy-2'-hydroxy-3'-prenyl-4'-methoxychalcone, 4-chloro-2'-acetoxy-3'-prenyl-4'-methoxychalcone, 4-chloro-2'-acetoxy-3'-geranyl-4'-methoxychalcone, 3,4,2'-trihydroxychalcone, 3,4,2',5'-tetrahydroxychalcone, chalcone, 4-chloro-2'-benzyloxychalcone, xanthoangelol B, xanthoangelol C, xanthoangelol D, xanthoangelol E and bavachromanol.

**5.** The therapeutic agent or prophylactic agent according to claim 1, wherein the derivative of the compound represented by the above general formula (Formula 1) is at least one compound selected from the group consisting of 4'-*O*-geranylnaringenin, isobavachin, prostratol F, 8-geranyl-4'-hydroxy-7-methoxyflavanone, 1-(2-hydroxy-3-(3-methylbutyl)-4-methoxyphenyl)-3-(4-hydroxyphenyl)-propan-1-one, 7-methoxy-8-prenyl-4'-hydroxyflavanone, 1-adamantyl-3-(3,4-dihydroxyphenyl)-2-propen-1-one, 1-adamantyl-3-hydroxy-4-(3,4-dihydroxyphenyl)-butan-1-one, and 1-adamantyl-4-(3,4-dihydroxyphenyl)- , 3-buten-1-one.

**6.** The therapeutic agent or prophylactic agent according to claim 2, wherein the compound represented by the above

general formula (Formula 5) is a compound represented by the following general formula (Formula 8):

(Formula 8)

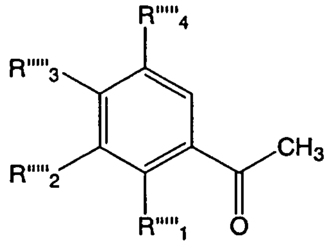

wherein R""'$_1$ is a hydroxyl group, a methyl group or a benzyloxy group; each of R""'$_2$ and R""'$_4$, which may be identical or different, is a hydrogen atom, a hydroxyl group, a methyl group, a methoxy group, an ethoxy group, a prenyl group, a methylbutyl group, a prenyloxy group, a geranyl group, a farnesyl group, a benzyl group, a benzyloxy group or a tetrahydropyranyloxy group; and R""'$_3$ is a hydrogen atom, a hydroxyl group, a methoxy group, an ethoxy group, an acetoxy group, a tetrahydropyranyloxy group, a benzyloxy group or a prenyloxy group.

**7.** The therapeutic agent or prophylactic agent according to claim 1, wherein the compound represented by the above general formula (Formula 1) is at least one compound selected from the group consisting of
2',4'-dihydroxy-5'-prenylacetophenone,
2'-hydroxy-4'-methoxy-5'-prenylacetophenone,
2'-hydroxy-3'-prenyl-4'-methoxyacetophenone,
2'-hydroxy-3'-methyl-4'-methoxyacetophenone,
2',4'-dihydroxy-3'-geranylacetophenone,
2'-hydroxy-3'-geranyl-4'-methoxyacetophenone,
2'-hydroxy-3'-(3-methylbutyl)-4'-methoxyacetophenone,
2'-hydroxy-3'-prenyl-4'-tetrahydropyranyloxyacetophenone,
2'-hydroxy-3'-geranyl-4'-tetrahydropyranyloxyacetophenone,
2'-hydroxy-3'-prenyl-4'-ethoxyacetophenone,
2'-hydroxy-3'-geranyl-4'-ethoxyacetophenone,
2',4'-dihydroxy-3'-farnesylacetophenone,
2'-hydroxy-3'-farnesyl-4'-methoxyacetophenone,
2',4'-dihydroxy-3'-benzylacetophenone,
2'-hydroxy-3'-benzyl-4'-methoxyacetophenone,
2'-methyl-4'-prenyloxyacetophenone,
2'-benzyloxyacetophenone and
2'-hydroxy-3'-geranyl-4'-benzyloxyacetophenone.

**8.** The therapeutic agent or prophylactic agent according to claim 1, wherein the compound represented by the above general formula (Formula 1) is a compound in which $X^0$ is a hydrogen atom, a 2-(2-furyl)vinyl group, a 2-(2-thienyl)vinyl group, a 4-phenyl-1,3-butadienyl group, or a 4-methyl-1,3-pentadienyl group; $R_1$ is a hydroxyl group; $R_2$ is a hydrogen atom or a prenyl group; $R_3$ is a methoxy group; and each of $R_4$ and $R_5$ is a hydrogen atom.

**9.** The therapeutic agent or prophylactic agent according to claim 1, wherein the compound represented by the above general formula (Formula 1) is at least one compound selected from the group consisting of 1-(2-hydroxy-4-methoxy-3-prenylphenyl)-3-(2-thienyl)-2-propen-1-one, 1-(2-hydroxy-4-methoxy-3-prenylphenyl)-5-phenyl-2,4-pentadien-1-one, 3-(2-furyl)-1-(2-hydroxy-3-prenyl-4-methoxyphenyl)-2-propen-1-one, 2-hydroxy-3-prenyl-4-methoxybenzaldehyde, 1-(2-hydroxy-3-prenyl-4-methoxyphenyl)-5-methyl-2,4-hexadien-1-one and 2-hydroxy-4-methoxybenzaldehyde.

**10.** The therapeutic agent or prophylactic agent according to claim 1, wherein the derivative of the compound represented by the above general formula (Formula 1) is 2-(2-furyl)-3-(2-furylmethylene)-2,3-dihydro-7-methoxy-8-prenyl-4H-1-benzopyran-4-one.

**11.** The therapeutic agent or prophylactic agent according to claim 1, wherein the compound represented by the above

general formula (Formula 2) is a compound represented by the following general formula (Formula 9):

(Formula 9)

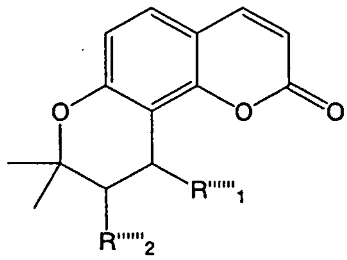

wherein each of R''''''$_1$ and R''''''$_2$, which may be identical or different, is a hydrogen atom, a hydroxyl group, an acetoxy group, or an angeloyloxy group.

**12.** The therapeutic agent or prophylactic agent according to claim 1, wherein the compound represented by the above general formula (Formula 2) is at least one compound selected from the group consisting of 3'-acetoxy-4'-angeloyloxy-3',4'-dihydroseselin, 3'-angeloyloxy-3',4'-dihydroseselin, 3'-angeloyloxy-4'-hydroxy-3',4'-dihydroseselin and xanthotoxin.

**13.** The therapeutic agent or prophylactic agent according to claim 1, wherein the compound represented by the above general formula (Formula 3) is sedanolide and/or n-butylidenephthalide.

**14.** An agent for an insulin-mimetic action **characterized in that** the agent comprises as an effective ingredient at least one compound selected from the group consisting of a compound represented by the general formula (Formula 1), a compound represented by the general formula (Formula 2), a compound represented by the general formula (Formula 3), derivatives thereof, and pharmacologically acceptable salts thereof, each as defined in claim 1.

**15.** The agent for an insulin-mimetic action according to claim 14, wherein the compound represented by the general formula (Formula 1) as defined in claim 1 is a compound represented by the general formula (Formula 4) and/or a compound represented by the general formula (Formula 5), each as defined in claim 2.

**16.** The agent for an insulin-mimetic action according to claim 15, wherein the compound represented by the general formula (Formula 4) as defined in claim 2 is a compound represented by the general formula (Formula 6) as defined in claim 3.

**17.** The agent for an insulin-mimetic action according to claim 14, wherein the compound represented by the general formula (Formula 1) as defined in claim 1 is at least one compound selected from the group consisting of xanthoangelol, 4-hydroxyderricin, xanthoangelol H, 1-(5,6,7,8,8a,10a-hexahydro-1,7-dihydroxy-8,8,10a-trimethyl-9H-xanthen-4-yl)-3-(4-hydroxyphenyl)-2-propen-1-one, 1-(3,4-dihydro-3,5-dihydroxy-2-(3-isohexenyl)-2-methyl-2H-benzopyran-8-yl)-3-(4-hydroxyphenyl)-2-propen-1-one, 1-[2,3-dihydro-4-hydroxy-2-(1-hydroxy-1,5-dimethyl-4-hexenyl)-benzofuran-5-yl]-3-(4-hydroxyphenyl)-2-propen-1-one, 1-[2,3-dihydro-2-(1-hydroxy-1-methylethyl)-4-methoxy-benzofuran-7-yl]-3-(4-hydroxyphenyl)-2-propen-1-one, 1-[2,4-dihydroxy-3-(6,7-dihydroxy-3,7-dimethyl-2-octenyl)phenyl]-3-(4-hydroxyphenyl)-2-propen-1-one, 1-[3-(7-ethoxy-6-hydroxy-3,7-dimethyl-2-octenyl)-2,4-dihydroxyphenyl]-3-(4-hydroxyphenyl)-2-propen-1-one, 1-[3-(2,5-epoxy-2,6,6-trimethyl-cyclohexylmethyl)-2-hydroxy-4-methoxyphenyl]-3-(4-hydroxyphenyl)-2-propen-1-one, 1-[2-hydroxy-3-(7-hydroperoxy-3,7-dimethyl-2,5-octadienyl)-4-methoxyphenyl]-3-(4-hydroxyphenyl)-2-propen-1-one, 1-[2-hydroxy-3-(6-hydroperoxy-3,7-dimethyl-2,7-octadienyl)-4-methoxyphenyl]-3-(4-hydroxyphenyl)-2-propen-1-one, 1-[2-hydroxy-3-(7-hydroxy-3,7-dimethyl-2,5-octadienyl)-4-methoxyphenyl]-3-(4-hydroxyphenyl)-2-propen-1-one, xanthoangelol G, xanthoangelol F, lespeol, isobavachalcone, xanthohumol, 4,2'-dihydroxy-4'-methoxychalcone, 4,2'-dihydroxy-3'-methyl-4'-methoxychalcone, bavachalcone, 4-tetrahydropyranyloxy-2'-hydroxy-3'-prenyl-4'-methoxychalcone, 3,4,2'-trihydroxy-3'-prenyl-4'-methoxychalcone, 4,2'-diacetoxy-3'-prenyl-4'-methoxychalcone, 4,4'-dimethoxy-2'-hydroxy-3'-prenylchalcone, 3,4-dihydroxy-2',4'-dichlorochalcone, 4,2'-dihydroxy-3'-(3-methylbutyl)-4'-methoxychalcone, 4,4',6'-trimethoxy-2'-hydroxy-3'-prenylchalcone, 4,2'-dihydroxy-3'-prenyl-4'-ethoxychalcone, 4,2'-dihydroxy-3'-geranyl-4'-ethoxychalcone, 4,2'-dihydroxy-3'-farnesyl-4'-methoxychalcone, 4,2'-dihydroxy-3'-benzyl-4'-methoxychalcone, 4,2',4'-trihydroxy-3'-benzylchalcone, 4,2',4'-trihydroxy-3'-farnesylchalcone, 4,2',4'-triacetoxy-3'-geranylchalcone, 4,2'-diacetoxy-3'-geranyl-4'-methoxychalcone, 2'-acetoxy-3'-geranyl-4-hydroxy-4'-methoxychalcone, 2,2'-dihydroxy-3,3'-diprenyl-4,4'-dimethoxychalcone, 4,2'-dihydroxy-3'-geranyl-4'-benzyloxychalcone, 4-chloro-2',4'-dihydroxy-3'-geranylchalcone, 4-chloro-2',4'-dihydroxy-3'-prenylchalcone, 3-nitro-2'-hydroxy-3'-prenyl-4'-methoxychalcone, 4-hydroxy-2'-benzyloxy-3'-prenyl-4'-methoxychalcone, 4-acetoxy-2'-hydroxy-3'-prenyl-4'-methoxychalcone, 4-chloro-2'-acetoxy-3'-prenyl-4'-methoxychalcone, 4-chloro-2'-acetoxy-3'-geranyl-4'-methoxychalcone, 3,4,2'-trihydroxychalcone, 3,4,2',5'-tetrahydroxychalcone, chalcone, 4-chloro-2'-benzyloxychalcone, xanthoangelol B, xanthoangelol C, xanthoangelol D, xanthoangelol E and bavachromanol.

**18.** The agent for an insulin-mimetic action according to claim 14, wherein the derivative of the compound represented by the general formula (Formula 1) as defined in claim 1 is at least one compound selected from the group consisting of 4'-*O*-geranylnaringenin, isobavachin, prostratol F, 8-geranyl-4'-hydroxy-7-methoxyflavanone, 1-(2-hydroxy-3-(3-methylbutyl)-4-methoxyphenyl)-3-(4-hydroxyphenyl)-propan-1-one, 7-methoxy-8-prenyl-4'-hydroxyflavanone, 1-adamantyl-3-(3,4-dihydroxyphenyl)-2-propen-1-one, 1-adamantyl-3-hydroxy-4-(3,4-dihydroxyphenyl)-butan-1-one, and 1-adamantyl-4-(3,4-dihydroxyphenyl)-3-buten-1-one.

**19.** The agent for an insulin-mimetic action according to claim 15, wherein the compound represented by the general formula (Formula 5) as defined in claim 2 is a compound represented by the general formula (Formula 8) as defined in claim 6.

**20.** The agent for an insulin-mimetic action according to claim 14, wherein the compound represented by the general formula (Formula 1) as defined in claim 1 is at least one compound selected from the group consisting of 2',4'-dihydroxy-5'-prenylacetophenone,
2'-hydroxy-4'-methoxy-5'-prenylacetophenone,
2'-hydroxy-3'-prenyl-4'-methoxyacetophenone,
2'-hydroxy-3'-methyl-4'-methoxyacetophenone,
2',4'-dihydroxy-3'-geranylacetophenone,
2'-hydroxy-3'-geranyl-4'-methoxyacetophenone,
2'-hydroxy-3'-(3-methylbutyl)-4'-methoxyacetophenone,
2'-hydroxy-3'-prenyl-4'-tetrahydropyranyloxyacetophenone,
2'-hydroxy-3'-geranyl-4'-tetrahydropyranyloxyacetophenone,
2'-hydroxy-3'-prenyl-4'-ethoxyacetophenone,
2'-hydroxy-3'-geranyl-4'-ethoxyacetophenone,
2',4'-dihydroxy-3'-farnesylacetophenone,
2'-hydroxy-3'-farnesyl-4'-methoxyacetophenone,
2',4'-dihydroxy-3'-benzylacetophenone,
2'-hydroxy-3'-benzyl-4'-methoxyacetophenone,
2'-methyl-4'-prenyloxyacetophenone,
2'-benzyloxyacetophenone and
2'-hydroxy-3'-geranyl-4'-benzyloxyacetophenone.

**21.** The agent for an insulin-mimetic action according to claim 14, wherein the compound represented by the general formula (Formula 1) as defined in claim 1 is a compound in which $X^0$ is a hydrogen atom, a 2-(2-furyl)vinyl group, a 2-(2-thienyl)vinyl group, a 4-phenyl-1,3-butadienyl group, or a 4-methyl-1,3-pentadienyl group; $R_1$ is a hydroxyl group; $R_2$ is a hydrogen atom or a prenyl group; $R_3$ is a methoxy group; and each of $R_4$ and $R_5$ is a hydrogen atom.

**22.** The agent for an insulin-mimetic action according to claim 14, wherein the compound represented by the general formula (Formula 1) as defined in claim 1 is at least one compound selected from the group consisting of 1-(2-hydroxy-4-methoxy-3-prenylphenyl)-3-(2-thienyl)-2-propen-1-one, 1-(2-hydroxy-4-methoxy-3-prenylphenyl)-5-phenyl-2,4-pentadien-1-one, 3-(2-furyl)-1-(2-hydroxy-3-prenyl-4-methoxyphenyl)-2-propen-1-one, 2-hydroxy-3-prenyl-4-methoxybenzaldehyde, 1-(2-hydroxy-3-prenyl-4-methoxyphenyl)-5-methyl-2,4-hexadien-1-one and 2-hydroxy-4-methoxybenzaldehyde.

**23.** The agent for an insulin-mimetic action according to claim 14, wherein the derivative of the compound represented by the general formula (Formula 1) as defined in claim 1 is 2-(2-furyl)-3-(2-furylmethylene)-2,3-dihydro-7-methoxy-8-prenyl-4H-1-benzopyran-4-one.

**24.** The agent for an insulin-mimetic action according to claim 14, wherein the compound represented by the general formula (Formula 2) as defined in claim 1 is a compound represented by the general formula (Formula 9) as defined

in claim 11.

**25.** The agent for an insulin-mimetic action according to claim 14, wherein the compound represented by the general formula (Formula 2) as defined in claim 1 is at least one compound selected from the group consisting of 3'-acetoxy-4'-angeloyloxy-3',4'-dihydroseselin, 3'-angeloyloxy-3',4'-dihydroseselin, 3'-angeloyloxy-4'-hydroxy-3',4'-dihydroseselin and xanthotoxin.

**26.** The agent for an insulin-mimetic action according to claim 14, wherein the compound represented by the general formula (Formula 3) as defined in claim 1 is sedanolide and/or n-butylidenephthalide.

**27.** A food, beverage or feed for treating or preventing a disease accompanying an abnormality in an amount of insulin or insulin response, **characterized in that** the food, beverage or feed comprises as an effective ingredient at least one compound selected from the group consisting of a compound represented by the general formula (Formula 1), a compound represented by the general formula (Formula 2), a compound represented by the general formula (Formula 3), derivatives thereof, and pharmacologically acceptable salts thereof, each as defined in claim 1.

**28.** The food, beverage or feed according to claim 27, wherein the compound represented by the general formula (Formula 1) as defined in claim 1 is a compound represented by the general formula (Formula 4) and/or a compound represented by the general formula (Formula 5), each as defined in claim 2.

**29.** The food, beverage or feed according to claim 28, wherein the compound represented by the general formula (Formula 4) as defined in claim 2 is a compound represented by the general formula (Formula 6) as defined in claim 3.

**30.** The food, beverage or feed according to claim 27, wherein the compound represented by the general formula (Formula 1) as defined in claim 1 is at least one compound selected from the group consisting of xanthoangelol, 4-hydroxyderricin, xanthoangelol H, 1-(5,6,7,8,8a,10a-hexahydro-1,7-dihydroxy-8,8,10a-trimethyl-9H-xanthen-4-yl)-3-(4-hydroxyphenyl)-2-propen-1-one, 1-(3,4-dihydro-3,5-dihydroxy-2-(3-isohexenyl)-2-methyl-2H-benzopyran-8-yl)-3-(4-hydroxyphenyl)-2-propen-1-one, 1-[2,3-dihydro-4-hydroxy-2-(1-hydroxy-1,5-dimethyl-4-hexenyl)-benzofuran-5-yl]-3-(4-hydroxyphenyl)-2-propen-1-one, 1-[2,3-dihydro-2-(1-hydroxy-1-methylethyl)-4-methoxy-benzofuran-7-yl]-3-(4-hydroxyphenyl)-2-propen-1-one, 1-[2,4-dihydroxy-3-(6,7-dihydroxy-3,7-dimethyl-2-octenyl)phenyl]-3-(4-hydroxyphenyl)-2-propen-1-one, 1-[3-(7-ethoxy-6-hydroxy-3,7-dimethyl-2-octenyl)-2,4-dihydroxyphenyl]-3-(4-hydroxyphenyl)-2-propen-1-one, 1-[3-(2,5-epoxy-2,6,6-trimethyl-cyclohexylmethyl)-2-hydroxy-4-methoxyphenyl]-3-(4-hydroxyphenyl)-2-propen-1-one, 1-[2-hydroxy-3-(7-hydroperoxy-3,7-dimethyl-2,5-octadienyl)-4-methoxyphenyl]-3-(4-hydroxyphenyl)-2-propen-1-one, 1-[2-hydroxy-3-(6-hydroperoxy-3,7-dimethyl-2,7-octadienyl)-4-methoxyphenyl]-3-(4-hydroxyphenyl)-2-propen-1-one, 1-[2-hydroxy-3-(7-hydroxy-3,7-dimethyl-2,5-octadienyl)-4-methoxyphenyl]-3-(4-hydroxyphenyl)-2-propen-1-one, xanthoangelol G, xanthoangelol F, lespeol, isobavachalcone, xanthohumol, 4,2'-dihydroxy-4'-methoxychalcone, 4,2'-dihydroxy-3'-methyl-4'-methoxychalcone, bavachalcone, 4-tetrahydropyranyloxy-2'-hydroxy-3'-prenyl-4'-methoxychalcone, 3,4,2'-trihydroxy-3'-prenyl-4'-methoxychalcone, 4,2'-diacetoxy-3'-prenyl-4'-methoxychalcone, 4,4'-dimethoxy-2'-hydroxy-3'-prenylchalcone, 3,4-dihydroxy-2',4'-dichlorochalcone, 4,2'-dihydroxy-3'-(3-methylbutyl)-4'-methoxychalcone, 4,4',6'-trimethoxy-2'-hydroxy-3'-prenylchalcone, 4,2'-dihydroxy-3'-prenyl-4'-ethoxychalcone, 4,2'-dihydroxy-3'-geranyl-4'-ethoxychalcone, 4,2'-dihydroxy-3'-farnesyl-4'-methoxychalcone, 4,2'-dihydroxy-3'-benzyl-4'-methoxychalcone, 4,2',4'-trihydroxy-3'-benzylchalcone, 4,2',4'-trihydroxy-3'-farnesylchalcone, 4,2',4'-triacetoxy-3'-geranylchalcone, 4,2'-diacetoxy-3'-geranyl-4'-methoxychalcone, 2'-acetoxy-3'-geranyl-4-hydroxy-4'-methoxychalcone, 2,2'-dihydroxy-3,3'-diprenyl-4,4'-dimethoxychalcone, 4,2'-dihydroxy-3'-geranyl-4'-benzyloxychalcone, 4-chloro-2',4'-dihydroxy-3'-geranylchalcone, 4-chloro-2',4'-dihydroxy-3'-prenylchalcone, 3-nitro-2'-hydroxy-3'-prenyl-4'-methoxychalcone, 4-hydroxy-2'-benzyloxy-3'-prenyl-4'-methoxychalcone, 4-acetoxy-2'-hydroxy-3'-prenyl-4'-methoxychalcone, 4-chloro-2'-acetoxy-3'-prenyl-4'-methoxychalcone, 4-chloro-2'-acetoxy-3'-geranyl-4'-methoxychalcone, 3,4,2'-trihydroxychalcone, 3,4,2',5'-tetrahydroxychalcone, chalcone, 4-chloro-2'-benzyloxychalcone, xanthoangelol B, xanthoangelol C, xanthoangelol D, xanthoangelol E and bavachromanol.

**31.** The food, beverage or feed according to claim 27, wherein the derivative of the compound represented by the general formula (Formula 1) as defined in claim 1 is at least one compound selected from the group consisting of 4'-*O*-geranylnaringenin, isobavachin, prostratol F, 8-geranyl-4'-hydroxy-7-methoxyflavanone, 1-(2-hydroxy-3-(3-methylbutyl)-4-methoxyphenyl)-3-(4-hydroxyphenyl)-propan-1-one, 7-methoxy-8-prenyl-4'-hydroxyflavanone, 1-adamantyl-3-(3,4-dihydroxyphenyl)-2-propan-1-one, 1-adamantyl-3-hydroxy-4-(3,4-dihydroxyphenyl)-butan-1-one, and 1-adamantyl-4-(3,4-dihydroxyphenyl)-3-buten-1-one.

**32.** The food, beverage or feed according to claim 28, wherein the compound represented by the general formula (Formula 5) as defined in claim 2 is a compound represented by the general formula (Formula 8) as defined in claim 6.

**33.** The food, beverage or feed according to claim 27, wherein the compound represented by the general formula (Formula 1) as defined in claim 1 is at least one compound selected from the group consisting of
2',4'-dihydroxy-5'-prenylacetophenone,
2'-hydroxy-4'-methoxy-5'-prenylacetophenone,
2'-hydroxy-3'-prenyl-4'-methoxyacetophenone,
2'-hydroxy-3'-methyl-4'-methoxyacetophenone,
2',4'-dihydroxy-3'-geranylacetophenone,
2'-hydroxy-3'-geranyl-4'-methoxyacetophenone,
2'-hydroxy-3'-(3-methylbutyl)-4'-methoxyacetophenone,
2'-hydroxy-3'-prenyl-4'-tetrahydropyranyloxyacetophenone,
2'-hydroxy-3'-geranyl-4'-tetrahydropyranyloxyacetophenone,
2'-hydroxy-3'-prenyl-4'-ethoxyacetophenone,
2'-hydroxy-3'-geranyl-4'-ethoxyacetophenone,
2',4'-dihydroxy-3'-farnesylacetophenone;
2'-hydroxy-3'-farnesyl-4'-methoxyacetophenone,
2',4'-dihydroxy-3'-benzylacetophenone,
2'-hydroxy-3'-benzyl-4'-methoxyacetophenone,
2'-methyl-4'-prenyloxyacetophenone,
2'-benzyloxyacetophenone and
2'-hydroxy-3'-geranyl-4'-benzyloxyacetophenone.

**34.** The food, beverage or feed according to claim 27, wherein the compound represented by the general formula (Formula 1) as defined in claim 1 is a compound in which $X^0$ is a hydrogen atom, a 2-(2-furyl)vinyl group, a 2-(2-thienyl)vinyl group, a 4-phenyl-1,3-butadienyl group, or a 4-methyl-1,3-pentadienyl group; $R_1$ is a hydroxyl group; $R_2$ is a hydrogen atom or a prenyl group; $R_3$ is a methoxy group; and each of $R_4$ and $R_5$ is a hydrogen atom.

**35.** The food, beverage or feed according to claim 27, wherein the compound represented by the general formula (Formula 1) as defined in claim 1 is at least one compound selected from the group consisting of 1-(2-hydroxy-4-methoxy-3-prenylphenyl)-3-(2-thienyl)-2-propen-1-one, 1-(2-hydroxy-4-methoxy-3-prenylphenyl)-5-phenyl-2,4-pentadien-1-one, 3-(2-furyl)-1-(2-hydroxy-3-prenyl-4-methoxyphenyl)-2-propen-1-one, 2-hydroxy-3-prenyl-4-methoxybenzaldehyde, 1-(2-hydroxy-3-prenyl-4-methoxyphenyl)-5-methyl-2,4-hexadien-1-one and 2-hydroxy-4-methoxybenzaldehyde.

**36.** The food, beverage or feed according to claim 27, wherein the derivative of the compound represented by the general formula (Formula 1) as defined in claim 1 is 2-(2-furyl)-3-(2-furylmethylene)-2,3-dihydro-7-methoxy-8-prenyl-4H-1-benzopyran-4-one.

**37.** The food, beverage or feed according to claim 27, wherein the compound represented by the general formula (Formula 2) as defined in claim 1 is a compound represented by the general formula (Formula 9) as defined in claim 11.

**38.** The food, beverage or feed according to claim 27, wherein the compound represented by the general formula (Formula 2) as defined in claim 1 is at least one compound selected from the group consisting of 3'-acetoxy-4'-angeloyloxy-3',4'-dihydroseselin, 3'-angeloyloxy-3',4'-dihydroseselin, 3'-angeloyloxy-4'-hydroxy-3',4'-dihydroseselin and xanthotoxin.

**39.** The food, beverage or feed according to claim 27, wherein the compound represented by the general formula (Formula 3) as defined in claim 1 is sedanolide and/or n-butylidenephthalide.

**40.** An agent for enhancing glucose uptake into a cell, **characterized in that** the agent comprises as an effective ingredient at least one compound selected from the group consisting of a compound represented by the general formula (Formula 1), a compound represented by the general formula (Formula 2), a compound represented by the general formula (Formula 3), derivatives thereof, and pharmacologically acceptable salts thereof, each as defined in claim 1.

**41.** The agent for enhancing glucose uptake into a cell according to claim 40, wherein the compound represented by

the general formula (Formula 1) as defined in claim 1 is a compound represented by the general formula (Formula 4) and/or a compound represented by the general formula (Formula 5) as defined in claim 2.

**42.** The agent for enhancing glucose uptake into a cell according to claim 41, wherein the compound represented by the general formula (Formula 4) as defined in claim 2 is a compound represented by the general formula (Formula 6) as defined in claim 3.

**43.** The agent for enhancing glucose uptake into a cell according to claim 40, wherein the compound represented by the general formula (Formula 1) as defined in claim 1 is at least one compound selected from the group consisting of xanthoangelol, 4-hydroxyderricin, xanthoangelol H, 1-(5,6,7,8,8a,10a-hexahydro-1,7-dihydroxy-8,8,10a-trimethyl-9H-xanthen-4-yl)-3-(4-hydroxyphenyl)-2-propen-1-one, 1-(3,4-dihydro-3,5-dihydroxy-2-(3-isohexenyl)-2-methyl-2H-benzopyran-8-yl)-3-(4-hydroxyphenyl)-2-propen-1-one, 1-[2,3-dihydro-4-hydroxy-2-(1-hydroxy-1,5-dimethyl-4-hexenyl)-benzofuran-5-yl]-3-(4-hydroxyphenyl)-2-propen-1-one, 1-[2,3-dihydro-2-(1-hydroxy-1-methylethyl)-4-methoxy-benzofuran-7-yl]-3-(4-hydroxyphenyl)-2-propen-1-one, 1-[2,4-dihydroxy-3-(6,7-dihydroxy-3,7-dimethyl-2-octenyl)phenyl]-3-(4-hydroxyphenyl)-2-propen-1-one, 1-[3-(7-ethoxy-6-hydroxy-3,7-dimethyl-2-octenyl)-2,4-dihydroxyphenyl]-3-(4-hydroxyphenyl)-2-propen-1-one, 1-[3-(2,5-epoxy-2,6,6-trimethyl-cyclohexylmethyl)-2-hydroxy-4-methoxyphenyl]-3-(4-hydroxyphenyl)-2-propen-1-one, 1-[2-hydroxy-3-(7-hydroperoxy-3,7-dimethyl-2,5-octadienyl)-4-methoxyphenyl]-3-(4-hydroxyphenyl)-2-propen-1-one, 1-[2-hydroxy-3-(6-hydroperoxy-3,7-dimethyl-2,7-octadienyl)-4-methoxyphenyl]-3-(4-hydroxyphenyl)-2-propen-1-one, 1-[2-hydroxy-3-(7-hydroxy-3,7-dimethyl-2,5-octadienyl)-4-methoxyphenyl]-3-(4-hydroxyphenyl)-2-propen-1-one, xanthoangelol G, xanthoangelol F, lespeol, isobavachalcone, xanthohumol, 4,2'-dihydroxy-4'-methoxychalcone, 4,2'-dihydroxy-3'-methyl-4'-methoxychalcone, bavachalcone, 4-tetrahydropyranyloxy-2'-hydroxy-3'-prenyl-4'-methoxychalcone, 3,4,2'-trihydroxy-3'-prenyl-4'-methoxychalcone, 4,2'-diacetoxy-3'-prenyl-4'-methoxychalcone, 4,4'-dimethoxy-2'-hydroxy-3'-prenylchalcone, 3,4-dihydroxy-2',4'-dichlorochalcone, 4,2'-dihydroxy-3'-(3-methylbutyl)-4'-methoxychalcone, 4,4',6'-trimethoxy-2'-hydroxy-3'-prenylchalcone, 4,2'-dihydroxy-3'-prenyl-4'-ethoxychalcone, 4,2'-dihydroxy-3'-geranyl-4'-ethoxychalcone, 4,2'-dihydroxy-3'-farnesyl-4'-methoxychalcone, 4,2'-dihydroxy-3'-benzyl-4'-methoxychalcone, 4,2',4'-trihydroxy-3'-benzylchalcone, 4,2',4'-trihydroxy-3'-farnesylchalcone, 4,2',4'-triacetoxy-3'-geranylchalcone, 4,2'-diacetoxy-3'-geranyl-4'-methoxychalcone, 2'-acetoxy-3'-geranyl-4-hydroxy-4'-methoxychalcone, 2,2'-dihydroxy-3,3'-diprenyl-4,4'-dimethoxychalcone, 4,2'-dihydroxy-3'-geranyl-4'-benzyloxychalcone, 4-chloro-2',4'-dihydroxy-3'-geranylchalcone, 4-chloro-2',4'-dihydroxy-3'-prenylchalcone, 3-nitro-2'-hydroxy-3'-prenyl-4'-methoxychalcone, 4-hydroxy-2'-benzyloxy-3'-prenyl-4'-methoxychalcone, 4-acetoxy-2'-hydroxy-3'-prenyl-4'-methoxychalcone, 4-chloro-2'- acetoxy-3'-prenyl-4'-methoxychalcone, 4-chloro-2'-acetoxy-3'-geranyl-4'-methoxychalcone, 3,4,2'-trihydroxychalcone, 3,4,2',5'-tetrahydroxychalcone, chalcone, 4-chloro-2'-benzyloxychalcone, xanthoangelol B, xanthoangelol C, xanthoangelol D, xanthoangelol E and bavachromanol.

**44.** The agent for enhancing glucose uptake into a cell according to claim 40, wherein the derivative of the compound represented by the general formula (Formula 1) as defined in claim 1 is at least one compound selected from the group consisting of 4'-*O*-geranylnaringenin, isobavachin, prostratol F, 8-geranyl-4'-hydroxy-7-methoxyflavanone, 1-(2-hydroxy-3-(3-methylbutyl)-4-methoxyphenyl)-3-(4-hydroxyphenyl)-propan-1-one, 7-methoxy-8-prenyl-4'-hydroxyflavanone, 1-adamantyl-3-(3,4-dihydroxyphenyl)-2-propan-1-one, 1-adamantyl-3-hydroxy-4-(3,4-dihydroxyphenyl)-butan-1-one, and 1-adamantyl-4-(3,4-dihydroxyphenyl)-3-buten-1-one.

**45.** The agent for enhancing glucose uptake into a cell according to claim 41, wherein the compound represented by the general formula (Formula 5) as defined in claim 2 is a compound represented by the general formula (Formula 8) as defined in claim 6.

**46.** The agent for enhancing glucose uptake into a cell according to claim 40, wherein the compound represented by the general formula (Formula 1) as defined in claim 1 is at least one compound selected from the group consisting of 2',4'-dihydroxy-5'-prenylacetophenone,
2'-hydroxy-4'-methoxy-5'-prenylacetophenone,
2'-hydroxy-3'-prenyl-4'-methoxyacetophenone,
2'-hydroxy-3'-methyl-4'-methoxyacetophenone,
2',4'-dihydroxy-3'-geranylacetophenone,
2'-hydroxy-3'-geranyl-4'-methoxyacetophenone,
2'-hydroxy-3'-(3-methylbutyl)-4'-methoxyacetophenone,
2'-hydroxy-3'-prenyl-4'-tetrahydropyranyloxyacetophenone,
2'-hydroxy-3'-geranyl-4'-tetrahydropyranyloxyacetophenone,
2'-hydroxy-3'-prenyl-4'-ethoxyacetophenone,

2'-hydroxy-3'-geranyl-4'-ethoxyacetophenone,
2',4'-dihydroxy-3'-farnesylacetophenone,
2'-hydroxy-3'-farnesyl-4'-methoxyacetophenone,
2',4'-dihydroxy-3'-benzylacetophenone,
2'-hydroxy-3'-benzyl-4'-methoxyacetophenone,
2'-methyl-4'-prenyloxyacetophenone,
2'-benzyloxyacetophenone and
2'-hydroxy-3'-geranyl-4'-benzyloxyacetophenone.

**47.** The agent for enhancing glucose uptake into a cell according to claim 40, wherein the compound represented by the general formula (Formula 1) as defined in claim 1 is a compound in which $X^0$ is a hydrogen atom, a 2-(2-furyl)vinyl group, a 2-(2-thienyl)vinyl group, a 4-phenyl-1,3-butadienyl group, or a 4-methyl-1,3-pentadienyl group; $R_1$ is a hydroxyl group; $R_2$ is a hydrogen atom or a prenyl group; $R_3$ is a methoxy group; and each of $R_4$ and $R_5$ is a hydrogen atom.

**48.** The agent for enhancing glucose uptake into a cell according to claim 40, wherein the compound represented by the general formula (Formula 1) as defined in claim 1 is at least one compound selected from the group consisting of 1-(2-hydroxy-4-methoxy-3-prenylphenyl)-3-(2-thienyl)-2-propen-1-one, 1-(2-hydroxy-4-methoxy-3-prenylphenyl)-5-phenyl-2,4-pentadien-1-one, 3-(2-furyl)-1-(2-hydroxy-3-prenyl-4-methoxyphenyl)-2-propen-1-one, 2-hydroxy-3-prenyl-4-methoxybenzaldehyde, 1-(2-hydroxy-3-prenyl-4-methoxyphenyl)-5-methyl-2,4-hexadien-1-one and 2-hydroxy-4-methoxybenzaldehyde.

**49.** The agent for enhancing glucose uptake into a cell according to claim 40, wherein the derivative of the compound represented by the general formula (Formula 1) as defined in claim 1 is 2-(2-furyl)-3-(2-furylmethylene)-2,3-dihydro-7-methoxy-8-prenyl-4H-1-benzopyran-4-one.

**50.** The agent for enhancing glucose uptake into a cell according to claim 40, wherein the compound represented by the general formula (Formula 2) as defined in claim 1 is a compound represented by the general formula (Formula 9) as defined in claim 11.

**51.** The agent for enhancing glucose uptake into a cell according to claim 40, wherein the compound represented by the general formula (Formula 2) as defined in claim 1 is at least one compound selected from the group consisting of 3'-acetoxy-4'-angeloyloxy-3',4'-dihydroseselin, 3'-angeloyloxy-3',4'-dihydroseselin, 3'-angeloyloxy-4'-hydroxy-3',4'-dihydroseselin and xanthotoxin.

**52.** The agent for enhancing glucose uptake into a cell according to claim 40, wherein the compound represented by the general formula (Formula 3) as defined in claim 1 is sedanolide and/or n-butylidenephthalide.

**53.** An agent for inducing differentiation into an adipocyte, **characterized in that** the agent comprises as an effective ingredient at least one compound selected from the group consisting of a compound represented by the general formula (Formula 1), a compound represented by the general formula (Formula 2), a compound represented by the general formula (Formula 3), derivatives thereof, and pharmacologically acceptable salts thereof, each as defined in claim 1.

**54.** The agent for inducing differentiation into an adipocyte according to claim 53, wherein the compound represented by the general formula (Formula 1) as defined in claim 1 is a compound represented by the general formula (Formula 4) and/or a compound represented by the general formula (Formula 5), each as defined in claim 2.

**55.** The agent for inducing differentiation into an adipocyte according to claim 54, wherein the compound represented by the general formula (Formula 4) as defined in claim 2 is a compound represented by the general formula (Formula 6) as defined in claim 3.

**56.** The agent for inducing differentiation into an adipocyte according to claim 53, wherein the compound represented by the general formula (Formula 1) as defined in claim 1 is at least one compound selected from the group consisting of xanthoangelol, 4-hydroxyderricin, xanthoangelol H, 1-(5,6,7,8,8a,10a-hexahydro-1,7-dihydroxy-8,8,10a-trimethyl-9H-xanthen-4-yl)-3-(4-hydroxyphenyl)-2-propen-1-one, 1-(3,4-dihydro-3,5-dihydroxy-2-(3-isohexenyl)-2-methyl-2H-benzopyran-8-yl)-3-(4-hydroxyphenyl)-2-propen-1-one, 1-[2,3-dihydro-4-hydroxy-2-(1-hydroxy-1,5-dimethyl-4-hexenyl)-benzofuran-5-yl]-3-(4-hydroxyphenyl)-2-propen-1-one, 1-[2,3-dihydro-2-(1-hydroxy-1-methylethyl)-4-methoxy-benzofuran-7-yl]-3-(4-hydroxyphenyl)-2-propen-1-one, 1-[2,4-dihydroxy-3-(6,7-dihydroxy-3,7-dimethyl-2-octenyl)phenyl]-3-(4-hydroxyphenyl)-2-propen-1-one, 1-[3-(7-ethoxy-6-hydroxy-3,7-dimethyl-2-octenyl)-2,4-dihydroxyphenyl]-3-(4-hydroxyphenyl)-2-propen-1-one, 1-[3-(2,5-epoxy-2,6,6-trimethyl-cyclohexylmethyl)-2-hydroxy-4-methoxyphenyl]-3-(4-hydroxyphenyl)-2-propen-1-one, 1-[2-hydroxy-3-(7-hydroperoxy-3,7-dimethyl-2,5-octadienyl)-4-methoxyphenyl]-3-(4-hydroxyphenyl)-2-propen-1-one, 1-[2-hydroxy-3-(6-hydroperoxy-3,7-dimethyl-2,7-octadienyl)-4-methoxyphenyl]-3-(4-hydroxyphenyl)-2-propen-1-one, 1-[2-hydroxy-3-(7-hydroxy-3,7-dimethyl-2,5-octadienyl)-4-methoxyphenyl]-3-(4-hydroxyphenyl)-2-propen-1-one, xanthoangelol G, xanthoangelol F, lespeol, isobavachalcone, xanthohumol, 4,2'-dihydroxy-4'-methoxychalcone, 4,2'-dihydroxy-3'-methyl-4'-methoxychalcone, bavachalcone, 4-tetrahydropyranyloxy-2'-hydroxy-3'-prenyl-4'-methoxychalcone, 3,4,2'-trihydroxy-3'-prenyl-4'-methoxychalcone, 4,2'-diacetoxy-3'-prenyl-4'-methoxychalcone, 4,4'-dimethoxy-2'-hydroxy-3'-prenylchalcone, 3,4-dihydroxy-2',4'-dichlorochalcone, 4,2'-dihydroxy-3'-(3-methylbutyl)-4'-methoxychalcone, 4,4',6'-trimethoxy-2'-hydroxy-3'-prenylchalcone, 4,2'-dihydroxy-3'-prenyl-4'-ethoxychalcone, 4,2'-dihydroxy-3'-geranyl-4'-ethoxychalcone, 4,2'-dihydroxy-3'-farnesyl-4'-methoxychalcone, 4,2'-dihydroxy-3'-benzyl-4'-methoxychalcone, 4,2',4'-trihydroxy-3'-benzylchalcone, 4,2',4'-trihydroxy-3'-farnesylchalcone, 4,2',4'-triacetoxy-3'-geranylchalcone, 4,2'-diacetoxy-3'-geranyl-4'-methoxychalcone, 2'-acetoxy-3'-geranyl-4-hydroxy-4'-methoxychalcone, 2,2'-dihydroxy-3,3'-diprenyl-4,4'-dimethoxychalcone, 4,2'-dihydroxy-3'-geranyl-4'-benzyloxychalcone, 4-chloro-2',4'-dihydroxy-3'-geranylchalcone, 4-chloro-2',4'-dihydroxy-3'-prenylchalcone, 3-nitro-2'-hydroxy-3'-prenyl-4'-methoxychalcone, 4-hydroxy-2'-benzyloxy-3'-prenyl-4'-methoxychalcone, 4-acetoxy-2'-hydroxy-3'-prenyl-4'-methoxychalcone, 4-chloro-2'-acetoxy-3'-prenyl-4'-methoxychalcone, 4-chloro-2'-acetoxy-3'-geranyl-4'-methoxychalcone, 3,4,2'-trihydroxychalcone, 3,4,2',5'-tetrahydroxychalcone, chalcone, 4-chloro-2'-benzyloxychalcone, xanthoangelol B, xanthoangelol C, xanthoangelol D, xanthoangelol E and bavachromanol.

**57.** The agent for inducing differentiation into an adipocyte according to claim 53, wherein the derivative of the compound represented by the general formula (Formula 1) as defined in claim 1 is at least one compound selected from the group consisting of 4'-*O*-geranylnaringenin, isobavachin, prostratol F, 8-geranyl-4'-hydroxy-7-methoxyflavanone, 1-(2-hydroxy-3-(3-methylbutyl)-4-methoxyphenyl)-3-(4-hydroxyphenyl)-propan-1-one, 7-methoxy-8-prenyl-4'-hydroxyflavanone, 1-adamantyl-3-(3,4-dihydroxyphenyl)-2-propen-1-one, 1-adamantyl-3-hydroxy-4-(3,4-dihydroxyphenyl)-butan-1-one, and 1-adamantyl-4-(3,4-dihydroxyphenyl)-3-buten-1-one.

**58.** The agent for inducing differentiation into an adipocyte according to claim 54, wherein the compound represented by the general formula (Formula 5) as defined in claim 2 is a compound represented by the general formula (Formula 8) as defined in claim 6.

**59.** The agent for inducing differentiation into an adipocyte according to claim 53, wherein the compound represented by the general formula (Formula 1) as defined in claim 1 is at least one compound selected from the group consisting of 2',4'-dihydroxy-5'-prenylacetophenone,
2'-hydroxy-4'-methoxy-5'-prenylacetophenone,
2'-hydroxy-3'-prenyl-4'-methoxyacetophenone,
2'-hydroxy-3'-methyl-4'-methoxyacetophenone,
2',4'-dihydroxy-3'-geranylacetophenone,
2'-hydroxy-3'-geranyl-4'-methoxyacetophenone,
2'-hydroxy-3'-(3-methylbutyl)-4'-methoxyacetophenone,
2'-hydroxy-3'-prenyl-4'-tetrahydropyranyloxyacetophenone,
2'-hydroxy-3'-geranyl-4'-tetrahydropyranyloxyacetophenone,
2'-hydroxy-3'-prenyl-4'-ethoxyacetophenone,
2'-hydroxy-3'-geranyl-4'-ethoxyacetophenone,
2',4'-dihydroxy-3'-farnesylacetophenone,
2'-hydroxy-3'-farnesyl-4'-methoxyacetophenone,
2',4'-dihydroxy-3'-benzylacetophenone,
2'-hydroxy-3'-benzyl-4'-methoxyacetophenone,
2'-methyl-4'-prenyloxyacetophenone,
2'-benzyloxyacetophenone and
2'-hydroxy-3'-geranyl-4'-benzyloxyacetophenone.

**60.** The agent for inducing differentiation into an adipocyte according to claim 53, wherein the compound represented by the general formula (Formula 1) as defined in claim 1 is a compound in which $X^0$ is a hydrogen atom, a 2-(2-furyl)vinyl group, a 2-(2-thienyl)vinyl group, a 4-phenyl-1,3-butadienyl group, or a 4-methyl-1,3-pentadienyl group; $R_1$ is a hydroxyl group; $R_2$ is a hydrogen atom or a prenyl group; $R_3$ is a methoxy group; and each of $R_4$ and $R_5$ is a

hydrogen atom.

**61.** The agent for inducing differentiation into an adipocyte according to claim 53, wherein the compound represented by the general formula (Formula 1) as defined in claim 1 is at least one compound selected from the group consisting of 1-(2-hydroxy-4-methoxy-3-prenylphenyl)-3-(2-thienyl)-2-propen-1-one, 1-(2-hydroxy-4-methoxy-3-prenylphenyl)-5-phenyl-2,4-pentadien-1-one, 3-(2-furyl)-1-(2-hydroxy-3-prenyl-4-methoxyphenyl)-2-propen-1-one, 2-hydroxy-3-prenyl-4-methoxybenzaldehyde, 1-(2-hydroxy-3-prenyl-4-methoxyphenyl)-5-methyl-2,4-hexadien-1-one and 2-hydroxy-4-methoxybenzaldehyde.

**62.** The agent for inducing differentiation into an adipocyte according to claim 53, wherein the derivative of the compound represented by the general formula (Formula 1) as defined in claim 1 is 2-(2-furyl)-3-(2-furylmethylene)-2,3-dihydro-7-methoxy-8-prenyl-4H-1-benzopyran-4-one.

**63.** The agent for inducing differentiation into an adipocyte according to claim 53, wherein the compound represented by the general formula (Formula 2) as defined in claim 1 is a compound represented by the general formula (Formula 9) as defined in claim 11.

**64.** The agent for inducing differentiation into an adipocyte according to claim 53, wherein the compound represented by the general formula (Formula 2) as defined in claim 1 is at least one compound selected from the group consisting of 3'-acetoxy-4'-angeloyloxy-3',4'-dihydroseselin, 3'-angeloyloxy-3' ,4'-dihydroseselin, 3'-angeloyloxy-4'-hydroxy-3',4'-dihydroseselin and xanthotoxin.

**65.** The agent for inducing differentiation into an adipocyte according to claim 53, wherein the compound represented by the general formula (Formula 3) as defined in claim 1 is sedanolide and/or n-butylidenephthalide.

**66.** A compound represented by the following formulas (Formula 10) to (Formula 44):

(Formula 10)

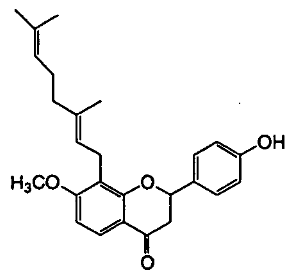

,

(Formula 11)

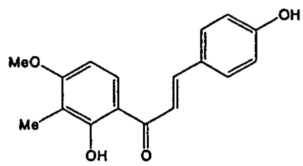

,

(Formula 12)

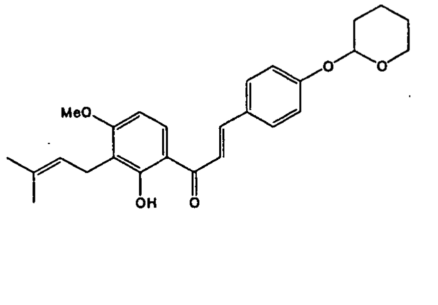

,

(Formula 13)

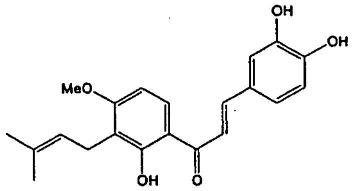

,

(Formula 14)

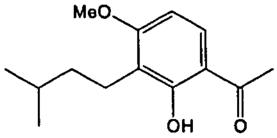

,

(Formula 15)

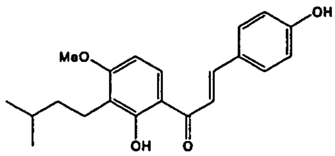

,

(Formula 16)

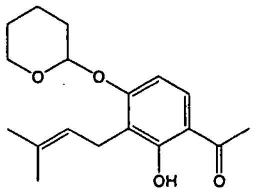

,

(Formula 17)

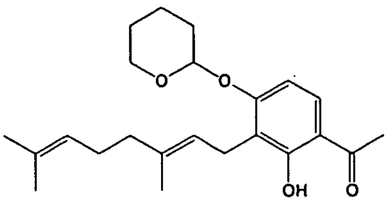

,

(Formula 18)

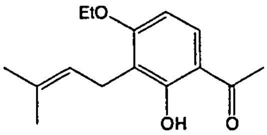

,

(Formula 19)

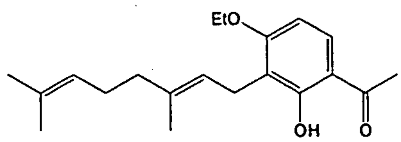

,

(Formula 20)

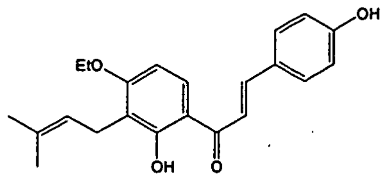

,

(Formula 21)

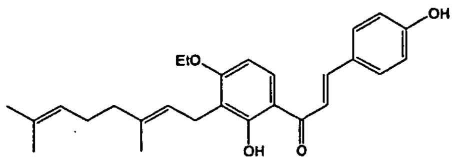

,

(Formula 22)

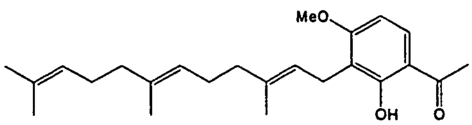

,

(Formula 23)

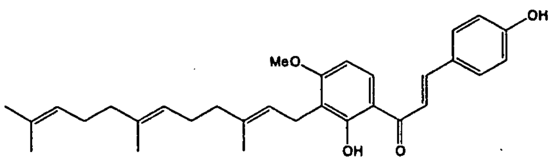

,

(Formula 24)

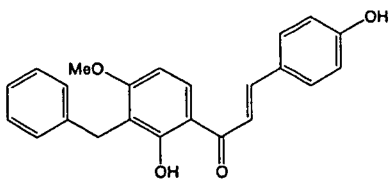

,

(Formula 25)

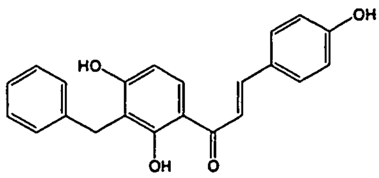

,

(Formula 26)

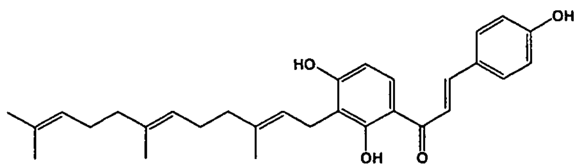

,

(Formula 27)

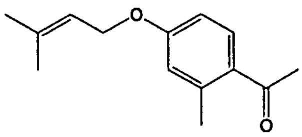

,

(Formula 28)

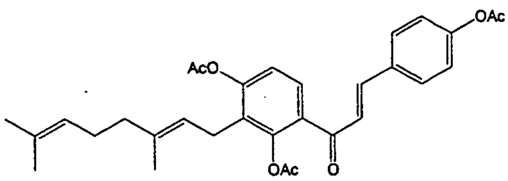

,

(Formula 29-1)

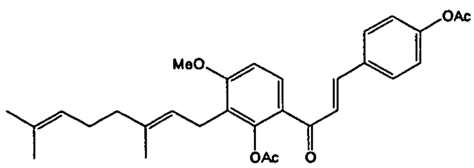

,

(Formula 29-2)

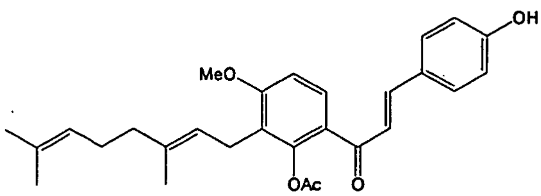

,

(Formula 30)

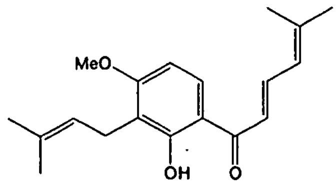

,

(Formula 31)

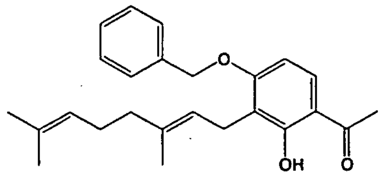

,

(Formula 32)

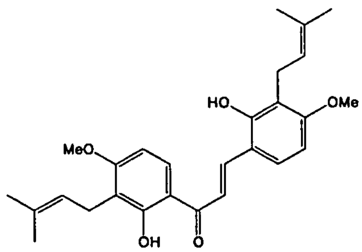

,

(Formula 33)

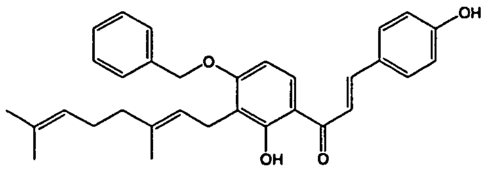

,

(Formula 34)

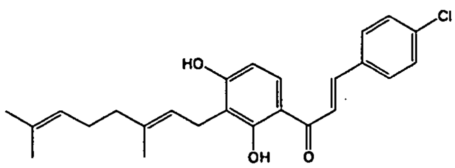

,

(Formula 35)

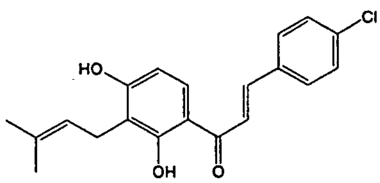

,

(Formula 36)

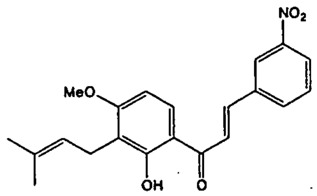

,

(Formula 37)

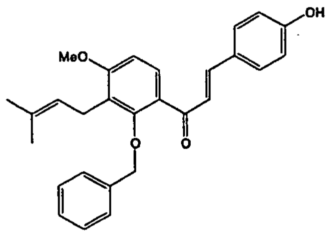

,

(Formula 38)

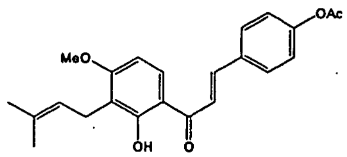

,

(Formula 39)

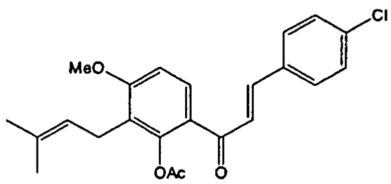

,

(Formula 40)

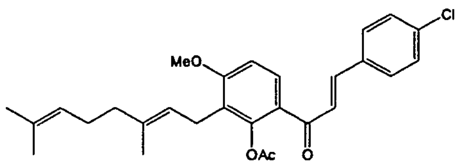

,

(Formula 41)

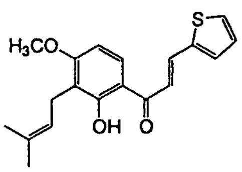

,

(Formula 42)

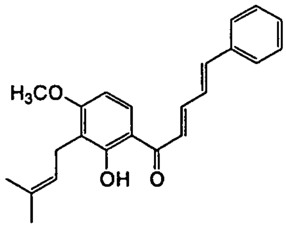

,

(Formula 43)

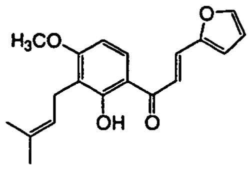

,

(Formula 44)

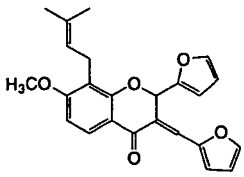

,

or a salt thereof.

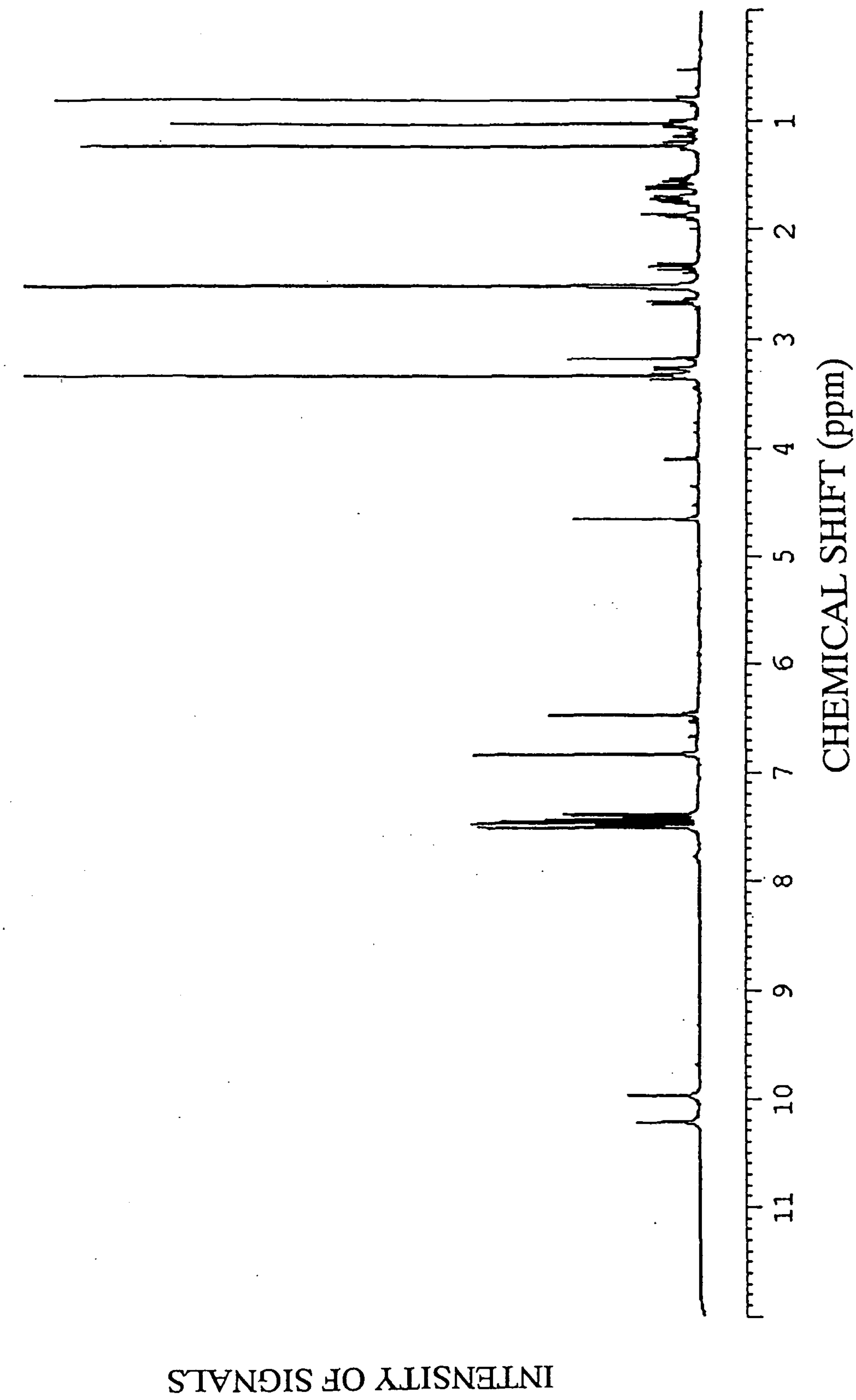


FIG. 1

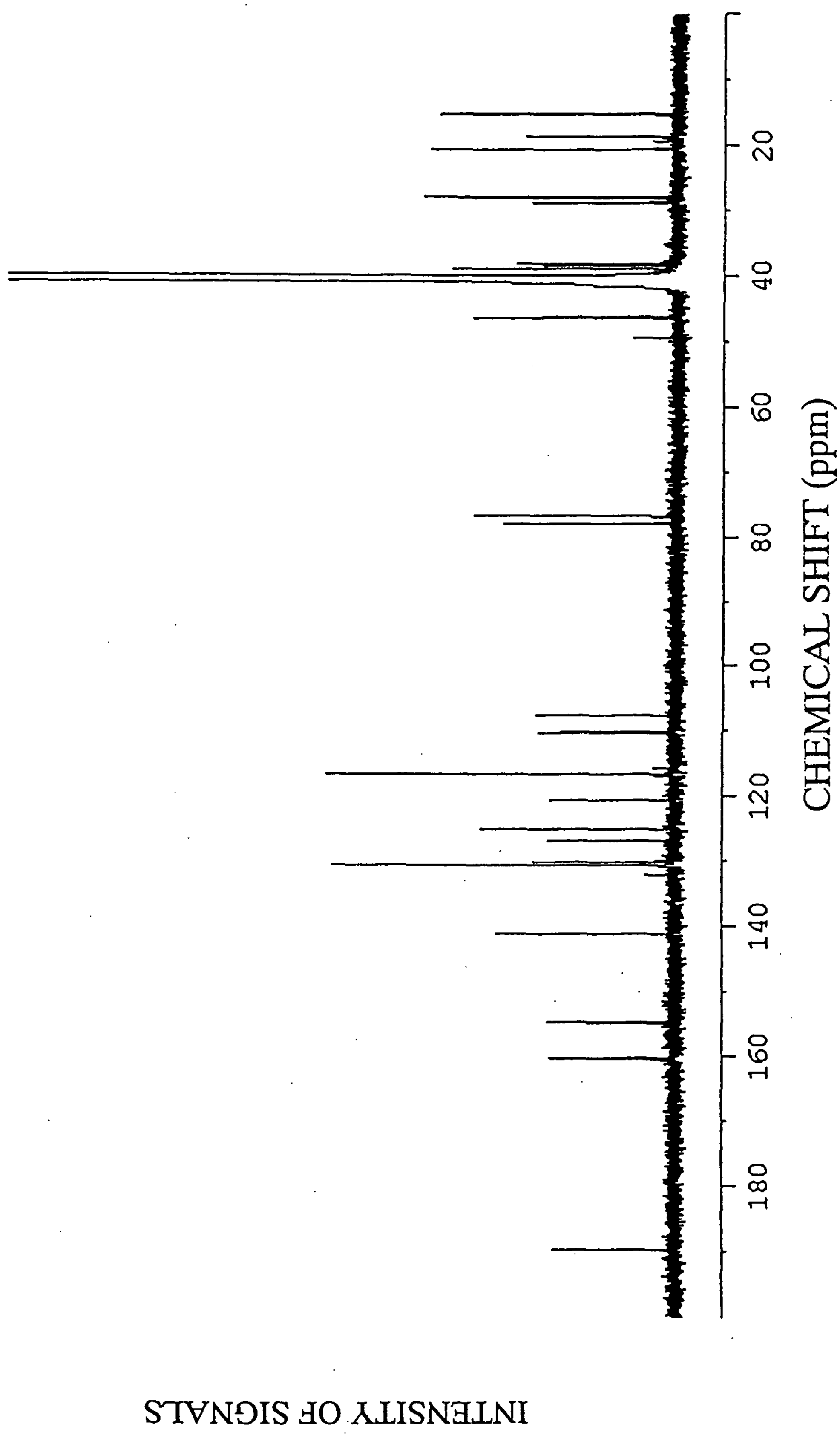
20
40
60
80
100
120
140
160
180
CHEMICAL SHIFT (ppm)
INTENSITY OF SIGNALS

FIG. 2

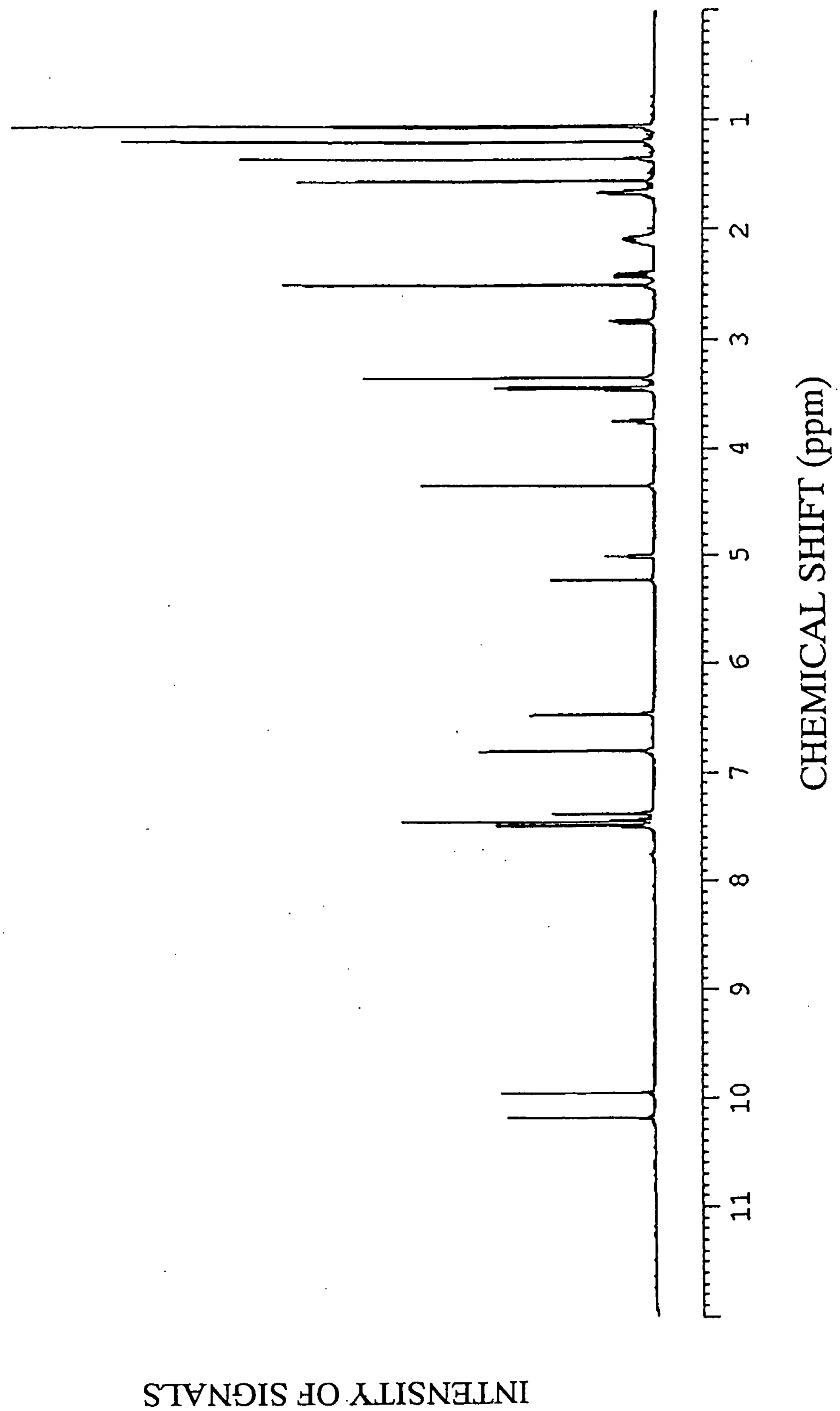


FIG. 3

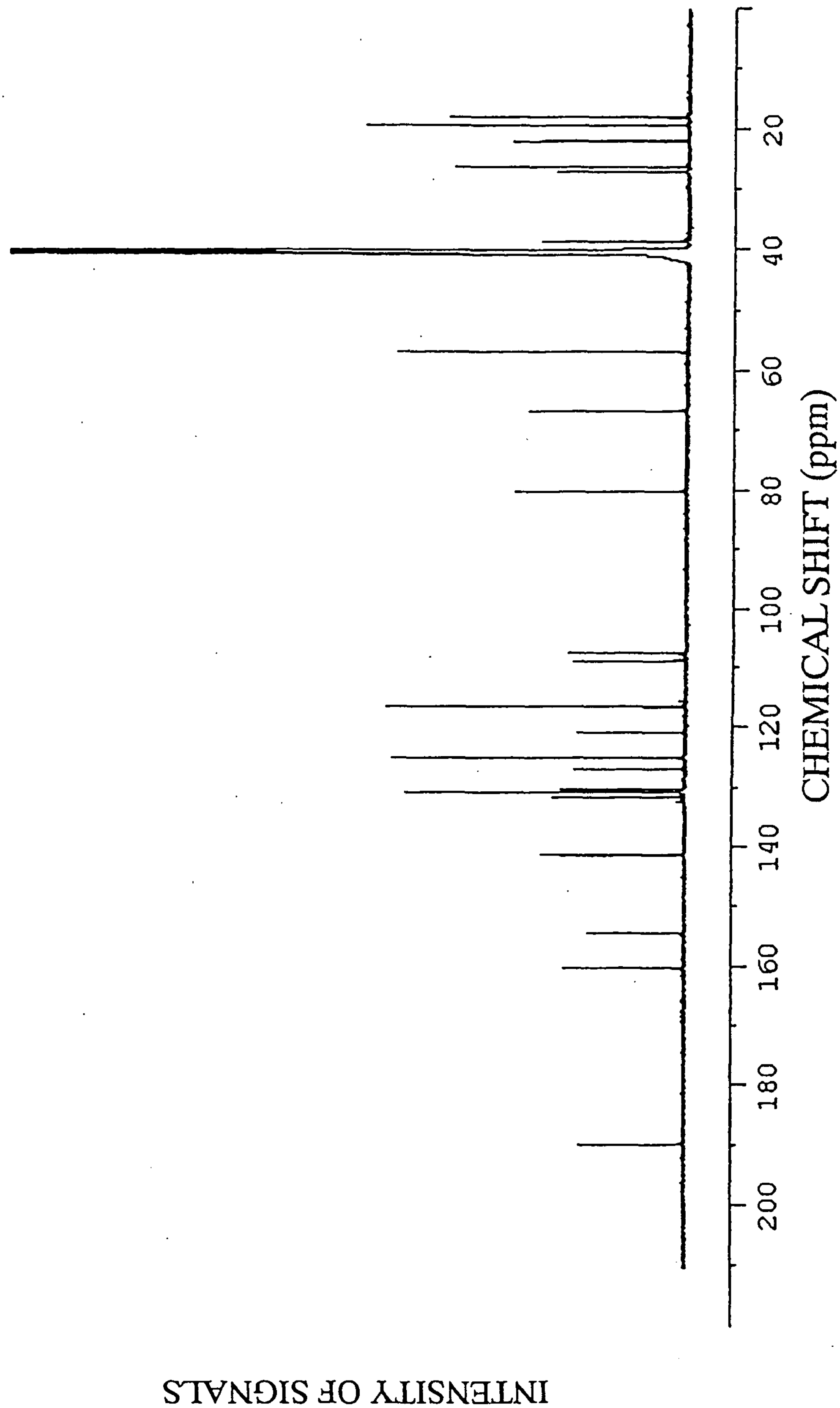
20
40
60
80
100
120
140
160
180
200
CHEMICAL SHIFT (ppm)
INTENSITY OF SIGNALS

FIG. 4

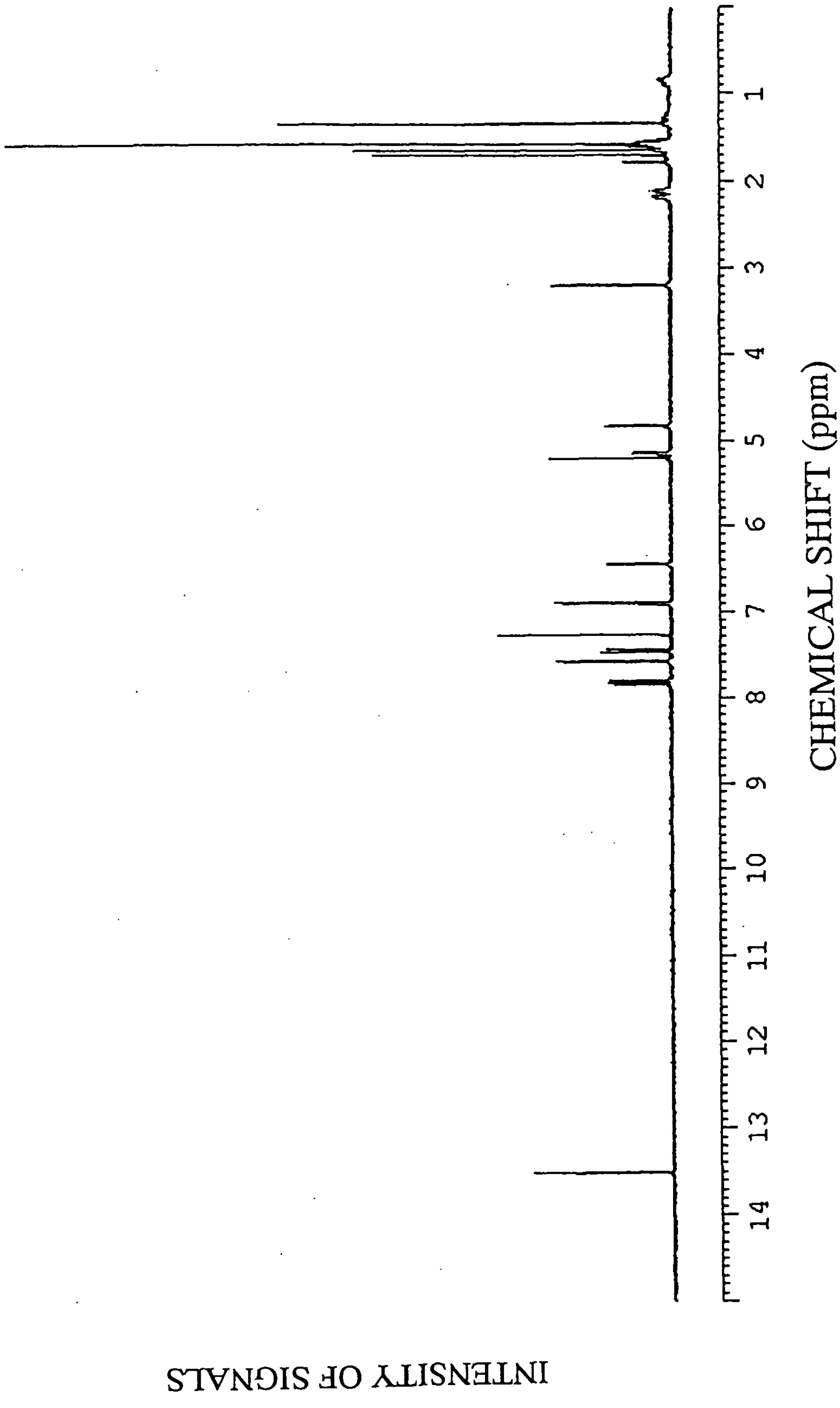
1
2
3
4
5
6
7
8
9
10
11
12
13
14
CHEMICAL SHIFT (ppm)
INTENSITY OF SIGNALS

FIG. 5

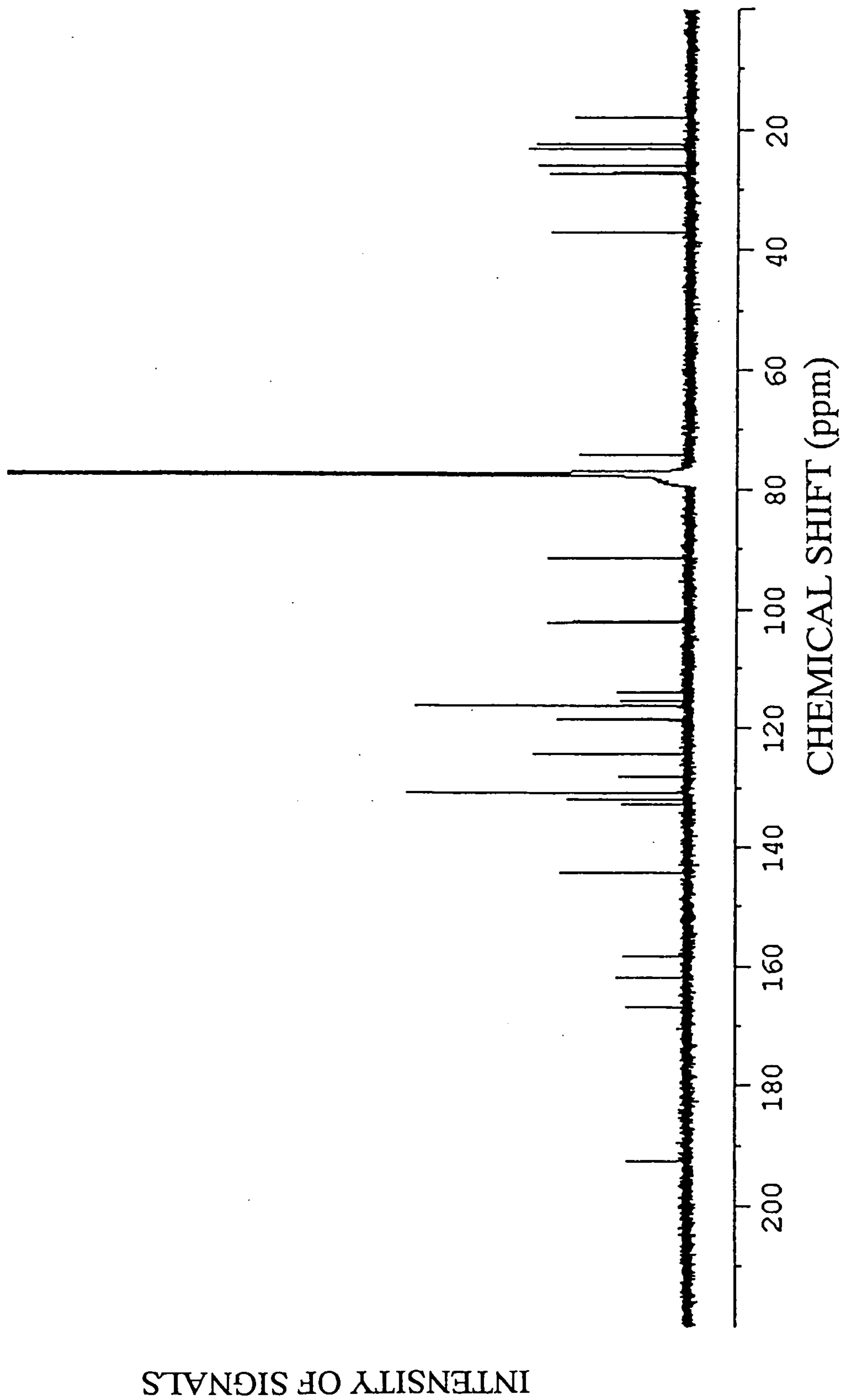
20
40
60
80
100
120
140
160
180
200
CHEMICAL SHIFT (ppm)
INTENSITY OF SIGNALS

FIG.6

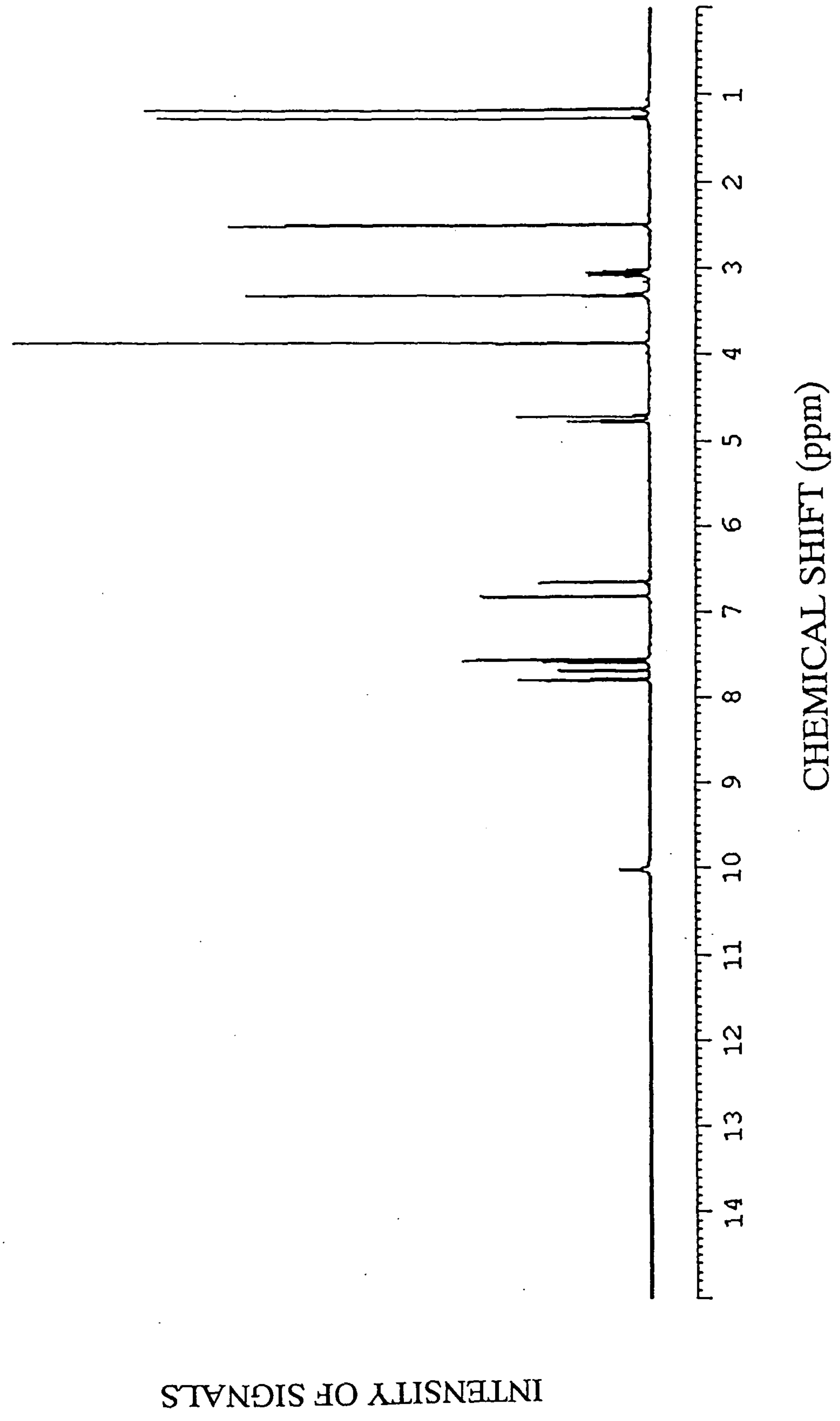
INTENSITY OF SIGNALS
CHEMICAL SHIFT (ppm)
1
2
3
4
5
6
7
8
9
10
11
12
13
14

FIG. 7

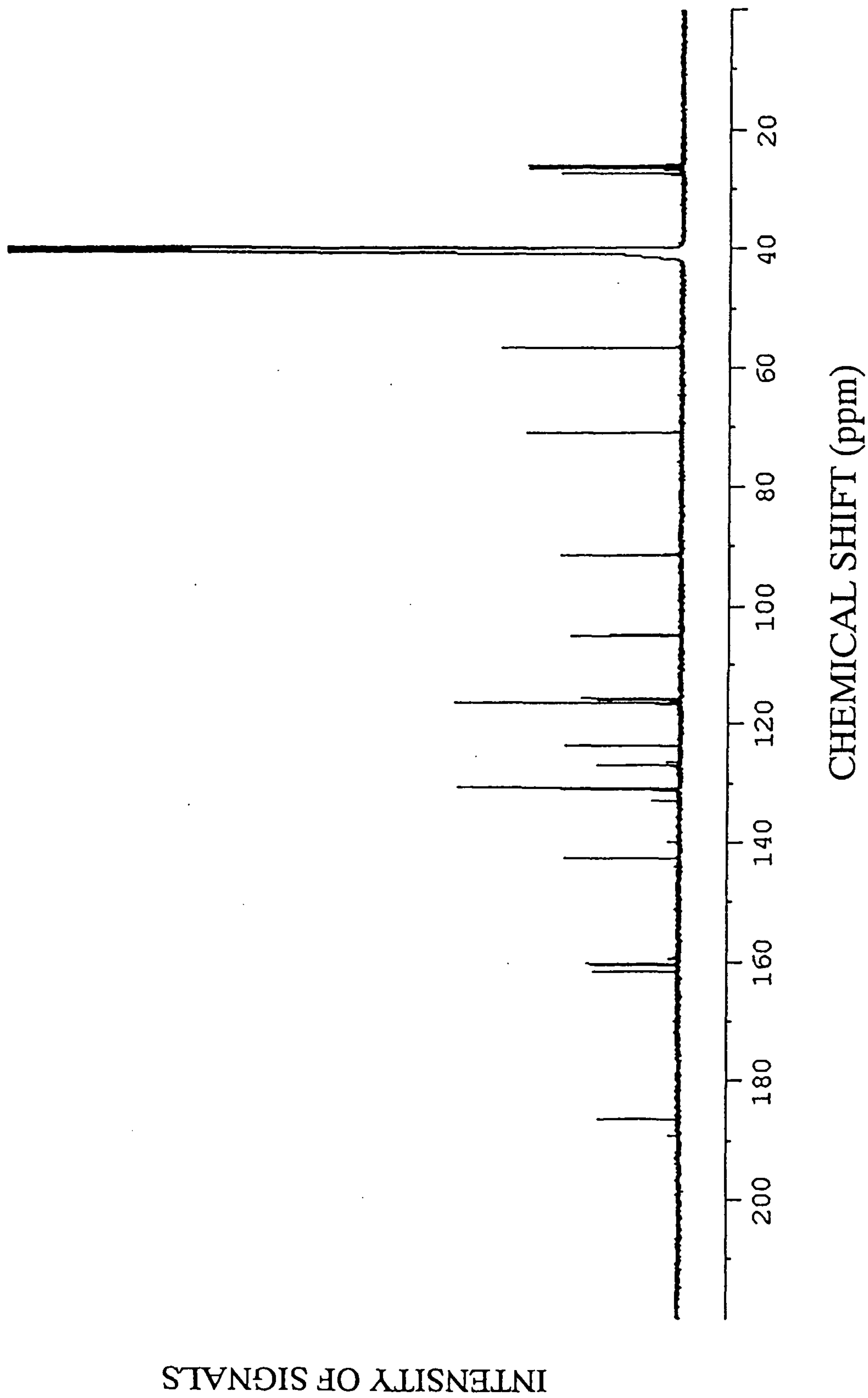
20
40
60
80
100
120
140
160
180
200
CHEMICAL SHIFT (ppm)
INTENSITY OF SIGNALS

FIG. 8

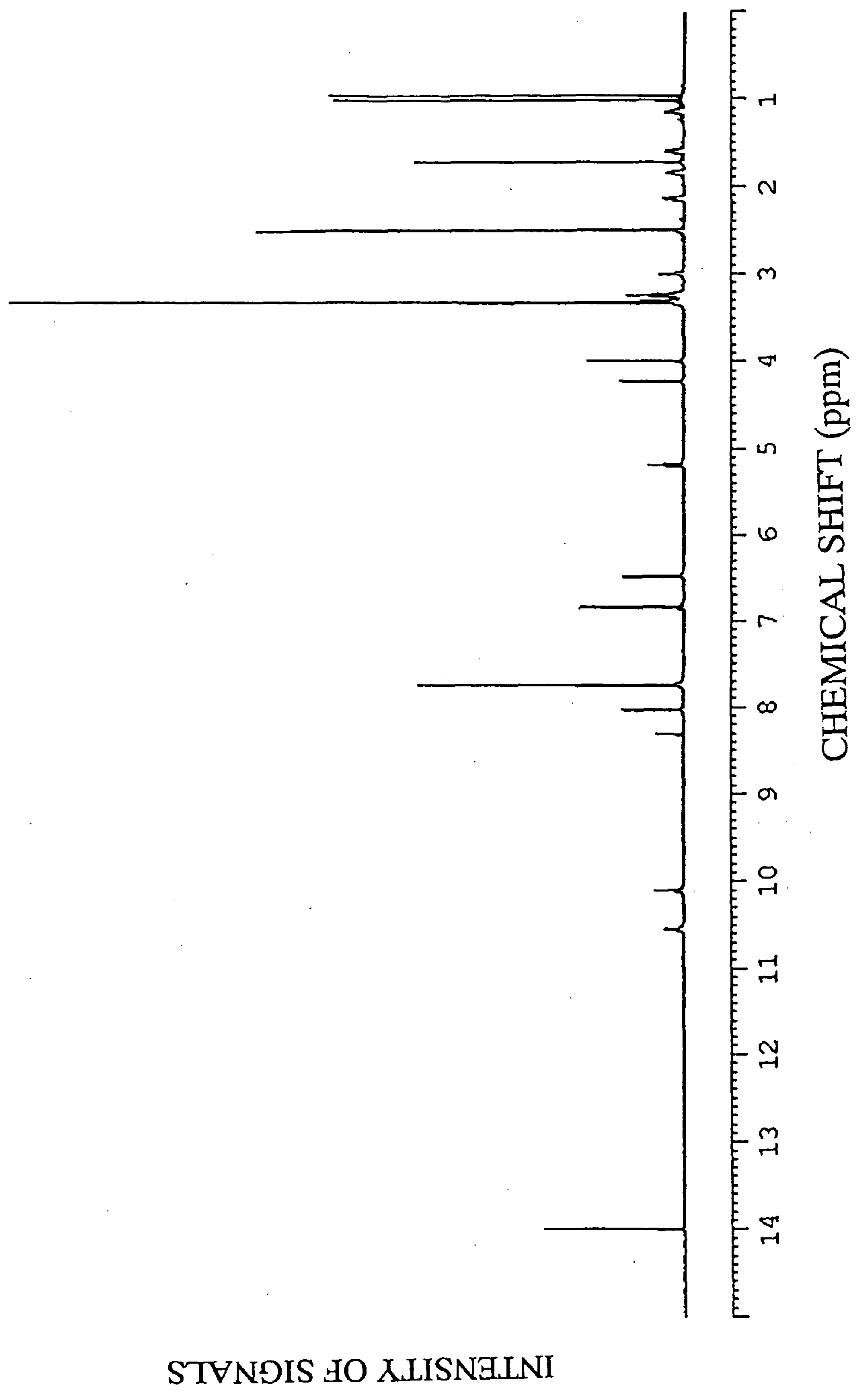
INTENSITY OF SIGNALS
CHEMICAL SHIFT (ppm)
1
2
3
4
5
6
7
8
9
10
11
12
13
14

FIG. 9

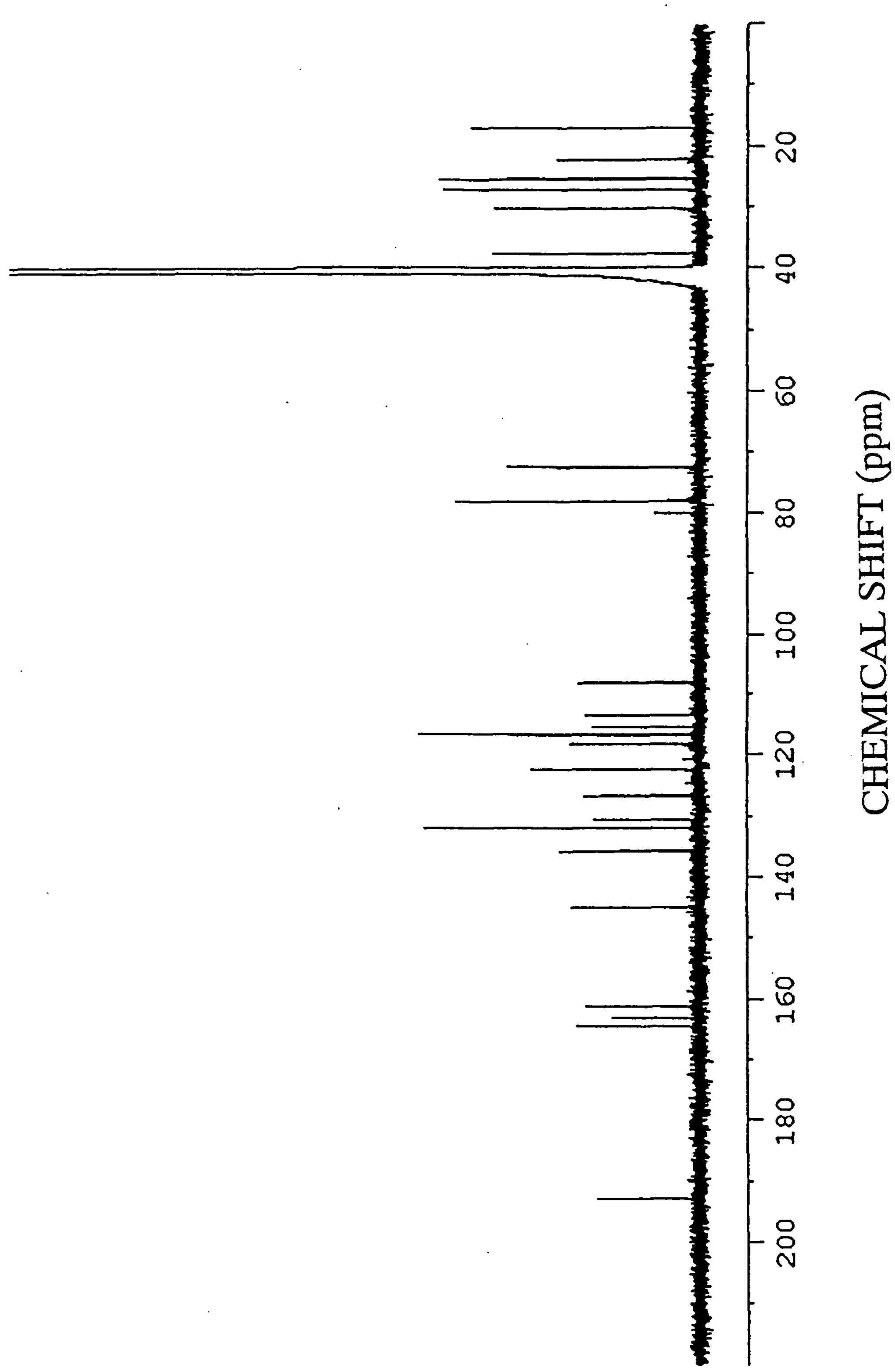
20
40
60
80
100
120
140
160
180
200
CHEMICAL SHIFT (ppm)
INTENSITY OF SIGNALS

FIG.10

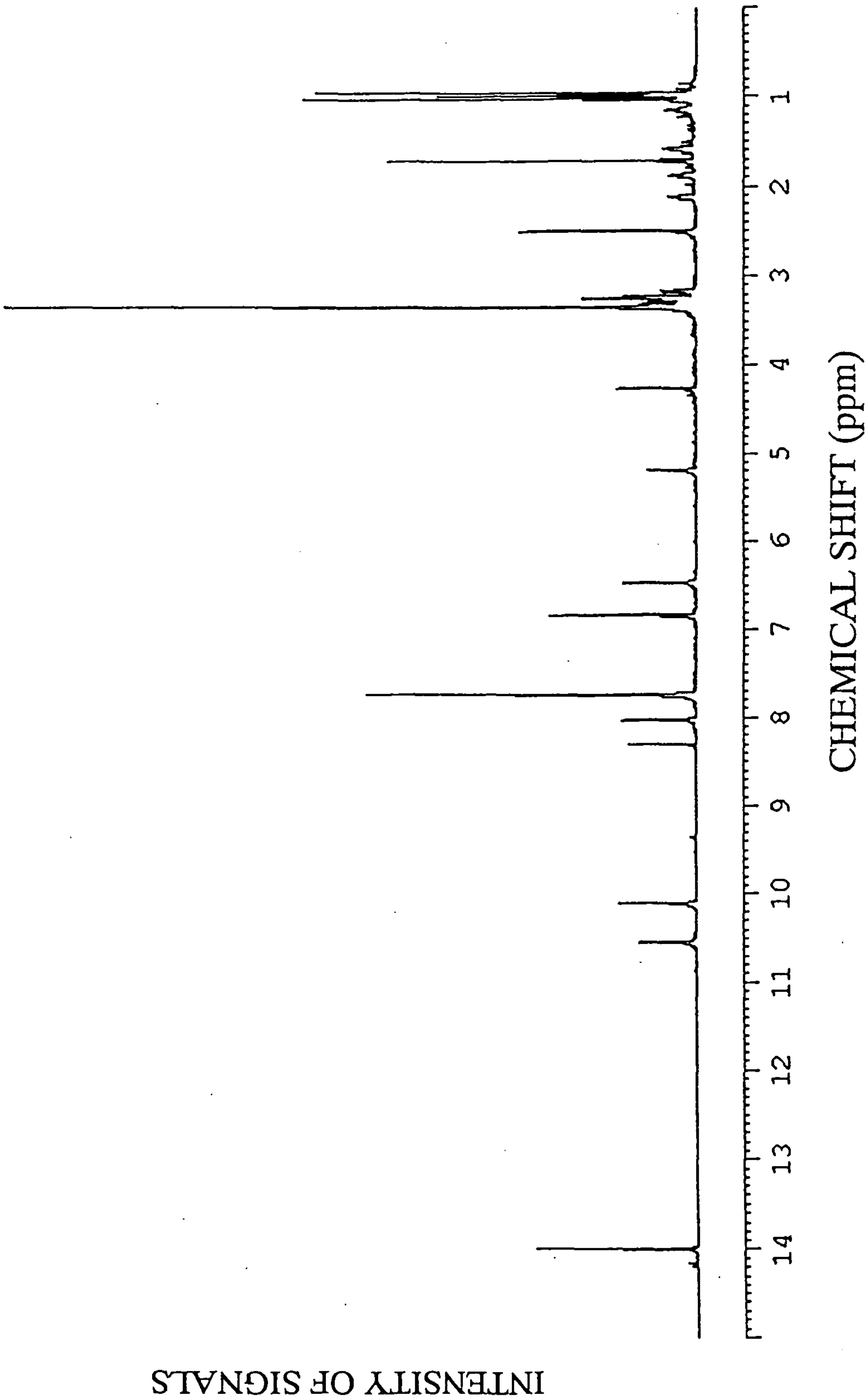
CHEMICAL SHIFT (ppm)
1
2
3
4
5
6
7
8
9
10
11
12
13
14
INTENSITY OF SIGNALS

FIG.11

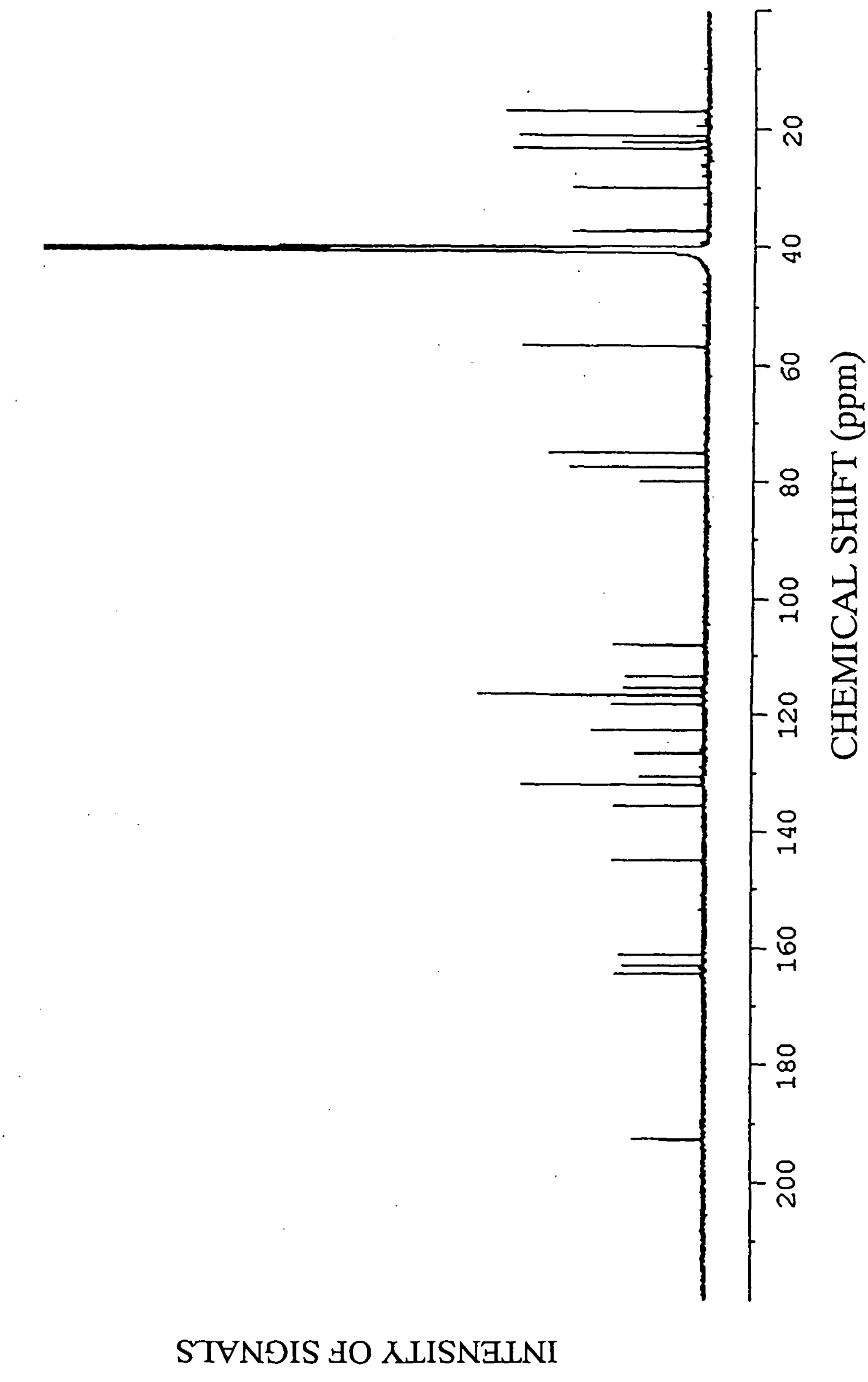
20
40
60
80
100
120
140
160
180
200
CHEMICAL SHIFT (ppm)
INTENSITY OF SIGNALS

FIG. 12

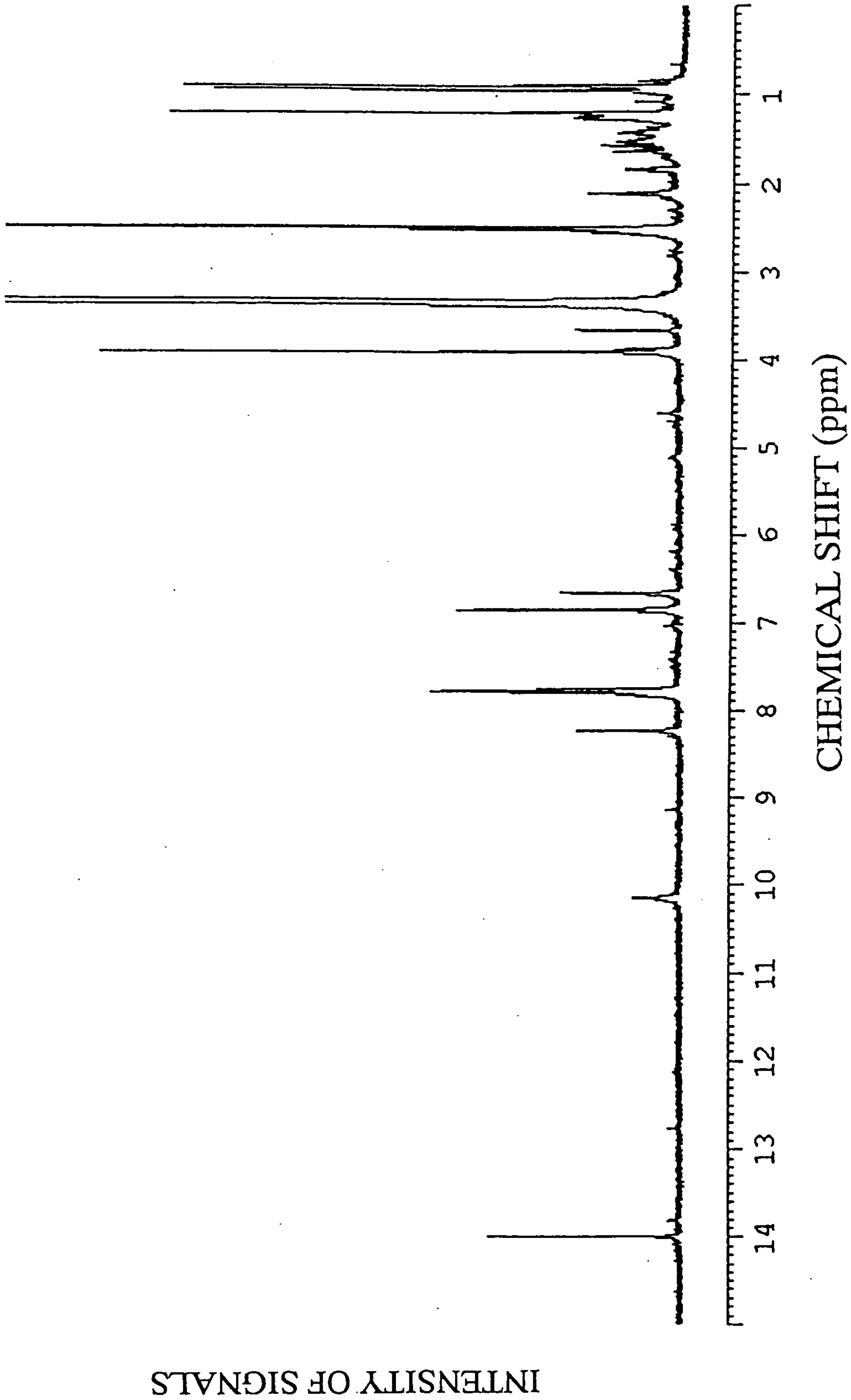
INTENSITY OF SIGNALS
CHEMICAL SHIFT (ppm)
1
2
3
4
5
6
7
8
9
10
11
12
13
14

FIG. 13

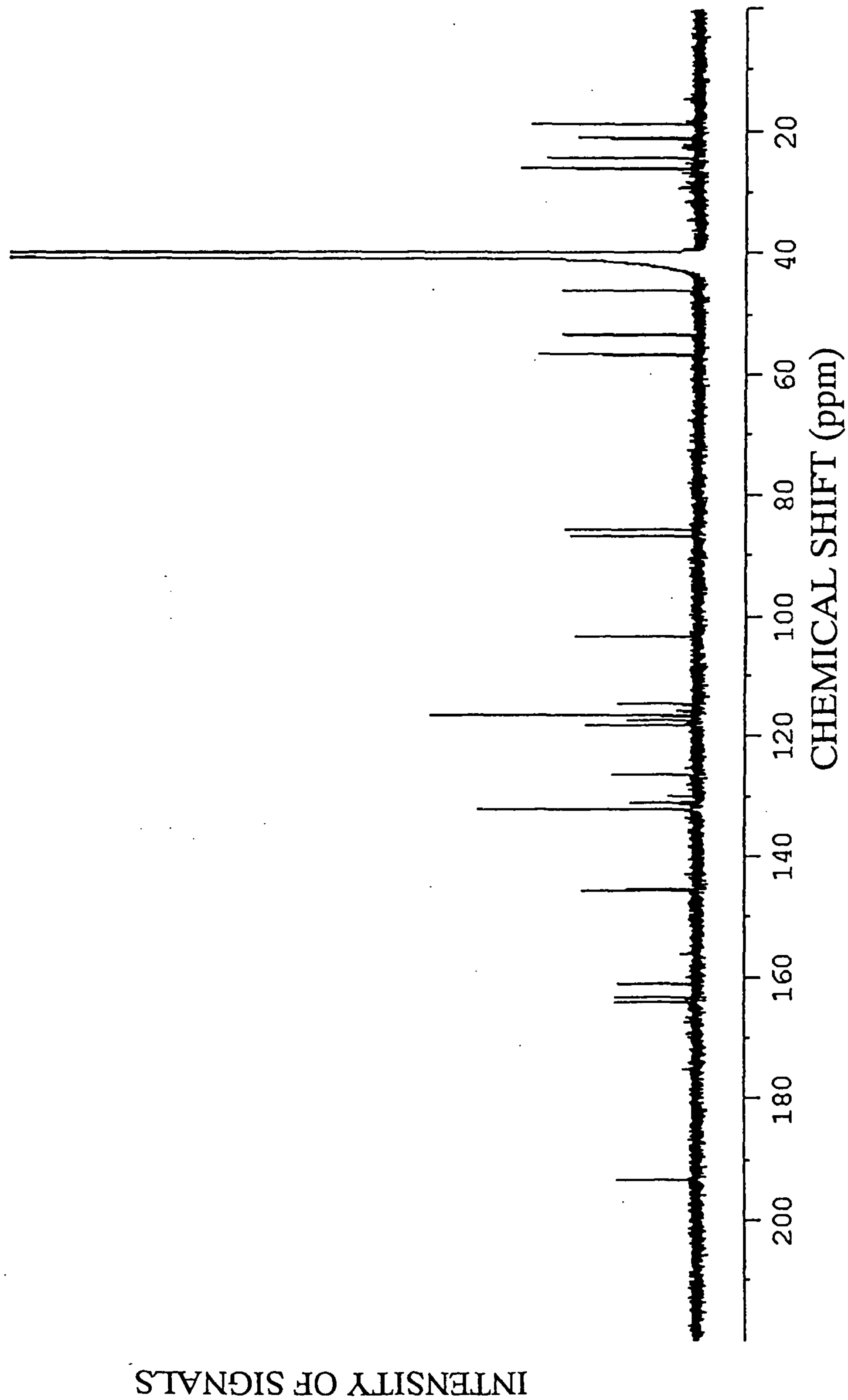
20
40
60
80
100
120
140
160
180
200
CHEMICAL SHIFT (ppm)
INTENSITY OF SIGNALS

FIG. 14

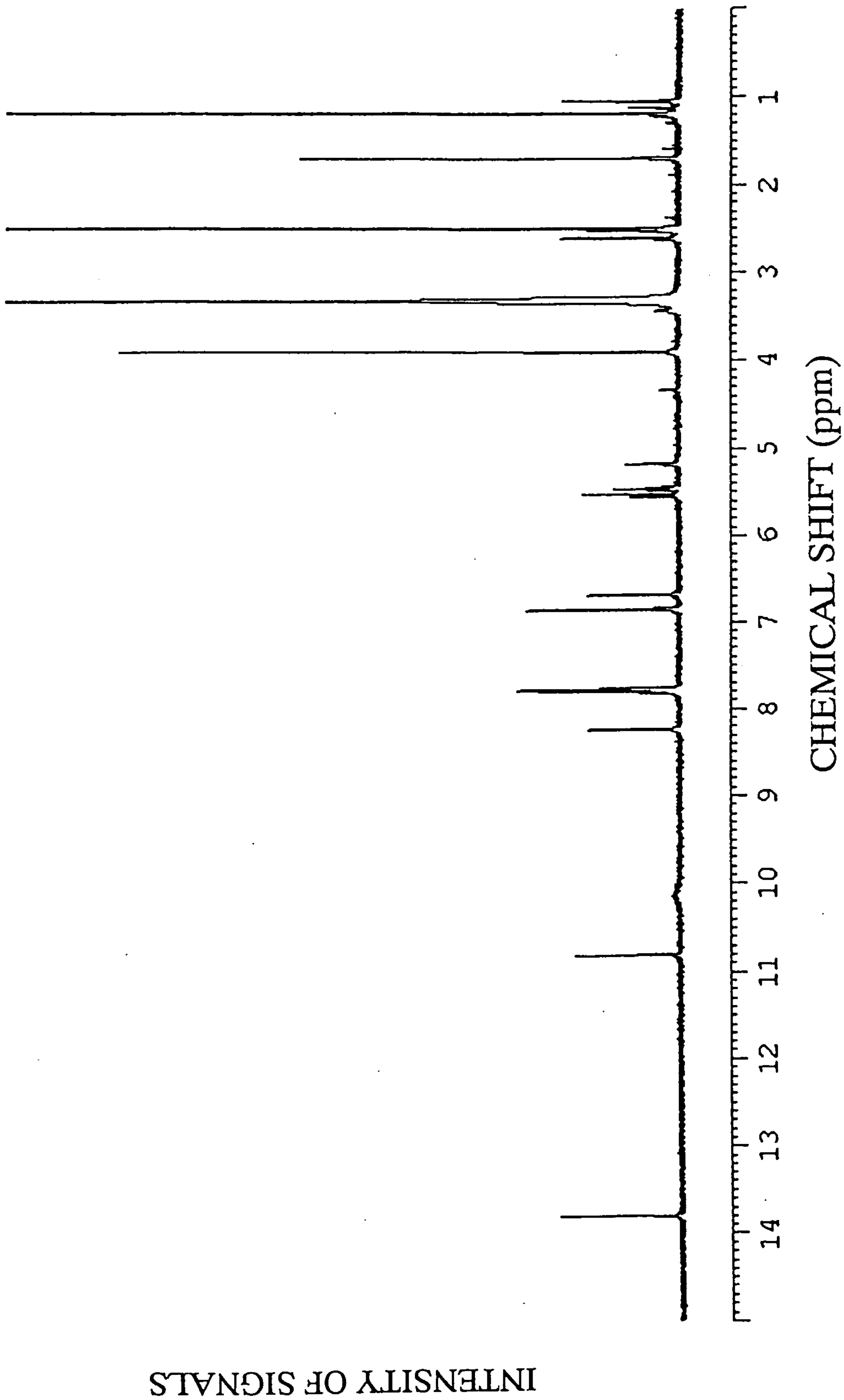
INTENSITY OF SIGNALS
CHEMICAL SHIFT (ppm)
1 2 3 4 5 6 7 8 9 10 11 12 13 14

FIG. 15

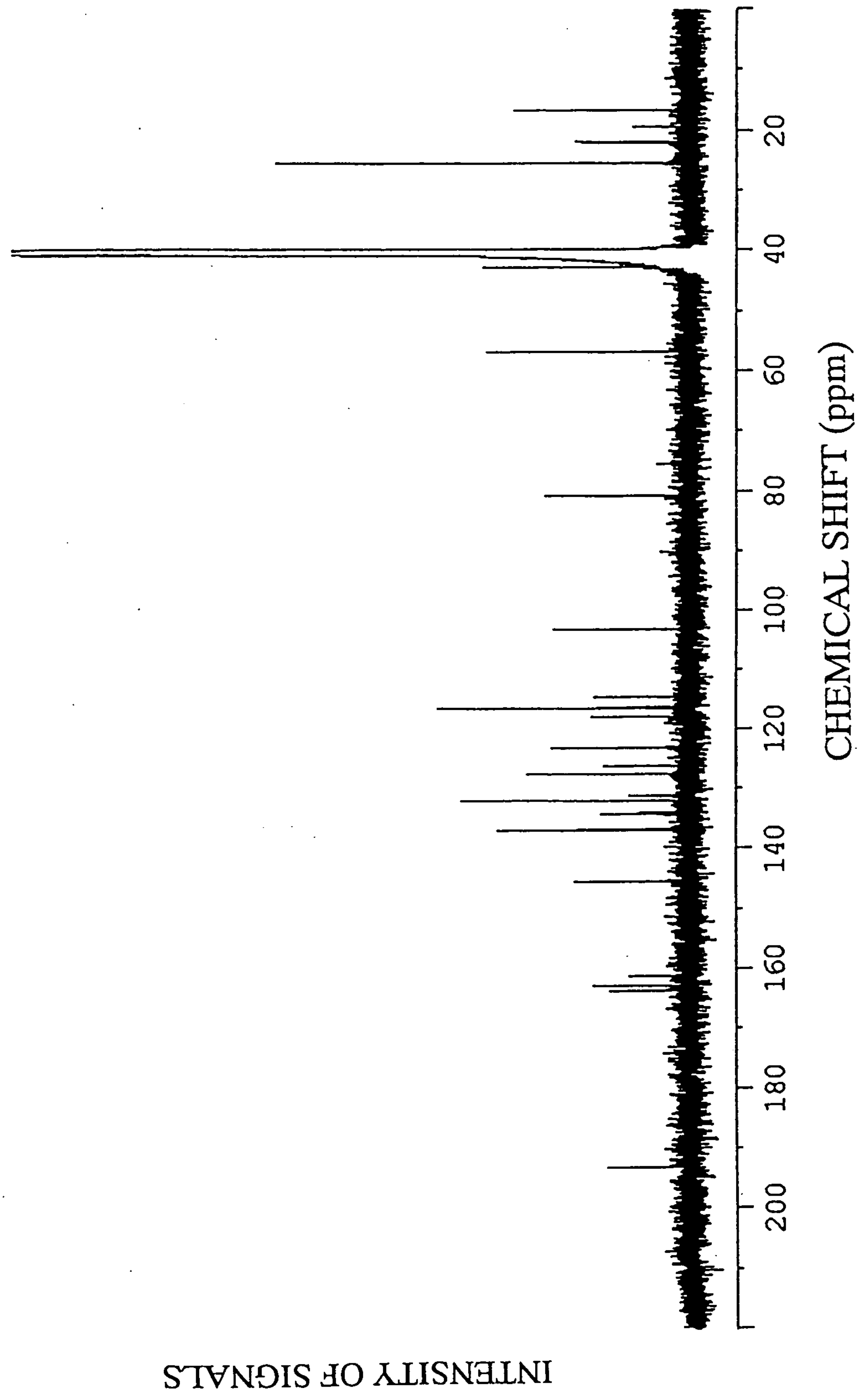
20
40
60
80
100
120
140
160
180
200
CHEMICAL SHIFT (ppm)
INTENSITY OF SIGNALS

FIG. 16

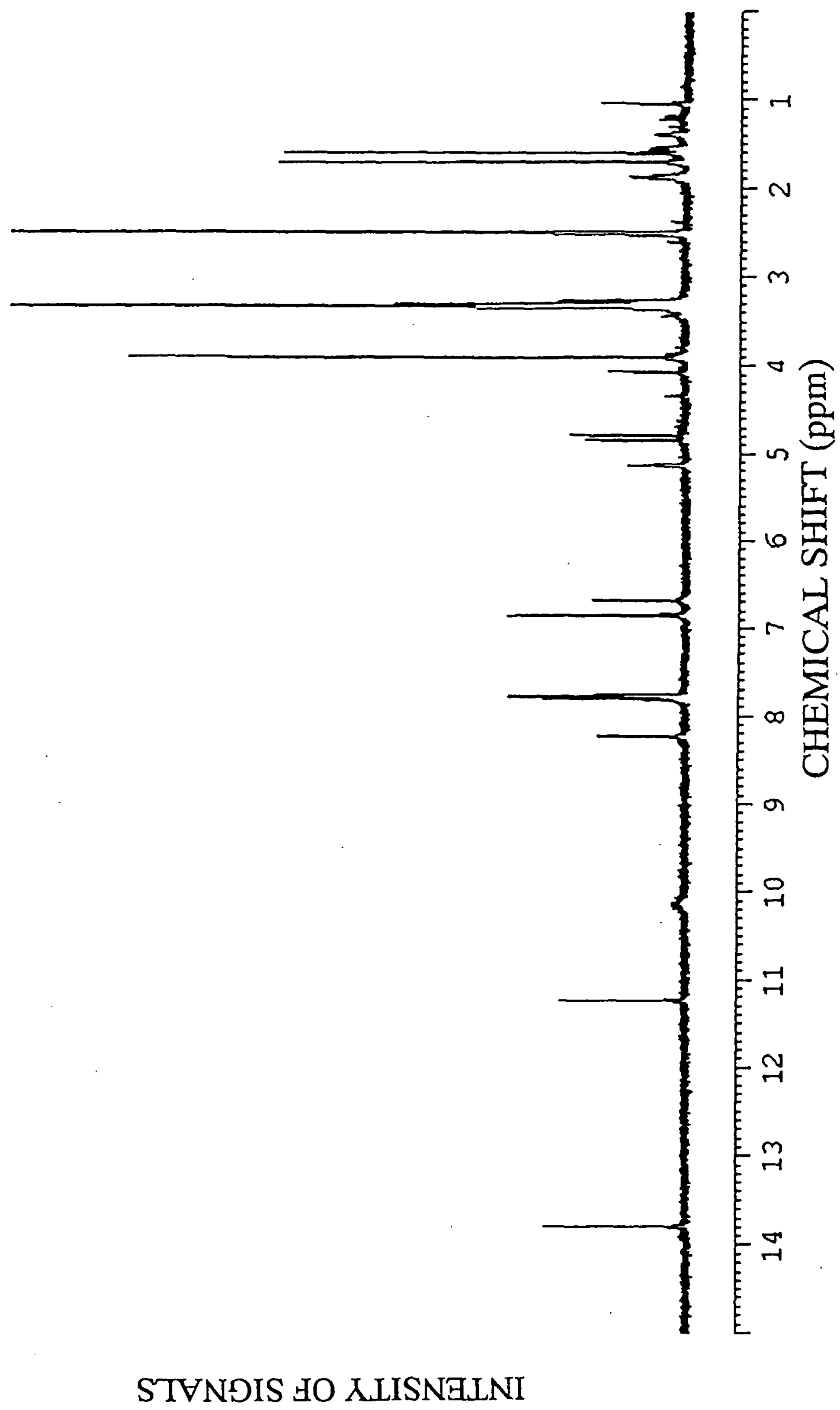
INTENSITY OF SIGNALS
CHEMICAL SHIFT (ppm)
1
2
3
4
5
6
7
8
9
10
11
12
13
14

FIG. 17

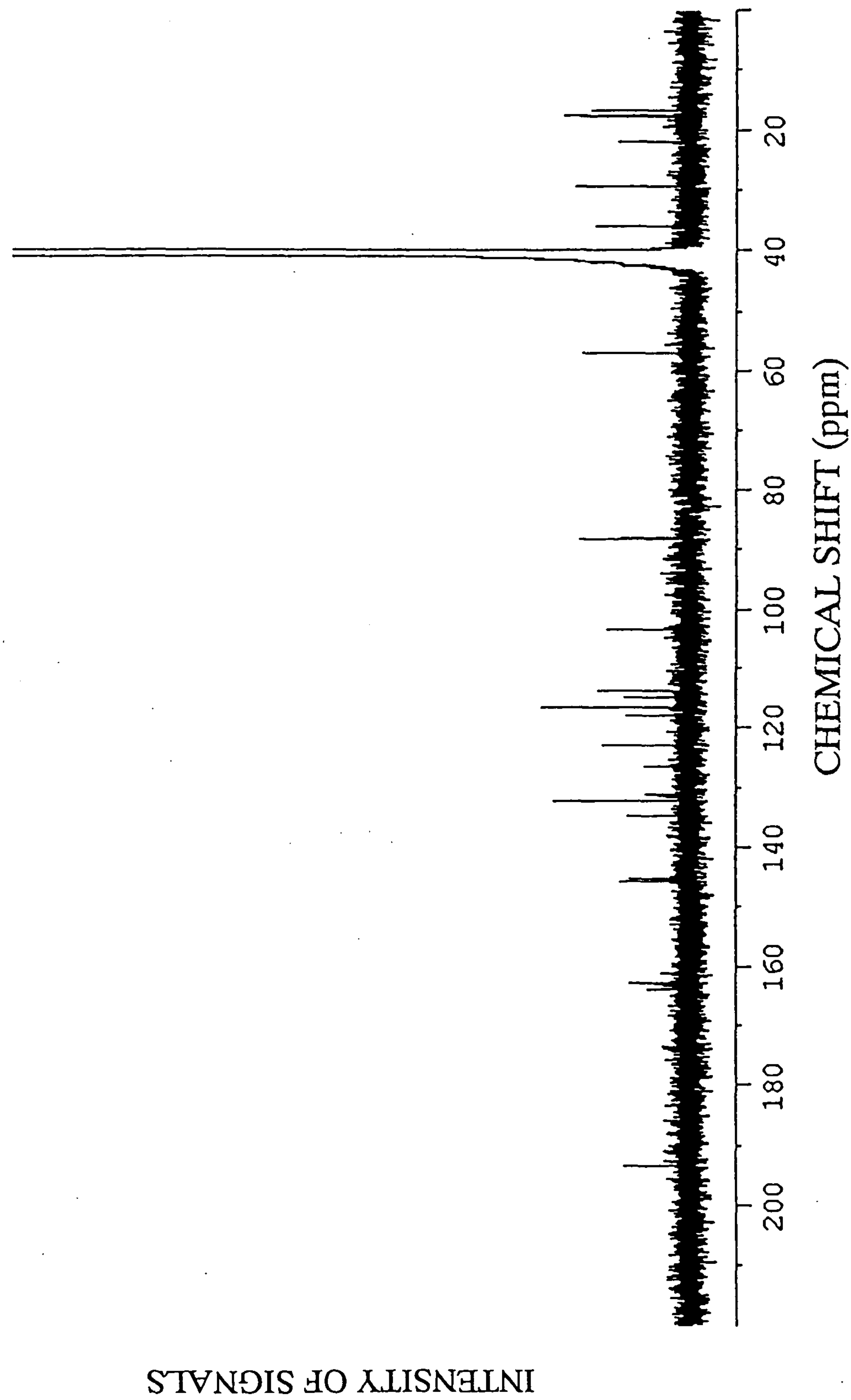

20
40
60
80
100
120
140
160
180
200
CHEMICAL SHIFT (ppm)
INTENSITY OF SIGNALS


FIG. 18

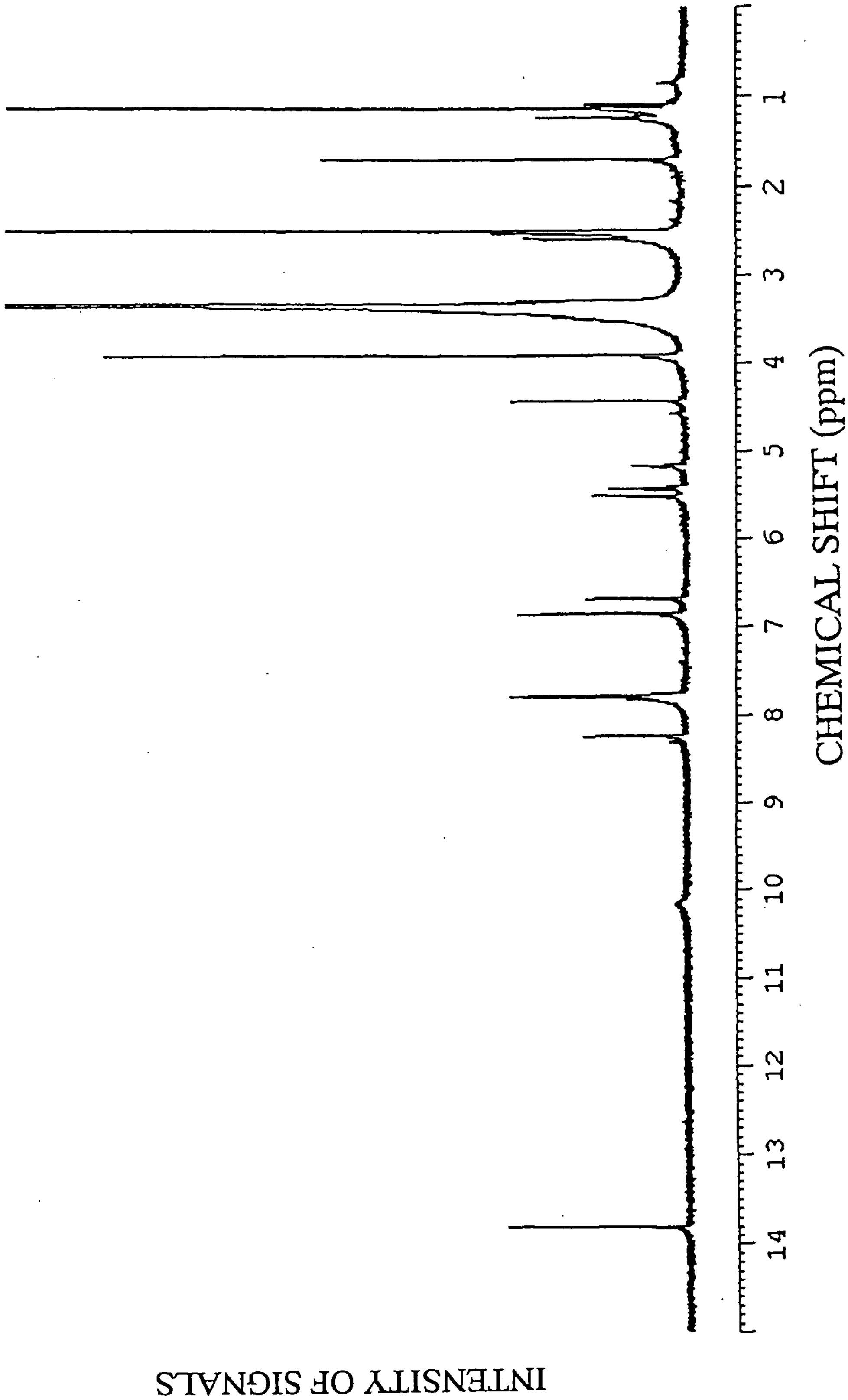
INTENSITY OF SIGNALS
CHEMICAL SHIFT (ppm)
1
2
3
4
5
6
7
8
9
10
11
12
13
14

FIG. 19

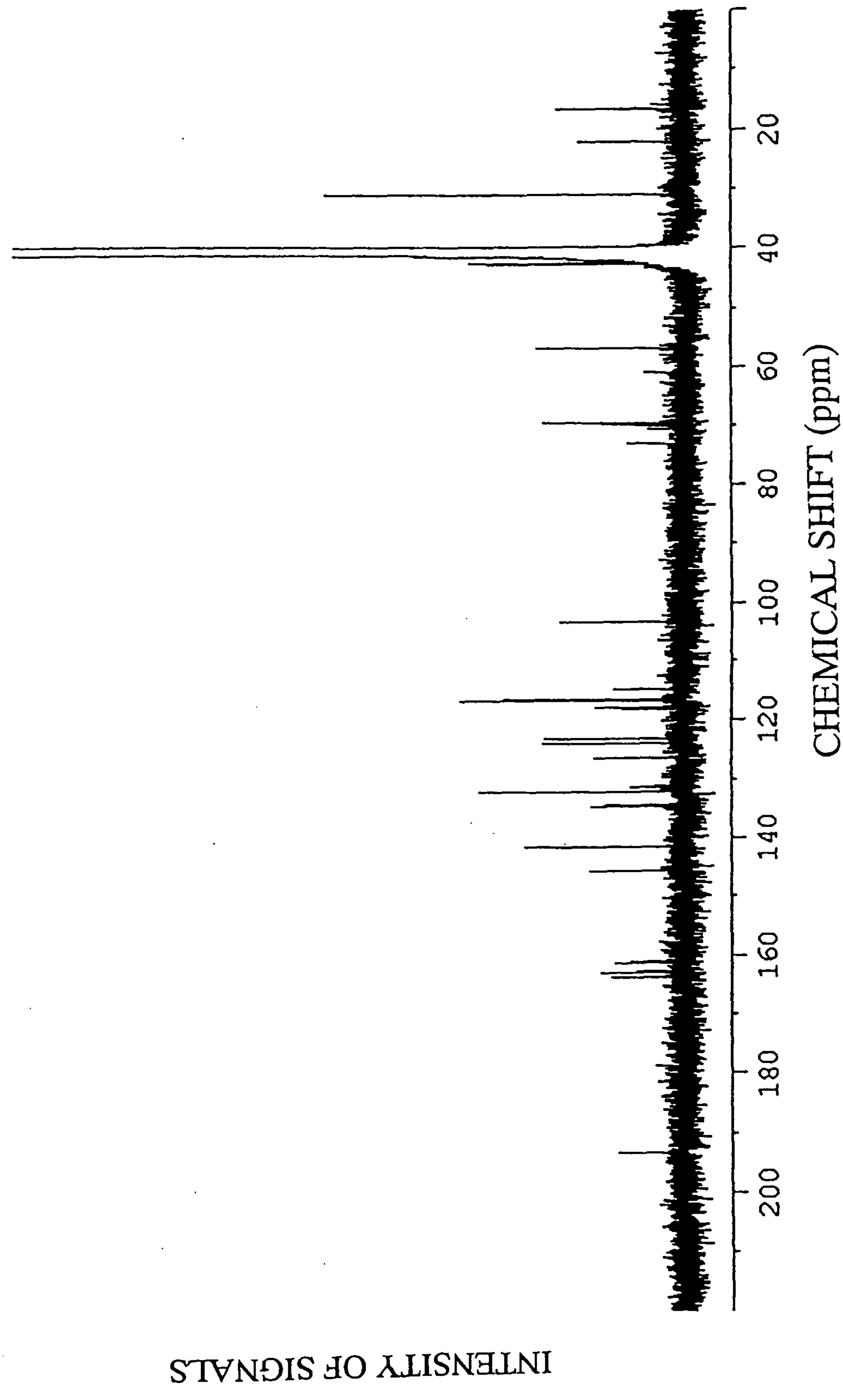
20
40
60
80
100
120
140
160
180
200
CHEMICAL SHIFT (ppm)
INTENSITY OF SIGNALS

FIG. 20

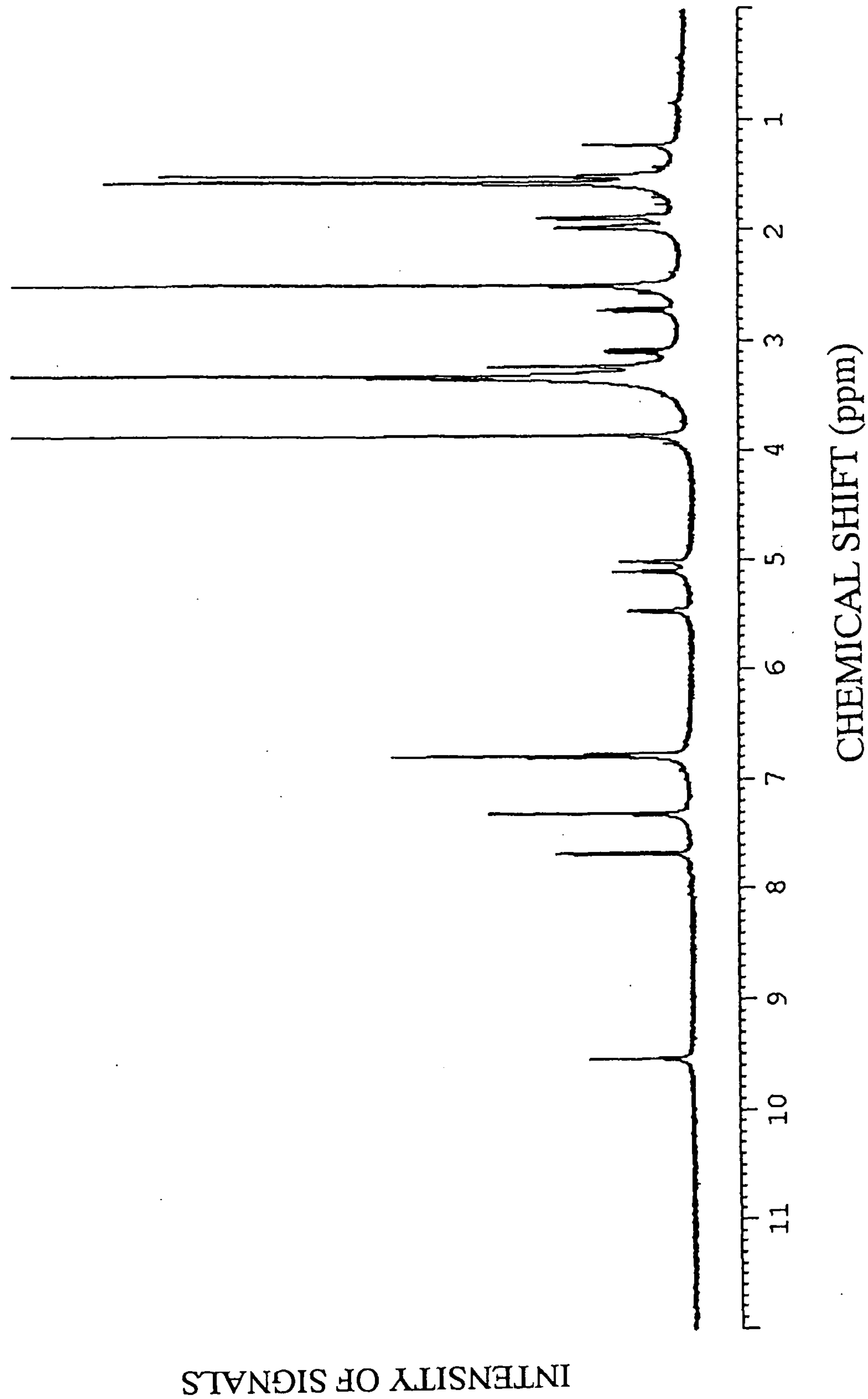


FIG. 21

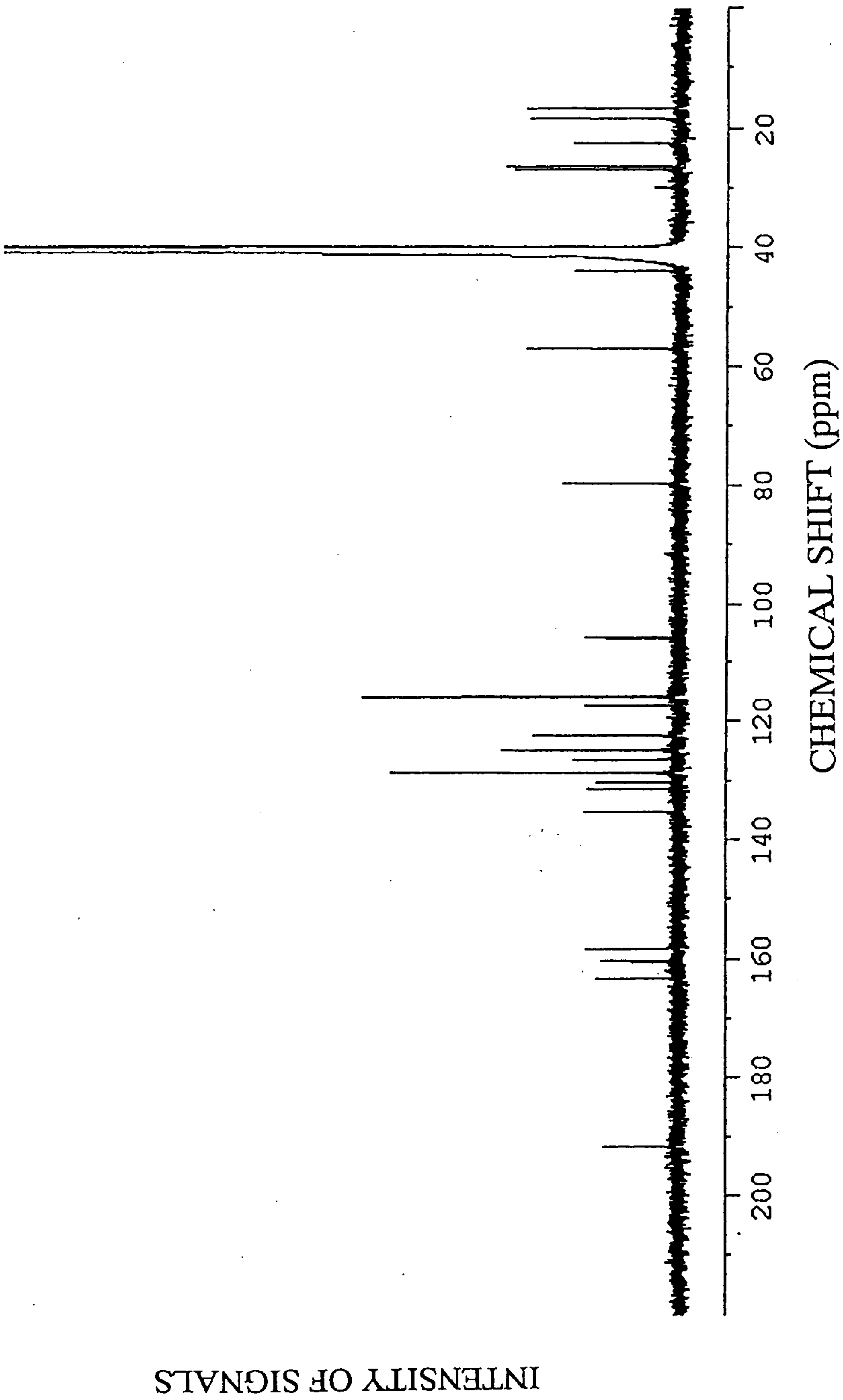
20
40
60
80
100
120
140
160
180
200
CHEMICAL SHIFT (ppm)
INTENSITY OF SIGNALS

FIG. 22

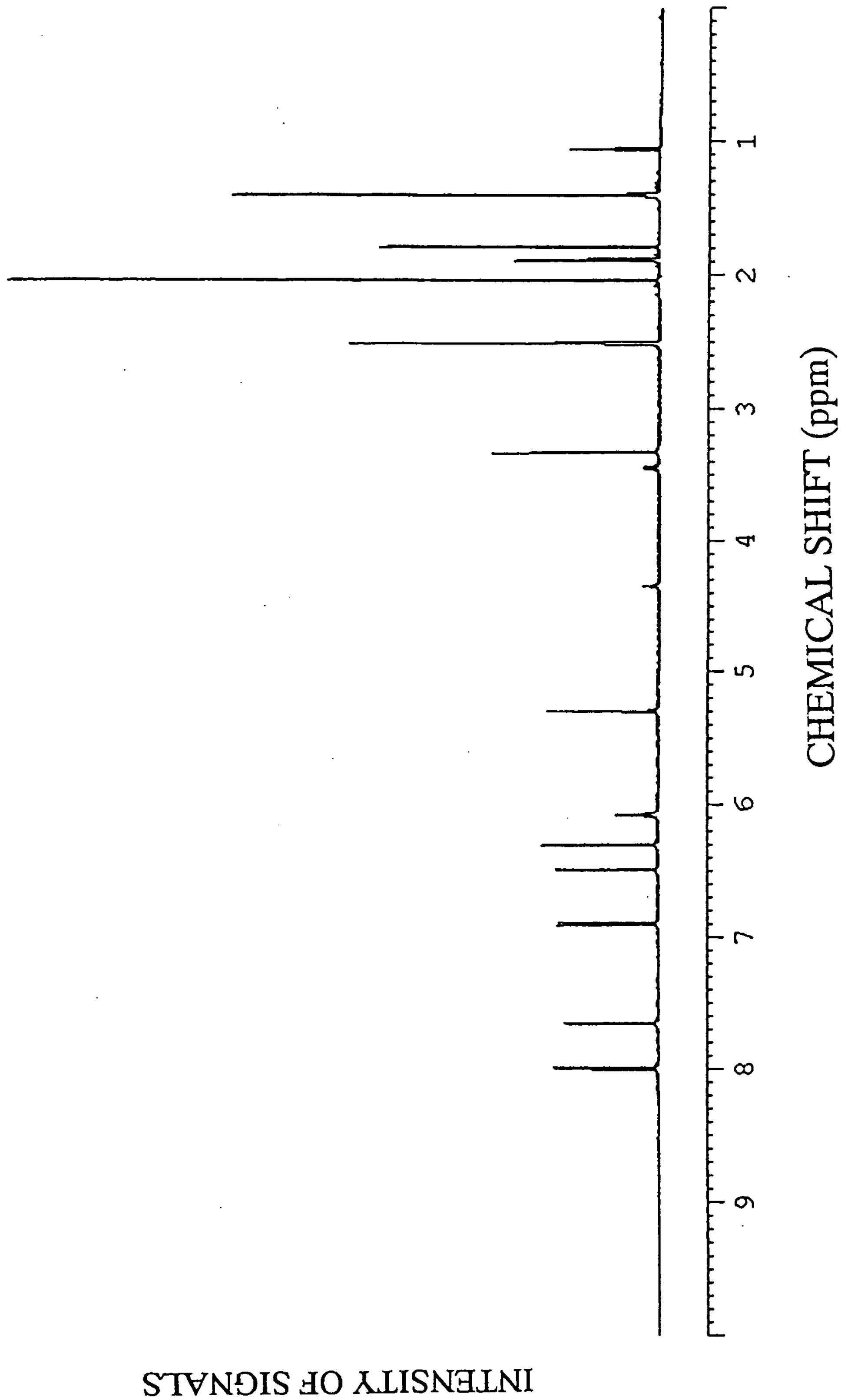


FIG. 23

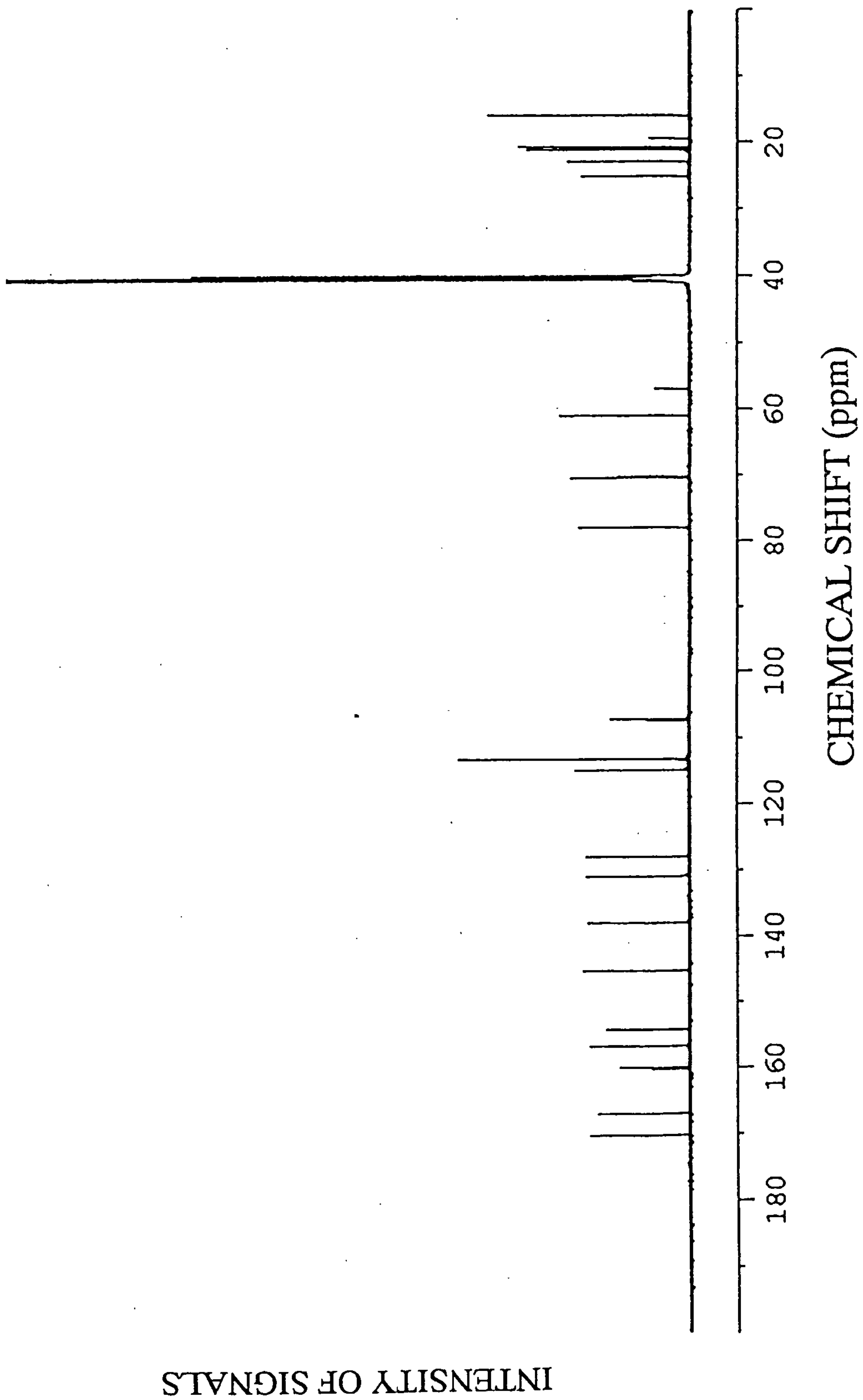
20
40
60
80
100
120
140
160
180
CHEMICAL SHIFT (ppm)
INTENSITY OF SIGNALS

FIG. 24

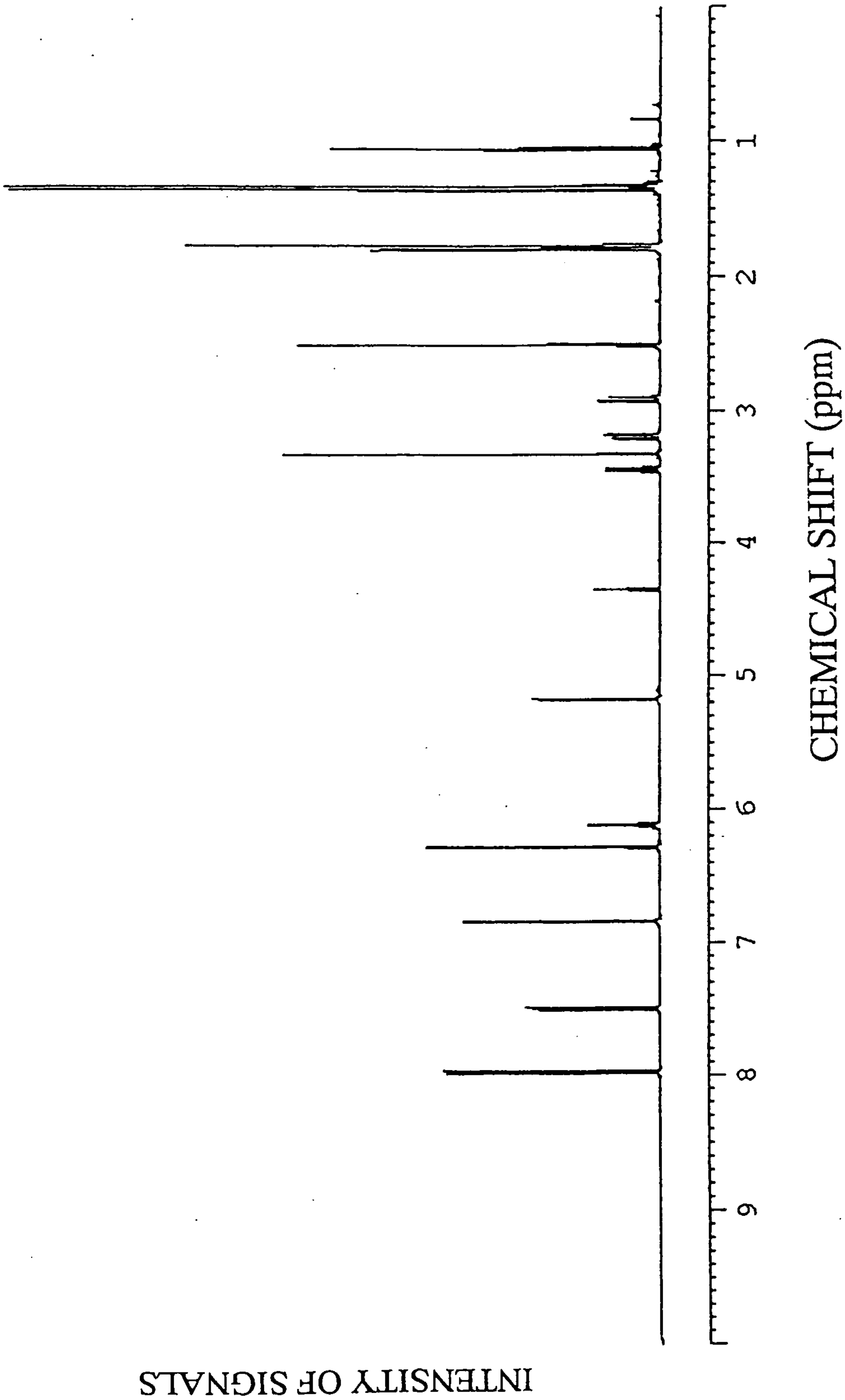


FIG. 25

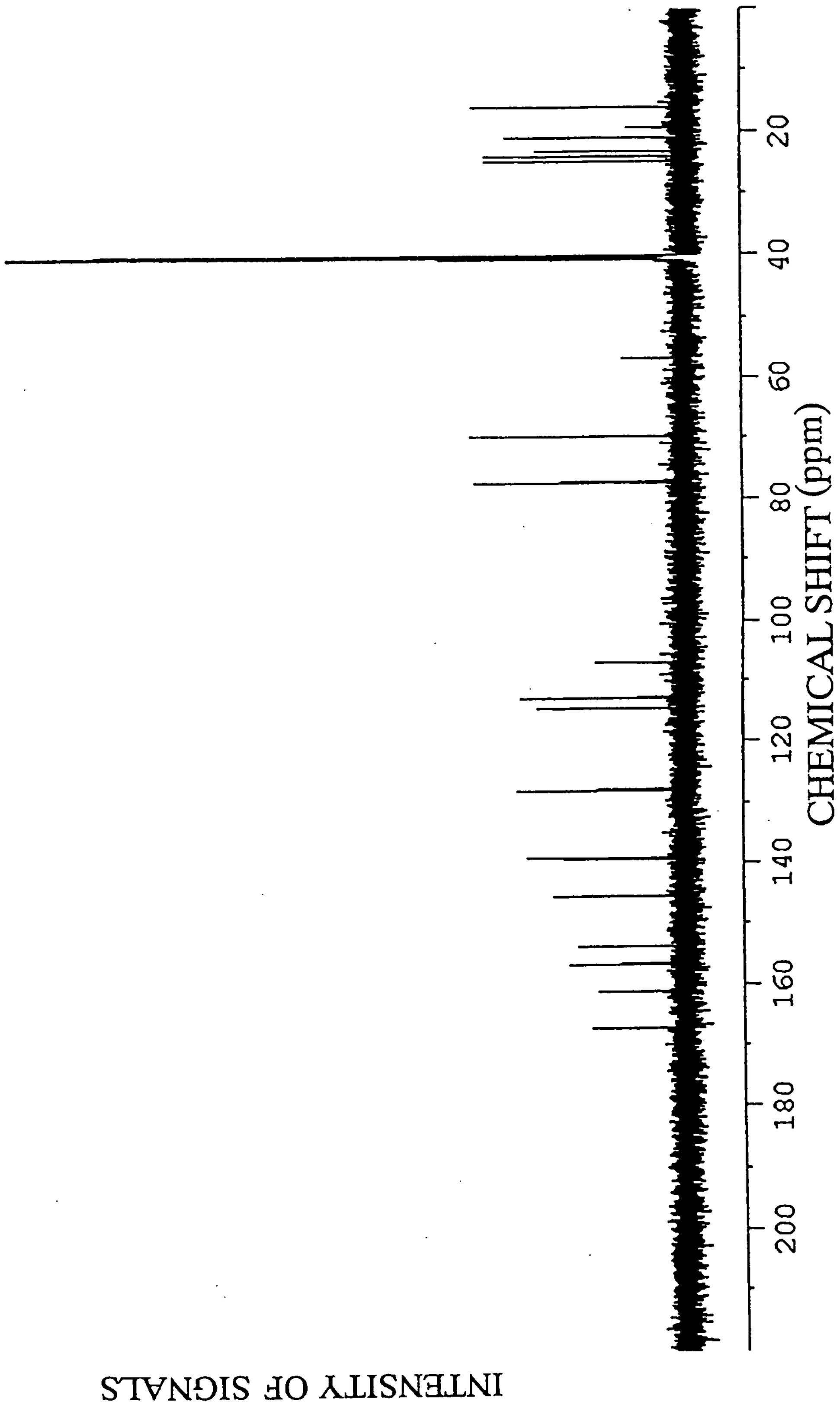
20
40
60
80
100
120
140
160
180
200
CHEMICAL SHIFT (ppm)
INTENSITY OF SIGNALS

FIG. 26

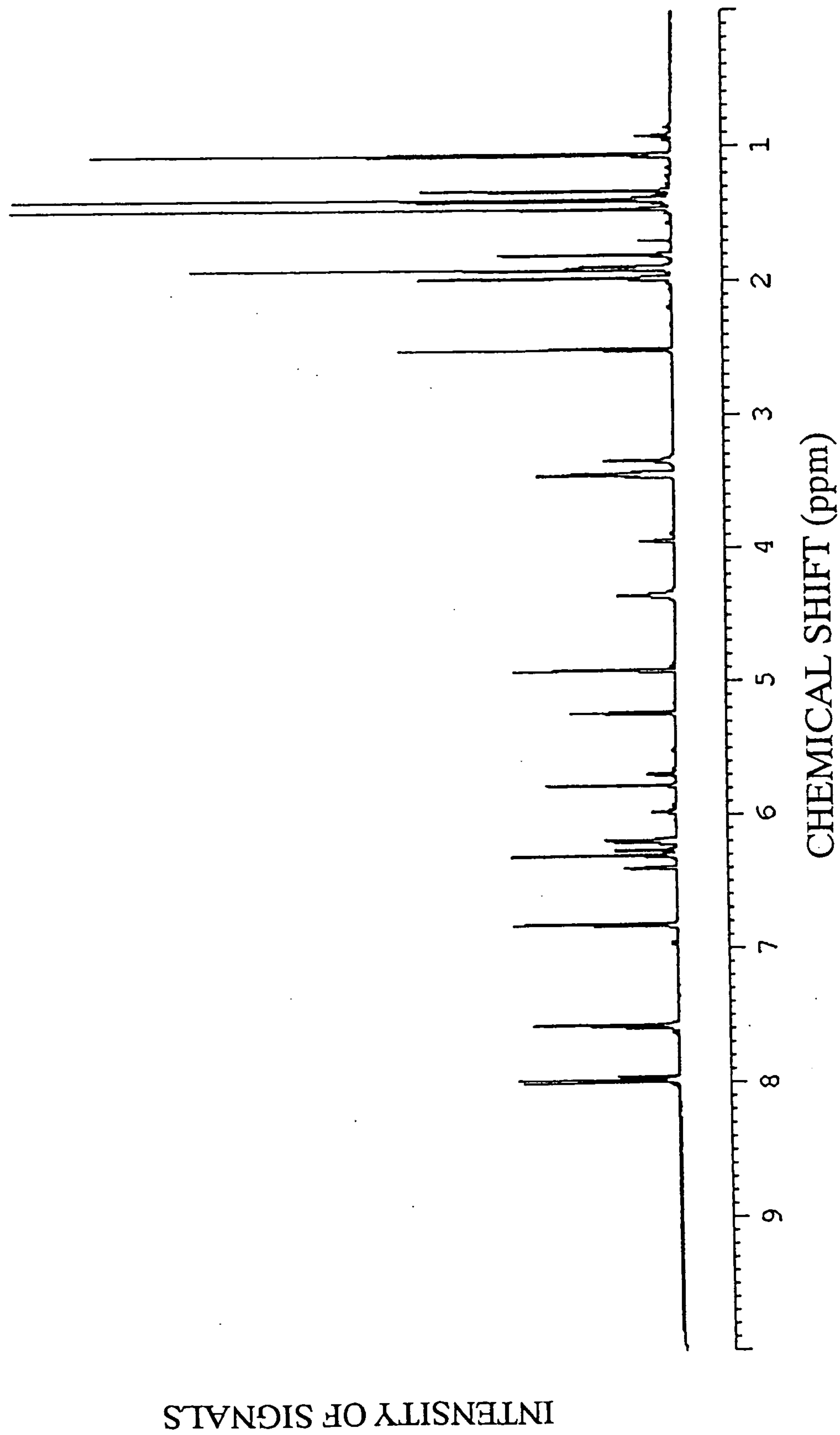


FIG. 27

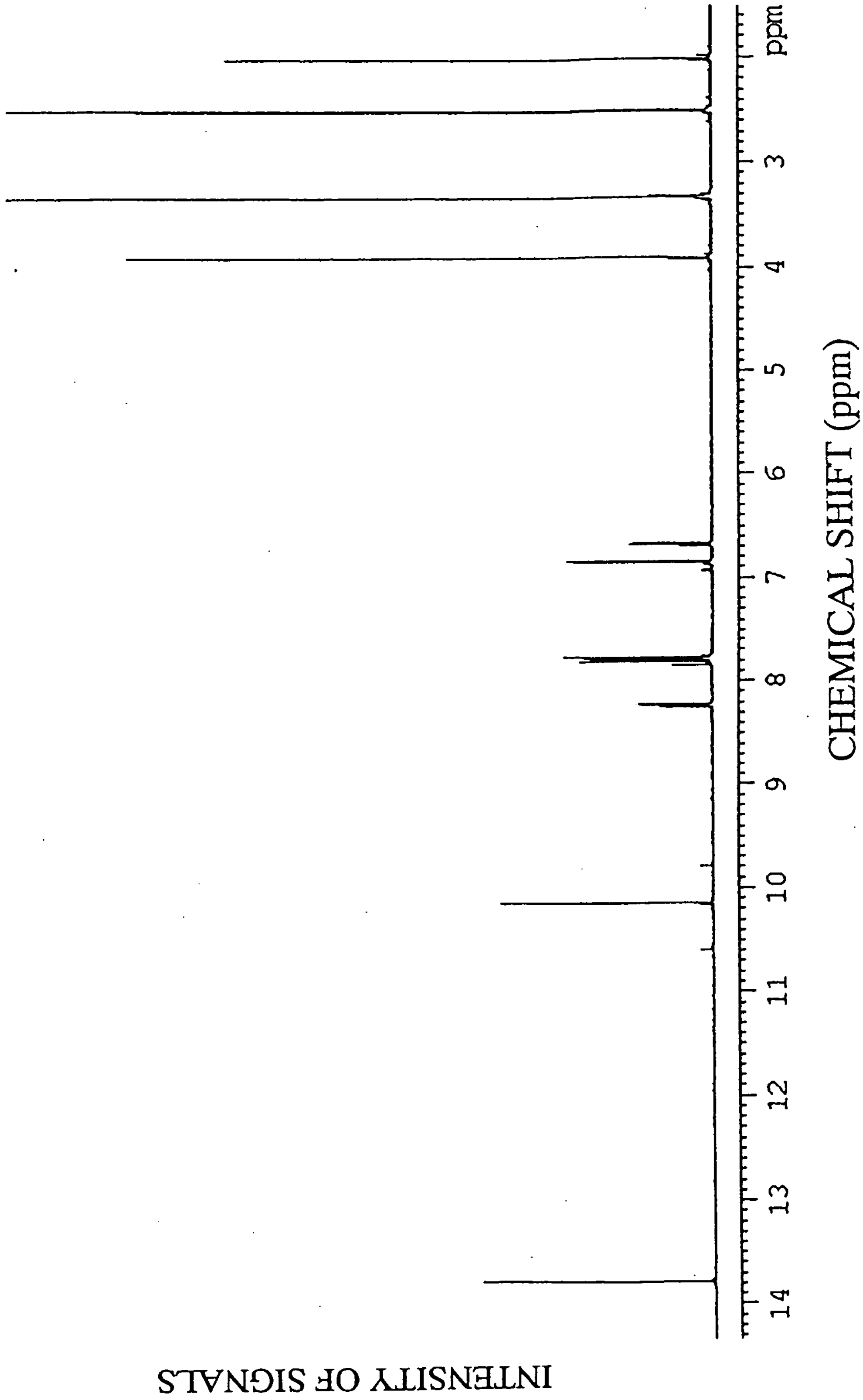
ppm
3
4
5
6
7
8
9
10
11
12
13
14
CHEMICAL SHIFT (ppm)
INTENSITY OF SIGNALS

FIG. 28

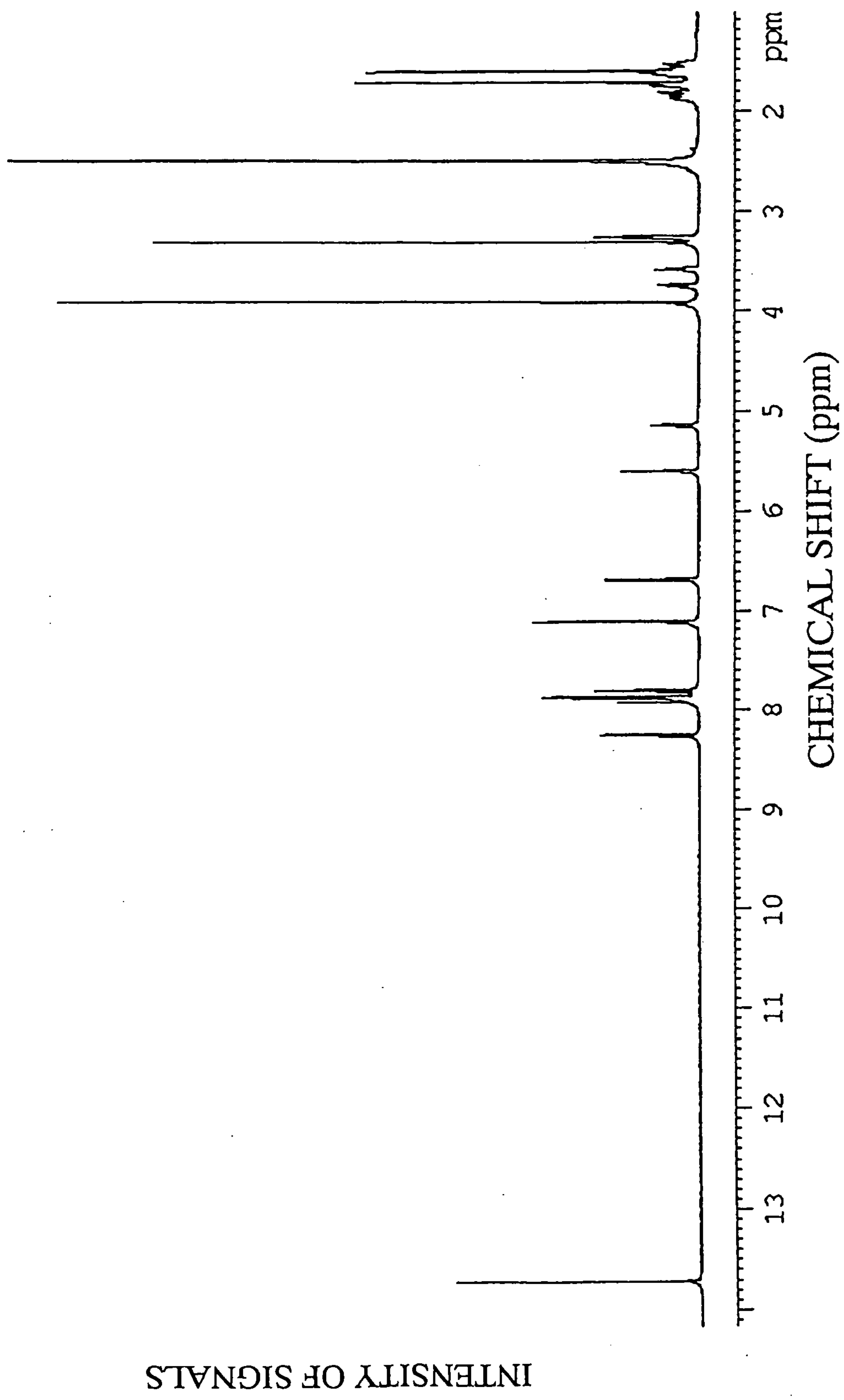
ppm
2
3
4
5
6
7
8
9
10
11
12
13
CHEMICAL SHIFT (ppm)
INTENSITY OF SIGNALS

FIG. 29

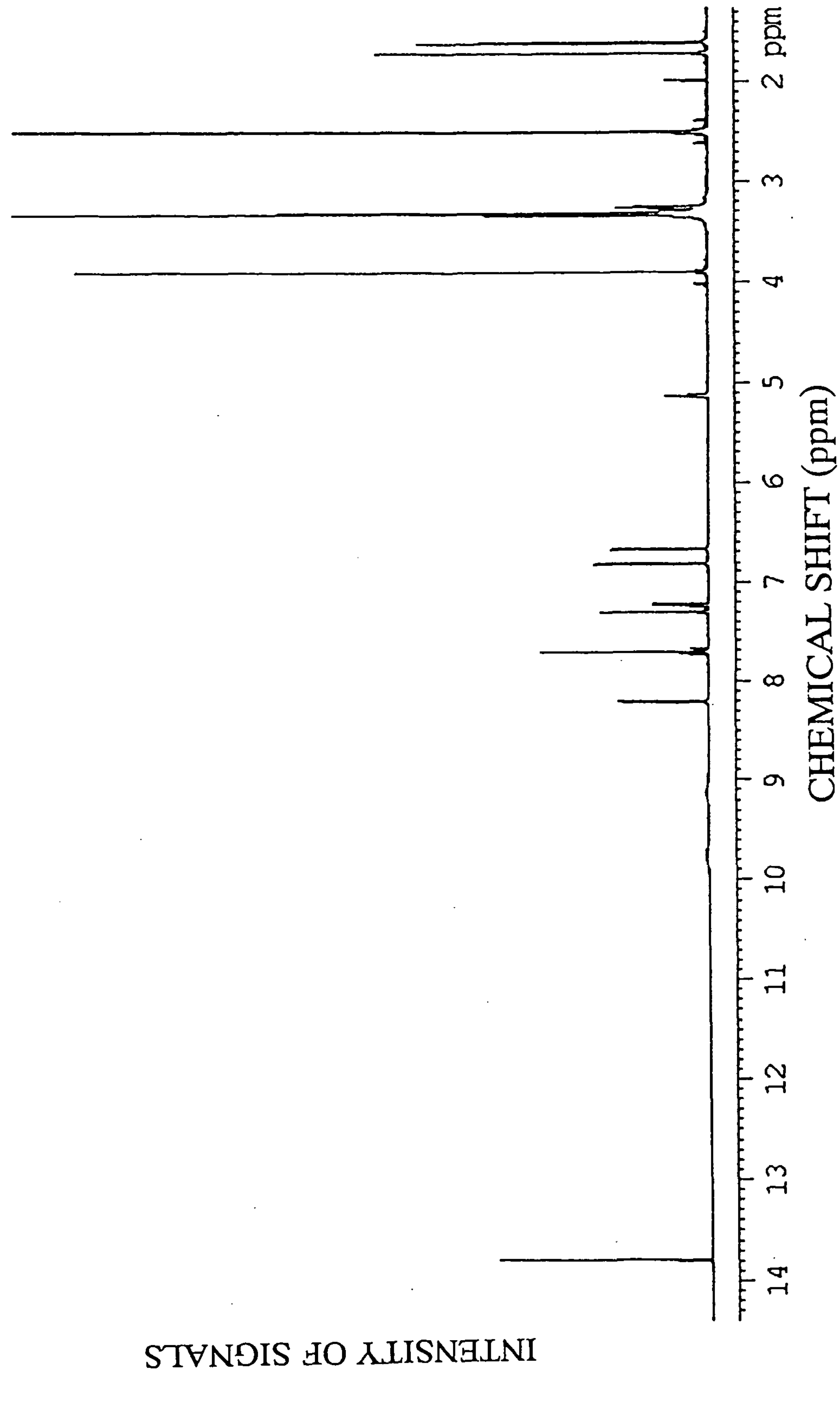

2 ppm
3
4
5
6
7
8
9
10
11
12
13
14
CHEMICAL SHIFT (ppm)
INTENSITY OF SIGNALS


FIG. 30

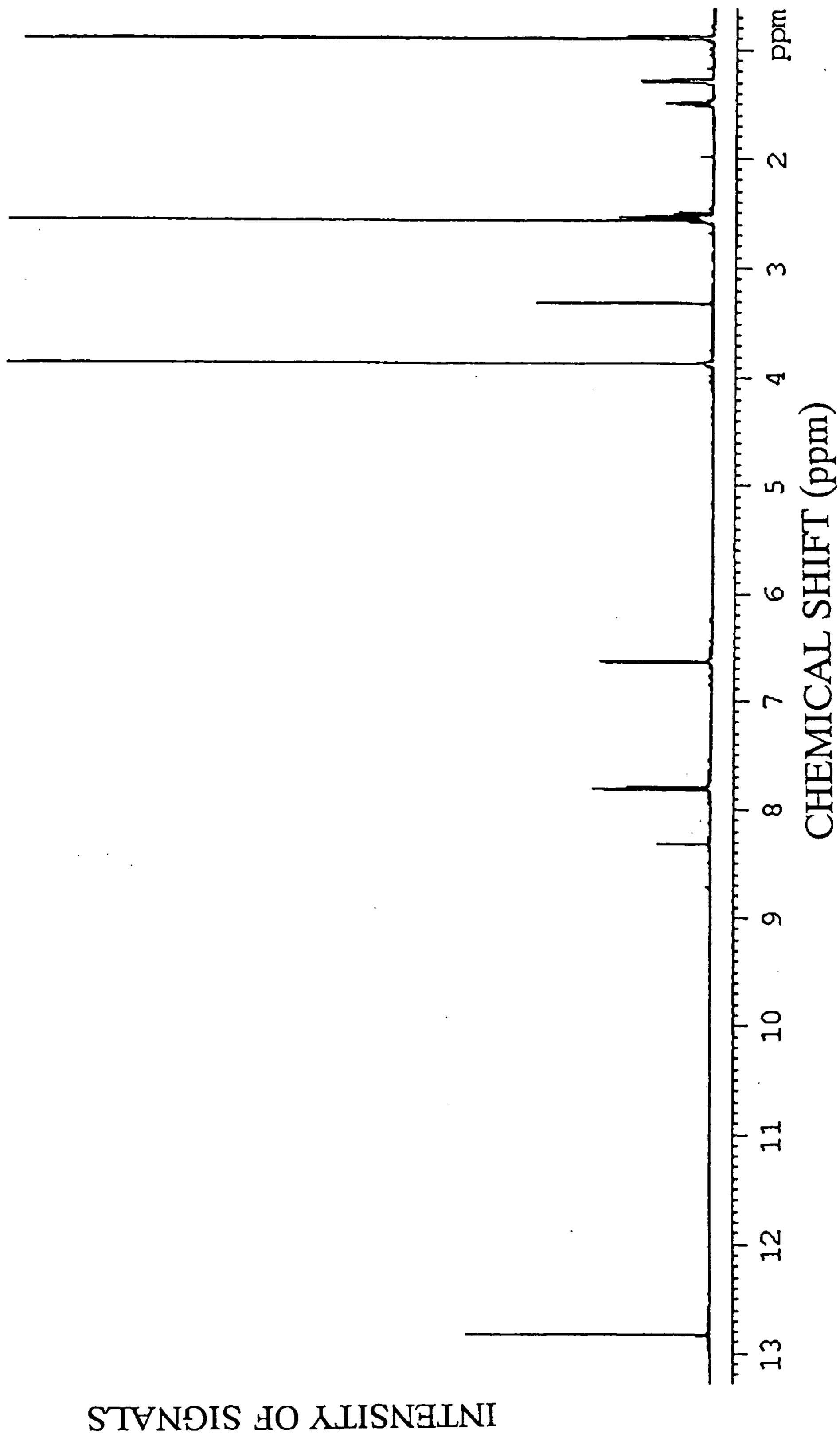
ppm
2
3
4
5
6
7
8
9
10
11
12
13
CHEMICAL SHIFT (ppm)
INTENSITY OF SIGNALS

FIG. 31

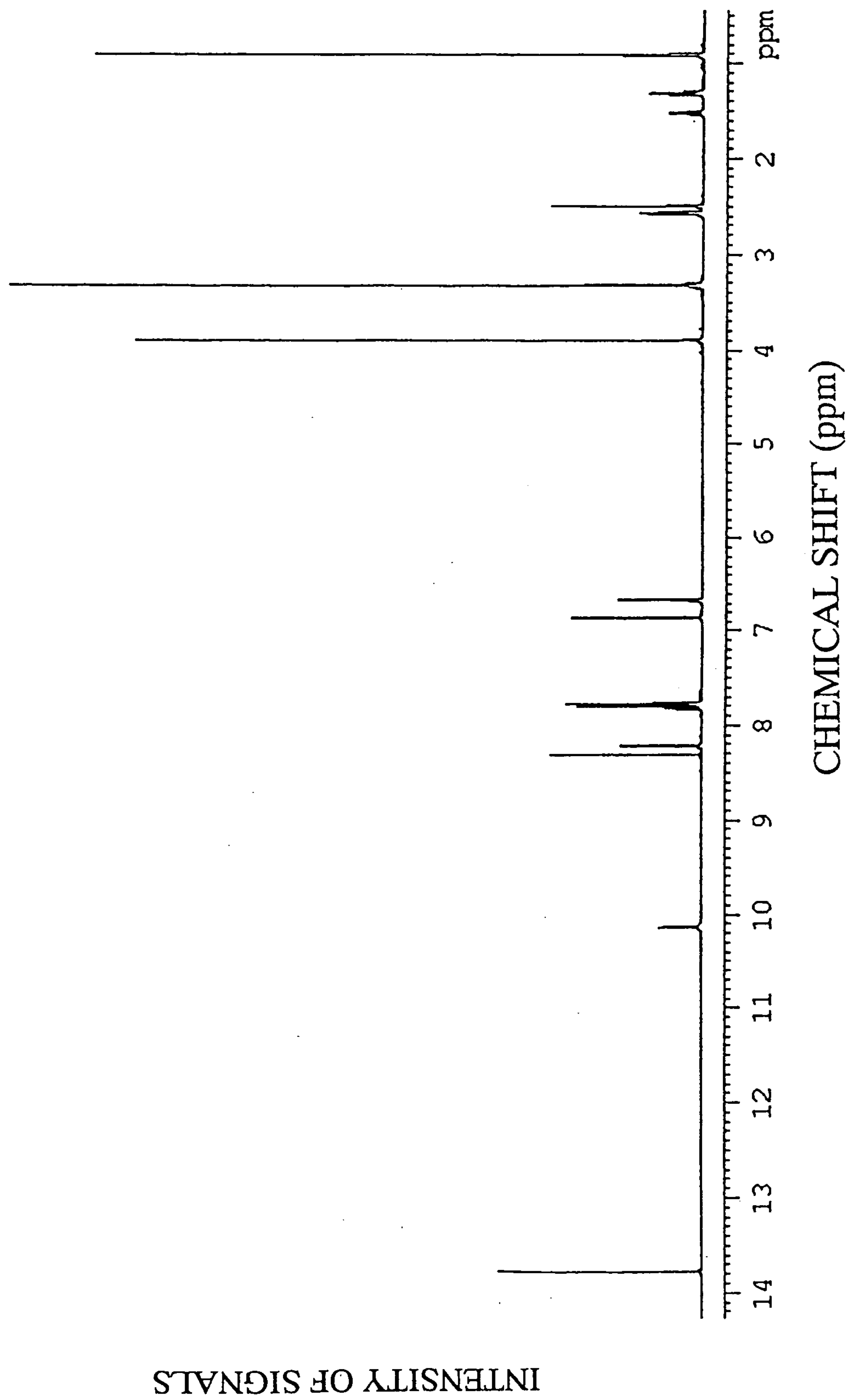
ppm
2
3
4
5
6
7
8
9
10
11
12
13
14
CHEMICAL SHIFT (ppm)
INTENSITY OF SIGNALS

FIG. 32

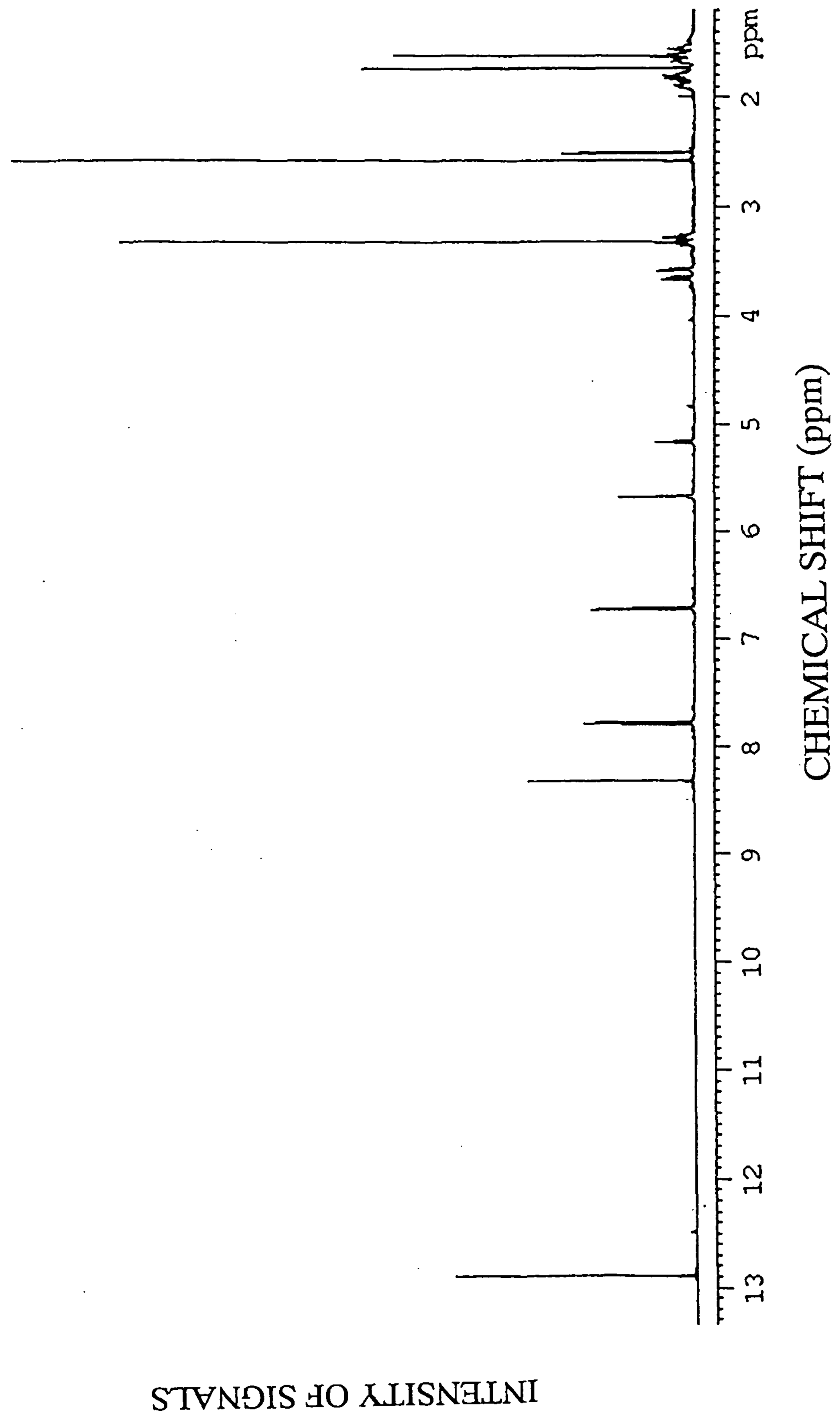
ppm
2
3
4
5
6
7
8
9
10
11
12
13
CHEMICAL SHIFT (ppm)
INTENSITY OF SIGNALS

FIG. 33

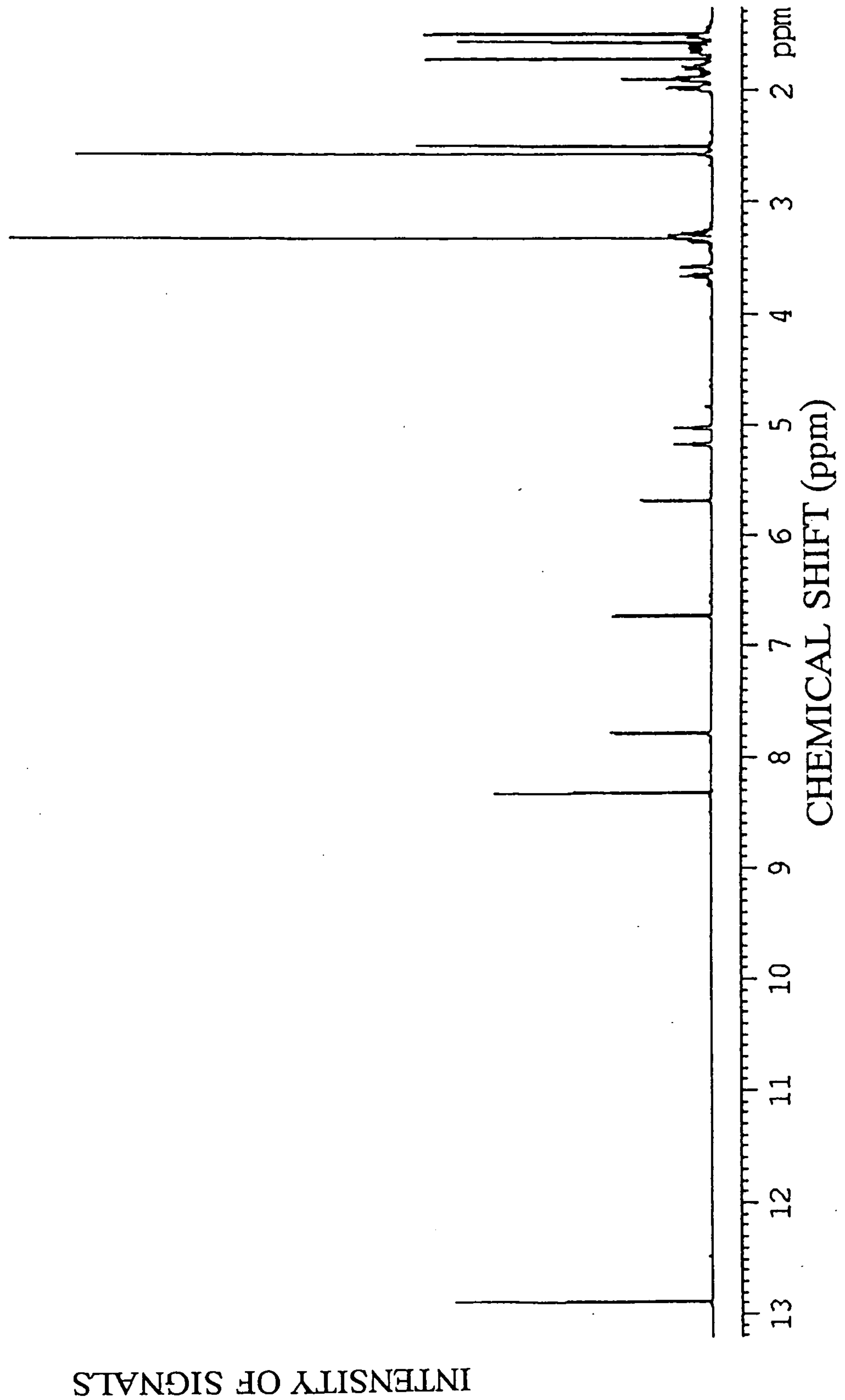

INTENSITY OF SIGNALS
CHEMICAL SHIFT (ppm)
ppm
2
3
4
5
6
7
8
9
10
11
12
13


FIG. 34

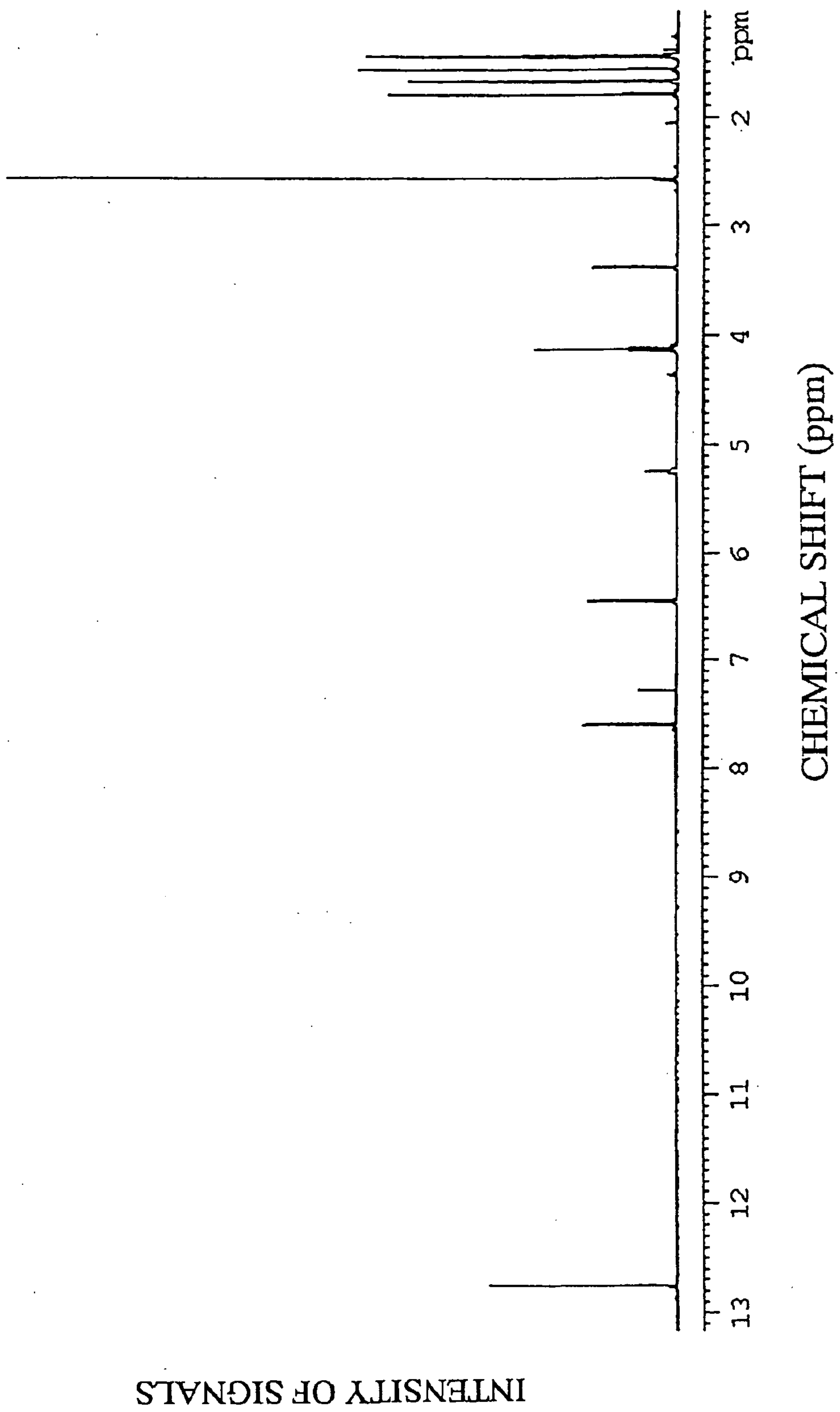
INTENSITY OF SIGNALS
13
12
11
10
9
8
7
6
5
4
3
2
ppm
CHEMICAL SHIFT (ppm)

FIG. 35

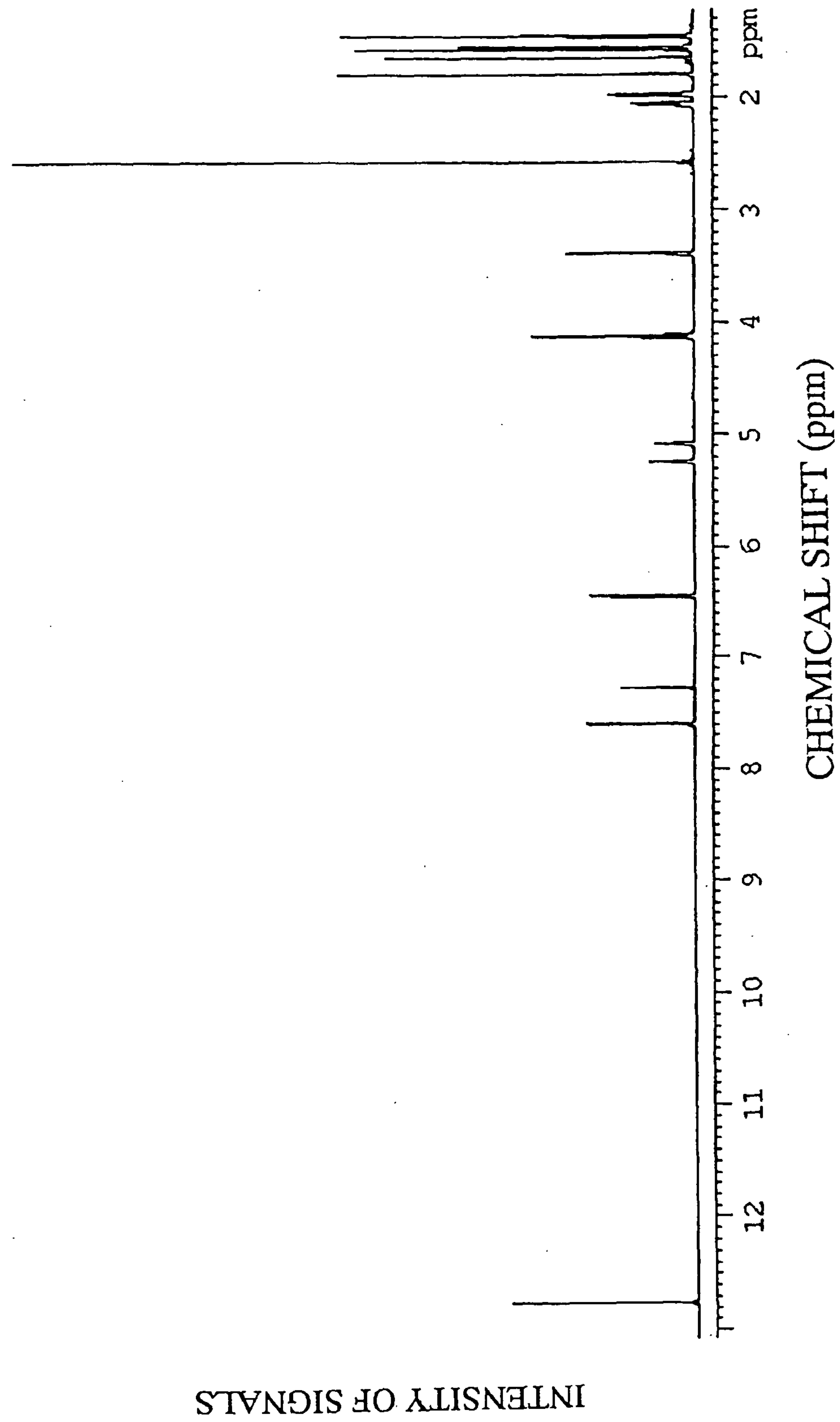
ppm
2
3
4
5
6
7
8
9
10
11
12
CHEMICAL SHIFT (ppm)
INTENSITY OF SIGNALS

FIG. 36

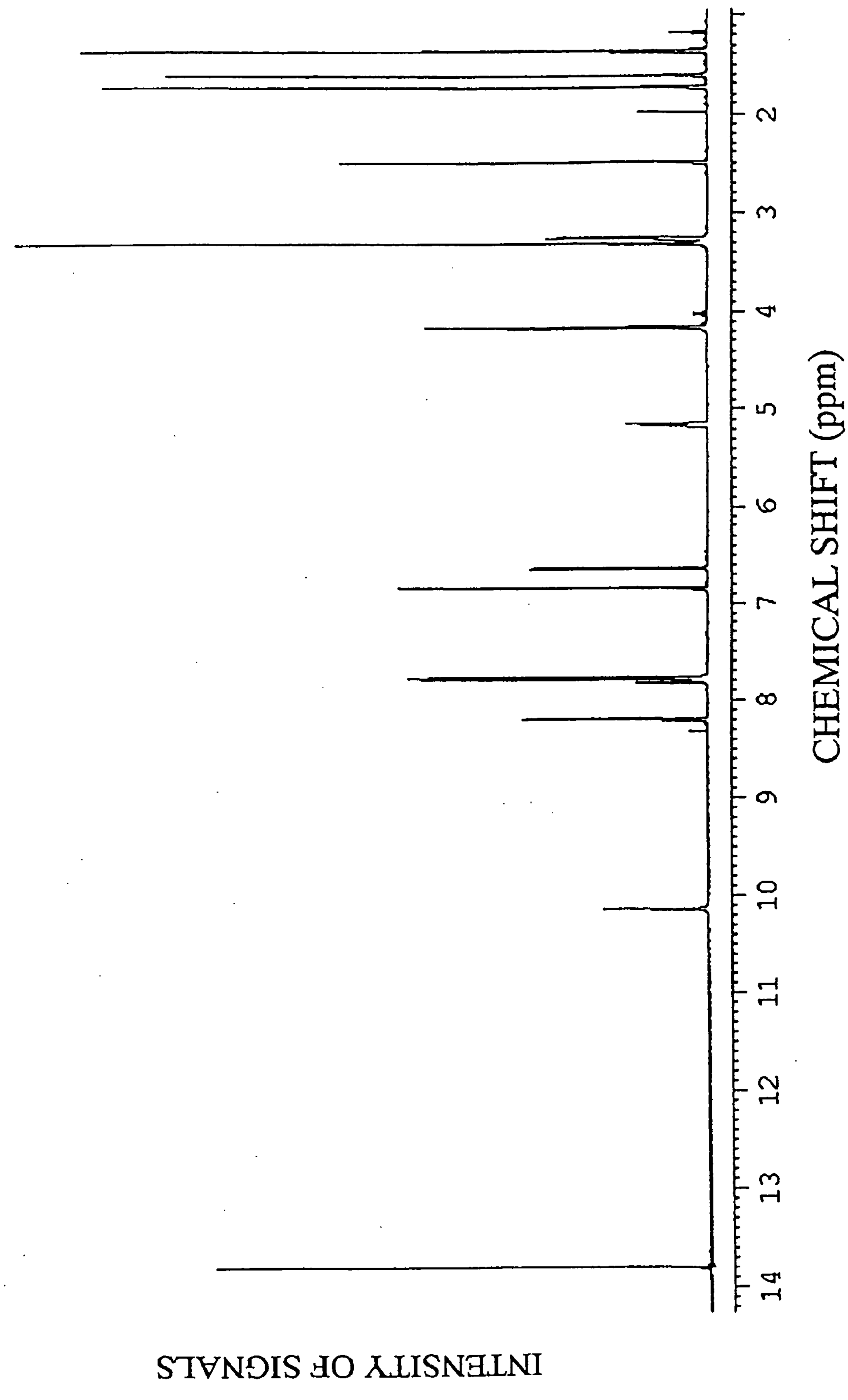
CHEMICAL SHIFT (ppm)
INTENSITY OF SIGNALS
2
3
4
5
6
7
8
9
10
11
12
13
14

FIG. 37

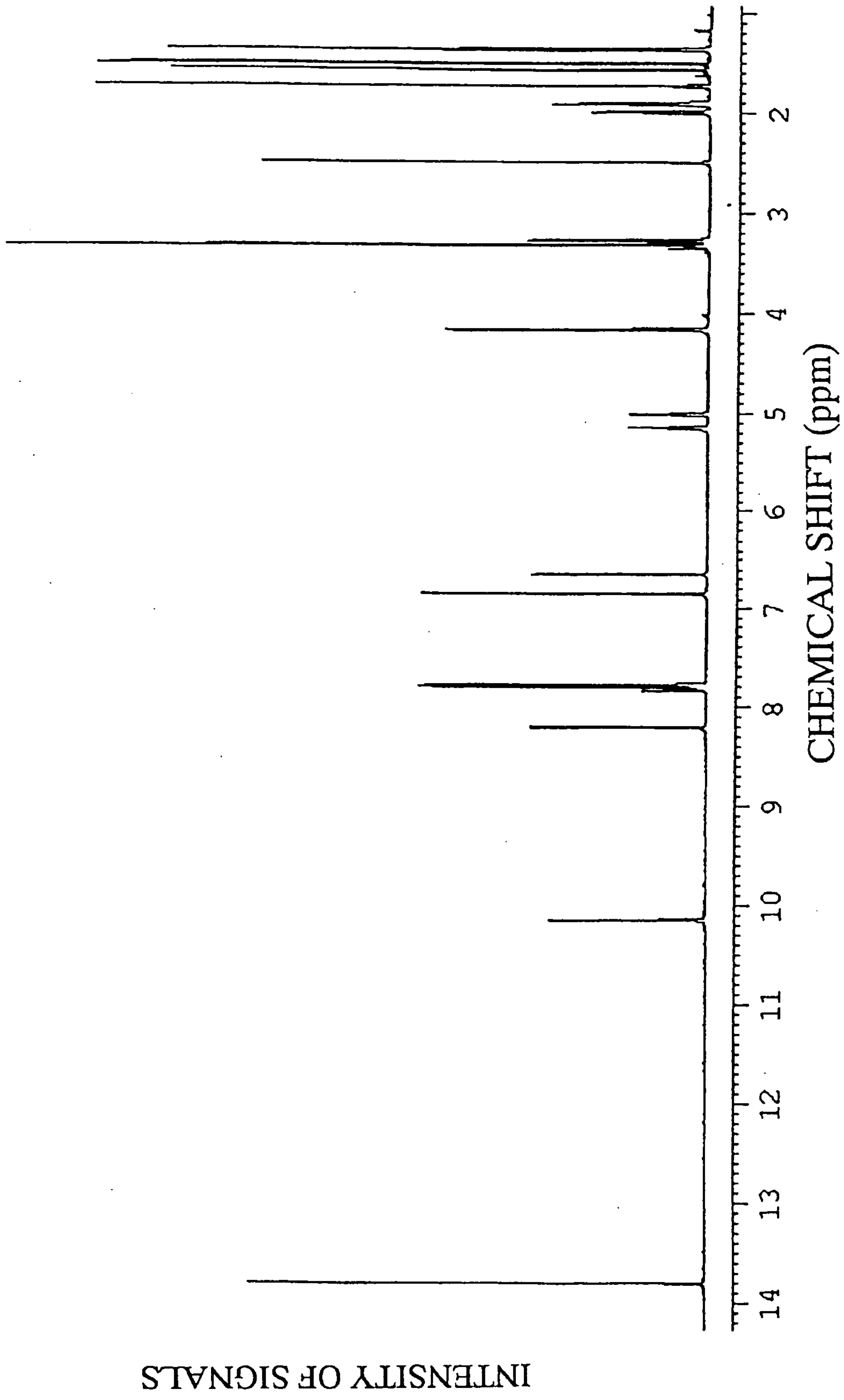
INTENSITY OF SIGNALS
CHEMICAL SHIFT (ppm)
14
13
12
11
10
9
8
7
6
5
4
3
2

FIG. 38

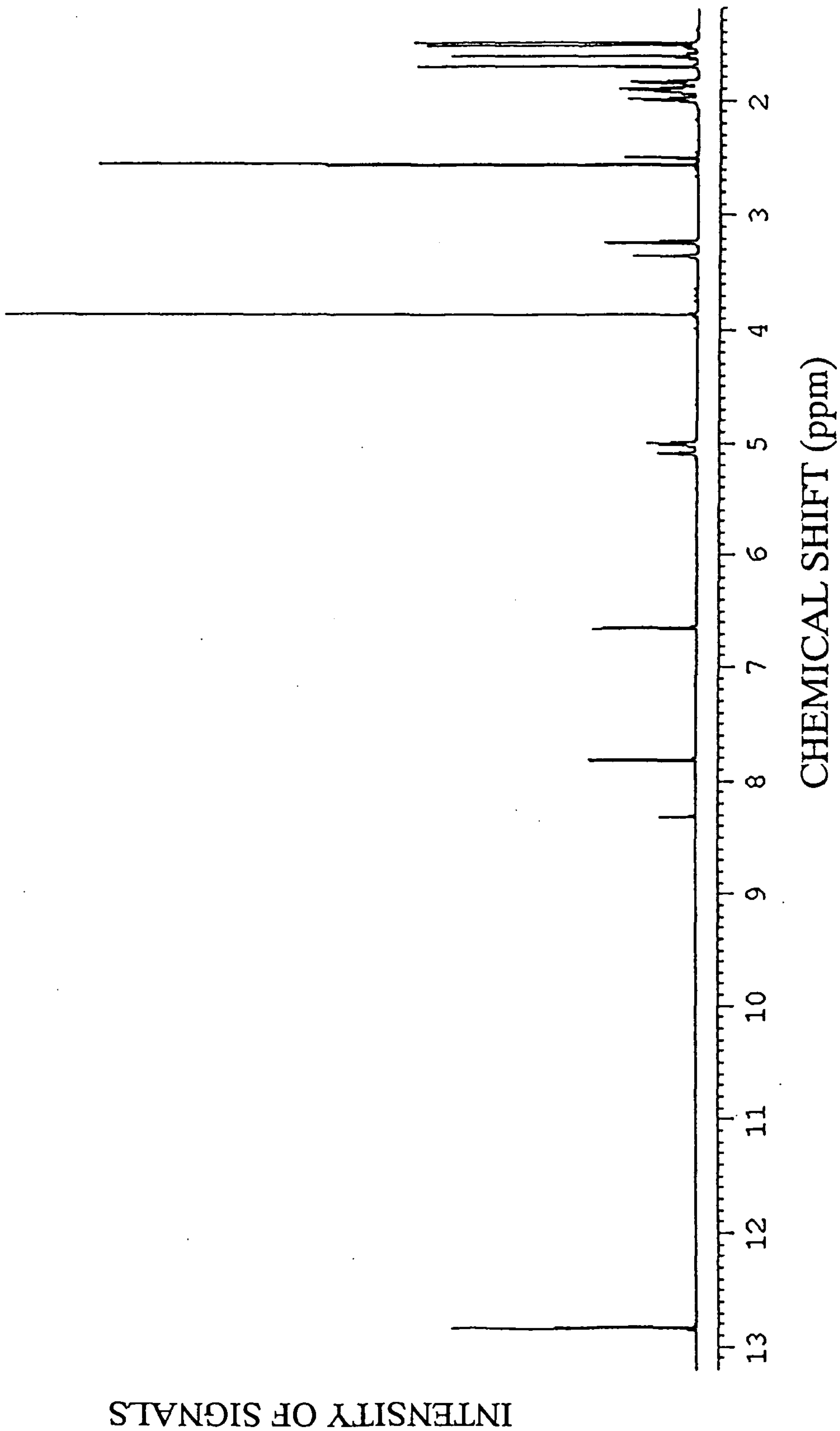
2
3
4
5
6
7
8
9
10
11
12
13
CHEMICAL SHIFT (ppm)
INTENSITY OF SIGNALS

FIG. 39

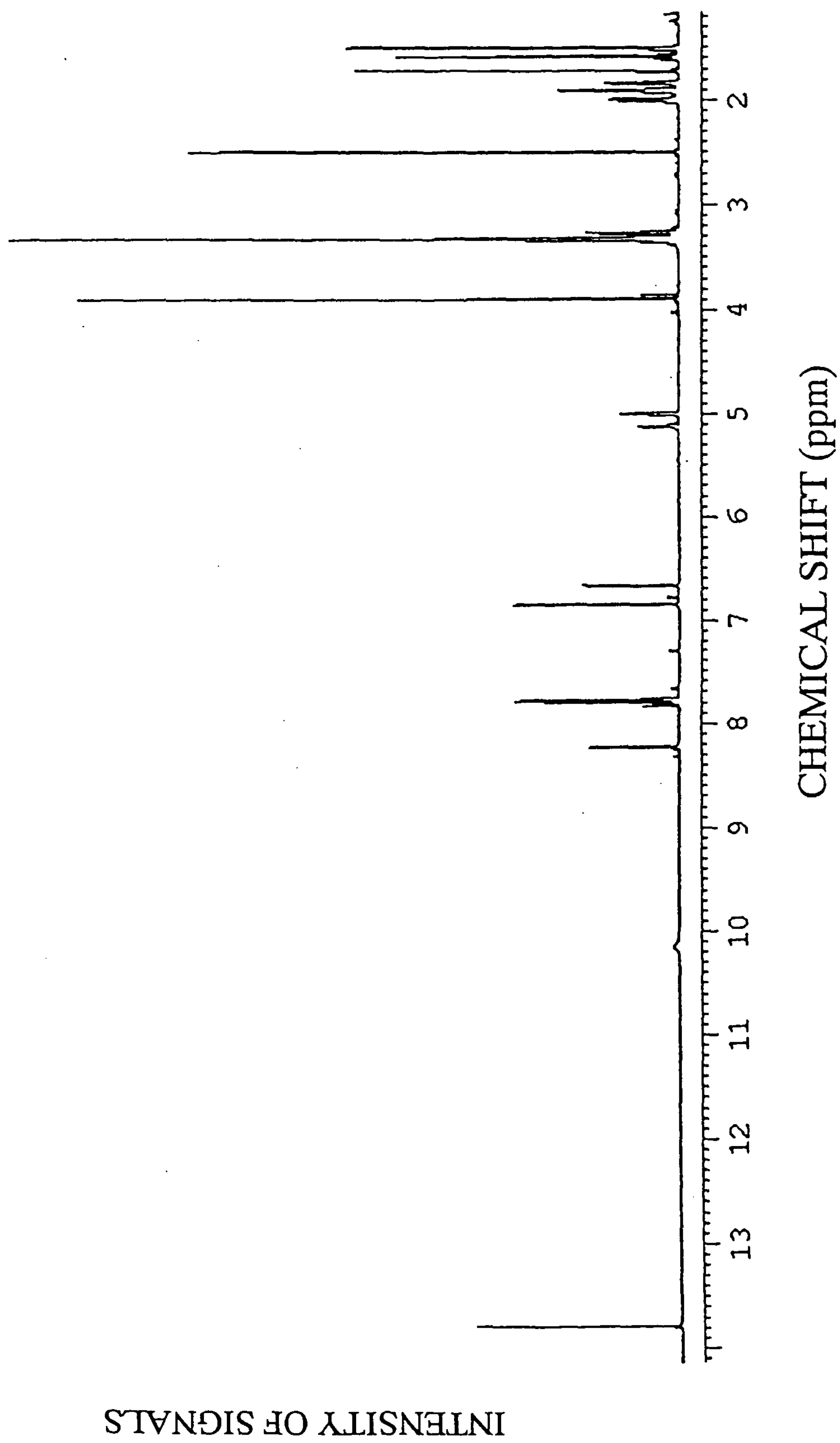
INTENSITY OF SIGNALS
CHEMICAL SHIFT (ppm)
2
3
4
5
6
7
8
9
10
11
12
13

FIG. 40

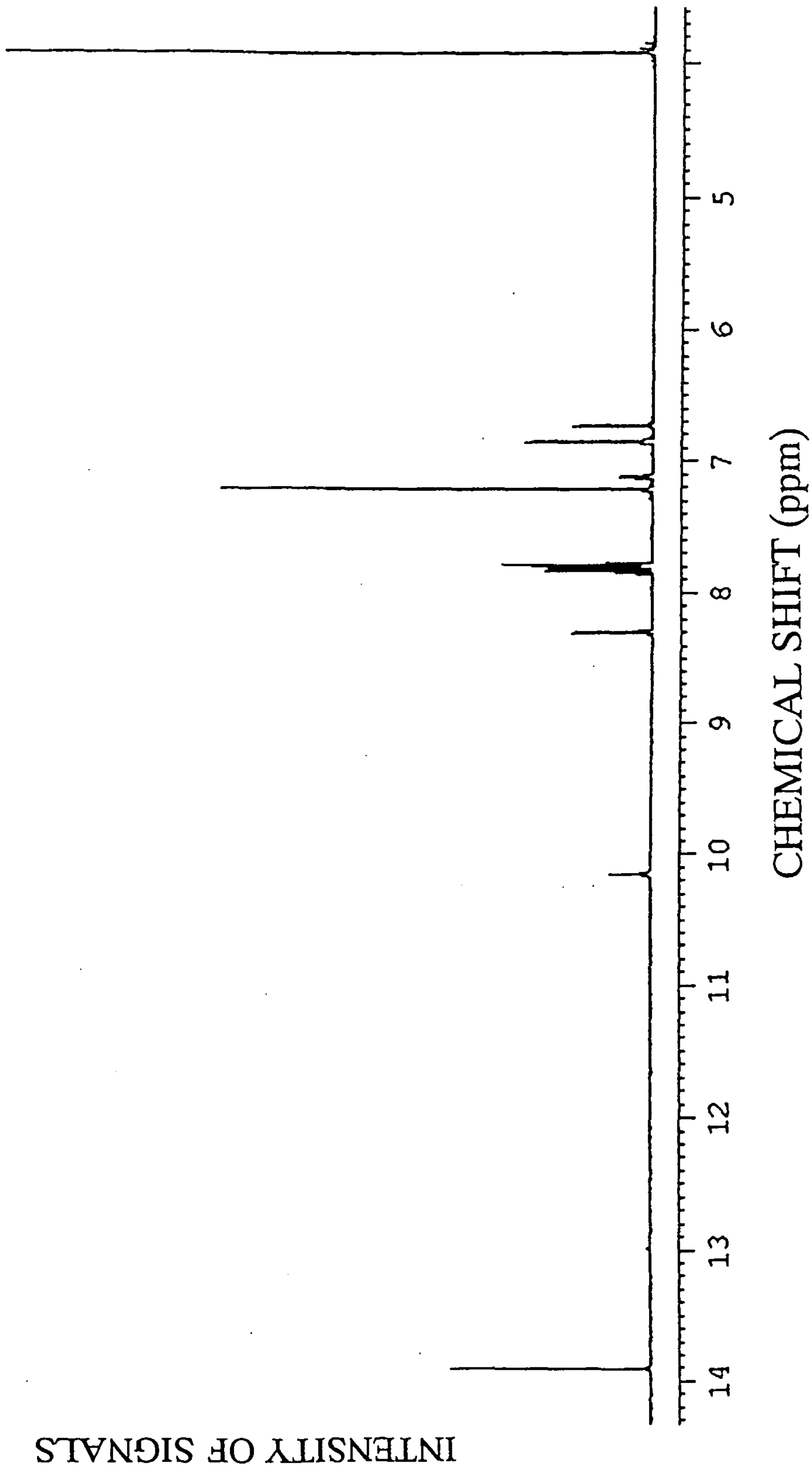


FIG. 41

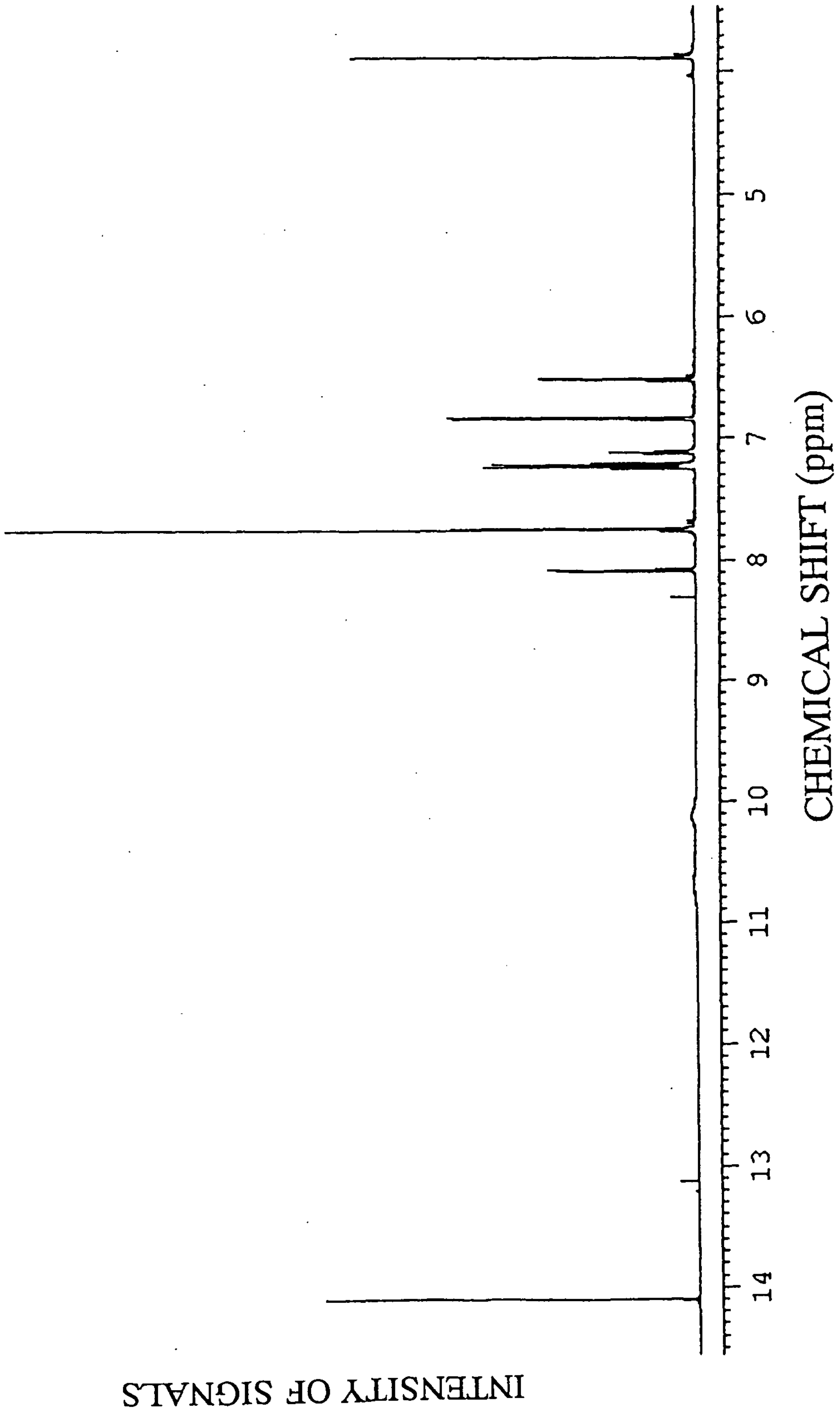


FIG. 42

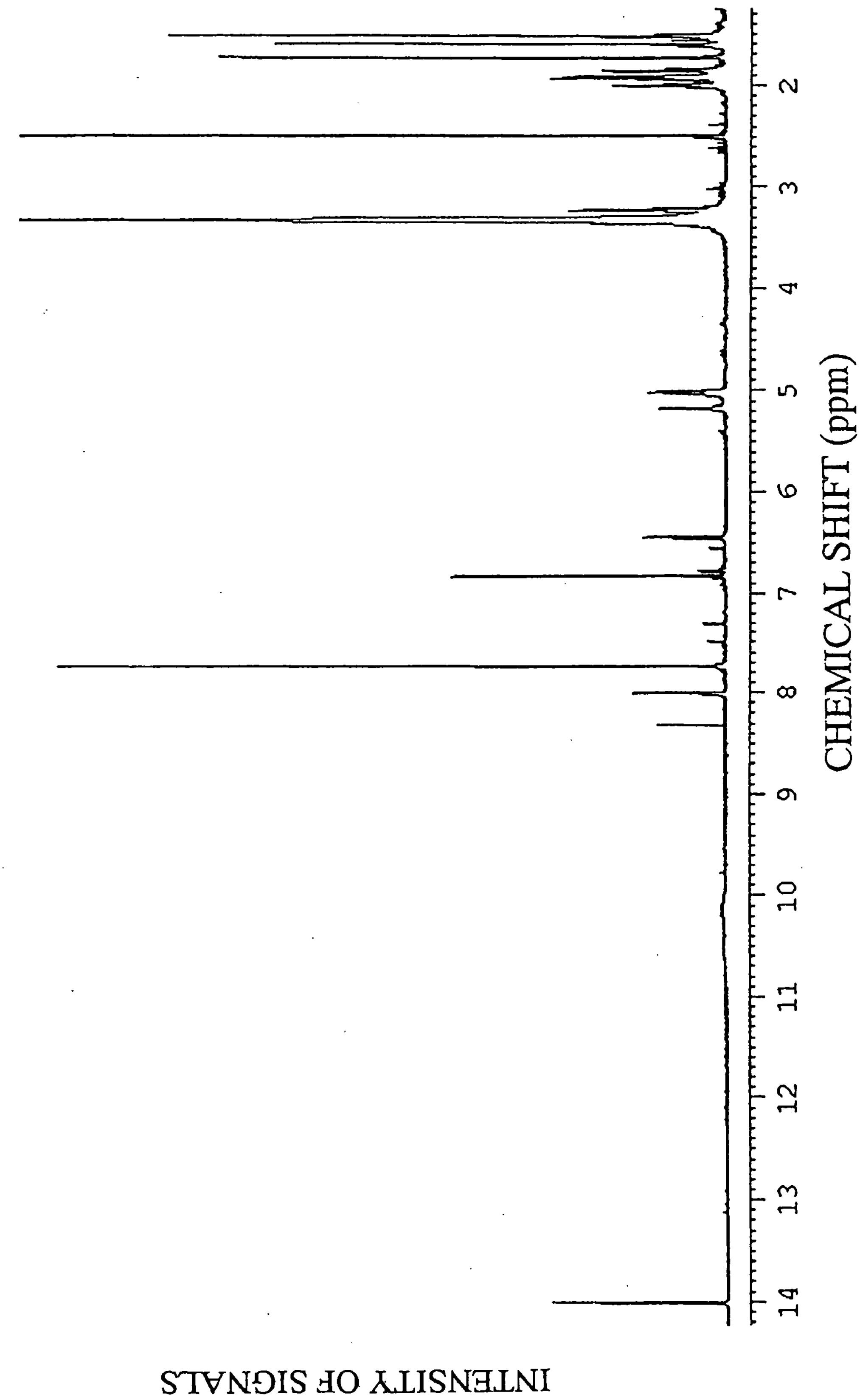
2
3
4
5
6
7
8
9
10
11
12
13
14
CHEMICAL SHIFT (ppm)
INTENSITY OF SIGNALS

FIG. 43

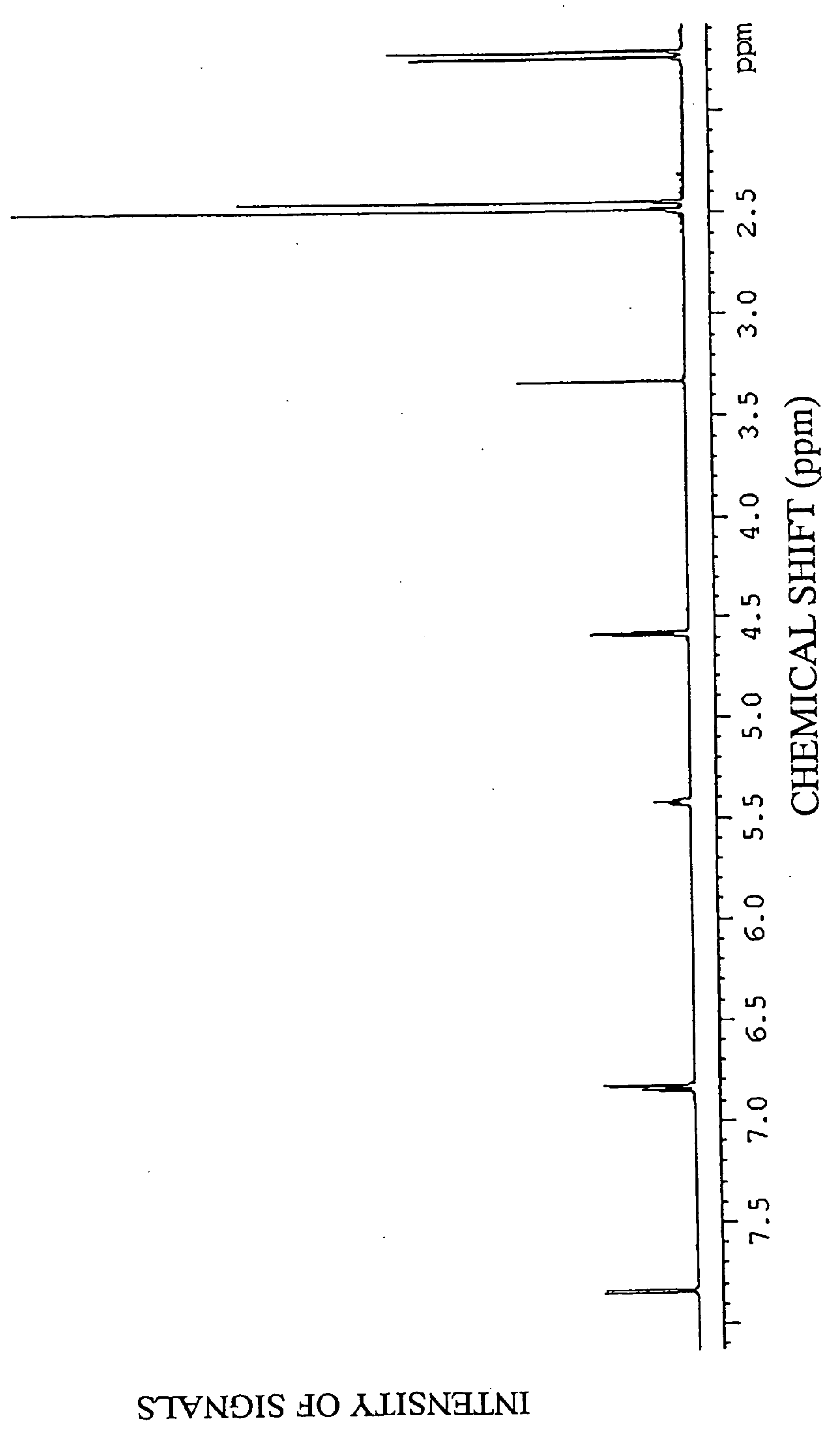
ppm
2.5
3.0
3.5
4.0
4.5
5.0
5.5
6.0
6.5
7.0
7.5
CHEMICAL SHIFT (ppm)
INTENSITY OF SIGNALS

FIG. 44

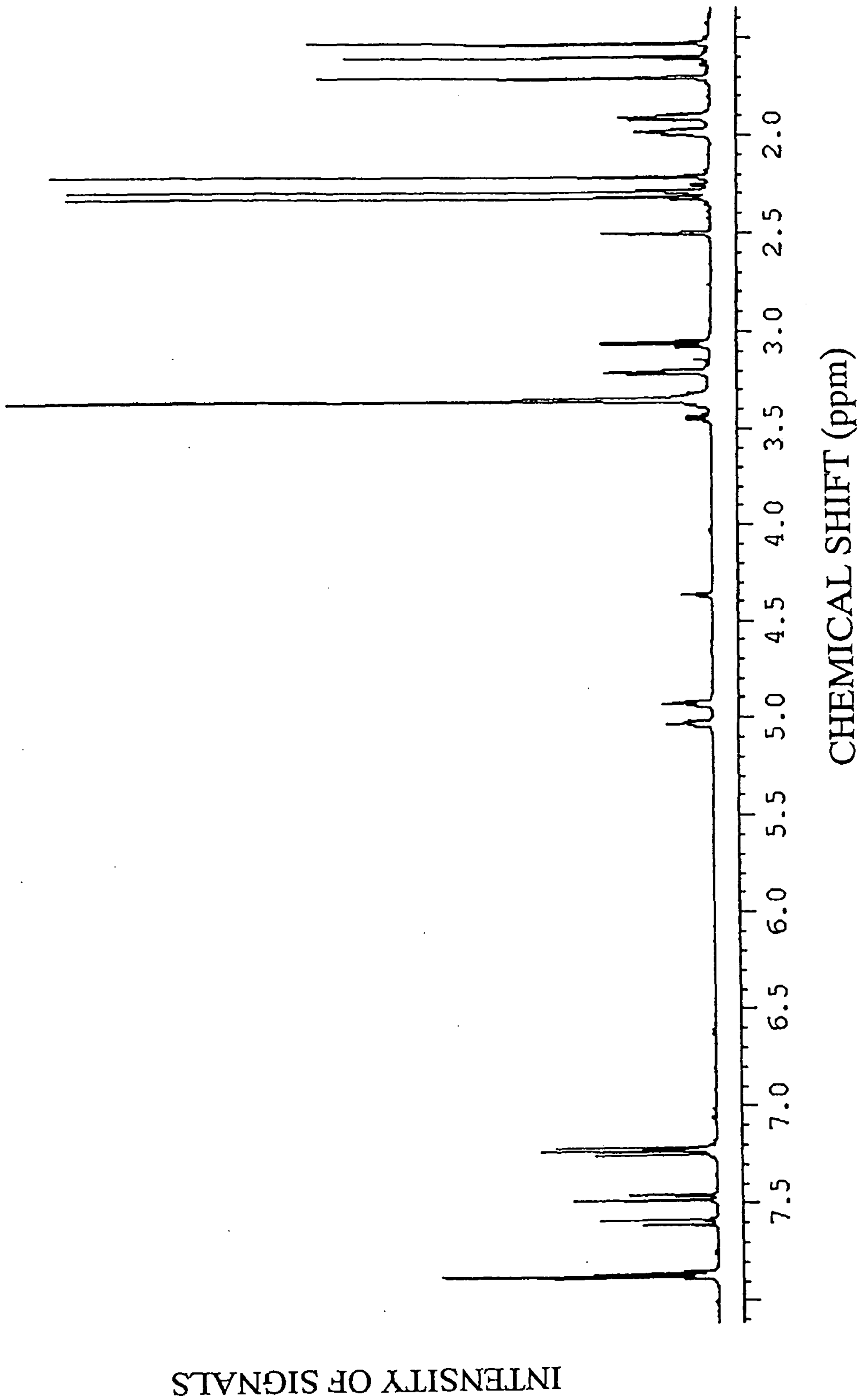
2.0
2.5
3.0
3.5
4.0
4.5
5.0
5.5
6.0
6.5
7.0
7.5
CHEMICAL SHIFT (ppm)
INTENSITY OF SIGNALS

FIG. 45

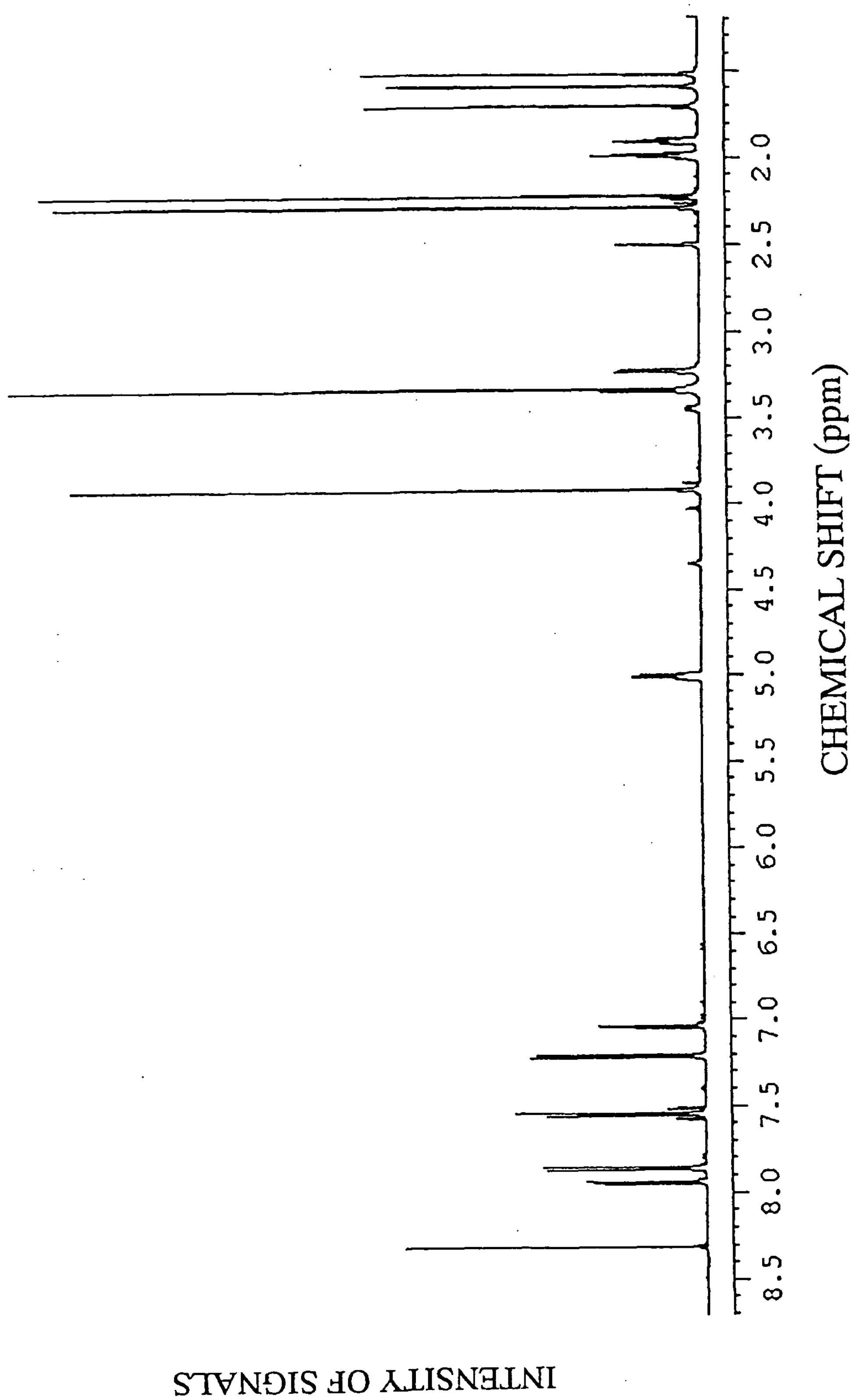
2.0
2.5
3.0
3.5
4.0
4.5
5.0
5.5
6.0
6.5
7.0
7.5
8.0
8.5
CHEMICAL SHIFT (ppm)
INTENSITY OF SIGNALS

FIG. 46

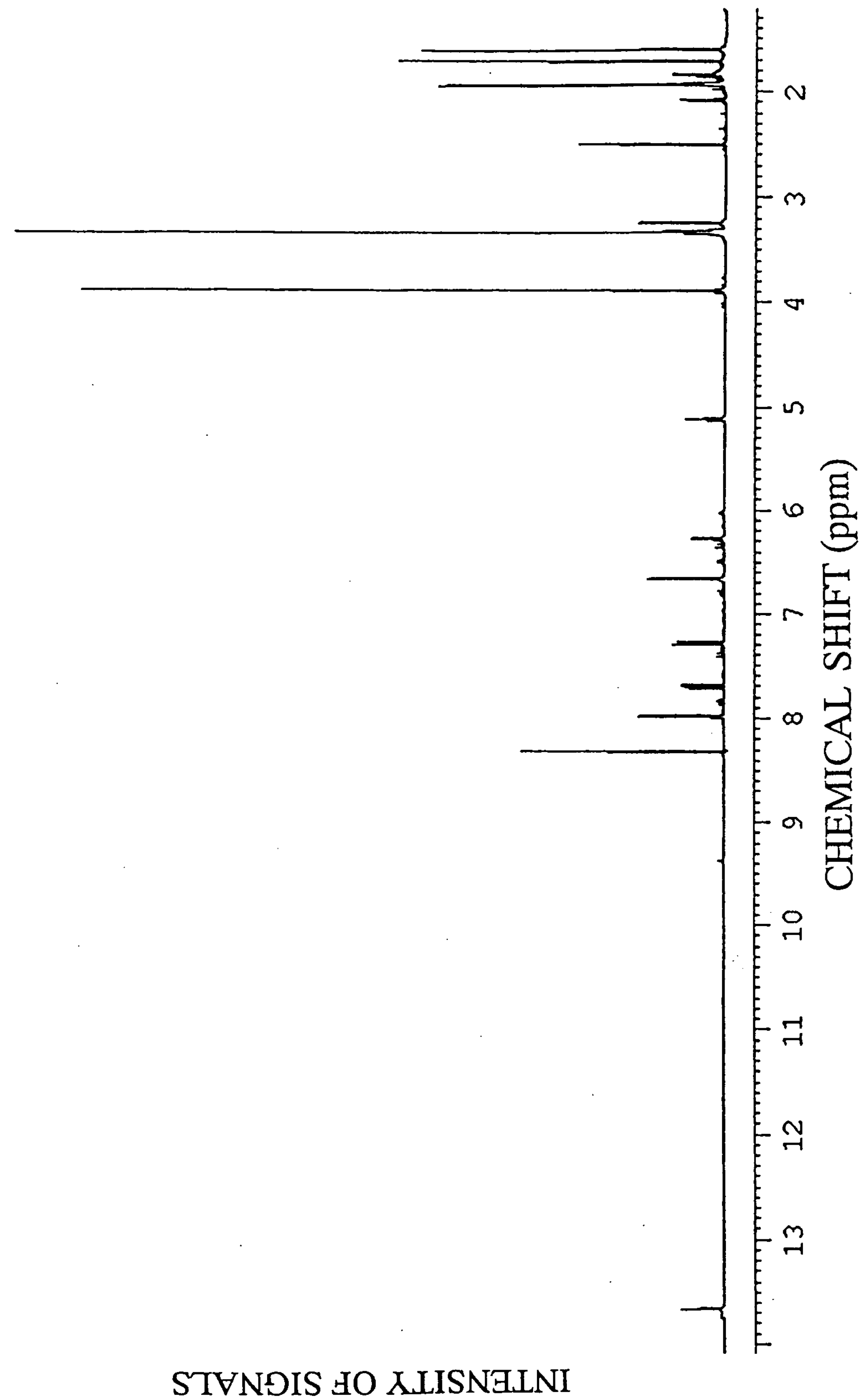
INTENSITY OF SIGNALS
CHEMICAL SHIFT (ppm)
2
3
4
5
6
7
8
9
10
11
12
13

FIG. 47

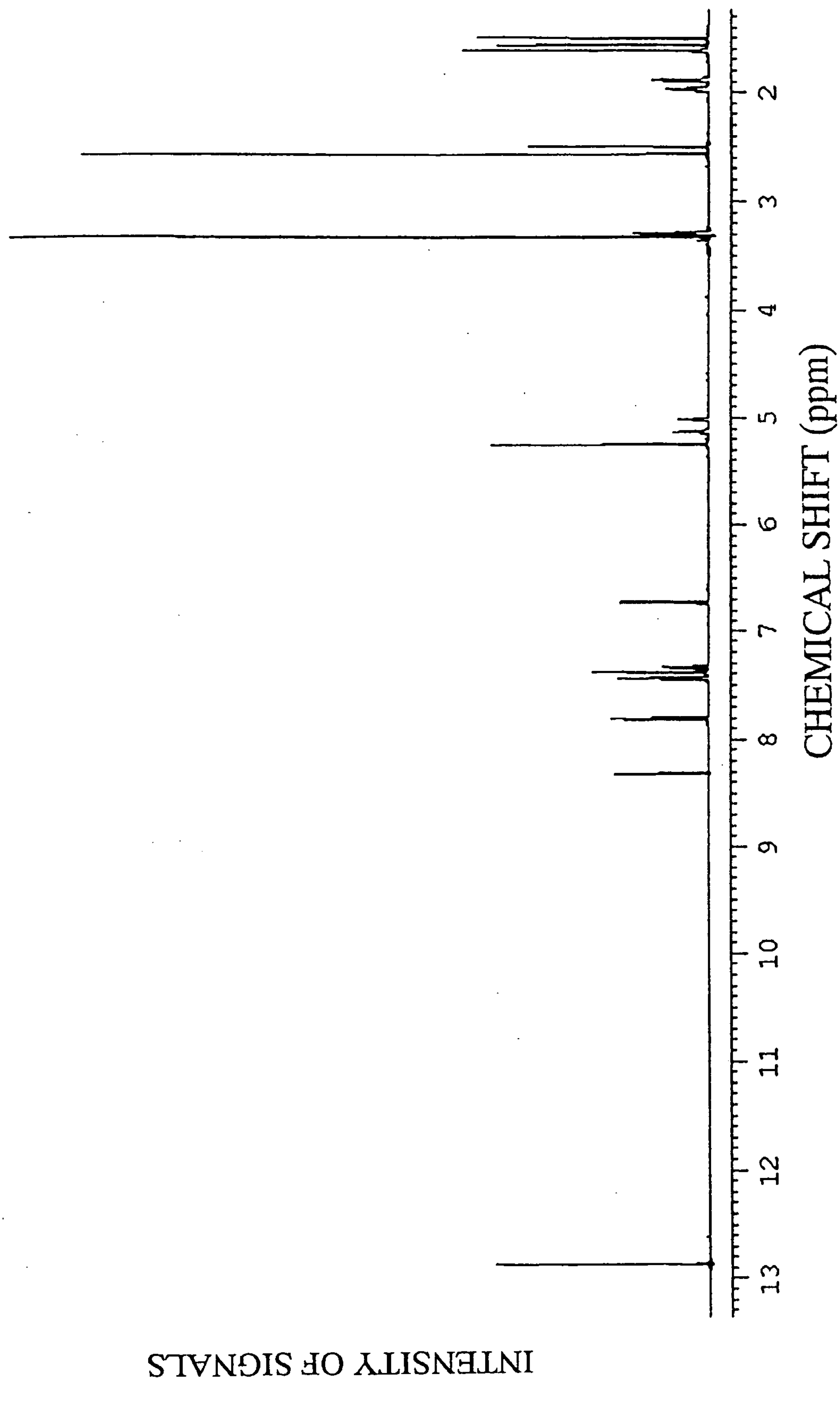
CHEMICAL SHIFT (ppm)
INTENSITY OF SIGNALS
2
3
4
5
6
7
8
9
10
11
12
13

FIG. 48

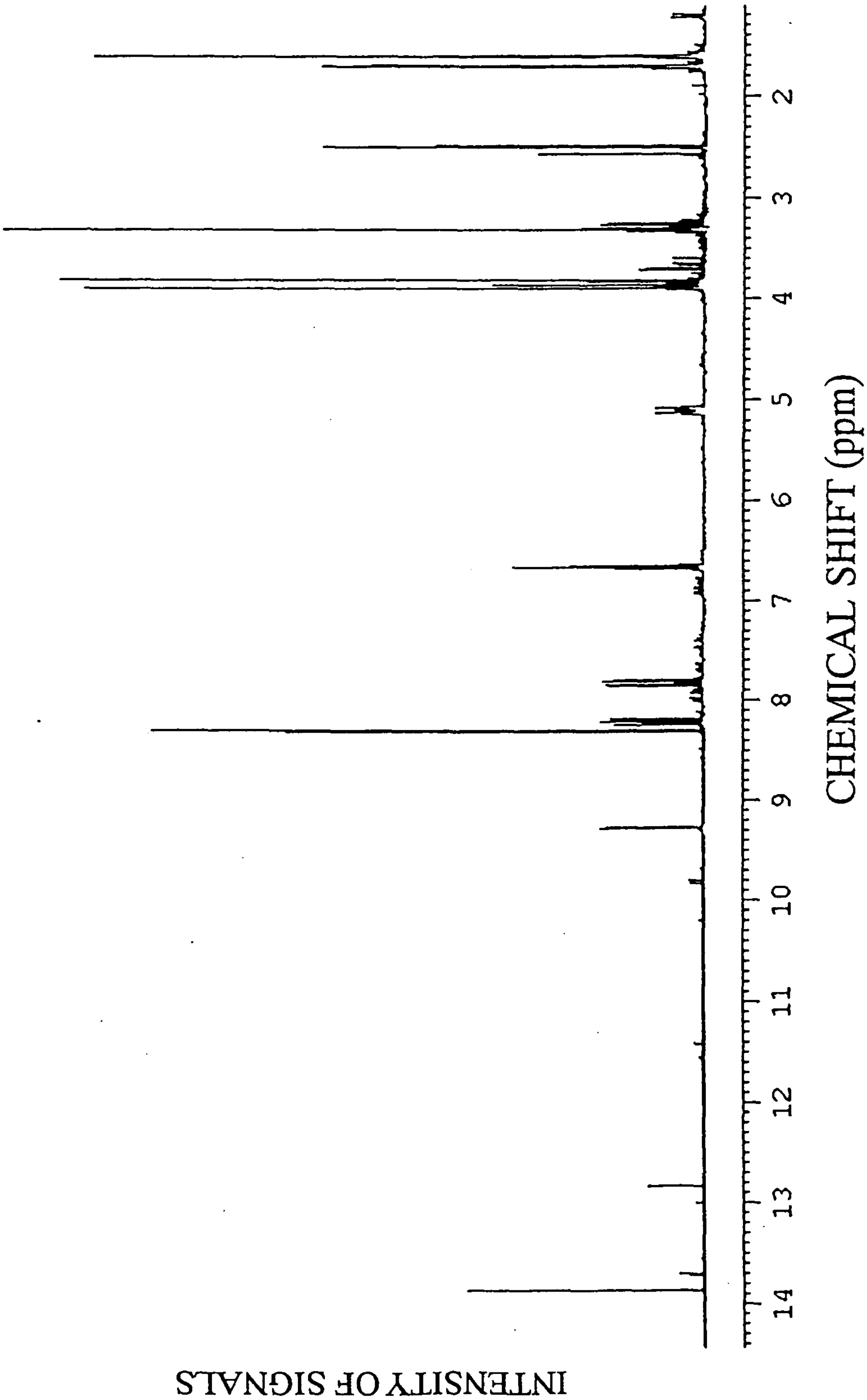
INTENSITY OF SIGNALS
CHEMICAL SHIFT (ppm)
2
3
4
5
6
7
8
9
10
11
12
13
14

FIG. 49

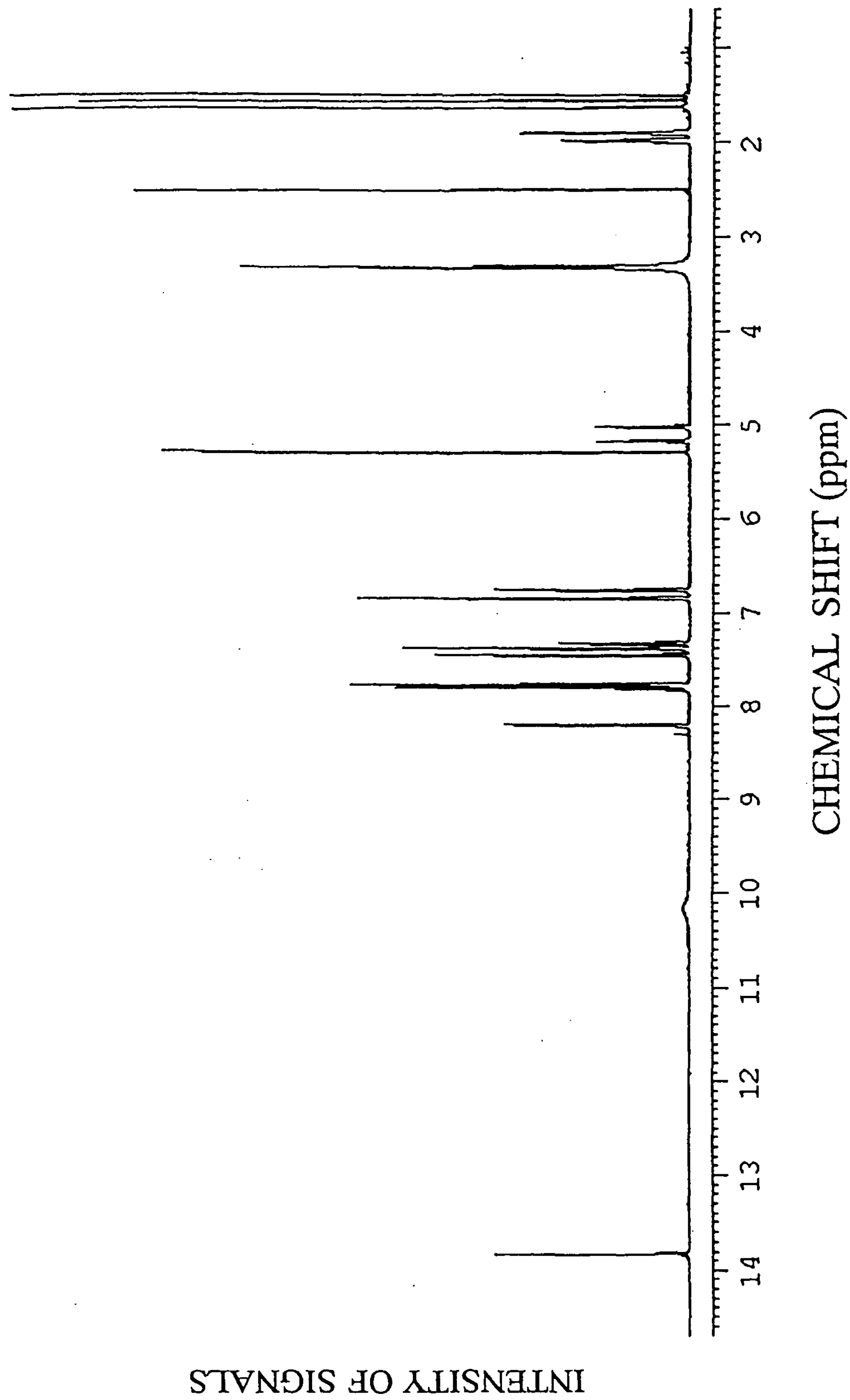
INTENSITY OF SIGNALS
CHEMICAL SHIFT (ppm)
2 3 4 5 6 7 8 9 10 11 12 13 14

FIG. 50

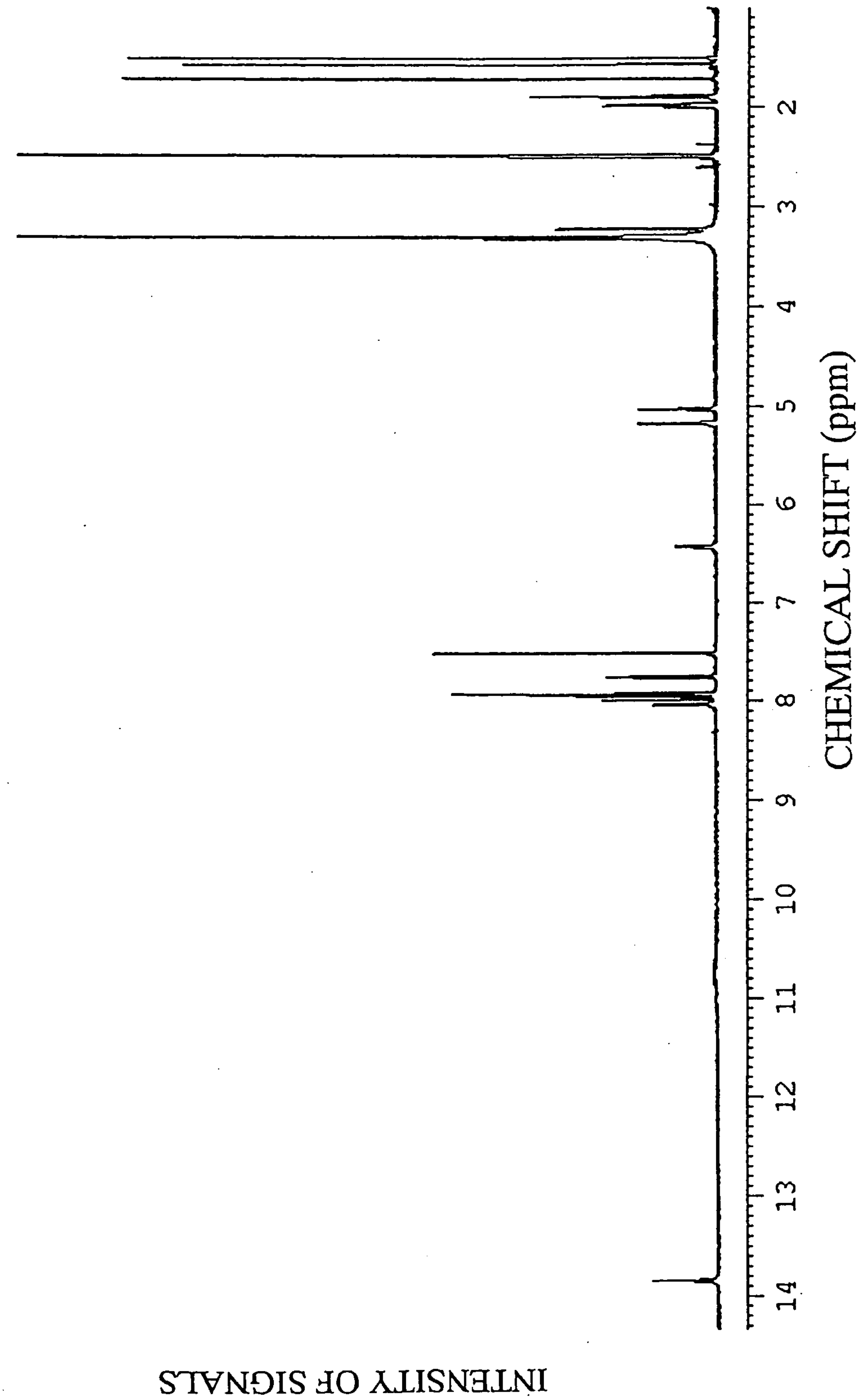
CHEMICAL SHIFT (ppm)
INTENSITY OF SIGNALS
2 3 4 5 6 7 8 9 10 11 12 13 14

FIG. 51

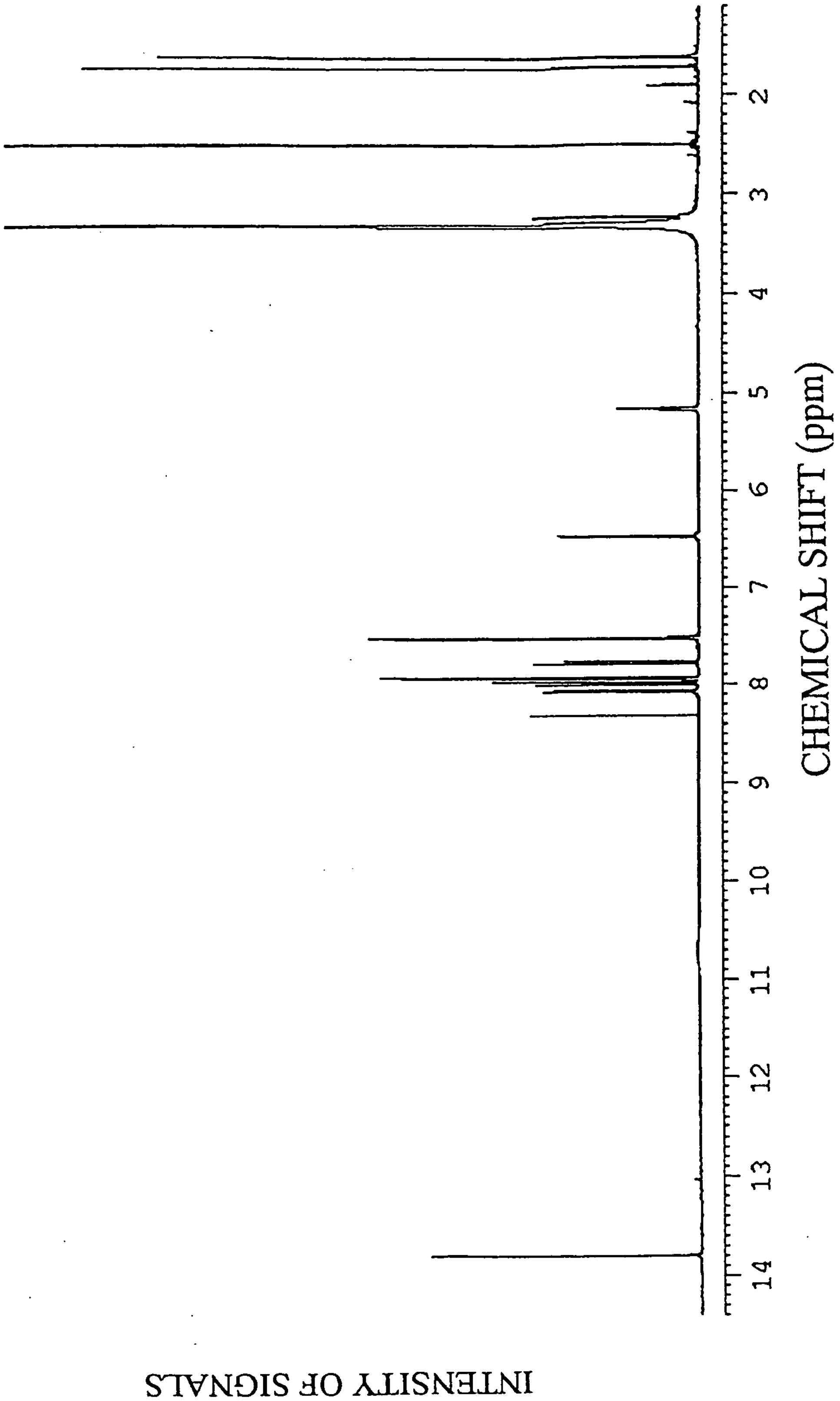
INTENSITY OF SIGNALS
CHEMICAL SHIFT (ppm)
2
3
4
5
6
7
8
9
10
11
12
13
14

FIG. 52

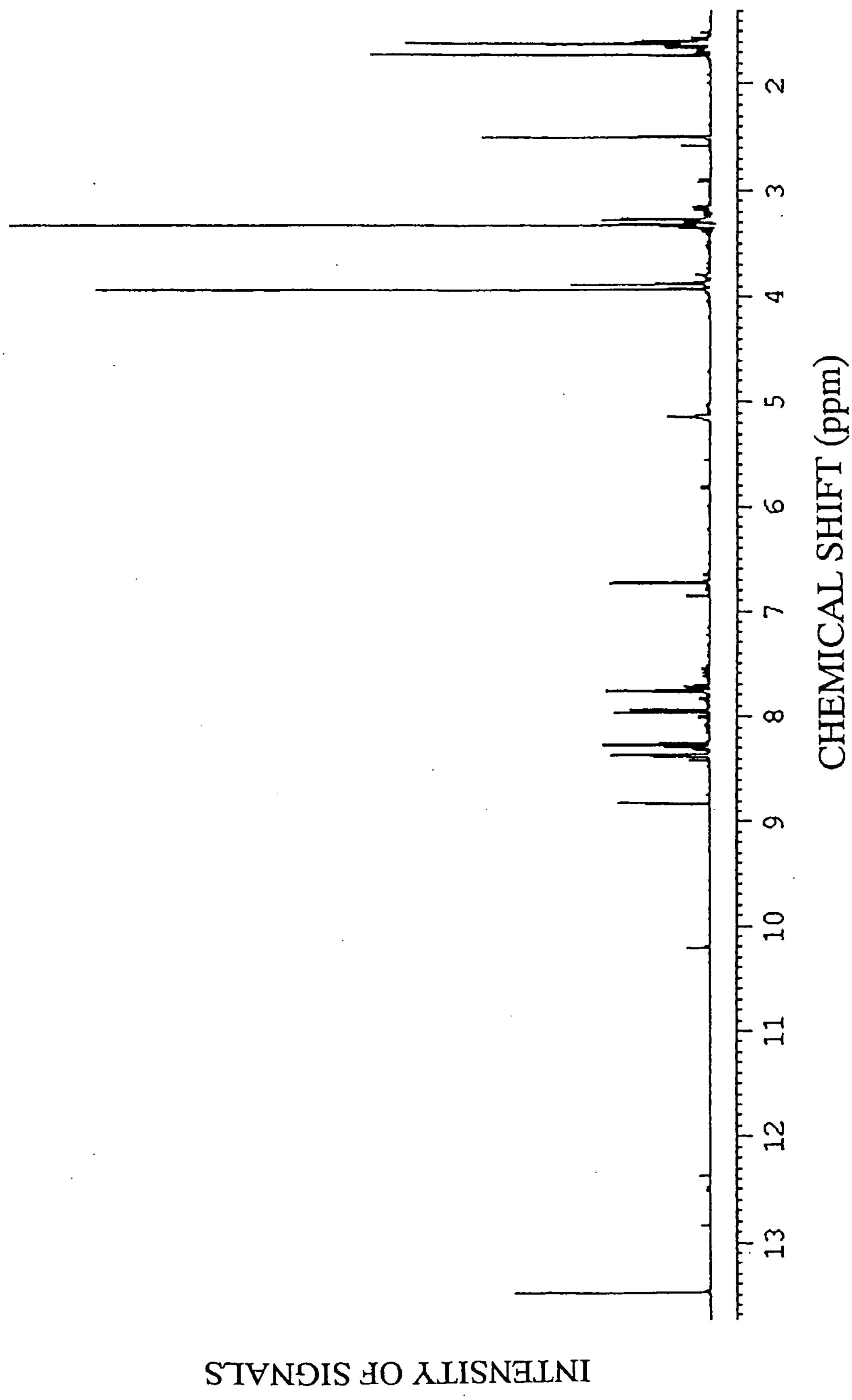
2
3
4
5
6
7
8
9
10
11
12
13
CHEMICAL SHIFT (ppm)
INTENSITY OF SIGNALS

FIG. 53

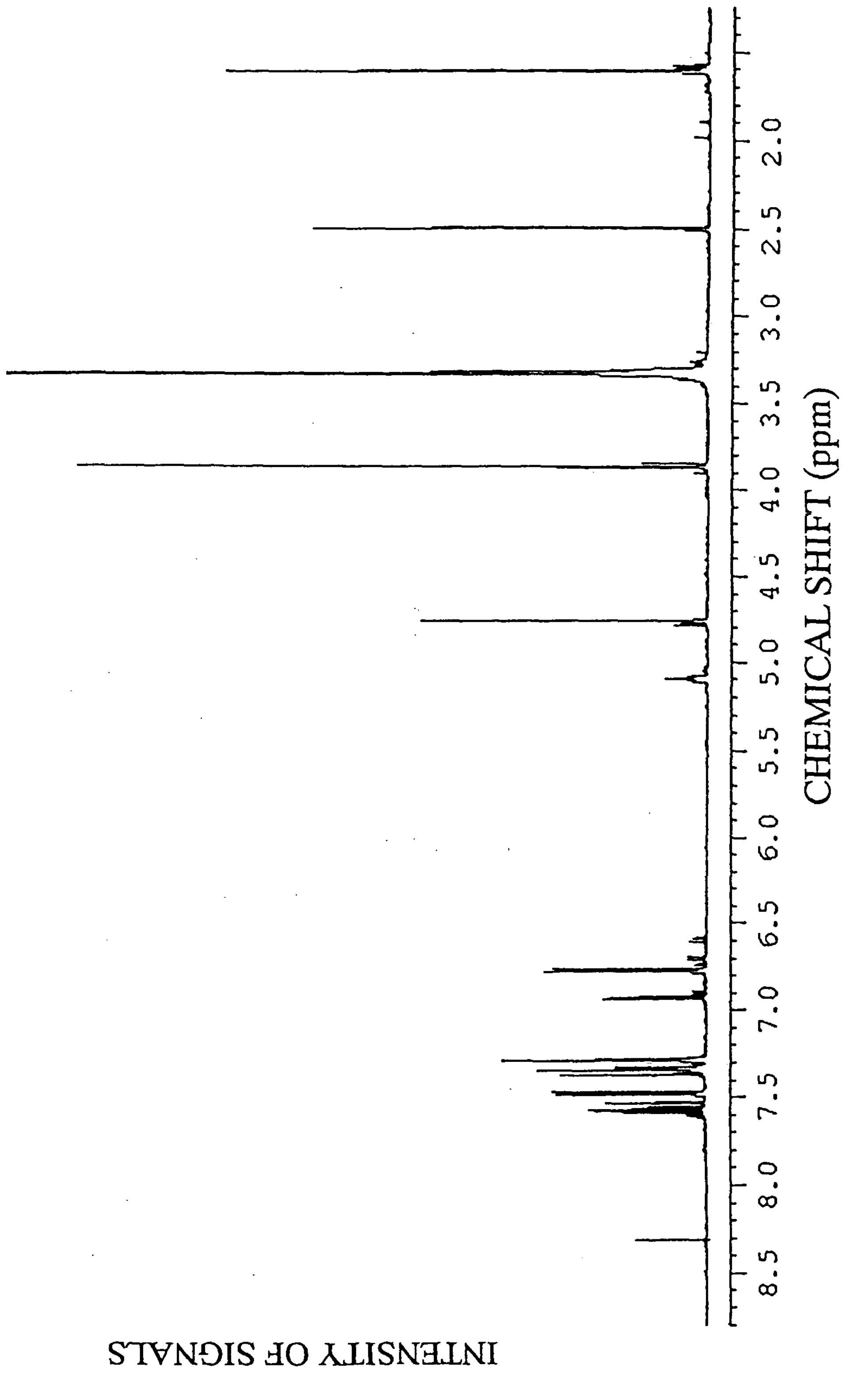
8.5
8.0
7.5
7.0
6.5
6.0
5.5
5.0
4.5
4.0
3.5
3.0
2.5
2.0
CHEMICAL SHIFT (ppm)
INTENSITY OF SIGNALS

FIG. 54

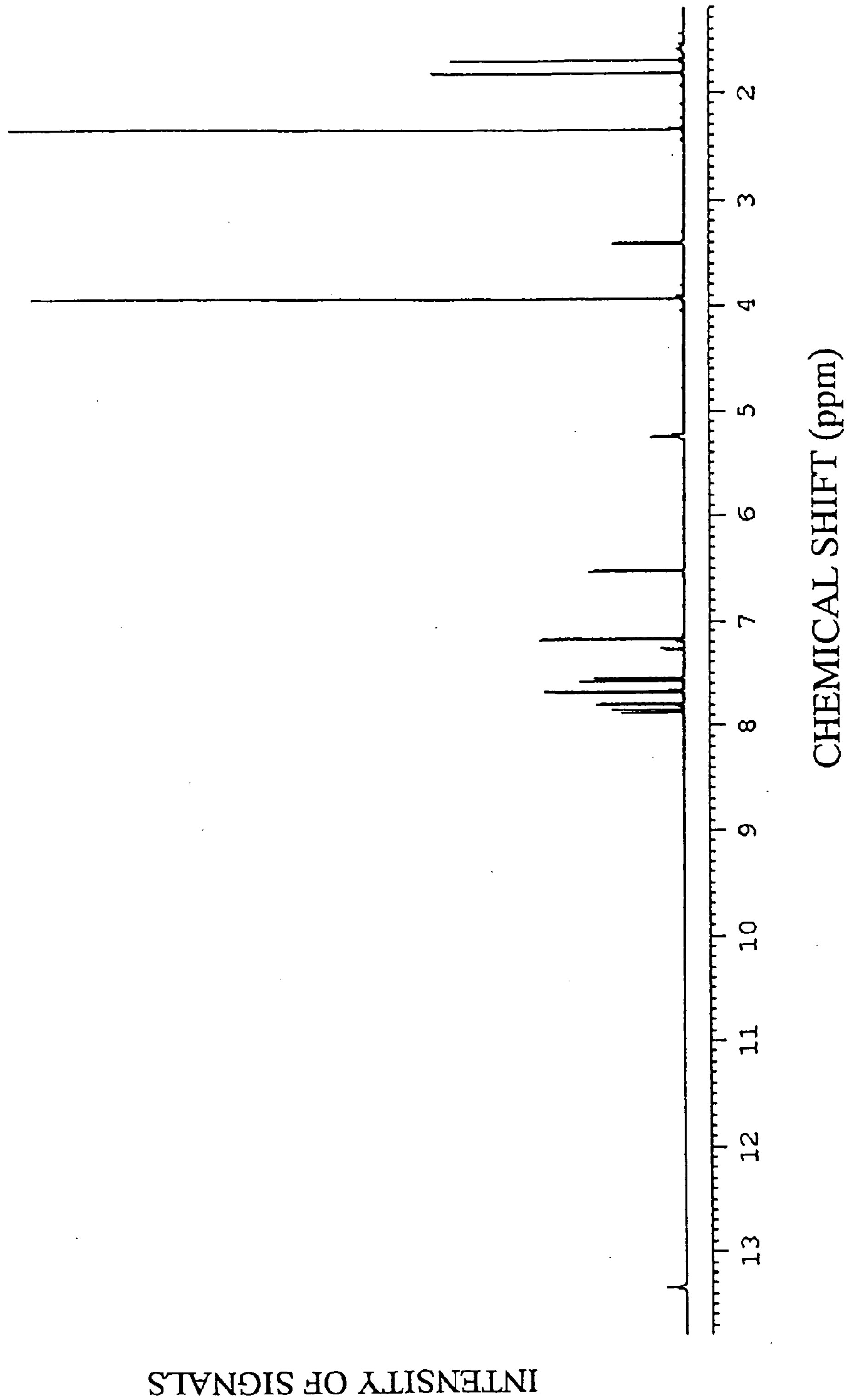
CHEMICAL SHIFT (ppm)
INTENSITY OF SIGNALS
2
3
4
5
6
7
8
9
10
11
12
13

FIG. 55

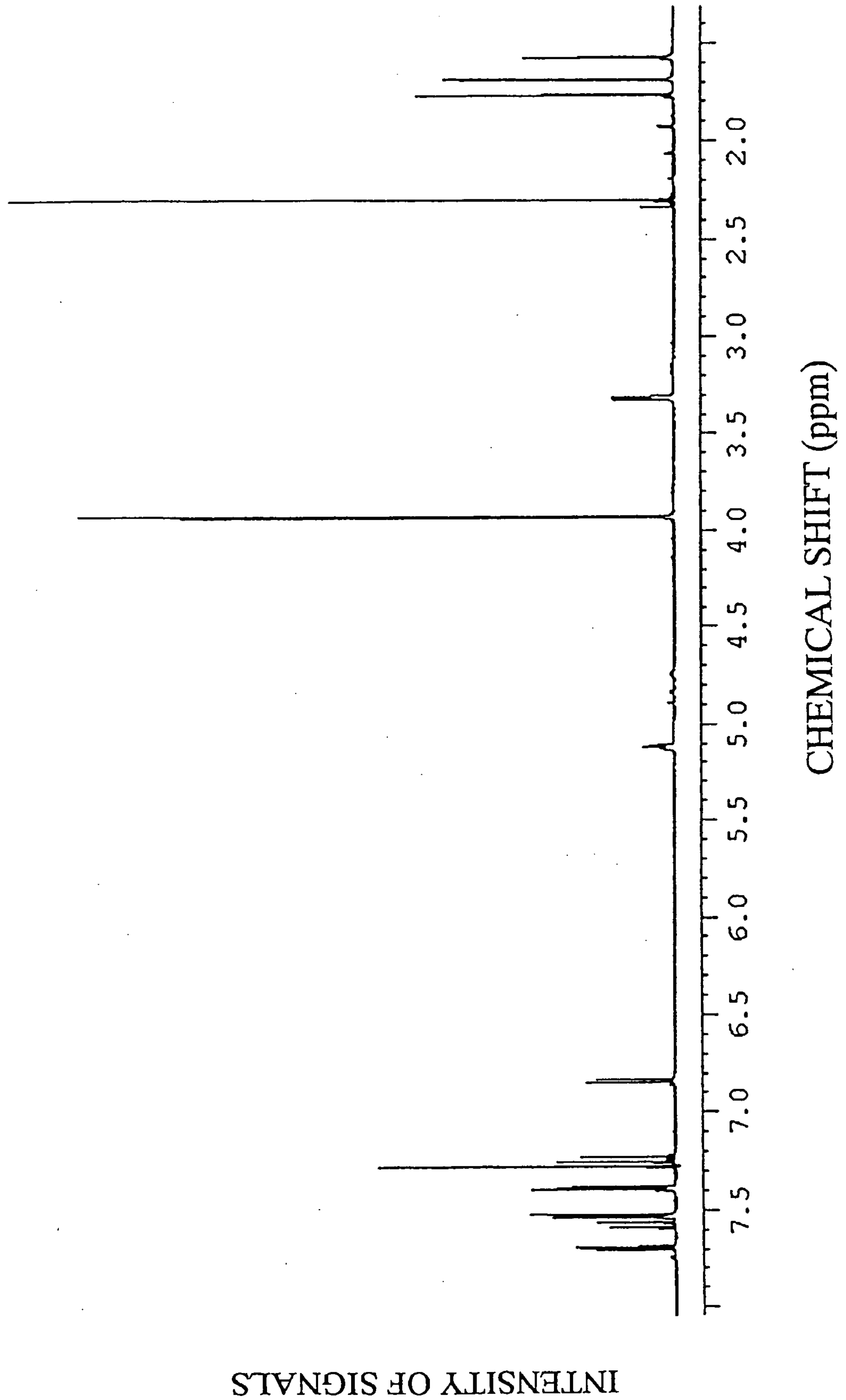
2.0
2.5
3.0
3.5
4.0
4.5
5.0
5.5
6.0
6.5
7.0
7.5
CHEMICAL SHIFT (ppm)
INTENSITY OF SIGNALS

FIG. 56

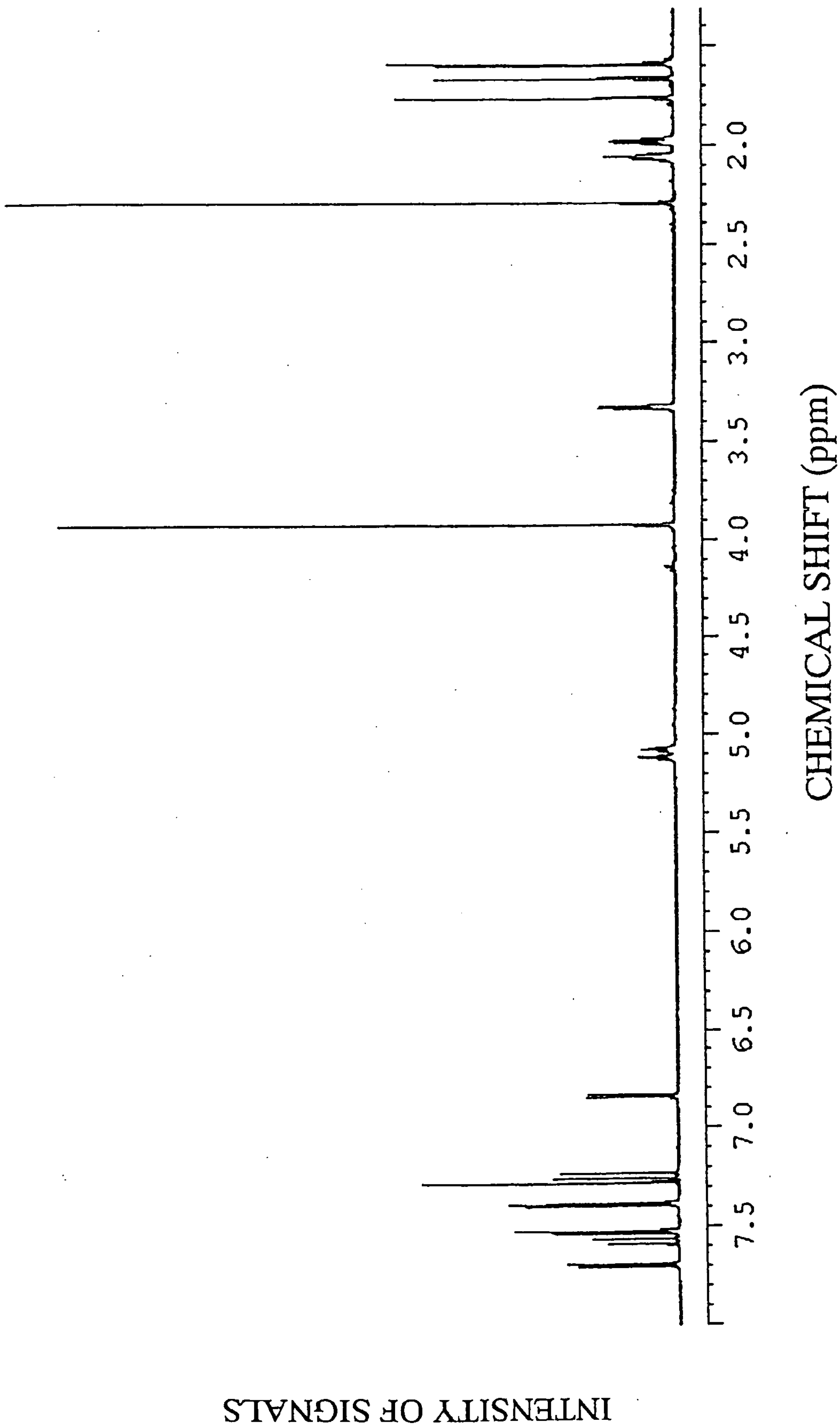
2.0
2.5
3.0
3.5
4.0
4.5
5.0
5.5
6.0
6.5
7.0
7.5
CHEMICAL SHIFT (ppm)
INTENSITY OF SIGNALS

FIG. 57

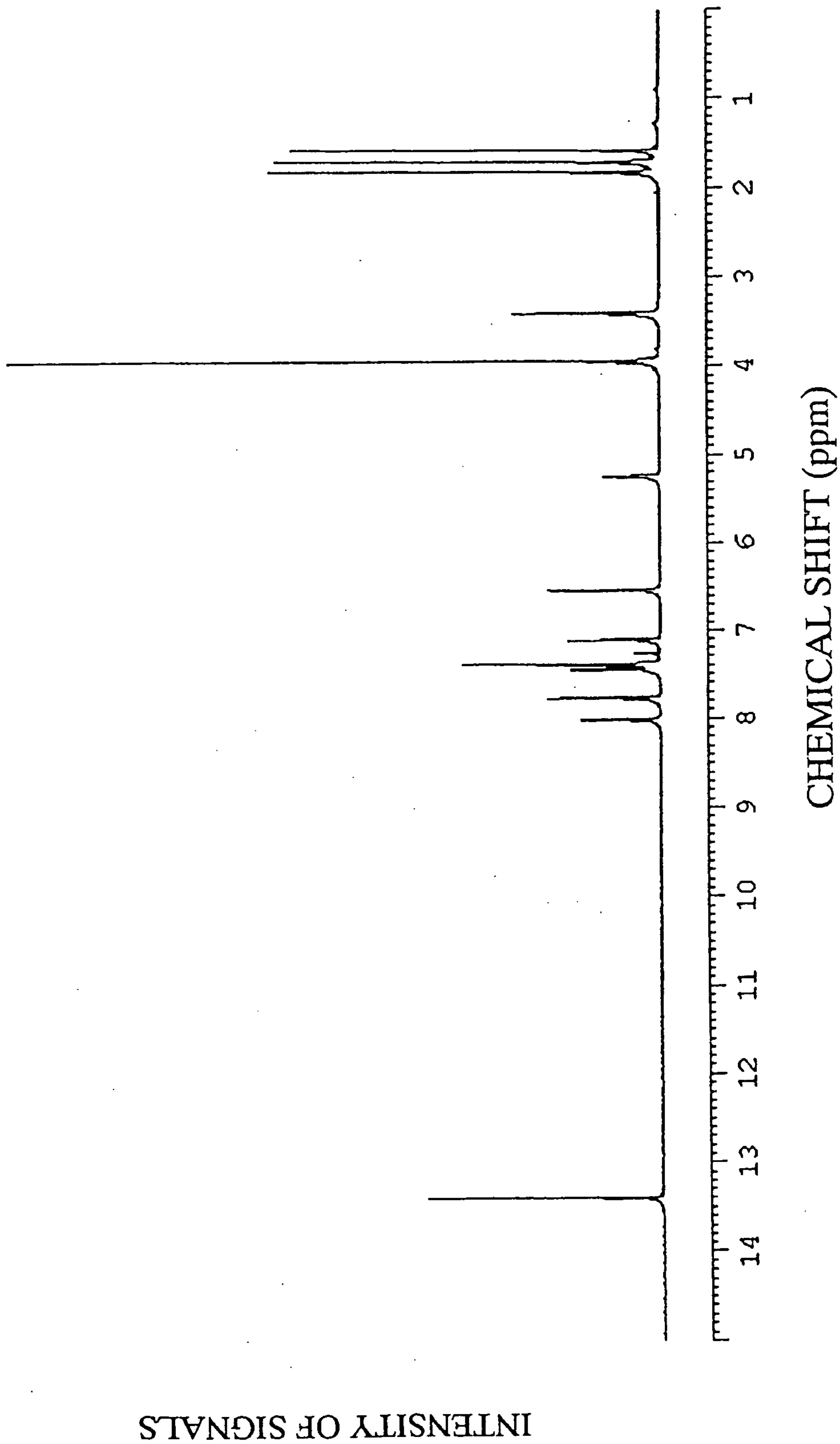
1
2
3
4
5
6
7
8
9
10
11
12
13
14
CHEMICAL SHIFT (ppm)
INTENSITY OF SIGNALS

FIG. 58

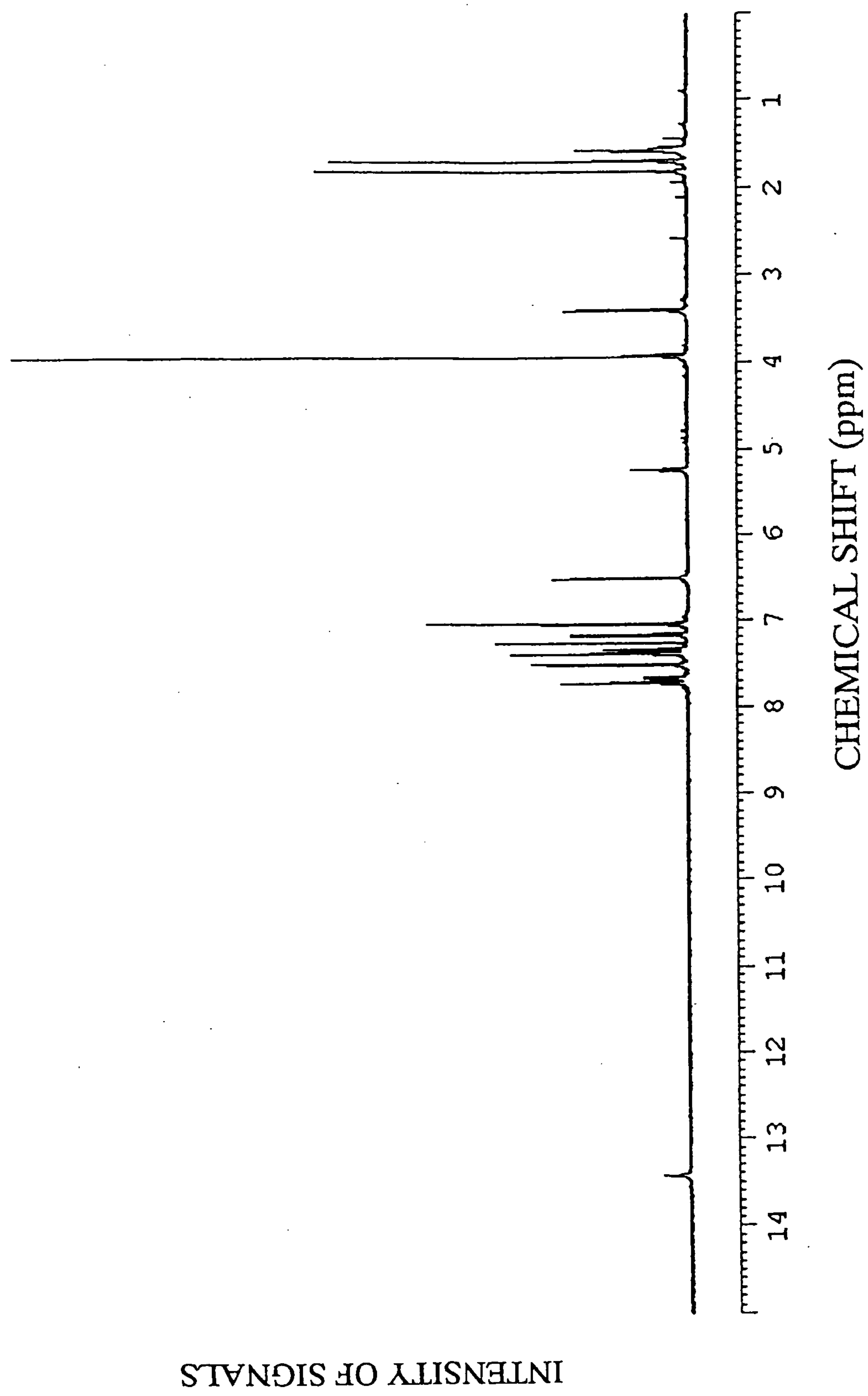
1
2
3
4
5
6
7
8
9
10
11
12
13
14
CHEMICAL SHIFT (ppm)
INTENSITY OF SIGNALS

FIG. 59

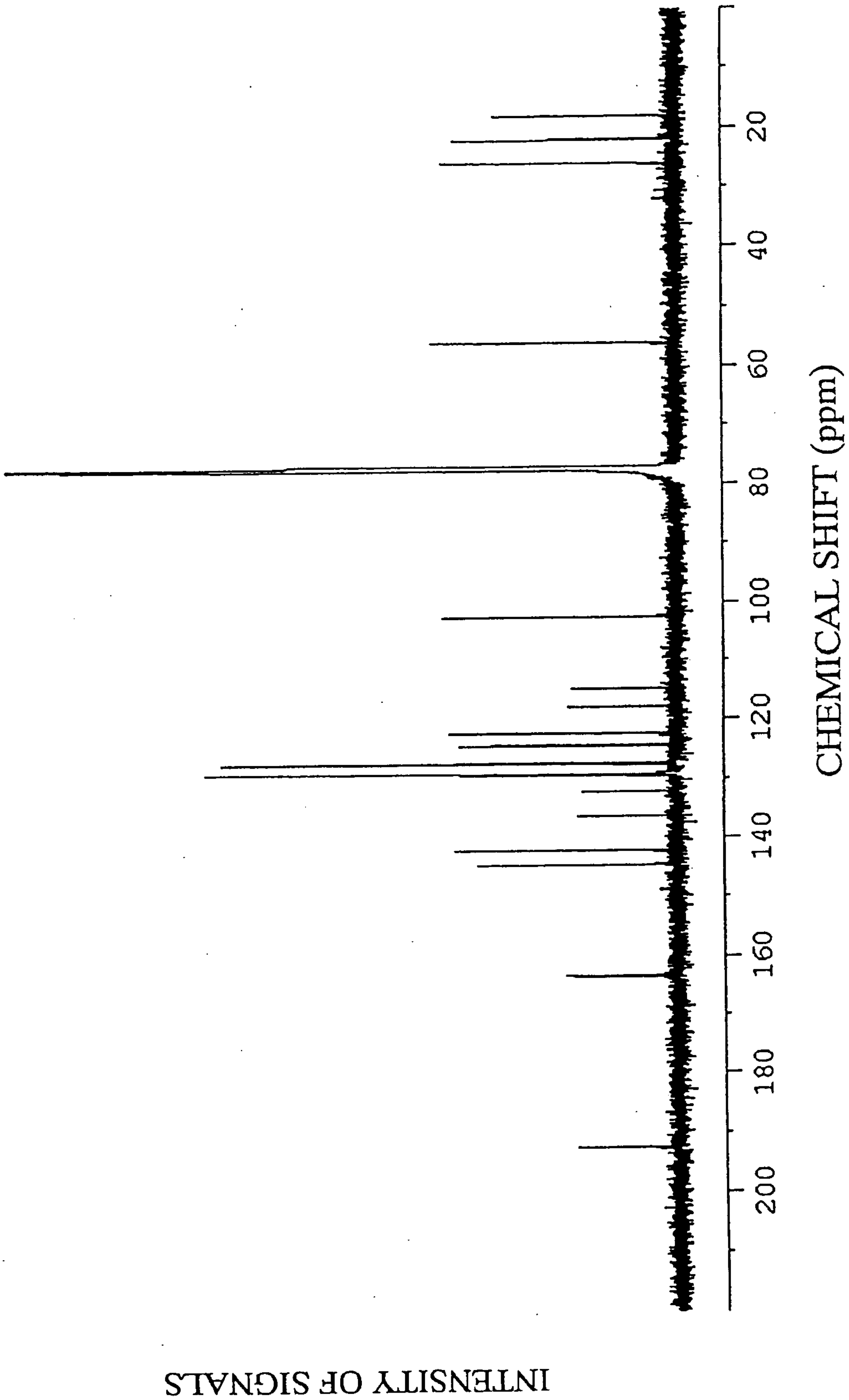
INTENSITY OF SIGNALS
CHEMICAL SHIFT (ppm)
20
40
60
80
100
120
140
160
180
200

FIG. 60

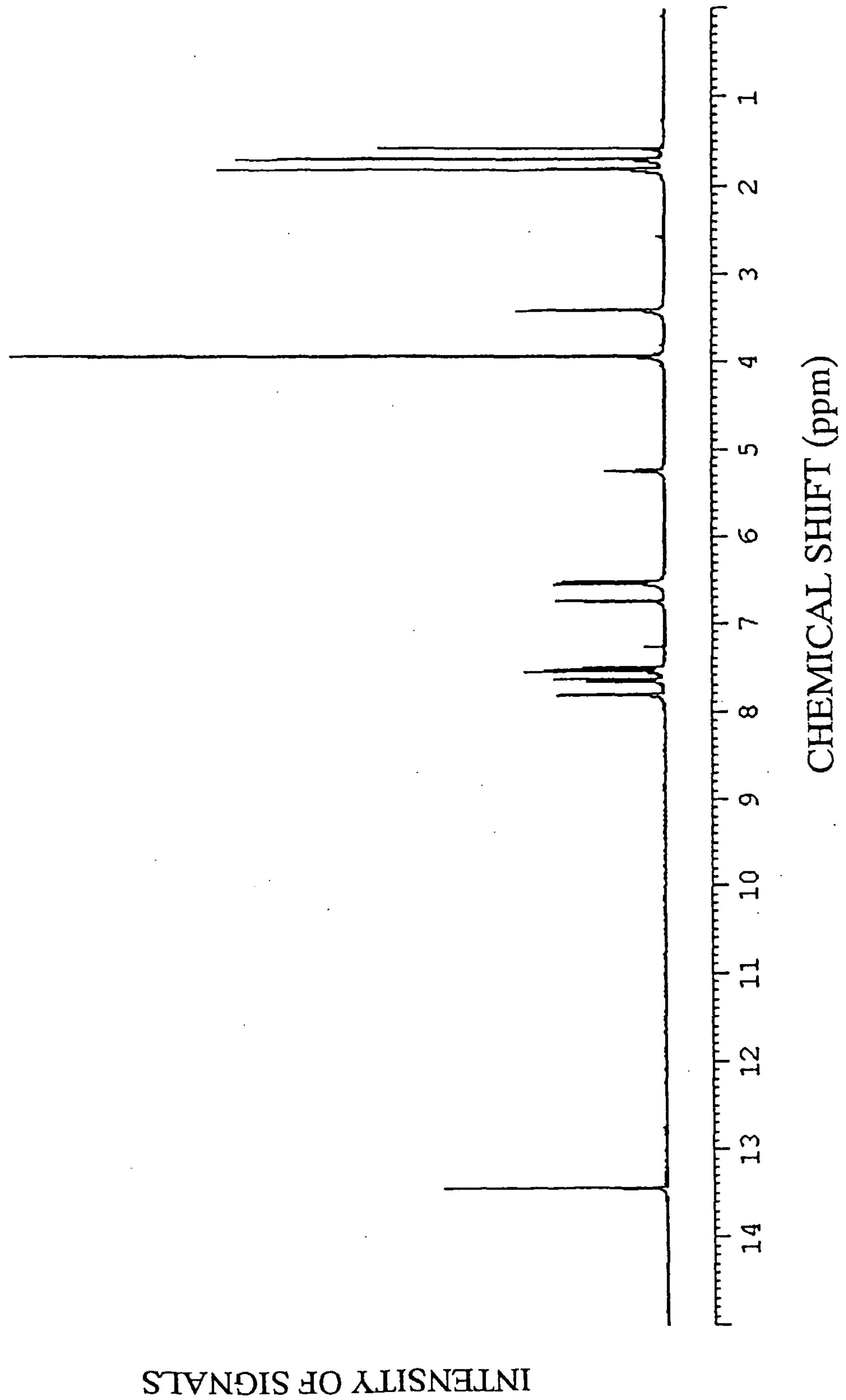
CHEMICAL SHIFT (ppm)
INTENSITY OF SIGNALS
1 2 3 4 5 6 7 8 9 10 11 12 13 14

FIG. 61

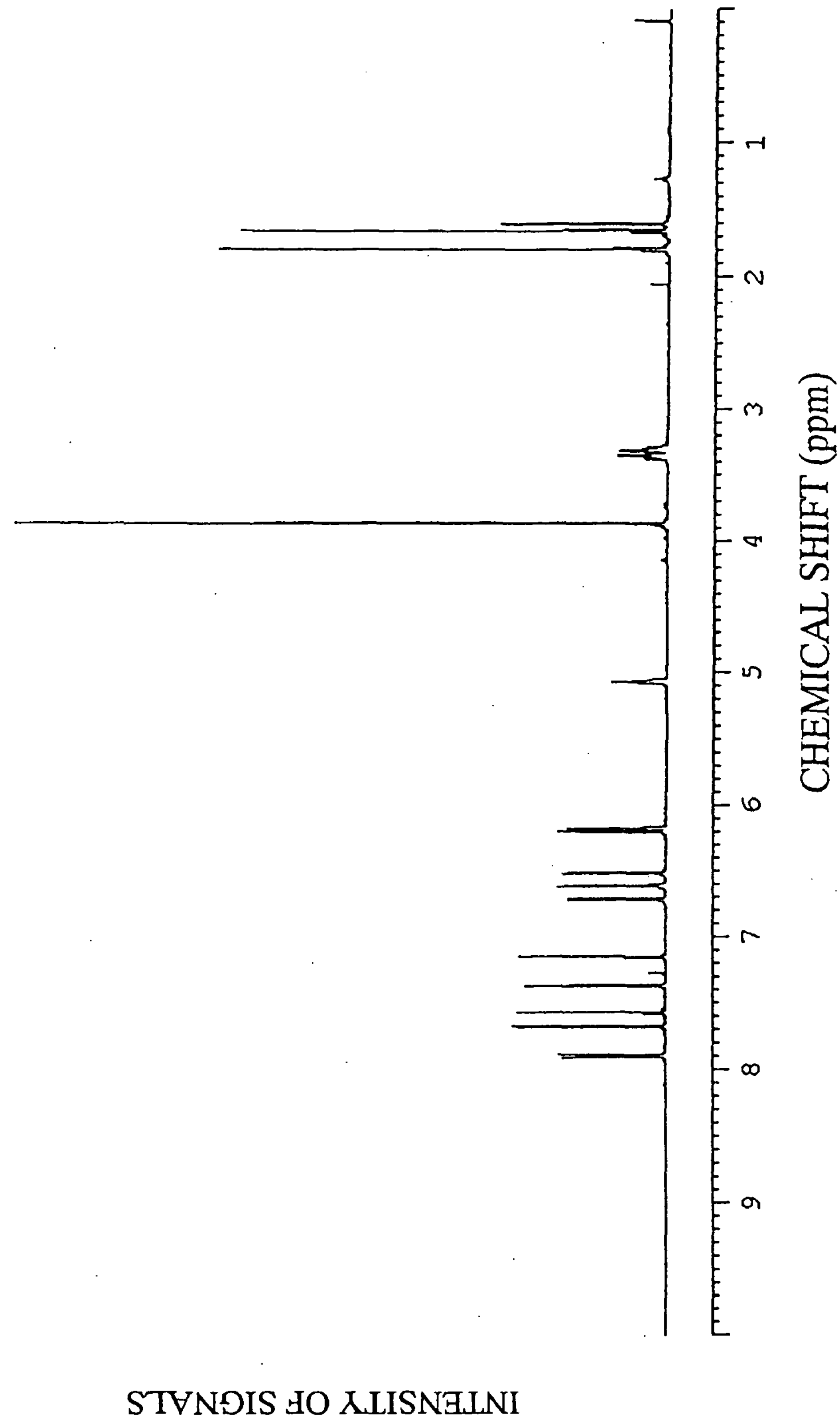


FIG. 62

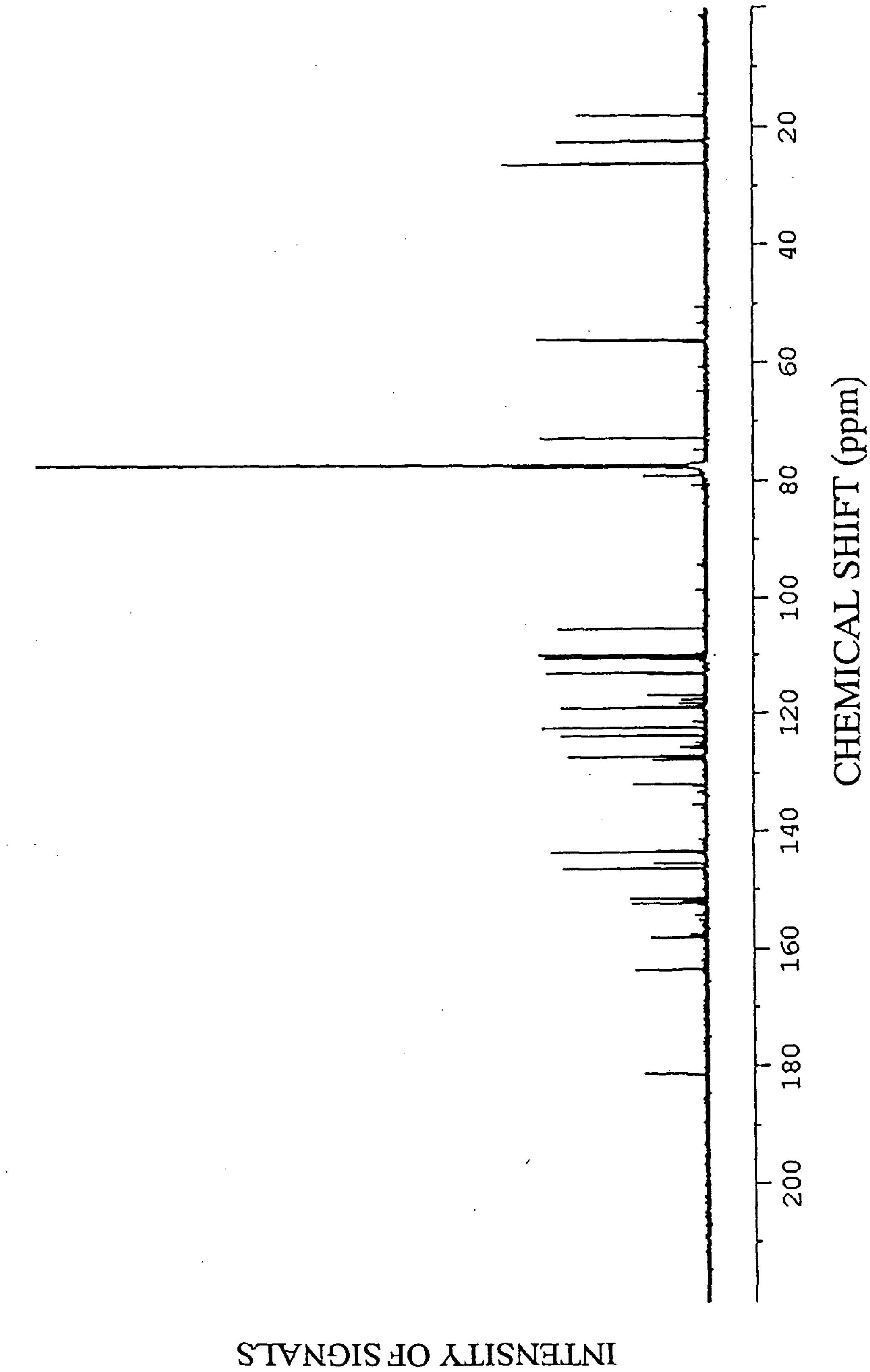
20
40
60
80
100
120
140
160
180
200
CHEMICAL SHIFT (ppm)
INTENSITY OF SIGNALS

FIG. 63

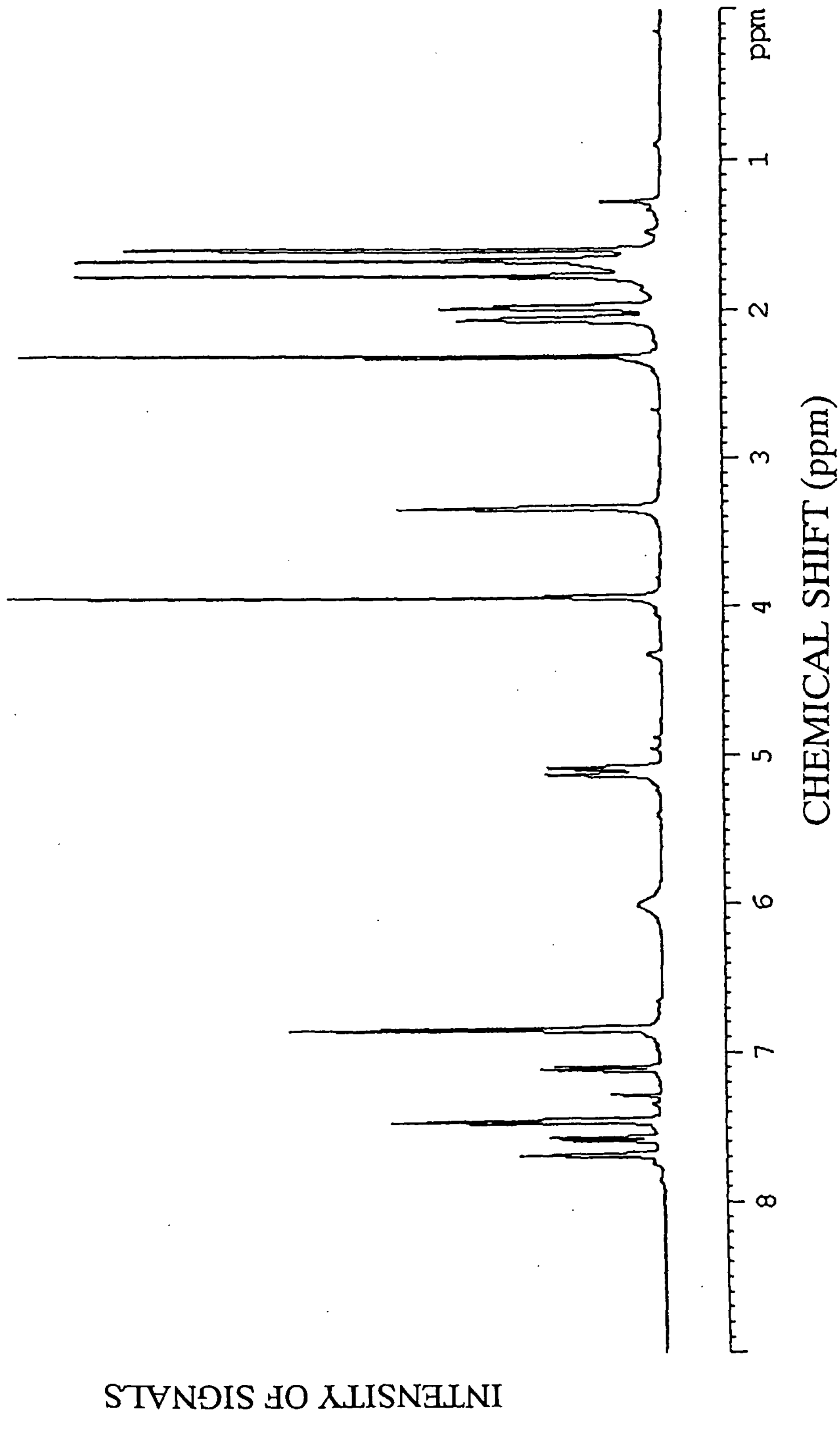


FIG. 64

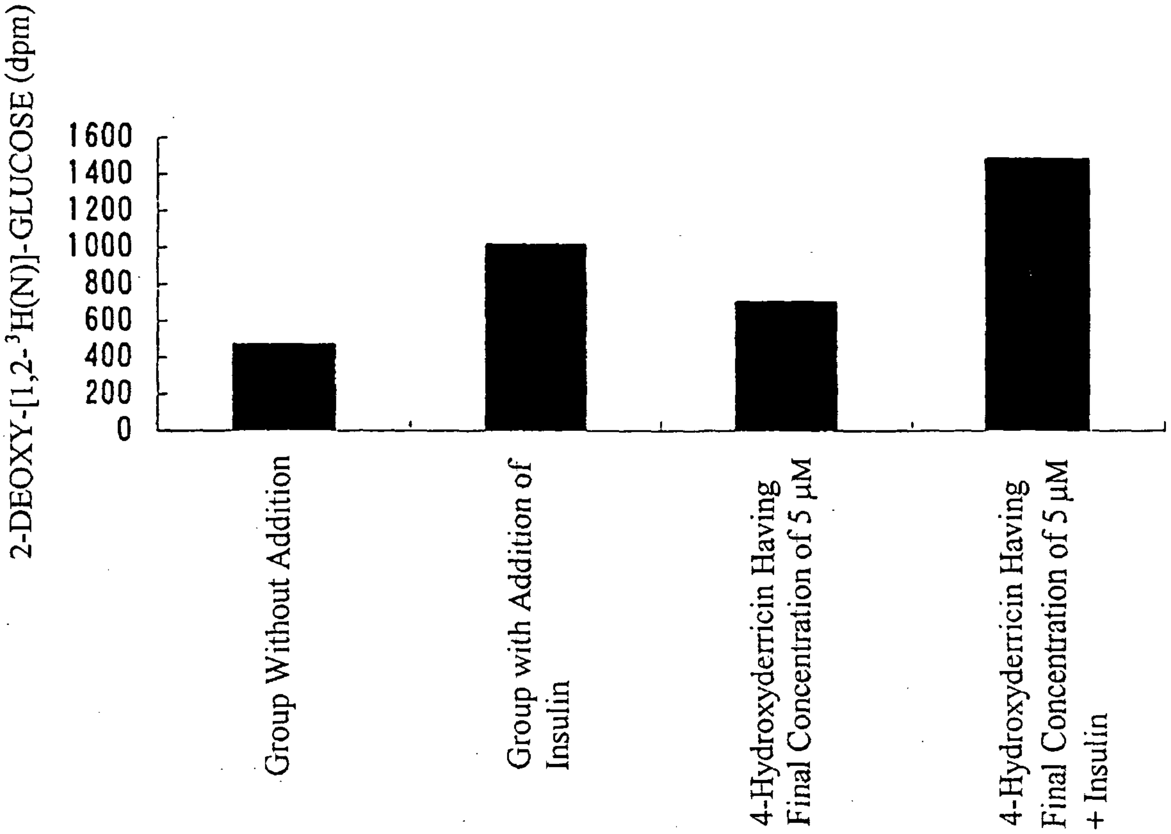
2-DEOXY-[1,2-$^3$H(N)]-GLUCOSE (dpm)
1600
1400
1200
1000
800
600
400
200
0
Group Without Addition
Group with Addition of Insulin
4-Hydroxyderricin Having Final Concentration of 5 μM
4-Hydroxyderricin Having Final Concentration of 5 μM + Insulin

FIG. 65

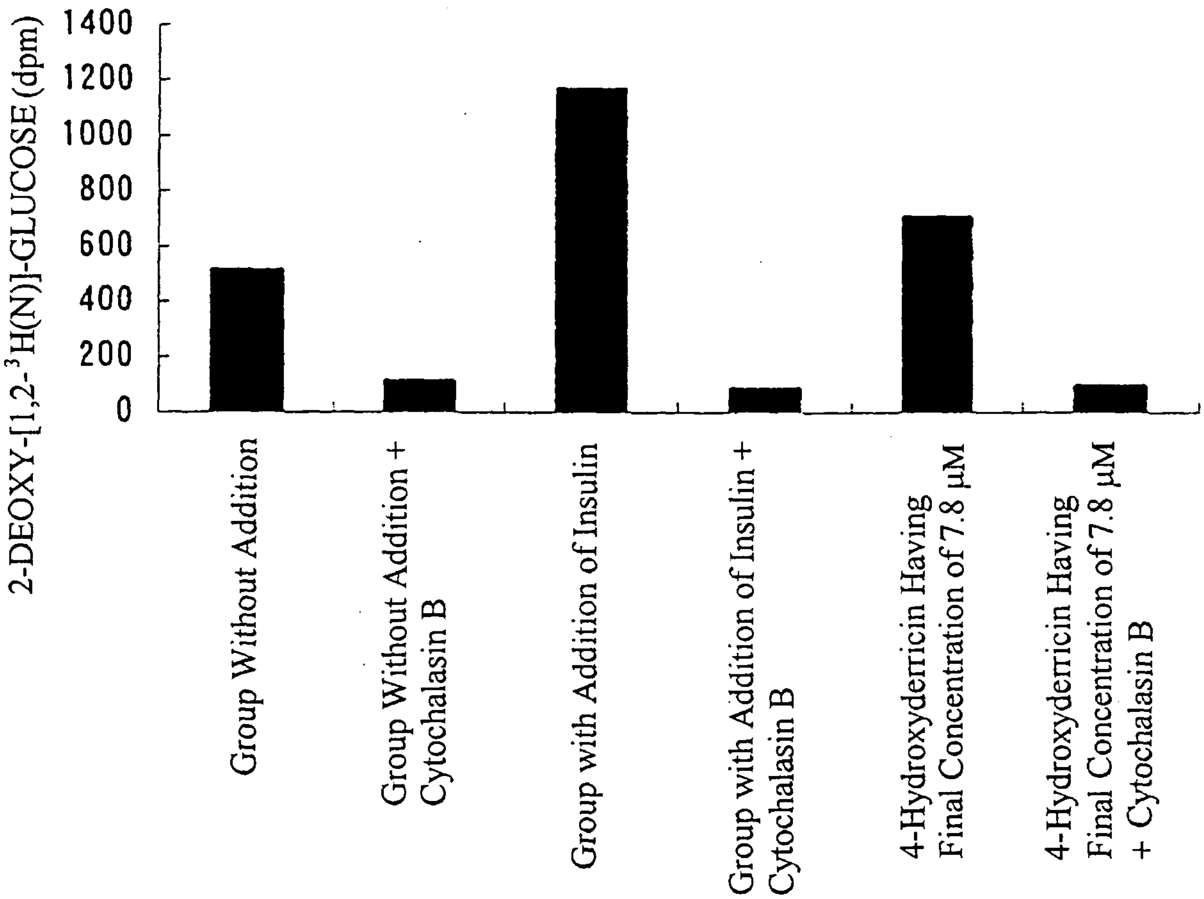
2-DEOXY-[1,2-3H(N)]-GLUCOSE (dpm)
1400
1200
1000
800
600
400
200
0
Group Without Addition
Group Without Addition + Cytochalasin B
Group with Addition of Insulin
Group with Addition of Insulin + Cytochalasin B
4-Hydroxyderricin Having Final Concentration of 7.8 μM
4-Hydroxyderricin Having Final Concentration of 7.8 μM + Cytochalasin B

FIG. 66

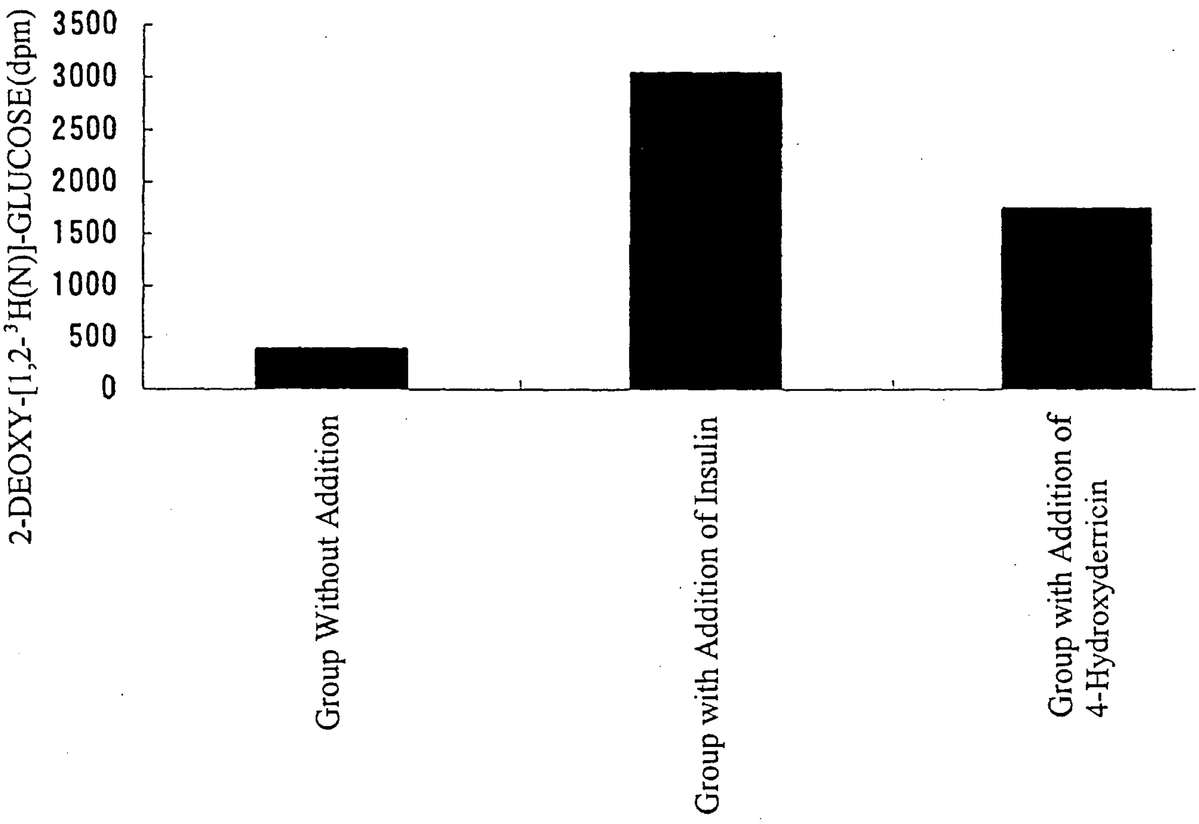
2-DEOXY-[1,2-$^3$H(N)]-GLUCOSE(dpm)
3500
3000
2500
2000
1500
1000
500
0
Group Without Addition
Group with Addition of Insulin
Group with Addition of 4-Hydroxyderricin

FIG. 67

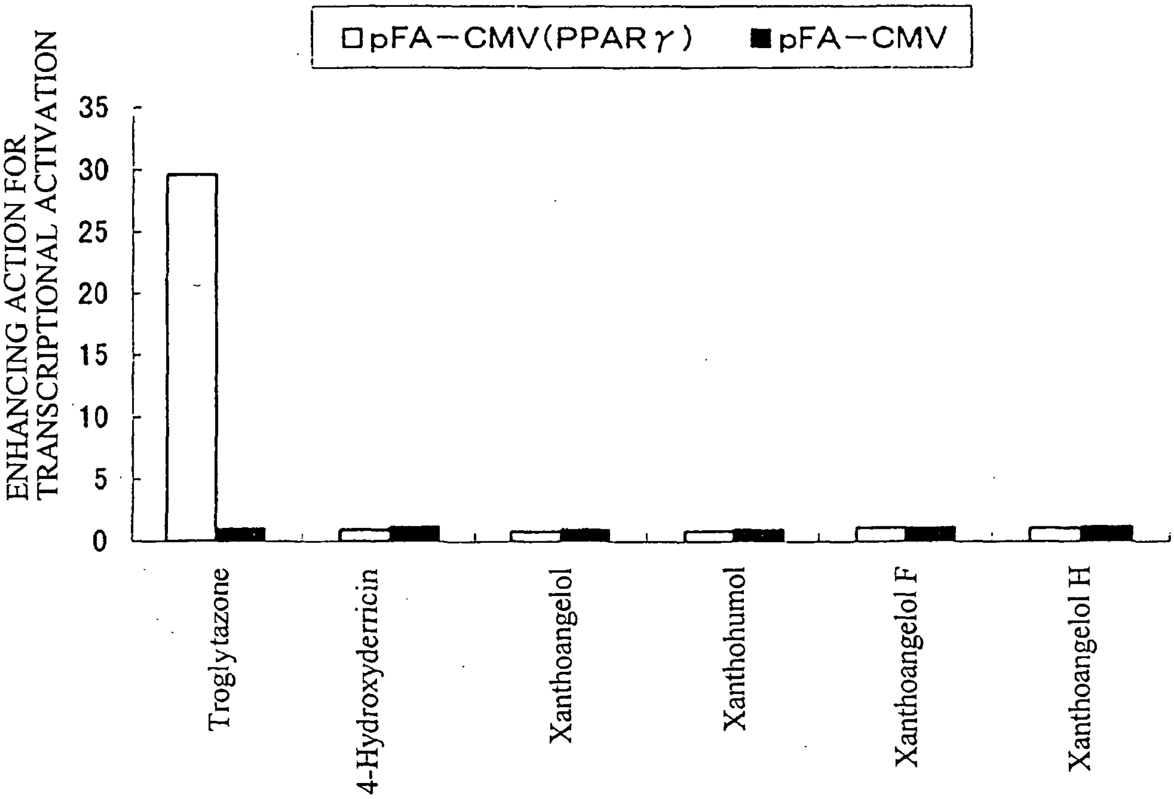
□pFA−CMV(PPARγ)
■pFA−CMV
ENHANCING ACTION FOR TRANSCRIPTIONAL ACTIVATION
35
30
25
20
15
10
5
0
Troglytazone
4-Hydroxyderricin
Xanthoangelol
Xanthohumol
Xanthoangelol F
Xanthoangelol H

FIG. 68

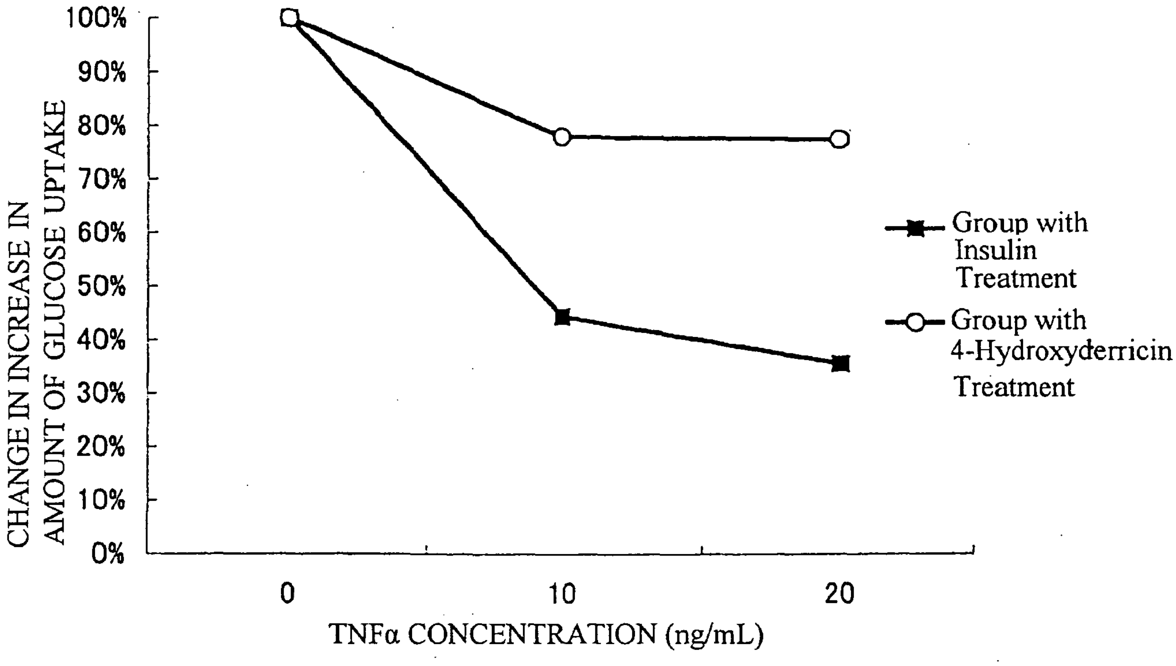
100%
90%
80%
70%
60%
50%
40%
30%
20%
10%
0%
CHANGE IN INCREASE IN AMOUNT OF GLUCOSE UPTAKE
0
10
20
TNFα CONCENTRATION (ng/mL)
Group with Insulin Treatment
Group with 4-Hydroxyderricin Treatment

FIG. 69

## INTERNATIONAL SEARCH REPORT

International application No.
PCT/JP2004/006282

**A. CLASSIFICATION OF SUBJECT MATTER**

Int.Cl[7] A61K31/121, 31/222, 31/343, 31/341, 31/365, 31/351, 31/381, 31/366, 31/352, A61P43/00, 3/10, 3/08, 5/50, C07D493/08, 309/12, 311/32, C07C49/84, 49/835, 69/18, 69/157, 69/16, 205/45,

According to International Patent Classification (IPC) or to both national classification and IPC

**B. FIELDS SEARCHED**

Minimum documentation searched (classification system followed by classification symbols)

Int.Cl[7] A61K31/121, 31/222, 31/343, 31/341, 31/365, 31/351, 31/381, 31/366, 31/352, A61P43/00, 3/10, 3/08, 5/50, C07D493/08, 309/12, 311/32, C07C49/84, 49/835, 69/18, 69/157, 69/16, 205/45,

Documentation searched other than minimum documentation to the extent that such documents are included in the fields searched

Electronic data base consulted during the international search (name of data base and, where practicable, search terms used)

CAPLUS(STN), REGISTRY(STN), MEDLINE(STN)

**C. DOCUMENTS CONSIDERED TO BE RELEVANT**

| Category* | Citation of document, with indication, where appropriate, of the relevant passages | Relevant to claim No. |
|---|---|---|
| X | JP 7-61927 A (Lotte Co., Ltd.), 07 March, 1995 (07.03.95), Full text (Family: none) | 1-3,5,10, 14-16,18,23, 27-29,31,36, 40-42,44,49, 53-55,57,62, 66 |
| X | JP 6-199695 A (Kabushiki Kaisha Yunie), 19 July, 1994 (19.07.94), Full text (Family: none) | 1-3,5,10, 14-16,18,23, 27-29,31,36, 40-42,44,49, 53-55,57,62, 66 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 July, 2004 (08.07.04) | 03 August, 2004 (03.08.04) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

International application No.
PCT/JP2004/006282

C (Continuation). DOCUMENTS CONSIDERED TO BE RELEVANT

| Category* | Citation of document, with indication, where appropriate, of the relevant passages | Relevant to claim No. |
|---|---|---|
| X | JP 5-509078 A (Beilar Collage of Medisin), 16 December, 1993 (16.12.93), Particularly, Claims 26, 33 & WO 91/17749 A1 28 November, 1991 (28.11.91) | 1-4,9,14-17, 22,27-30,35, 40-43,48, 53-56,61,66 |
| X | JP 2001-58969 A (Nettairin Saisei Gijutsu Kenkyu Kumiai), 06 March, 2001 (06.03.01), Full text (Family: none) | 1-3,9,14-16, 22,27-29,35, 40-42,48, 53-55,61,66 |
| X | JP 9-176010 A (Kureha Chemical Industry Co., Ltd.), 08 July, 1997 (08.07.97), Particularly, Par. Nos. [0012], [0014] to [0022] (Family: none) | 1-5,9-10, 14-18,22-23, 27-31,35-36, 40-44,48-49, 53-57,61-62, 66 |
| X | EP 950411 A1 (GEHRLICHER GMBH & CO. KG, PHARMAZEUTISCHE EXTRAKTE), 08 March, 1999 (08.03.99), Full text (Family: none) | 1,11-12,14, 24-25,27, 37-38,40, 50-51,53,63, 66 |
| X | JP 1-207233 A (Tsumura & Co.), 21 August, 1989 (21.08.89), Full text (Family: none) | 1,13-14, 26-27,39-40, 52-53,65 |
| X | JP 11-507946 A (The Research Foundation of State University of New York), 13 July, 1999 (13.07.99), Claims 13, 19 & WO 97/19679 A1 05 June, 1997 (05.06.97) | 1-2,6-8, 14-15,19-21, 27-28,32-34, 40-41,45-47, 53-54,58-60, 66 |
| X | WO 01/076580 A1 (Takara Bio Inc.), 18 October, 2001 (18.10.01), Particularly, Claims; descriptions, page 8, line 29 & EP 1283037 A1 12 February, 2003 (12.02.03) | 1-5,9-10, 14-18,22-23, 27-31,35-36, 40-44,48-49, 53-57,61-62, 66 |
| X | JP 6-65115 A (Bristol-Myers Squibb Co.), 08 March, 1994 (08.03.94), Particularly, tables 1, 2 & EP 571928 A1 01 December, 1993 (01.12.93) | 1-2,6-8, 14-15,19-21, 27-28,32-34, 40-41,45-47, 53-54,58-60, 66 |

Form PCT/ISA/210 (continuation of second sheet) (January 2004)

**INTERNATIONAL SEARCH REPORT**

International application No.
PCT/JP2004/006282

C (Continuation). DOCUMENTS CONSIDERED TO BE RELEVANT

| Category* | Citation of document, with indication, where appropriate, of the relevant passages | Relevant to claim No. |
|---|---|---|
| X | JP 10-511346 A (INDENA S.P.A.), 04 November, 1998 (04.11.98), Particularly, compound S2 & WO 96/19209 A1 27 June, 1996 (27.06.96) | 66 |
| P,X | JP 2004-18376 A (Nikken Chemicals Co., Ltd.), 22 January, 2004 (22.01.04), Full text (Family: none) | 1,14,27,40, 53 |
| P,X | WO 03/037316 A (Kaneka Corp.), 08 May, 2003 (08.05.03), Full text (Family: none) | 1-12,14-25, 27-38,40-51, 53-64 |
| A | JP 8-27057 A (Asahi Breweries, Ltd.), 30 January, 1996 (30.01.96), Full text (Family: none) | 1-66 |

Form PCT/ISA/210 (continuation of second sheet) (January 2004)

**INTERNATIONAL SEARCH REPORT**

International application No.
PCT/JP2004/006282

**Box No. II Observations where certain claims were found unsearchable (Continuation of item 2 of first sheet)**

This international search report has not been established in respect of certain claims under Article 17(2)(a) for the following reasons:

1. [X] Claims Nos.:
because they relate to subject matter not required to be searched by this Authority, namely:

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
(See extra sheet.)

1. [X] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest.
[X] No protest accompanied the payment of additional search fees.

Form PCT/ISA/210 (continuation of first sheet (2)) (January 2004)

**INTERNATIONAL SEARCH REPORT**

International application No.
PCT/JP2004/006282

Continuation of A. CLASSIFICATION OF SUBJECT MATTER
(International Patent Classification (IPC))

Int.Cl$^7$ A23L1/30//C07D307/80, 307/46, 307/88, 333/16, 407/14, 311/58

(According to International Patent Classification (IPC) or to both national classification and IPC)

Continuation of B. FIELDS SEARCHED
Minimum documentation searched (International Patent Classification (IPC))

Int.Cl$^7$ A23L1/30//C07D307/80, 307/46, 307/88, 333/16, 407/14, 311/58

Minimum documentation searched (classification system followed by classification symbols)

Continuation of Box No.III of continuation of first sheet(2)

It appears that the chemical structure common to the formulae 1, 2 and 3 shown in the claims is a 6-membered carbon ring. However, the usefulness of the compound with this structure in the treatment of diabetes mellitus is publicly known as described in prior art references such as WO 99/15520 A1, JP 2001-58969 A and JP 11-349521 A. Consequently, the above chemical structure cannot be regarded as being an important chemical structure element.

Further, it appears that the chemical structure common to a group of compounds represented by the formula 1 is a moiety other than Xo. However, the usefulness of the compound with this structure in the treatment of diabetes mellitus is also publicly known as described in the above prior art references.

The "special technical features" of the inventions of this international application, with respect to claims 1 to 65, comprehend six drug inventions respectively comprising a compound of araliphatic Xo, a compound of hydrogen Xo, a compound of aliphatic Xo, a compound of aromatic Xo, a compound of the formula 2 and a compound of the formula 3.

With respect to claim 66, there are comprehended three inventions respectively directed to a 4-oxoacetophenone derivative having a carbon group bonded at 2-position and a 4H-1-benzopyran-4-one derivative and 2,4-oxoacetophenone derivative having a carbon group bonded at 3-position and having hydrogen bonded at 5-position.

It does not appear that there is a technical relationship among these inventions involving one or more of the same or corresponding special technical features.

Therefore, this international application involves nine inventions which do not satisfy the requirement of unity of invention.

Form PCT/ISA/210 (extra sheet) (January 2004)